(19) Europäisches Patentamt / European Patent Office / Office européen des brevets

(11) **EP 3 981 429 A1**

(12) **EUROPEAN PATENT APPLICATION**
published in accordance with Art. 153(4) EPC

(43) Date of publication:
**13.04.2022 Bulletin 2022/15**

(21) Application number: **20822960.9**

(22) Date of filing: **10.06.2020**

(51) International Patent Classification (IPC):
**A61K 39/395** (2006.01)  **A61K 45/00** (2006.01)
**A61P 35/00** (2006.01)  **A61P 43/00** (2006.01)
**C07K 16/28** (2006.01)  **C07K 16/46** (2006.01)
**C12N 15/13** (2006.01)  **A61K 31/573** (2006.01)

(52) Cooperative Patent Classification (CPC):
**A61K 31/573; A61K 39/395; A61K 45/00;**
**A61P 35/00; A61P 43/00; C07K 16/28; C07K 16/46**

(86) International application number:
**PCT/JP2020/022771**

(87) International publication number:
**WO 2020/250915 (17.12.2020 Gazette 2020/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.06.2019 JP 2019107894**
**03.07.2019 JP 2019124364**

(71) Applicant: **CHUGAI SEIYAKU KABUSHIKI KAISHA**
**Tokyo 115-8543 (JP)**

(72) Inventors:
• **ISHIGURO, Takahiro**
**Tokyo 103-8324 (JP)**
• **KISHISHITA, Shohei**
**Tokyo 103-8324 (JP)**
• **NAKAMURA, Mikiko**
**Tokyo 103-8324 (JP)**
• **MORLEY, Roland Kaneo**
**Tokyo 103-8324 (JP)**

(74) Representative: **Vossius & Partner**
**Patentanwälte Rechtsanwälte mbB**
**Siebertstraße 3**
**81675 München (DE)**

(54) **ANTI-T CELL ANTIGEN-BINDING MOLECULE TO BE USED IN COMBINATION WITH CYTOKINE INHIBITOR**

(57) The present disclosure provides combination therapies with an anti-T cell antigen-binding molecule and a cytokine inhibitor. Antibodies that recruit T cells as effector cells into tumor tissues are called T cell redirecting antibodies, and are known as means for treating tumors. On the other hand, when systemic cytokine production is stimulated by binding of antibodies to T cells, it is feared that this systemic action will lead to aberrations such as CRS. The present disclosure provides means for alleviating systemic cytokine production, and will enable safer use of anti-T cell antigen-binding molecules in tumor treatment.

FIG. 12

EP 3 981 429 A1

**Description**

[Technical Field]

The present disclosure relates to anti-T cell antigen-binding molecules for use in combination with cytokine inhibitors. Furthermore, the present disclosure relates to pharmaceutical compositions and methods for preventing, alleviating, or treating adverse reactions caused by cytokine production associated with administration of anti-T cell antigen-binding molecules.

[Background Art]

Antibodies are drawing attention as pharmaceuticals because of their high stability in plasma and few adverse reactions (NPL 1 and NPL 2). Antibodies are known to induce not only an antigen-binding action, an agonistic action, and an antagonistic action, but also effector cell-mediated cytotoxic activities (also called effector functions) such as antibody-dependent cytotoxicity (ADCC), antibody dependent cell phagocytosis (ADCP), and complement-dependent cytotoxicity (CDC), and exhibit antitumor effects against cancer cells (NPL 3). A number of therapeutic antibodies showing excellent anti-tumor effects have been developed as pharmaceuticals aimed for cancer treatment (NPL 4); and while existing therapeutic antibodies have shown excellent actions, the therapeutic outcome achieved by administration of these antibodies is still not satisfactory.

Antibodies (bispecific antibodies) that bind to two or more types of antigens with one molecule are being studied as molecules that inhibit multiple targets. If all antigens recognized by a bispecific antibody are antigens specifically expressed in cancer, the bispecific antibody exhibits cytotoxic activity to cancer cells when it binds to any of the antigens; therefore, in comparison to a conventional antibody pharmaceutical that recognizes one antigen, a more efficient anti-tumor effect can be expected from such an antibody.

As one of the bispecific antibodies, a T cell-redirecting antibody, which is an antibody whose mechanisms of antitumor effect is cytotoxicity mediated by the recruitment of T cells as effector cells, has been known from the 1980s (NPLs 5, 6, and 7). Unlike antibodies that employ ADCC, which is mediated by the recruitment of NK cells or macrophages as effector cells, as the mechanism for their antitumor effects, a T cell-redirecting antibody is a bispecific antibody comprising an antibody against any one of the subunits constituting the T-cell receptor (TCR) complex on T cells, in particular, an antibody that binds to the CD3 epsilon chain; and an antibody that binds to an antigen on the target cancer cell. T cells come close to cancer cells via simultaneous binding of the T cell-redirecting antibody to the CD3 epsilon chain and a cancer antigen. As a result, antitumor effects against cancer cells are considered to be exerted through the cytotoxic activity possessed by T cells. For example, in recent years, novel T cell-redirecting antibodies have been provided, which are antibodies whose two Fabs respectively bind to a cancer antigen (GPC3) and the CD3ε chain expressed on T cells, and have an Fc region with reduced FcγR-binding activity (PTL 1 and 2).

On the other hand, occurrence of toxicity associated with exertion of high antitumor activity by the T cell-redirecting antibody, for example, development of cytokine release syndrome (CRS), has been reported (NPL 8).

[Citation List]

[Patent Literature]

[PTL 1] WO2012/073985
[PTL 2] WO2016/047722

[Non-Patent Literature]

[NPL 1] Nat. Biotechnol. (2005) 23, 1073-1078
[NPL 2] Eur J Pharm Biopharm. (2005) 59 (3), 389-396
[NPL 3] Drug Des Devel Ther (2009) 3, 7-16
[NPL 4] Clin Cancer Res. (2010) 16 (1), 11-20
[NPL 5] Nature (1985) 314 (6012), 628-31
[NPL 6] Int J Cancer (1988) 41 (4), 609-15.
[NPL 7] Proc Natl Acad Sci USA (1986) 83 (5), 1453-7

[NPL 8] Cancer J. 2014 Mar-Apr; 20(2): 119-122.

[Summary of Invention]

[Technical Problem]

In a non-limiting embodiment, the present disclosure relates to combination therapies involving an anti-T cell antigen-binding molecule and a cytokine inhibitor, and a pharmaceutical composition and such for use in the combination therapies.

[Solution to Problem]

In a non-limiting embodiment, the present inventors found that administration of a cytokine inhibitor can prevent, alleviate, or treat cytokine release syndrome (CRS) associated with administration of an anti-T cell antigen-binding molecule in an individual.

In a non-limiting embodiment, the present disclosure relates to the following:

(A1) a pharmaceutical composition comprising an anti-T cell antigen-binding molecule, wherein the pharmaceutical composition is for use in its combination therapy with a cytokine inhibitor;

(A1-1) the pharmaceutical composition of (A1), wherein the cytokine inhibitor is administered before, simultaneously with, or after administration of the pharmaceutical composition;

(A1-2) the pharmaceutical composition of (A1) or (A1-1), wherein the cytokine inhibitor is administered before or simultaneously with the administration of the pharmaceutical composition;

(A1-3) the pharmaceutical composition of any one of (A1) to (A1-2), wherein the cytokine inhibitor is administered 6 days, 5 days, 4 days, 3 days, 2 days, or 1 day before the administration of the pharmaceutical composition, or on the same day as but before said administration;

(A1-4) the pharmaceutical composition of any one of (A1) to (A1-3), wherein the cytokine inhibitor is further administered on the same day as, or 1 day, 2 days, 3 days, 4 days, 5 days, or 6 days after the administration of the pharmaceutical composition;

(A1-5) the pharmaceutical composition of any one of (A1) to (A1-4), wherein the cytokine inhibitor is further administered when CRS or signs of CRS develop after the administration of the pharmaceutical composition;

(A1-6) the pharmaceutical composition of (A1-5), wherein the CRS is CRS in Grade 2 or higher or Grade 3 or higher;

(A1-7) the pharmaceutical composition of (A1) or (A1-1), wherein the cytokine inhibitor is administered after the administration of the pharmaceutical composition;

(A1-8) the pharmaceutical composition of (A1) or (A1-7), wherein the cytokine inhibitor is administered when CRS or signs of CRS develop after the administration of the pharmaceutical composition;

(A1-9) the pharmaceutical composition of (A1-7) or (A1-8), wherein the CRS is CRS in Grade 2 or higher or Grade 3 or higher;

(A1-10) the pharmaceutical composition of any one of (A1) to (A1-9), wherein the cytokine inhibitor is an inhibitor of one or more cytokines selected from IL-1β, IL-2, IL-4, IL-5, IL-6, IL-8, IL-10, IL-12, IL-13, IL-15, IL-17, IL-1Ra, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE;

(A1-11) the pharmaceutical composition of any one of (A1) to (A1-10), wherein the cytokine inhibitor is an anti-IL6R antibody;

(A1-12) the pharmaceutical composition of any one of (A1) to (A1-11), wherein the cytokine inhibitor is Tocilizumab, and wherein Tocilizumab is administered at 8 mg/kg or less per dose for a patient weighing 30 kg or more and at 12 mg/kg or less per dose for a patient weighing less than 30 kg;

(A1-13) the pharmaceutical composition of any one of (A1) to (A1-12), wherein a corticosteroid is not administered before or simultaneously with the administration of the pharmaceutical composition;

(A1-14) the pharmaceutical composition of any one of (A1) to (A1-12), wherein a corticosteroid is further administered before, simultaneously with, or after the administration of the pharmaceutical composition;

(A1-15) the pharmaceutical composition of (A1-13) or (A1-14), wherein the corticosteroid is dexamethasone, a pharmaceutically acceptable salt thereof, or a derivative thereof;

(A1-16) the pharmaceutical composition of any one of (A1) to (A1-15), wherein the anti-T cell antigen-binding molecule is a bispecific antigen-binding molecule comprising:

(1) a domain comprising an antibody variable region having T cell receptor complex-binding activity; and
(2) a domain comprising an antibody variable region having cancer antigen-binding activity;

(A1-17) the pharmaceutical composition of any one of (A1) to (A1-16), wherein the anti-T cell antigen-binding molecule is a bispecific antibody comprising:

(1) a domain comprising an antibody variable region having T cell receptor complex-binding activity;
(2) a domain comprising an antibody variable region having glypican 3-binding activity; and
(3) a domain comprising an Fc region with reduced binding activity to an Fcγ receptor;

(A1-18) the pharmaceutical composition of any one of (A1) to (A1-17), which is for treatment of cancer; and
(A1-19) the pharmaceutical composition of any one of (A1) to (A1-18), which is for treating cancer while preventing or alleviating cytokine release syndrome associated with administration of the anti-T cell antigen-binding molecule.

The present disclosure also provides administration of an anti-T cell antigen-binding molecule that is accompanied with pre-administration of a cytokine inhibitor, which includes each of the following embodiments (when pre-administration of a cytokine inhibitor is performed for each administration of an anti-T cell antigen-binding molecule):

(A2) a pharmaceutical composition comprising an anti-T cell antigen-binding molecule, wherein the pharmaceutical composition is for use in its combination therapy with a cytokine inhibitor, wherein in the combination therapy,

(i) the pharmaceutical composition is administered repeatedly, and
(ii) the cytokine inhibitor is administered before or simultaneously with each administration of the pharmaceutical composition;

(A2-1) the pharmaceutical composition of (A2), wherein the pharmaceutical composition is administered repeatedly at the same dose;
(A2-2) the pharmaceutical composition of (A2) or (A2-1), wherein the pharmaceutical composition is administered repeatedly at intervals of once every week, once every 2 weeks, or once every 3 weeks;
(A2-3) the pharmaceutical composition of any one of (A2) to (A2-2), wherein the cytokine inhibitor is administered 6 days, 5 days, 4 days, 3 days, 2 days, or 1 day before each administration of the pharmaceutical composition, or on the same day as but before said each administration;
(A2-4) the pharmaceutical composition of any one of (A2) to (A2-3), wherein the cytokine inhibitor is further administered on the same day as, or 1 day, 2 days, 3 days, 4 days, 5 days, or 6 days after the administration of the pharmaceutical composition;
(A2-5) the pharmaceutical composition of any one of (A2) to (A2-4), wherein the cytokine inhibitor is further administered when CRS or signs of CRS develop after the administration of the pharmaceutical composition;
(A2-6) the pharmaceutical composition of (A2-5), wherein the CRS is CRS in Grade 2 or higher or Grade 3 or higher;
(A2-7) the pharmaceutical composition of any one of (A2) to (A2-6), wherein the cytokine inhibitor is an inhibitor of one or more cytokines selected from IL-1β, IL-2, IL-4, IL-5, IL-6, IL-8, IL-10, IL-12, IL-13, IL-15, IL-17, IL-1Ra, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE;
(A2-8) the pharmaceutical composition of any one of (A2) to (A2-7), wherein the cytokine inhibitor is an anti-IL6R antibody;
(A2-9) the pharmaceutical composition of any one of (A2) to (A2-8), wherein the cytokine inhibitor is Tocilizumab, and wherein Tocilizumab is administered at 8 mg/kg or less per dose for a patient weighing 30 kg or more and at 12 kg/kg or less per dose for a patient weighing less than 30 kg;
(A2-10) the pharmaceutical composition of any one of (A2) to (A2-9), wherein a corticosteroid is not administered before or simultaneously with the administration of the pharmaceutical composition;
(A2-11) the pharmaceutical composition of any one of (A2) to (A2-9), wherein a corticosteroid is further administered before, simultaneously with, or after the administration of the pharmaceutical composition;
(A2-12) the pharmaceutical composition of (A2-10) or (A2-11), wherein the corticosteroid is dexamethasone, a pharmaceutically acceptable salt thereof, or a derivative thereof;
(A2-13) the pharmaceutical composition of any one of (A2) to (A2-12), wherein the anti-T cell antigen-binding molecule is a bispecific antigen-binding molecule comprising:

(1) a domain comprising an antibody variable region having T cell receptor complex-binding activity; and
(2) a domain comprising an antibody variable region having cancer antigen-binding activity; (A2-14) the pharmaceutical composition of any one of (A2) to (A2-13), wherein the anti-T cell antigen-binding molecule is a bispecific antibody comprising:

(1) a domain comprising an antibody variable region having T cell receptor complex-binding activity;
(2) a domain comprising an antibody variable region having glypican 3-binding activity; and
(3) a domain comprising an Fc region with reduced binding activity to an Fcγ receptor; (A2-15) the pharmaceutical composition of any one of (A2) to (A2-14), which is for treatment of cancer; and

(A2-16) the pharmaceutical composition of any one of (A2) to (A2-15), which is for treating cancer while preventing or alleviating cytokine release syndrome associated with administration of the anti-T cell antigen-binding molecule.

Alternatively, the present disclosure provides each of the following embodiments (when pre-administration of a cytokine inhibitor is performed for the first or multiple administrations of an anti-T cell antigen-binding molecule):

(A3) a pharmaceutical composition comprising an anti-T cell antigen-binding molecule, wherein the pharmaceutical composition is for use in its combination therapy with a cytokine inhibitor, wherein in the combination therapy,

(i) the pharmaceutical composition is administered repeatedly, and
(ii) the cytokine inhibitor is administered before or simultaneously with the first or each administration of the first to second, the first to third, the first to fourth, or the first to fifth administrations of the pharmaceutical composition;

(A3-1) the pharmaceutical composition of (A3), wherein the pharmaceutical composition is administered repeatedly at the same dose;
(A3-2) the pharmaceutical composition of (A3) or (A3-1), wherein the pharmaceutical composition is administered repeatedly at intervals of once every week, once every 2 weeks, or once every 3 weeks;
(A3-3) the pharmaceutical composition of any one of (A3) to (A3-2), wherein the cytokine inhibitor is administered 6 days, 5 days, 4 days, 3 days, 2 days, or 1 day before the first, or each administration of the first to second, the first to third, the first to fourth, or the first to fifth administrations of the pharmaceutical composition, or on the same day as but before said first or each administration;
(A3-4) the pharmaceutical composition of any one of (A3) to (A3-3), wherein the cytokine inhibitor is further administered on the same day as, or 1 day, 2 days, 3 days, 4 days, 5 days, or 6 days after the first, the first to second, the first to third, the first to fourth, or the first to fifth administrations of the pharmaceutical composition;
(A3-5) the pharmaceutical composition of any one of (A3) to (A3-4), wherein the cytokine inhibitor is further administered when CRS or signs of CRS develop after the administration of the pharmaceutical composition;
(A3-6) the pharmaceutical composition of (A3-5), wherein the CRS is CRS in Grade 2 or higher or Grade 3 or higher;
(A3-7) the pharmaceutical composition of any one of (A3) to (A3-6), wherein the cytokine inhibitor is an inhibitor of one or more cytokines selected from IL-1β, IL-2, IL-4, IL-5, IL-6, IL-8, IL-10, IL-12, IL-13, IL-15, IL-17, IL-1Ra, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE;
(A3-8) the pharmaceutical composition of any one of (A3) to (A3-7), wherein the cytokine inhibitor is an anti-IL6R antibody;
(A3-9) the pharmaceutical composition of any one of (A3) to (A3-8), wherein the cytokine inhibitor is Tocilizumab, and wherein Tocilizumab is administered at 8 mg/kg or less per dose for a patient weighing 30 kg or more and at 12 kg/kg or less per dose for a patient weighing less than 30 kg;
(A3-10) the pharmaceutical composition of any one of (A3) to (A3-9), wherein a corticosteroid is not administered before or simultaneously with the administration of the pharmaceutical composition;
(A3-11) the pharmaceutical composition of any one of (A3) to (A3-9), wherein a corticosteroid is further administered before, simultaneously with, or after the administration of the pharmaceutical composition;
(A3-12) the pharmaceutical composition of (A3-10) or (A3-11), wherein the corticosteroid is dexamethasone, a pharmaceutically acceptable salt thereof, or a derivative thereof;
(A3-13) the pharmaceutical composition of any one of (A3) to (A3-12), wherein the anti-T cell antigen-binding molecule is a bispecific antigen-binding molecule comprising:

(1) a domain comprising an antibody variable region having T cell receptor complex-binding activity; and
(2) a domain comprising an antibody variable region having cancer antigen-binding activity; (A3-14) the pharmaceutical composition of any one of (A3) to (A3-13), wherein the anti-T cell antigen-binding molecule is a bispecific antibody comprising:

(1) a domain comprising an antibody variable region having T cell receptor complex-binding activity;
(2) a domain comprising an antibody variable region having glypican 3-binding activity; and
(3) a domain comprising an Fc region with reduced binding activity to an Fcγ receptor; (A3-15) the pharmaceutical composition of any one of (A3) to (A3-14), which is for treatment of cancer; and

(A3-16) the pharmaceutical composition of any one of (A3) to (A3-15), which is for treating cancer while preventing or alleviating cytokine release syndrome associated with administration of the anti-T cell antigen-binding molecule.

Alternatively, the present disclosure provides each of the following embodiments (when pre-administration of a cytokine inhibitor is performed in every dosing cycle of an anti-T cell antigen-binding molecule):

(A4) a pharmaceutical composition comprising an anti-T cell antigen-binding molecule, wherein the pharmaceutical composition is for use in its combination therapy with a cytokine inhibitor, wherein in the combination therapy,

(i) the pharmaceutical composition is administered repeatedly in dosing cycles where 2, 3, 4, or 5 administrations constitute one cycle, and
(ii) the cytokine inhibitor is administered before or simultaneously with the first administration of the pharmaceutical composition in each of the dosing cycles;

(A4-1) the pharmaceutical composition of (A4), wherein the pharmaceutical composition is administered repeatedly at the same dose;
(A4-2) the pharmaceutical composition of (A4) or (A4-1), wherein the pharmaceutical composition is administered repeatedly at intervals of once every week, once every 2 weeks, or once every 3 weeks;
(A4-3) the pharmaceutical composition of any one of (A4) to (A4-2), wherein the cytokine inhibitor is administered 6 days, 5 days, 4 days, 3 days, 2 days, or 1 day before the first administration of the pharmaceutical composition in each of the dosing cycles, or on the same day as but before the said first administration;
(A4-4) the pharmaceutical composition of any one of (A4) to (A4-3), wherein the cytokine inhibitor is further administered on the same day as, or 1 day, 2 days, 3 days, 4 days, 5 days, or 6 days after the first administration of the pharmaceutical composition in each of the dosing cycles;
(A4-5) the pharmaceutical composition of any one of (A4) to (A4-4), wherein the cytokine inhibitor is further administered when CRS or signs of CRS develop after the administration of the pharmaceutical composition;
(A4-6) the pharmaceutical composition of (A4-5), wherein the CRS is CRS in Grade 2 or higher or Grade 3 or higher;
(A4-7) the pharmaceutical composition of any one of (A4) to (A4-6), wherein the cytokine inhibitor is an inhibitor of one or more cytokines selected from IL-1β, IL-2, IL-4, IL-5, IL-6, IL-8, IL-10, IL-12, IL-13, IL-15, IL-17, IL-1Ra, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE;
(A4-8) the pharmaceutical composition of any one of (A4) to (A4-7), wherein the cytokine inhibitor is an anti-IL6R antibody;
(A4-9) the pharmaceutical composition of any one of (A4) to (A4-8), wherein the cytokine inhibitor is Tocilizumab, and wherein Tocilizumab is administered at 8 mg/kg or less per dose for a patient weighing 30 kg or more and at 12 kg/kg or less per dose for a patient weighing less than 30 kg;
(A4-10) the pharmaceutical composition of any one of (A4) to (A4-9), wherein a corticosteroid is not administered before or simultaneously with the administration of the pharmaceutical composition;
(A4-11) the pharmaceutical composition of any one of (A4) to (A4-9), wherein a corticosteroid is further administered before, simultaneously with, or after the administration of the pharmaceutical composition;
(A4-12) the pharmaceutical composition of (A4-10) or (A4-11), wherein the corticosteroid is dexamethasone, a pharmaceutically acceptable salt thereof, or a derivative thereof;
(A4-13) the pharmaceutical composition of any one of (A4) to (A4-12), wherein the anti-T cell antigen-binding molecule is a bispecific antigen-binding molecule comprising:

(1) a domain comprising an antibody variable region having T cell receptor complex-binding activity; and
(2) a domain comprising an antibody variable region having cancer antigen-binding activity;
(A4-14) the pharmaceutical composition of any one of (A4) to (A4-13), wherein the anti-T cell antigen-binding molecule is a bispecific antibody comprising:

(1) a domain comprising an antibody variable region having T cell receptor complex-binding activity;
(2) a domain comprising an antibody variable region having glypican 3-binding activity; and
(3) a domain comprising an Fc region with reduced binding activity to an Fcγ receptor; (A4-15) the pharmaceutical composition of any one of (A4) to (A4-14), which is for treatment of cancer; and

(A4-16) the pharmaceutical composition of any one of (A4) to (A4-15), which is for treating cancer while preventing or alleviating cytokine release syndrome associated with administration of the anti-T cell antigen-binding molecule.

Alternatively, the present disclosure comprises each of the following embodiments (when pre-administration of a cytokine inhibitor is performed in every dosing cycle of an anti-T cell antigen-binding molecule):

(A5) a pharmaceutical composition comprising an anti-T cell antigen-binding molecule, wherein the pharmaceutical composition is for use in its combination therapy with a cytokine inhibitor, wherein in the combination therapy,

(i) the pharmaceutical composition is administered repeatedly in dosing cycles where 2, 3, 4, or 5 administrations constitute one cycle, and
(ii) (a) the cytokine inhibitor is administered before or simultaneously with each administration of the pharmaceutical composition in the first dosing cycle, and (b) the cytokine inhibitor is administered before or simultaneously with the first administration of the pharmaceutical composition in each of the second and subsequent dosing cycles;

(A5-1) the pharmaceutical composition of (A5), wherein the pharmaceutical composition is administered repeatedly at the same dose;
(A5-2) the pharmaceutical composition of (A5) or (A5-1), wherein the pharmaceutical composition is administered repeatedly at intervals of once every week, once every 2 weeks, or once every 3 weeks;
(A5-3) the pharmaceutical composition of any one of (A5) to (A5-2), wherein the cytokine inhibitor is administered

(a) 6 days, 5 days, 4 days, 3 days, 2 days, or 1 day before each administration of the pharmaceutical composition in the first dosing cycle, or on the same day as but before said each administration, and
(b) 6 days, 5 days, 4 days, 3 days, 2 days, or 1 day before the first administration of the pharmaceutical composition in each of the second and subsequent dosing cycles, or on the same day as but before said first administration;

(A5-4) the pharmaceutical composition of any one of (A5) to (A5-3), wherein the cytokine inhibitor is further administered

(a) on the same day as, or 1 day, 2 days, 3 days, 4 days, 5 days, or 6 days after each administration of the pharmaceutical composition in the first dosing cycle, and
(b) on the same day as, or 1 day, 2 days, 3 days, 4 days, 5 days, or 6 days after the first administration of the pharmaceutical composition in each of the second and subsequent dosing cycles;

(A5-5) the pharmaceutical composition of any one of (A5) to (A5-4), wherein the cytokine inhibitor is further administered when CRS or signs of CRS develop after the administration of the pharmaceutical composition;
(A5-6) the pharmaceutical composition of (A5-5), wherein the CRS is CRS in Grade 2 or higher or Grade 3 or higher;
(A5-7) the pharmaceutical composition of any one of (A5) to (A5-6), wherein the cytokine inhibitor is an inhibitor of one or more cytokines selected from IL-1β, IL-2, IL-4, IL-5, IL-6, IL-8, IL-10, IL-12, IL-13, IL-15, IL-17, IL-1Ra, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE;
(A5-8) the pharmaceutical composition of any one of (A5) to (A5-7), wherein the cytokine inhibitor is an anti-IL6R antibody;
(A5-9) the pharmaceutical composition of any one of (A5) to (A5-8), wherein the cytokine inhibitor is Tocilizumab, and wherein Tocilizumab is administered at 8 mg/kg or less per dose for a patient weighing 30 kg or more and at 12 kg/kg or less per dose for a patient weighing less than 30 kg;
(A5-10) the pharmaceutical composition of any one of (A5) to (A5-9), wherein a corticosteroid is not administered before or simultaneously with the administration of the pharmaceutical composition;
(A5-11) the pharmaceutical composition of any one of (A5) to (A5-9), wherein a corticosteroid is further administered before, simultaneously with, or after the administration of the pharmaceutical composition;
(A5-12) the pharmaceutical composition of (A5-10) or (A5-11), wherein the corticosteroid is dexamethasone, a pharmaceutically acceptable salt thereof, or a derivative thereof;
(A5-13) the pharmaceutical composition of any one of (A5) to (A5-12), wherein the anti-T cell antigen-binding molecule is a bispecific antigen-binding molecule comprising:

(1) a domain comprising an antibody variable region having T cell receptor complex-binding activity; and
(2) a domain comprising an antibody variable region having cancer antigen-binding activity;

(A5-14) the pharmaceutical composition of any one of (A5) to (A5-13), wherein the anti-T cell antigen-binding molecule is a bispecific antibody comprising:

(1) a domain comprising an antibody variable region having T cell receptor complex-binding activity;
(2) a domain comprising an antibody variable region having glypican 3-binding activity; and
(3) a domain comprising an Fc region with reduced binding activity to an Fcγ receptor;

(A5-15) the pharmaceutical composition of any one of (A5) to (A5-14), which is for treatment of cancer; and
(A5-16) the pharmaceutical composition of any one of (A5) to (A5-15), which is for treating cancer while preventing or alleviating cytokine release syndrome associated with administration of the anti-T cell antigen-binding molecule.

Furthermore, the present disclosure relates to administration of an anti-T cell antigen-binding molecule that is accompanied with pre-administration of a cytokine inhibitor, which comprises the following embodiments (when pre-administration of a cytokine inhibitor is performed for each administration of an anti-T cell antigen-binding molecule, in a regimen comprising a step-up dosing period and a subsequent maintenance dosing period):

(A6) a pharmaceutical composition comprising an anti-T cell antigen-binding molecule, wherein the pharmaceutical composition is for use in its combination therapy with a cytokine inhibitor, wherein in the combination therapy

(i) a step-up dosing period and a subsequent maintenance dosing period are comprised for the pharmaceutical composition and
(ii) the cytokine inhibitor is administered before or simultaneously with each administration of the pharmaceutical composition during the step-up dosing period and the maintenance dosing period;

(A6-1) the pharmaceutical composition of (A6), wherein the dose of the pharmaceutical composition is increased stepwise during the step-up dosing period;
(A6-2) the pharmaceutical composition of (A6) or (A6-1), wherein the pharmaceutical composition is administered twice, 3 times, 4 times, or 5 times during the step-up dosing period;
(A6-3) the pharmaceutical composition of any one of (A6) to (A6-2), wherein the pharmaceutical composition is administered at intervals of once every week, once every 2 weeks, or once every 3 weeks during the step-up dosing period;
(A6-4) the pharmaceutical composition of any one of (A6) to (A6-3), wherein the pharmaceutical composition is administered repeatedly at the same dose during the maintenance dosing period;
(A6-5) the pharmaceutical composition of any one of (A6) to (A6-4), wherein the pharmaceutical composition is administered repeatedly at intervals of once every week, once every 2 weeks, or once every 3 weeks during the maintenance dosing period;
(A6-6) the pharmaceutical composition of any one of (A6) to (A6-5), wherein the cytokine inhibitor is administered 6 days, 5 days, 4 days, 3 days, 2 days, or 1 day before each administration of the pharmaceutical composition during the step-up dosing period and the maintenance dosing period, or on the same day as but before said each administration;
(A6-7) the pharmaceutical composition of any one of (A6) to (A6-6), wherein the cytokine inhibitor is further administered on the same day as, or 1 day, 2 days, 3 days, 4 days, 5 days, or 6 days after each administration of the pharmaceutical composition during the step-up dosing period and the maintenance dosing period;
(A6-8) the pharmaceutical composition of any one of (A6) to (A6-7), wherein the cytokine inhibitor is further administered when CRS or signs of CRS develop after the administration of the anti-T cell antigen-binding molecule;
(A6-9) the pharmaceutical composition of (A6-8), wherein the CRS is CRS in Grade 2 or higher or Grade 3 or higher;
(A6-10) the pharmaceutical composition of any one of (A6) to (A6-9), wherein the cytokine inhibitor is an inhibitor of one or more cytokines selected from IL-1β, IL-2, IL-4, IL-5, IL-6, IL-8, IL-10, IL-12, IL-13, IL-15, IL-17, IL-1Ra, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE;
(A6-11) the pharmaceutical composition of any one of (A6) to (A6-10), wherein the cytokine inhibitor is an anti-IL6R antibody;
(A6-12) the pharmaceutical composition of any one of (A6) to (A6-11), wherein the cytokine inhibitor is Tocilizumab, and wherein Tocilizumab is administered at 8 mg/kg or less per dose for a patient weighing 30 kg or more and at 12 kg/kg or less per dose for a patient weighing less than 30 kg;
(A6-13) the pharmaceutical composition of any one of (A6) to (A6-12), wherein a corticosteroid is not administered before or simultaneously with the administration of the pharmaceutical composition;
(A6-14) the pharmaceutical composition of any one of (A6) to (A6-12), wherein a corticosteroid is further administered before, simultaneously with, or after the administration of the pharmaceutical composition;

(A6-15) the pharmaceutical composition of (A6-13) or (A6-14), wherein the corticosteroid is dexamethasone, a pharmaceutically acceptable salt thereof, or a derivative thereof;

(A6-16) the pharmaceutical composition of any one of (A6) to (A6-15), wherein the anti-T cell antigen-binding molecule is a bispecific antigen-binding molecule comprising:

(1) a domain comprising an antibody variable region having T cell receptor complex-binding activity; and
(2) a domain comprising an antibody variable region having cancer antigen-binding activity; (A6-17) the pharmaceutical composition of any one of (A6) to (A6-16), wherein the anti-T cell antigen-binding molecule is a bispecific antibody comprising:

(1) a domain comprising an antibody variable region having T cell receptor complex-binding activity;
(2) a domain comprising an antibody variable region having glypican 3-binding activity; and
(3) a domain comprising an Fc region with reduced binding activity to an Fcγ receptor; (A6-18) the pharmaceutical composition of any one of (A6) to (6-17), which is for treatment of cancer; and

(A6-19) the pharmaceutical composition of any one of (A6) to (A6-18), which is for treating cancer while preventing or alleviating cytokine release syndrome associated with administration of the anti-T cell antigen-binding molecule.

Furthermore, the present disclosure relates to each of the following embodiments (when, in a regimen comprising a step-up dosing period and a subsequent maintenance dosing period, pre-administration of a cytokine inhibitor is performed for administration of an anti-T cell antigen-binding molecule during the step-up dosing period):

(A7) a pharmaceutical composition comprising an anti-T cell antigen-binding molecule, wherein the pharmaceutical composition is for use in its combination therapy with a cytokine inhibitor, wherein in the combination therapy

(i) a step-up dosing period and a subsequent maintenance dosing period are comprised for the pharmaceutical composition, and
(ii) the cytokine inhibitor is administered before or simultaneously with each administration of the pharmaceutical composition during the step-up dosing period;

(A7-1) the pharmaceutical composition of (A7), wherein the dose of the pharmaceutical composition is increased stepwise during the step-up dosing period;

(A7-2) the pharmaceutical composition of (A7) or (A7-1), wherein the pharmaceutical composition is administered twice, 3 times, 4 times, or 5 times during the step-up dosing period;

(A7-3) the pharmaceutical composition of any one of (A7) to (A7-2), wherein the pharmaceutical composition is administered at intervals of once every week, once every 2 weeks, or once every 3 weeks during the step-up dosing period;

(A7-4) the pharmaceutical composition of any one of (A7) to (A7-3), wherein the pharmaceutical composition is administered repeatedly at the same dose during the maintenance dosing period;

(A7-5) the pharmaceutical composition of any one of (A7) to (A7-4), wherein the pharmaceutical composition is administered repeatedly at intervals of once every week, once every 2 weeks, or once every 3 weeks during the maintenance dosing period;

(A7-6) the pharmaceutical composition of any one of (A7) to (A7-5), wherein the cytokine inhibitor is administered 6 days, 5 days, 4 days, 3 days, 2 days, or 1 day before each administration of the pharmaceutical composition during the step-up dosing period, or on the same day as but before said each administration;

(A7-7) the pharmaceutical composition of any one of (A7) to (A7-6), wherein the cytokine inhibitor is further administered on the same day as, or 1 day, 2 days, 3 days, 4 days, 5 days, or 6 days after each administration of the pharmaceutical composition during the step-up dosing period;

(A7-8) the pharmaceutical composition of any one of (A7) to (A7-7), wherein the cytokine inhibitor is further administered when CRS or signs of CRS develop after the administration of the pharmaceutical composition;

(A7-9) the pharmaceutical composition of (A7-8), wherein the CRS is CRS in Grade 2 or higher or Grade 3 or higher;

(A7-10) the pharmaceutical composition of any one of (A7) to (A7-9), wherein the cytokine inhibitor is an inhibitor of one or more cytokines selected from IL-1β, IL-2, IL-4, IL-5, IL-6, IL-8, IL-10, IL-12, IL-13, IL-15, IL-17, IL-1Ra, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE;

(A7-11) the pharmaceutical composition of any one of (A7) to (A7-10), wherein the cytokine inhibitor is an anti-IL6R antibody;

(A7-12) the pharmaceutical composition of any one of (A7) to (A7-11), wherein the cytokine inhibitor is Tocilizumab, and wherein Tocilizumab is administered at 8 mg/kg or less per dose for a patient weighing 30 kg or more and at 12 kg/kg or less per dose for a patient weighing less than 30 kg;

(A7-13) the pharmaceutical composition of any one of (A7) to (A7-12), wherein a corticosteroid is not administered before or simultaneously with the administration of the pharmaceutical composition;

(A7-14) the pharmaceutical composition of any one of (A7) to (A7-12), wherein a corticosteroid is further administered before, simultaneously with, or after the administration of the pharmaceutical composition;

(A7-15) the pharmaceutical composition of (A7-13) or (A7-14), wherein the corticosteroid is dexamethasone, a pharmaceutically acceptable salt thereof, or a derivative thereof;

(A7-16) the pharmaceutical composition of any one of (A7) to (A7-15), wherein the anti-T cell antigen-binding molecule is a bispecific antigen-binding molecule comprising

(1) a domain comprising an antibody variable region having T cell receptor complex-binding activity, and

(2) a domain comprising an antibody variable region having cancer antigen-binding activity; (A7-17) the pharmaceutical composition of any one of (A7) to (A7-16), wherein the anti-T cell antigen-binding molecule is a bispecific antibody comprising

(1) a domain comprising an antibody variable region having T cell receptor complex-binding activity,

(2) a domain comprising an antibody variable region having glypican 3-binding activity, and

(3) a domain comprising an Fc region with reduced binding activity to an Fc$\gamma$ receptor; (A7-18) the pharmaceutical composition of any one of (A7) to (A7-17), which is for treatment of cancer; and

(A7-19) the pharmaceutical composition of any one of (A7) to (A7-18), which is for treating cancer while preventing or alleviating cytokine release syndrome associated with administration of the anti-T cell antigen-binding molecule.

Alternatively, the present disclosure provides each of the following embodiments (when, in a regimen comprising a step-up dosing period and a subsequent maintenance dosing period, pre-administration of a cytokine inhibitor is performed for administration of an anti-T cell antigen-binding molecule for each administration during the step-up dosing period, and for the first or multiple administrations during the maintenance dosing period):

(A8) a pharmaceutical composition comprising an anti-T cell antigen-binding molecule, wherein the pharmaceutical composition is for use in its combination therapy with a cytokine inhibitor, wherein in the combination therapy

(i) a step-up dosing period and a subsequent maintenance dosing period are comprised for the pharmaceutical composition, and

(ii) (a) the cytokine inhibitor is administered before or simultaneously with each administration of the pharmaceutical composition during the step-up dosing period, and (b) the cytokine inhibitor is administered before or simultaneously with the first administration or each of the first to second, the first to third, the first to fourth, or the first to fifth administrations of the pharmaceutical composition during the maintenance dosing period;

(A8-1) the pharmaceutical composition of (A8), wherein the dose of the pharmaceutical composition is increased stepwise during the step-up dosing period;

(A8-2) the pharmaceutical composition of (A8) or (A8-1), wherein the pharmaceutical composition is administered twice, 3 times, 4 times, or 5 times during the step-up dosing period;

(A8-3) the pharmaceutical composition of any one of (A8) to (A8-2), wherein the pharmaceutical composition is administered at intervals of once every week, once every 2 weeks, or once every 3 weeks during the step-up dosing period;

(A8-4) the pharmaceutical composition of any one of (A8) to (A8-3), wherein the pharmaceutical composition is administered repeatedly at the same dose during the maintenance dosing period;

(A8-5) the pharmaceutical composition of any one of (A8) to (A8-4), wherein the pharmaceutical composition is administered repeatedly at intervals of once every week, once every 2 weeks, or once every 3 weeks during the maintenance dosing period;

(A8-6) the pharmaceutical composition of any one of (A8) to (A8-5), wherein the cytokine inhibitor is administered

(a) 6 days, 5 days, 4 days, 3 days, 2 days, or 1 day before each administration of the pharmaceutical composition during the step-up dosing period, or on the same day as but before said each administration, and

(b) 6 days, 5 days, 4 days, 3 days, 2 days, or 1 day before the first administration or each administration of the first to second, the first to third, the first to fourth, or the first to fifth administrations of the pharmaceutical composition during the maintenance dosing period, or on the same day as but before said first or each administration;

(A8-7) the pharmaceutical composition of any one of (A8) to (A8-6), wherein the cytokine inhibitor is further administered

(a) on the same day as, or 1 day, 2 days, 3 days, 4 days, 5 days, or 6 days after each administration of the pharmaceutical composition during the step-up dosing period, and
(b) on the same day as, or 1 day, 2 days, 3 days, 4 days, 5 days, or 6 days after the first administration or each administration of the first to second, the first to third, the first to fourth, or the first to fifth administrations of the pharmaceutical composition during the maintenance dosing period;

(A8-8) the pharmaceutical composition of any one of (A8) to (A8-7), wherein the cytokine inhibitor is further administered when CRS or signs of CRS develop after the administration of the pharmaceutical composition;
(A8-9) the pharmaceutical composition of (A8-8), wherein the CRS is CRS in Grade 2 or higher or Grade 3 or higher;
(A8-10) the pharmaceutical composition of any one of (A8) to (A8-9), wherein the cytokine inhibitor is an inhibitor of one or more cytokines selected from IL-1β, IL-2, IL-4, IL-5, IL-6, IL-8, IL-10, IL-12, IL-13, IL-15, IL-17, IL-1Ra, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE;
(A8-11) the pharmaceutical composition of any one of (A8) to (A8-10), wherein the cytokine inhibitor is an anti-IL6R antibody;
(A8-12) the pharmaceutical composition of any one of (A8) to (A8-11), wherein the cytokine inhibitor is Tocilizumab, and wherein Tocilizumab is administered at 8 mg/kg or less per dose for a patient weighing 30 kg or more and at 12 kg/kg or less per dose for a patient weighing less than 30 kg;
(A8-13) the pharmaceutical composition of any one of (A8) to (A8-12), wherein a corticosteroid is not administered before or simultaneously with the administration of the pharmaceutical composition;
(A8-14) the pharmaceutical composition of any one of (A8) to (A8-12), wherein a corticosteroid is further administered before, simultaneously with, or after the administration of the pharmaceutical composition;
(A8-15) the pharmaceutical composition of (A8-13) or (A8-14), wherein the corticosteroid is dexamethasone, a pharmaceutically acceptable salt thereof, or a derivative thereof;
(A8-16) the pharmaceutical composition of any one of (A8) to (A8-15), wherein the anti-T cell antigen-binding molecule is a bispecific antigen-binding molecule comprising:

(1) a domain comprising an antibody variable region having T cell receptor complex-binding activity; and
(2) a domain comprising an antibody variable region having cancer antigen-binding activity;

(A8-17) the pharmaceutical composition of any one of (A8) to (A8-16), wherein the anti-T cell antigen-binding molecule is a bispecific antibody comprising:

(1) a domain comprising an antibody variable region having T cell receptor complex-binding activity;
(2) a domain comprising an antibody variable region having glypican 3-binding activity; and
(3) a domain comprising an Fc region with reduced binding activity to an Fcγ receptor;

(A8-18) the pharmaceutical composition of any one of (A8) to (A8-17), which is for treatment of cancer; and
(A8-19) the pharmaceutical composition of any one of (A8) to (A8-18), which is for treating cancer while preventing or alleviating cytokine release syndrome associated with administration of the anti-T cell antigen-binding molecule.

Alternatively, the present disclosure relates to each of the following embodiments (when, in a regimen comprising a step-up dosing period and a subsequent maintenance dosing period, pre-administration of a cytokine inhibitor is performed for administration of an anti-T cell antigen-binding molecule for each administration during the step-up dosing period, and for every dosing cycle during the maintenance dosing period):

(A9) a pharmaceutical composition comprising an anti-T cell antigen-binding molecule, wherein the pharmaceutical composition is for use in its combination therapy with a cytokine inhibitor, wherein in the combination therapy

(i) a step-up dosing period and a subsequent maintenance dosing period are comprised for the pharmaceutical composition,
(ii) the pharmaceutical composition is administered repeatedly in dosing cycles where 2, 3, 4, or 5 administrations constitute one cycle during the maintenance dosing period, and

(iii) (a) the cytokine inhibitor is administered before or simultaneously with each administration of the pharmaceutical composition during the step-up dosing period, and (b) the cytokine inhibitor is administered before or simultaneously with the first administration of the pharmaceutical composition in each of the dosing cycles during the maintenance dosing period;

(A9-1) the pharmaceutical composition of (A9), wherein the dose of the pharmaceutical composition is increased stepwise during the step-up dosing period;

(A9-2) the pharmaceutical composition of (A9) or (A9-1), wherein the pharmaceutical composition is administered twice, 3 times, 4 times, or 5 times during the step-up dosing period;

(A9-3) the pharmaceutical composition of any one of (A9) to (A9-2), wherein the pharmaceutical composition is administered at intervals of once every week, once every 2 weeks, or once every 3 weeks during the step-up dosing period;

(A9-4) the pharmaceutical composition of any one of (A9) to (A9-3), wherein the pharmaceutical composition is administered repeatedly at the same dose during the maintenance dosing period;

(A9-5) the pharmaceutical composition of any one of (A9) to (A9-4), wherein the pharmaceutical composition is administered repeatedly at intervals of once every week, once every 2 weeks, or once every 3 weeks during the maintenance dosing period;

(A9-6) the pharmaceutical composition of any one of (A9) to (A9-5), wherein the cytokine inhibitor is administered

(a) 6 days, 5 days, 4 days, 3 days, 2 days, or 1 day before each administration of the pharmaceutical composition during the step-up dosing period, or on the same day as but before said each administration, and
(b) 6 days, 5 days, 4 days, 3 days, 2 days, or 1 day before the first administration of the pharmaceutical composition in each of the dosing cycles during the maintenance dosing period, or on the same day as but before said first administration;

(A9-7) the pharmaceutical composition of any one of (A9) to (A9-6), wherein the cytokine inhibitor is further administered

(a) on the same day as, or 1 day, 2 days, 3 days, 4 days, 5 days, or 6 days after each administration of the pharmaceutical composition during the step-up dosing period, and
(b) on the same day as, or 1 day, 2 days, 3 days, 4 days, 5 days, or 6 days after the first administration of the pharmaceutical composition in each of the dosing cycles during the maintenance dosing period;

(A9-8) the pharmaceutical composition of any one of (A9) to (A9-7), wherein the cytokine inhibitor is further administered when CRS or signs of CRS develop after the administration of the pharmaceutical composition;

(A9-9) the pharmaceutical composition of (A9-8), wherein the CRS is CRS in Grade 2 or higher or Grade 3 or higher;

(A9-10) the pharmaceutical composition of any one of (A9) to (A9-9), wherein the cytokine inhibitor is an inhibitor of one or more cytokines selected from IL-1β, IL-2, IL-4, IL-5, IL-6, IL-8, IL-10, IL-12, IL-13, IL-15, IL-17, IL-1Ra, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE;

(A9-11) the pharmaceutical composition of any one of (A9) to (A9-10), wherein the cytokine inhibitor is an anti-IL6R antibody;

(A9-12) the pharmaceutical composition of any one of (A9) to (A9-11), wherein the cytokine inhibitor is Tocilizumab, and wherein Tocilizumab is administered at 8 mg/kg or less per dose for a patient weighing 30 kg or more and at 12 kg/kg or less per dose for a patient weighing less than 30 kg;

(A9-13) the pharmaceutical composition of any one of (A9) to (A9-12), wherein a corticosteroid is not administered before or simultaneously with the administration of the pharmaceutical composition;

(A9-14) the pharmaceutical composition of any one of (A9) to (A9-12), wherein a corticosteroid is further administered before, simultaneously with, or after the administration of the pharmaceutical composition;

(A9-15) the pharmaceutical composition of (A9-13) or (A9-14), wherein the corticosteroid is dexamethasone, a pharmaceutically acceptable salt thereof, or a derivative thereof;

(A9-16) the pharmaceutical composition of any one of (A9) to (A9-15), wherein the anti-T cell antigen-binding molecule is a bispecific antigen-binding molecule comprising:

(1) a domain comprising an antibody variable region having T cell receptor complex-binding activity; and
(2) a domain comprising an antibody variable region having cancer antigen-binding activity;

(A9-17) the pharmaceutical composition of any one of (A9) to (A9-16), wherein the anti-T cell antigen-binding molecule is a bispecific antibody comprising:

(1) a domain comprising an antibody variable region having T cell receptor complex-binding activity;
(2) a domain comprising an antibody variable region having glypican 3-binding activity; and
(3) a domain comprising an Fc region with reduced binding activity to an Fcγ receptor;

(A9-18) the pharmaceutical composition of any one of (A9) to (A9-17), which is for treatment of cancer; and
(A9-19) the pharmaceutical composition of any one of (A9) to (A9-18), which is for treating cancer while preventing or alleviating cytokine release syndrome associated with administration of the anti-T cell antigen-binding molecule.

Alternatively, in a non-limiting embodiment, the present disclosure relates to the following:

(A10) a kit comprising (i) a container, (ii) a pharmaceutical composition in the container, the composition comprising an anti-T cell antigen-binding molecule, and (iii) a document instructing to administer a cytokine inhibitor before, simultaneously with, or after administration of the pharmaceutical composition;
(A10-1) the kit of (A10-1), further comprising a label attached to the container, wherein the label indicates that the pharmaceutical composition can be used for treatment of cancer;
(A10-2) the kit of (A10) or (A10-1), wherein prescription of the pharmaceutical composition is performed according to the instructions in the document;
(A10-3) the kit of (A10) or (A10-1), which is for treating cancer by the prescription instructed by the document;
(A11) use of an anti-T cell antigen-binding molecule in producing a pharmaceutical composition for its combination therapy with a cytokine inhibitor; and
(A12) use of an anti-T cell antigen-binding molecule in its combination therapy with a cytokine inhibitor.

Alternatively, in a non-limiting embodiment, the present disclosure relates to the following:
(A13) a method of treating cancer by administering an anti-T cell antigen-binding molecule to a subject while preventing, alleviating, and/or treating cytokine release syndrome associated with the administration of the anti-T cell antigen-binding molecule, the method comprising using a cytokine inhibitor in combination.

Alternatively, the present disclosure comprises the following embodiments:

(B1) a pharmaceutical composition comprising a cytokine inhibitor, wherein the pharmaceutical composition is for use in its combination therapy with an anti-T cell antigen-binding molecule;
(B1-1) the pharmaceutical composition of (B1), which is for administration before, simultaneously with, or after administration of the anti-T cell antigen-binding molecule;
(B1-2) the pharmaceutical composition of (B1) or (B1-1), which is for administration before or simultaneously with the administration of the anti-T cell antigen-binding molecule;
(B1-3) the pharmaceutical composition of any one of (B1) to (B1-2), which is for administration 6 days, 5 days, 4 days, 3 days, 2 days, or 1 day before the administration of the anti-T cell antigen-binding molecule, or on the same day as but before said administration;
(B1-4) the pharmaceutical composition of any one of (B1) to (B1-3), which is for further administration on the same day as, or 1 day, 2 days, 3 days, 4 days, 5 days, or 6 days before the administration of the anti-T cell antigen-binding molecule;
(B1-5) the pharmaceutical composition of any one of (B1) to (B1-4), which is for further administration when CRS or signs of CRS develop after the administration of the anti-T cell antigen-binding molecule;
(B1-6) the pharmaceutical composition of (B1-5), wherein the CRS is CRS in Grade 2 or higher or Grade 3 or higher;
(B1-7) the pharmaceutical composition of (B1) or (B1-1), which is further administered after the administration of the anti-T cell antigen-binding molecule;
(B1-8) the pharmaceutical composition of (B1) or (B1-7), which is further administered when CRS or signs of CRS develop after the administration of the anti-T cell antigen-binding molecule;
(B1-9) the pharmaceutical composition of (B1-7) or (B1-8), wherein the CRS is CRS in Grade 2 or higher or Grade 3 or higher;
(B1-10) the pharmaceutical composition of any one of (B1) to (B1-9), wherein the cytokine inhibitor is an inhibitor of one or more cytokines selected from IL-1β, IL-2, IL-4, IL-5, IL-6, IL-8, IL-10, IL-12, IL-13, IL-15, IL-17, IL-1Ra, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE;
(B1-11) the pharmaceutical composition of any one of (B1) to (B1-10), wherein the cytokine inhibitor is an anti-IL6R antibody;

(B1-12) the pharmaceutical composition of any one of (B1) to (B1-11), wherein the cytokine inhibitor is Tocilizumab, and wherein Tocilizumab is administered at 8 mg/kg or less per dose for a patient weighing 30 kg or more and at 12 mg/kg or less per dose for a patient weighing less than 30 kg;

(B1-13) the pharmaceutical composition of any one of (B1) to (B1-12), wherein a corticosteroid is not administered before or simultaneously with the administration of the anti-T cell antigen-binding molecule;

(B1-14) the pharmaceutical composition of any one of (B1) to (B1-12), wherein a corticosteroid is further administered before, simultaneously with, or after the administration of the anti-T cell antigen-binding molecule;

(B1-15) the pharmaceutical composition of (B1-13) or (B1-14), wherein the corticosteroid is dexamethasone, a pharmaceutically acceptable salt thereof, or a derivative thereof;

(B1-16) the pharmaceutical composition of any one of (B1) to (B1-15), wherein the anti-T cell antigen-binding molecule is a bispecific antigen-binding molecule comprising:

(1) a domain comprising an antibody variable region having T cell receptor complex-binding activity; and
(2) a domain comprising an antibody variable region having cancer antigen-binding activity; (B1-17) the pharmaceutical composition of any one of (B1) to (B1-16), wherein the anti-T cell antigen-binding molecule is a bispecific antibody comprising:

(1) a domain comprising an antibody variable region having T cell receptor complex-binding activity;
(2) a domain comprising an antibody variable region having glypican 3-binding activity; and
(3) a domain comprising an Fc region with reduced binding activity to an Fcγ receptor; and (B1-18) the pharmaceutical composition of any one of (B1) to (B1-17), which is for preventing or alleviating cytokine release syndrome associated with administration of the anti-T cell antigen-binding molecule.

Alternatively, the present disclosure comprises the following embodiments:

(B2) a pharmaceutical composition comprising a cytokine inhibitor, wherein the pharmaceutical composition is for use in its combination therapy with an anti-T cell antigen-binding molecule, wherein in the combination therapy,

(i) the anti-T cell antigen-binding molecule is administered repeatedly, and
(ii) the pharmaceutical composition is for administration before or simultaneously with each administration of the repeated administration;

(B3) a pharmaceutical composition comprising a cytokine inhibitor, wherein the pharmaceutical composition is for use in its combination therapy with an anti-T cell antigen-binding molecule, wherein in the combination therapy,

(i) the anti-T cell antigen-binding molecule is administered repeatedly, and
(ii) the pharmaceutical composition is for administration after or simultaneously with the first or each of the first to second, the first to third, the first to fourth, or the first to fifth administrations in the repeated administration;

(B4) a pharmaceutical composition comprising a cytokine inhibitor, wherein the pharmaceutical composition is for use in its combination therapy with an anti-T cell antigen-binding molecule, wherein in the combination therapy,

(i) the anti-T cell antigen-binding molecule is administered repeatedly in dosing cycles where 2, 3, 4, or 5 administrations constitute one cycle, and
(ii) the pharmaceutical composition is for administration before or simultaneously with the first administration of the anti-T cell antigen-binding molecule in each of the dosing cycles;

(B5) a pharmaceutical composition comprising a cytokine inhibitor, wherein the pharmaceutical composition is for use in its combination therapy with an anti-T cell antigen-binding molecule, wherein in the combination therapy,

(i) the anti-T cell antigen-binding molecule is administered repeatedly in dosing cycles where 2, 3, 4, or 5 administrations constitute one cycle, and
(ii) the pharmaceutical composition is for (a) administration before or simultaneously with each administration of the anti-T cell antigen-binding molecule during the first dosing cycle, and (b) administration before or simultaneously with the first administration of the anti-T cell antigen-binding molecule in each of the second and subsequent dosing cycles;

(B6) a pharmaceutical composition comprising a cytokine inhibitor, wherein the pharmaceutical composition is for use in its combination therapy with an anti-T cell antigen-binding molecule, wherein in the combination therapy

(i) a step-up dosing period and a subsequent maintenance dosing period are comprised for the anti-T cell antigen-binding molecule, and
(ii) the pharmaceutical composition is for administration before or simultaneously with each administration of the anti-T cell antigen-binding molecule during the step-up dosing period and the maintenance dosing period;

(B7) a pharmaceutical composition comprising a cytokine inhibitor, wherein the pharmaceutical composition is for use in its combination therapy with an anti-T cell antigen-binding molecule, wherein in the combination therapy,

(i) the anti-T cell antigen-binding molecule is administered repeatedly in dosing cycles where 2, 3, 4, or 5 administrations constitute one cycle, and
(ii) the pharmaceutical composition is for (a) administration before or simultaneously with each administration of the anti-T cell antigen-binding molecule during the first dosing cycle, and (b) administration before or simultaneously with the first administration of the anti-T cell antigen-binding molecule in each of the second and subsequent dosing cycles;

(B8) a pharmaceutical composition comprising a cytokine inhibitor, wherein the pharmaceutical composition is for use in its combination therapy with an anti-T cell antigen-binding molecule, wherein in the combination therapy

(i) a step-up dosing period and a subsequent maintenance dosing period are comprised for the anti-T cell antigen-binding molecule, and
(ii) the pharmaceutical composition is for administration (a) before or simultaneously with each administration of the anti-T cell antigen-binding molecule during the step-up dosing period, and (b) before or simultaneously with the first administration or each of the first to second, the first to third, the first to fourth, or the first to fifth administrations of the anti-T cell antigen-binding molecule during the maintenance dosing period; and

(B9) a pharmaceutical composition comprising a cytokine inhibitor, wherein the pharmaceutical composition is for use in its combination therapy with a T cell antigen-binding molecule, wherein in the combination therapy

(i) a step-up dosing period and a subsequent maintenance dosing period are comprised for the anti-T cell antigen-binding molecule,
(ii) the anti-T cell antigen-binding molecule is administered repeatedly in dosing cycles where 2, 3, 4, or 5 administrations constitute one cycle during the maintenance dosing period, and
(iii) the pharmaceutical composition is for administration (a) before or simultaneously with each administration of the anti-T cell antigen-binding molecule during the step-up dosing period, and (b) before or simultaneously with the first administration of the anti-T cell antigen-binding molecule in each of the dosing cycles during the maintenance dosing period.

Alternatively, in a non-limiting embodiment, the present disclosure relates to the following:

(B10) a kit comprising (i) a container, (ii) a pharmaceutical composition in the container, the composition comprising a cytokine inhibitor, and (iii) a document instructing to administer the pharmaceutical composition before, simultaneously with, or after administration of an anti-T cell antigen-binding molecule;
(B10-1) the kit of (B10-1), further comprising a label attached to the container, wherein the label indicates that the pharmaceutical composition can be used for prevention, alleviation, and/or treatment of cytokine release syndrome associated with the administration of the anti-T cell antigen-binding molecule;
(B 10-2) the kit of (B10) or (B10-1), wherein prescription of the pharmaceutical composition is performed according to the instructions in the document;
(B10-3) the kit of (B10) or (B10-1), which is for preventing, alleviating, and/or treating cytokine release syndrome associated with the administration of the anti-T cell antigen-binding molecule by a prescription instructed by the document;
(B11) use of a cytokine inhibitor in producing a pharmaceutical composition for its combination therapy with an anti-T cell antigen-binding molecule; and
(B12) use of a cytokine inhibitor in its combination therapy with an anti-T cell antigen-binding molecule.

Alternatively, in a non-limiting embodiment, the present disclosure relates to the following:

(B13) a method of preventing, alleviating, and/or treating cytokine release syndrome associated with administration of an anti-T cell antigen-binding molecule, wherein the method comprises administering a cytokine inhibitor to a subject; and

(B14) a method of preventing and/or alleviating cytokine release syndrome associated with administration of an anti-T cell antigen-binding molecule, wherein the method comprises administering a cytokine inhibitor to a subject before or simultaneously with the administration of the anti-T cell antigen-binding molecule.

Alternatively, in a non-limiting embodiment, the present disclosure relates to the following:

(C1) a method of treating cancer, the method comprising administering an anti-T cell antigen-binding molecule and a cytokine inhibitor to a subject;

(C1-1) the method of (C1), wherein the cytokine inhibitor is administered before, simultaneously with, or after administration of the anti-T cell antigen-binding molecule;

(C1-2) the method of (C1) or (C1-1), wherein the cytokine inhibitor is administered before or simultaneously with the administration of the anti-T cell antigen-binding molecule;

(C1-3) the method of any one of (C1) to (CI-2), wherein the cytokine inhibitor is administered 6 days, 5 days, 4 days, 3 days, 2 days, or 1 day before the administration of the anti-T cell antigen-binding molecule, or on the same day as but before said administration; and

(C1-4) the method of any one of (C1) to (C1-3), wherein the cytokine inhibitor is further administered on the same day as, or 1 day, 2 days, 3 days, 4 days, 5 days, or 6 days after the administration of the anti-T cell antigen-binding molecule.

In a non-limiting embodiment, the present disclosure relates to combination therapies of various embodiments disclosed herein, which comprise administering an anti-T cell antigen-binding molecule and cytokine inhibitor; methods for treating cancer by the combination therapies; and methods for preventing, alleviating, and/or treating, by the combination therapies, cytokine release syndrome associated with administration of an anti-T cell antigen-binding molecule. Alternatively, in the methods for treating cancer comprising administering an anti-T cell antigen-binding molecule, the present disclosure provides combination therapies aimed at any one or a combination selected from prevention, alleviation, and treatment of cytokine release syndrome associated with administration of the anti-T cell antigen-binding molecule.

Alternatively, in a non-limiting embodiment, the present disclosure relates to the following:

(C2) a method for a combination therapy comprising steps of administering an anti-T cell antigen-binding molecule and administering a cytokine inhibitor to a subject;

(C3) a combinatorial therapy, comprising an anti-T cell antigen-binding molecule and a cytokine inhibitor;

(C4) a combination of an anti-T cell antigen-binding molecule and a cytokine inhibitor;

(C5) a pharmaceutical composition comprising an anti-T cell antigen-binding molecule and a cytokine inhibitor; and

(C5-1) the pharmaceutical composition of (C5), which is for treatment of cancer.

[Effects of the Invention]

Anti-T cell antigen-binding molecules which bind to T cell antigens such as CD3 are drawing attention as novel means for treating cancer that utilizes the antitumor effects of T cells possessed by living bodies. However, since they target T cells that produce cytokines, possibility of causing adverse reactions due to cytokine production (for example, cytokine release syndrome) has been pointed out. Therefore, concerns of unexpected adverse reactions caused by cytokine production had existed for administration of anti-T cell antigen-binding molecules. In the present disclosure, an anti-T cell antigen-binding molecule is administered in combination with a cytokine inhibitor in a preplanned manner; therefore, unexpected adverse reactions caused by cytokines can be prevented or alleviated.

[Brief Description of Drawings]

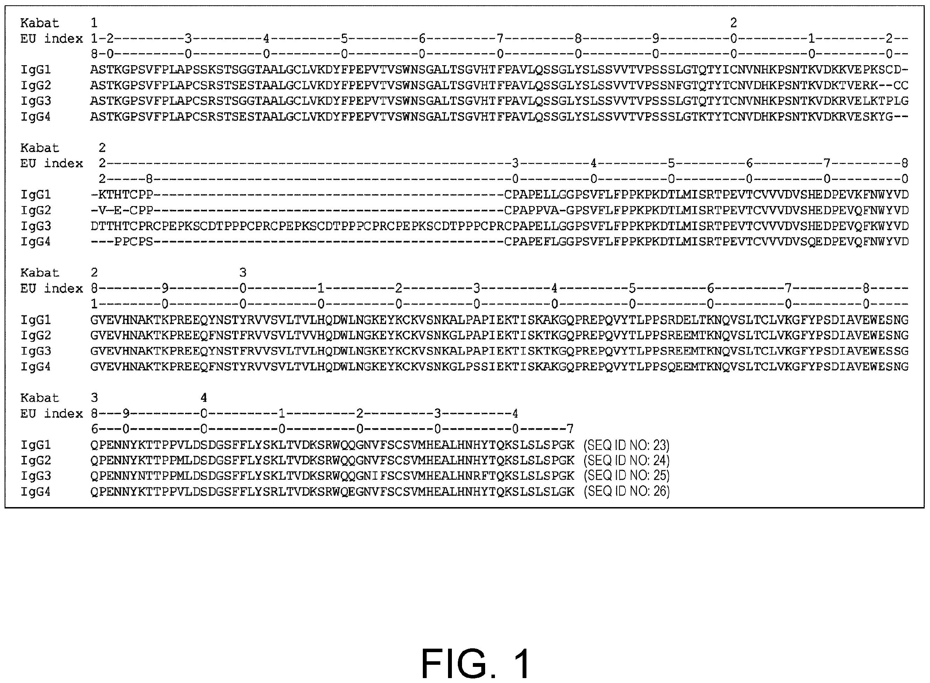

[Fig. 1] Fig. 1 shows the relationship between the amino acid residues constituting the Fc regions of IgG1, IgG2, IgG3, and IgG4, and the Kabat EU numbering system (herein, also referred to as EU INDEX).

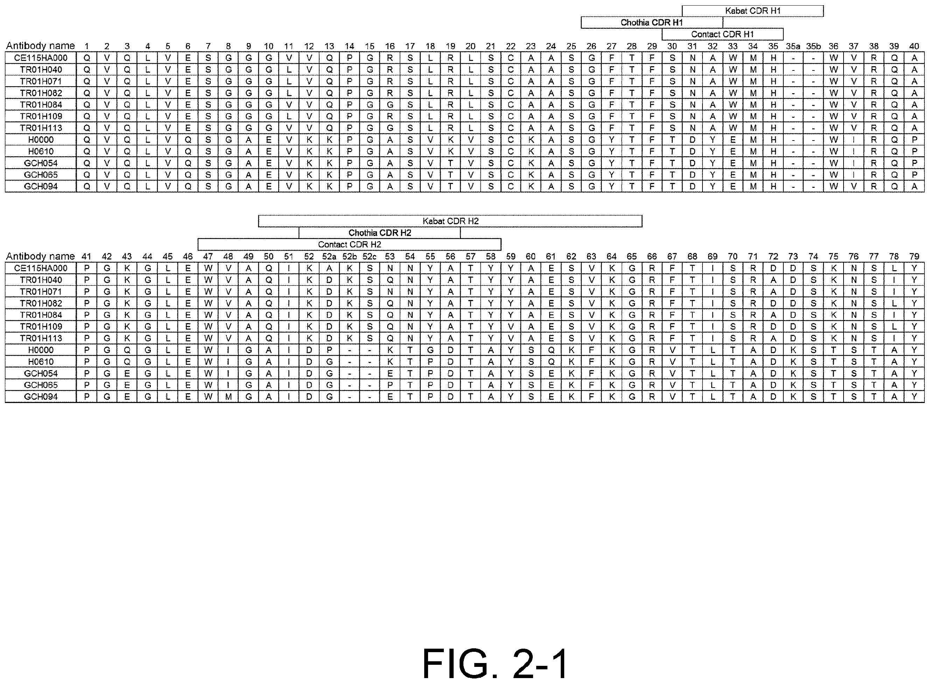

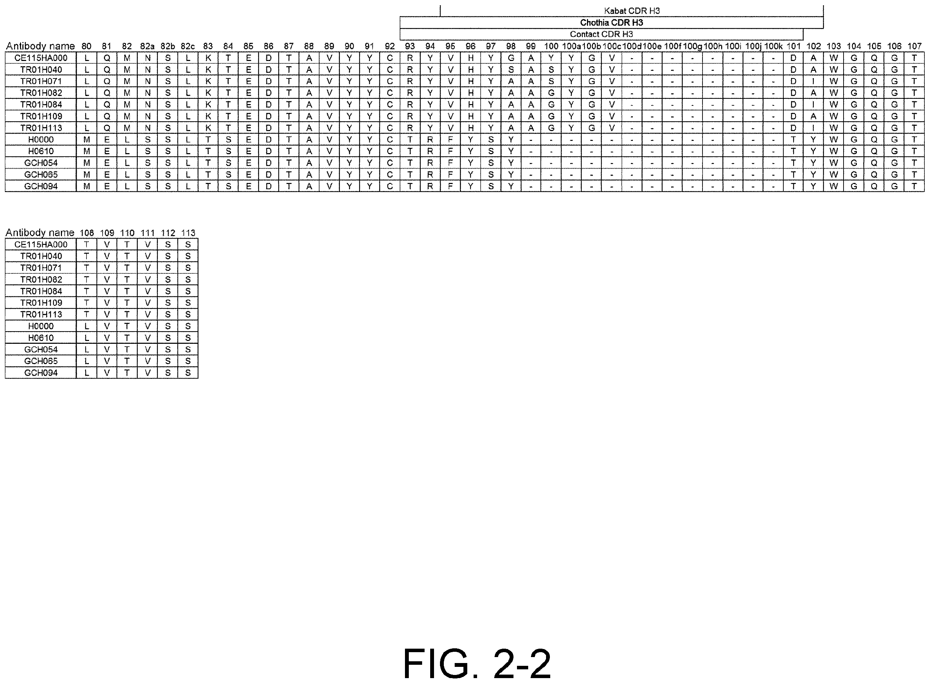

[Fig. 2] Fig. 2-1 shows the heavy chain variable region sequences and their various numbering according to Kabat et al.

[Fig. 2-2] Fig. 2-2 shows the heavy chain variable region sequences and their various numbering according to Kabat et al.

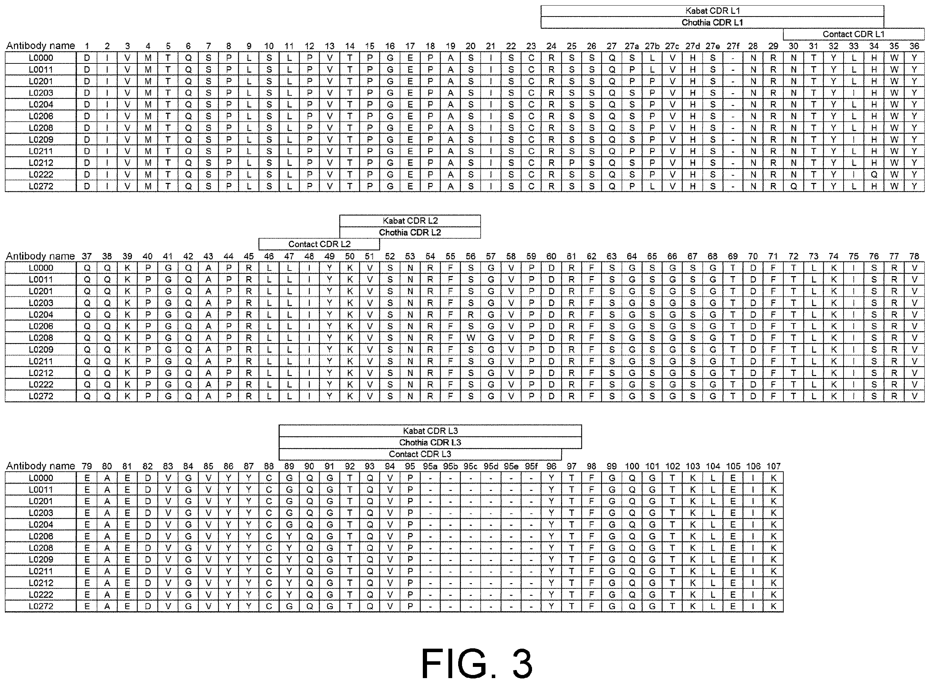

[Fig. 3] Fig. 3 shows the light chain variable region sequences and their various numbering according to Kabat et al.

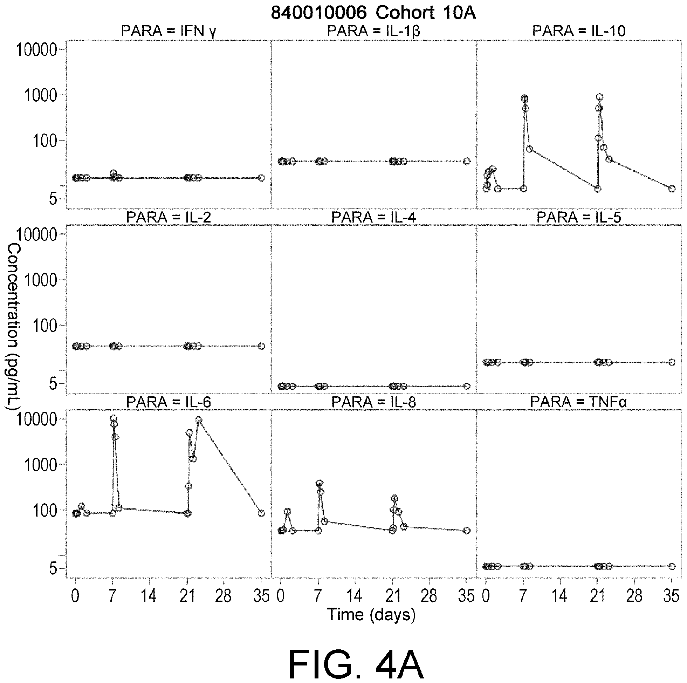

[Fig. 4A] Fig. 4A shows cytokine detection in patient ID840010006 of cohort 10A. The vertical axis represents the serum cytokine concentration (pg/mL), and the horizontal axis represents the number of days from the first day of administration; provided that, the number of days on the horizontal axis is the nominal time calculated based on the clinical trial visit and blood sampling time defined in the study protocol. In the present figure, the administration start day (first day) is displayed as Day 0, and ERY974 was administered on Days 0, 7, and 21 on the present figure (drug interruption on Day 14, and dose reduction on Day 21).

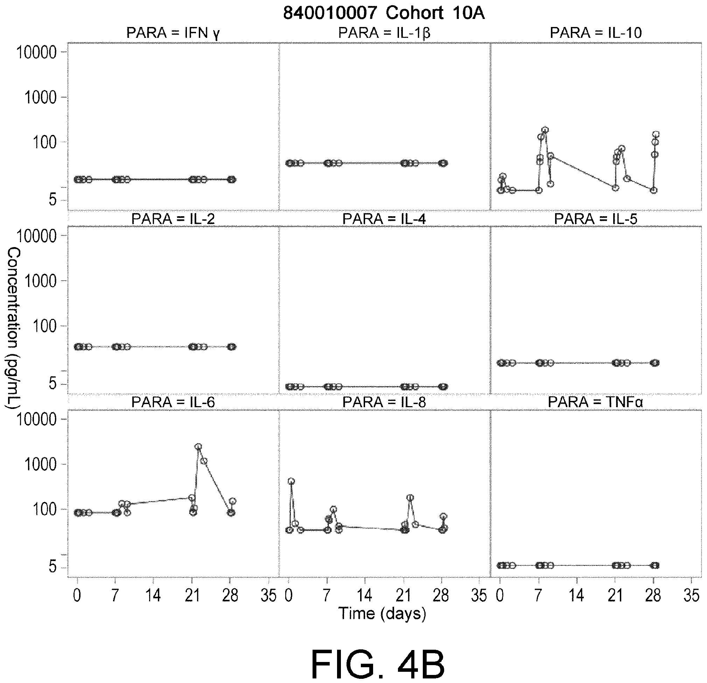

[Fig. 4B] Fig. 4B shows cytokine detection in patient ID840010007 of cohort 10A. The vertical axis represents the serum cytokine concentration (pg/mL), and the horizontal axis represents the number of days from the first day of administration. In the present figure, the administration start day (first day) is displayed as Day 0, and ERY974 was administered on Days 0, 7, 21, and 28 in the present figure (drug interruption on Day 14, and dose-reduction on Day 21). As in Fig. 4A, the number of days on the horizontal axis is the nominal time.

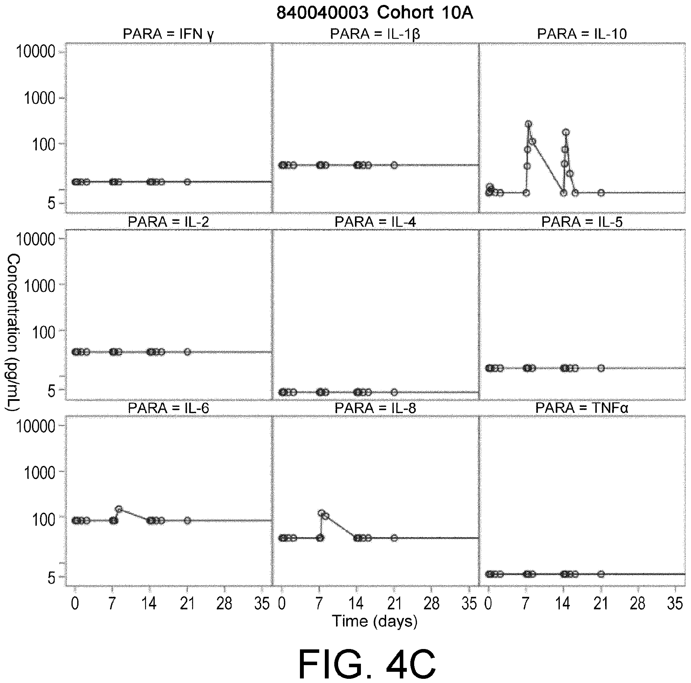

[Fig. 4C] Fig. 4C shows cytokine detection in patient ID840040003 of cohort 10A. The vertical axis represents the serum cytokine concentration (pg/mL), and the horizontal axis represents the number of days from the first day of administration. In the present figure, the administration start day (first day) is displayed as Day 0, and ERY974 was administered on Days 0, 7, 14, 21, and 28 in the present figure (dose reduction on Day 14 and onwards). As in Fig. 4A, the number of days on the horizontal axis is the nominal time.

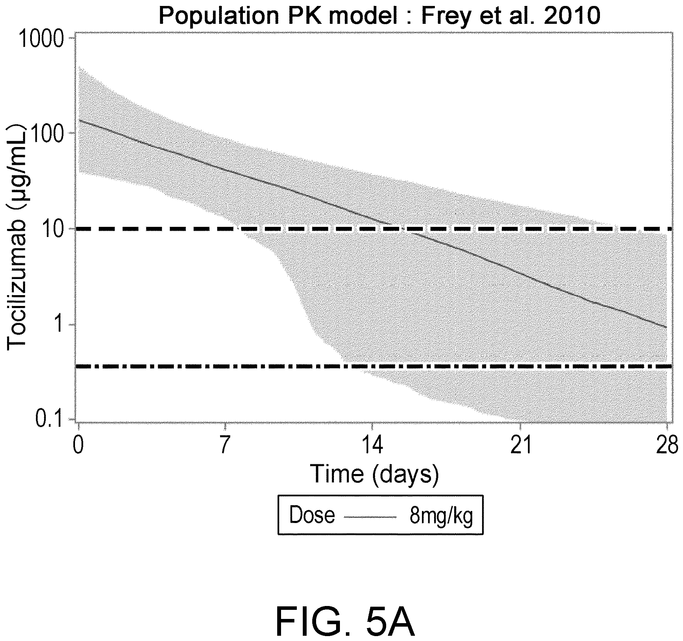

[Fig. 5A] Fig. 5A is a simulation of changes in serum Tocilizumab concentration when Tocilizumab is administered at 8 mg/kg (intravenous administration: IV) on Day 0, based on the population pharmacokinetic model reported in the article by Frey et al. The vertical axis represents the serum Tocilizumab concentration ($\mu$g/mL), and the horizontal axis represents the number of days after administration of Tocilizumab. The dashed line shows the target concentration of Tocilizumab (10 $\mu$g/mL), and the alternate long and short dash line depicts, as a reference, the Michaelis coefficient (Km value) (0.367 $\mu$g/mL) in the article by Gibiansky et al.

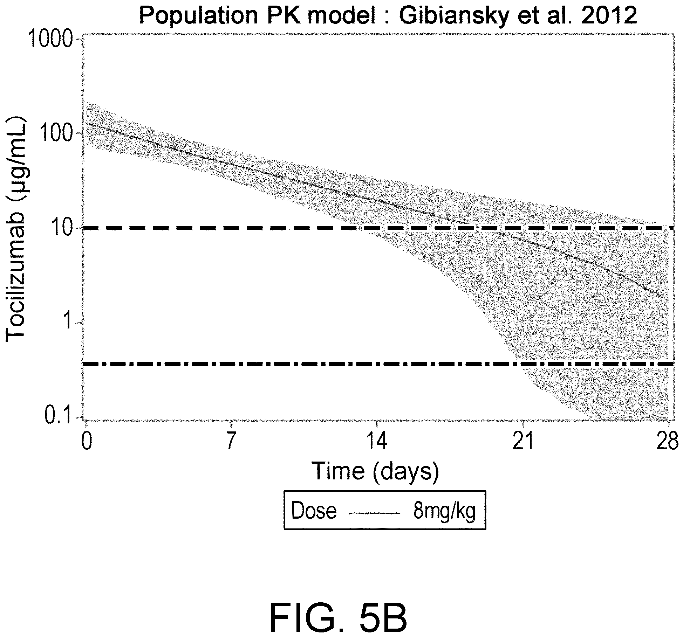

[Fig. 5B] Fig. 5B is a simulation of changes in serum Tocilizumab concentration when Tocilizumab is administered at 8 mg/kg (IV) on Day 0, based on the population pharmacokinetic model reported in the article by Gibiansky et al. The vertical axis represents the serum Tocilizumab concentration ($\mu$g/mL), and the horizontal axis represents the number of days after administration of Tocilizumab. The dashed line shows the target concentration of Tocilizumab (10 $\mu$g/mL), and the alternate long and short dash line depicts, as a reference, the Michaelis coefficient (Km value) (0.367 $\mu$g/mL) in the article by Gibiansky et al.

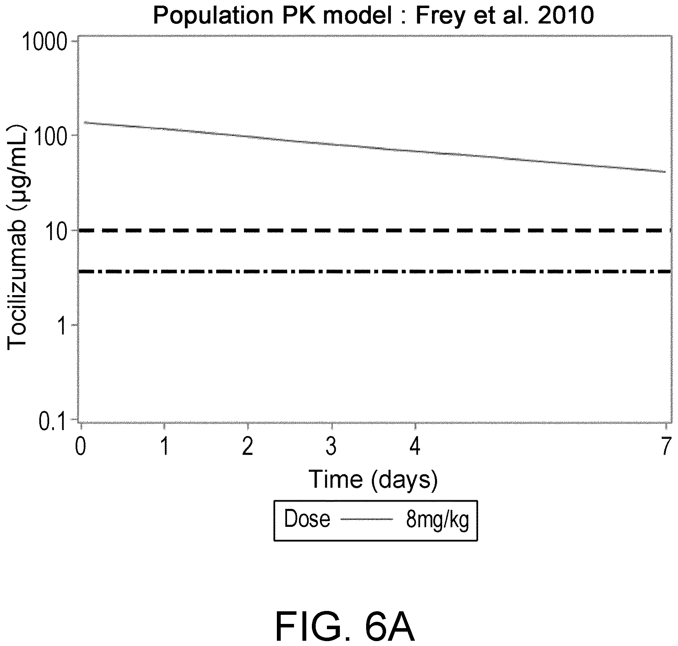

[Fig. 6A] Fig. 6A is a simulation of changes in serum Tocilizumab concentration when Tocilizumab is intravenously administered (IV) at 8 mg/kg on Day 0, based on the population pharmacokinetic model reported in the article by Frey et al. The vertical axis represents the serum Tocilizumab concentration ($\mu$g/mL), and the horizontal axis represents the number of days after administration of Tocilizumab.

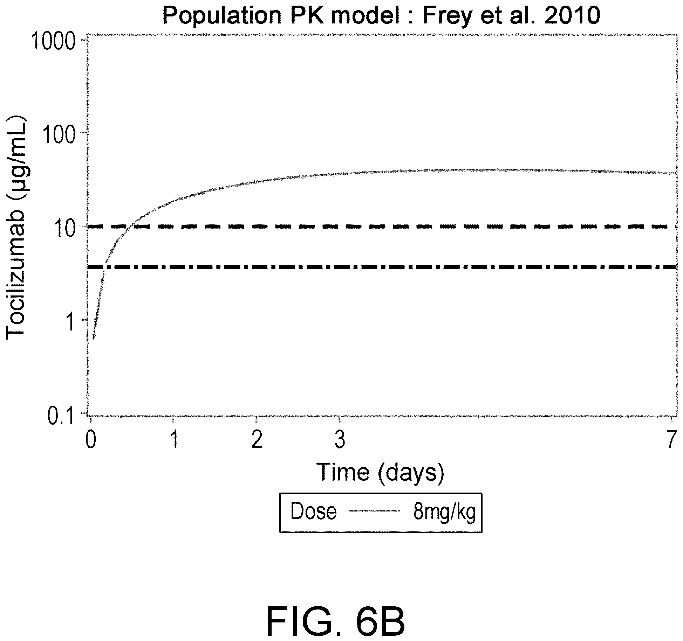

[Fig. 6B] Fig. 6B is a simulation of changes in serum Tocilizumab concentration when Tocilizumab is subcutaneously administered (SC) at 8 mg/kg on Day 0, based on the population pharmacokinetic model reported in the article by Frey et al. The vertical axis represents the serum Tocilizumab concentration ($\mu$g/mL), and the horizontal axis represents the number of days after administration of Tocilizumab. The simulation used as F (bioavailability) and Ka (absorption rate constant), values reported in Abdallah, Hisham et al. "Pharmacokinetic and Pharmacodynamic Analysis of Subcutaneous Tocilizumab in Patients with Rheumatoid Arthritis From 2 Randomized, Controlled Trials: SUMMACTA and BREVACTA." Journal of clinical pharmacology vol. 57,4 (2017): 459-468.

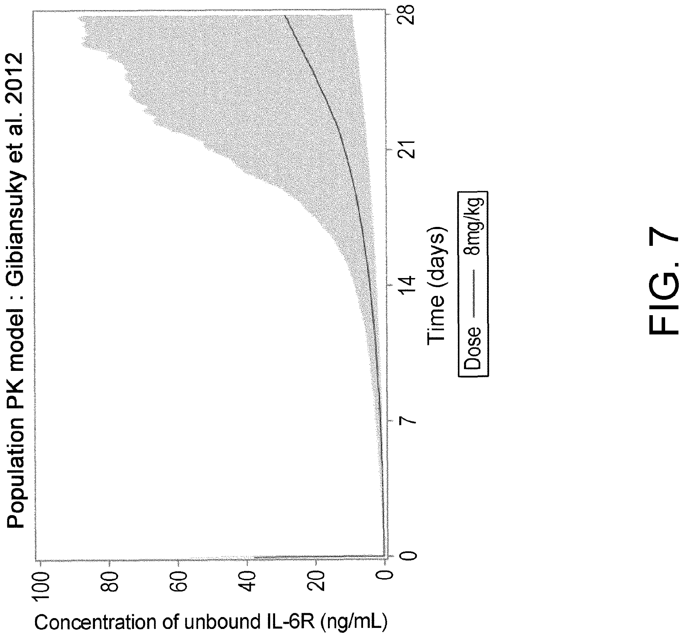

[Fig. 7] Fig. 7 is a simulation of changes in the concentration of unbound IL-6R (IL-6R not complexed with Tocilizumab) in serum when Tocilizumab is administered at 8 mg/kg on Day 0, based on the population pharmacokinetic model reported in the article by Gibiansky et al. The vertical axis represents the unbound IL-6R concentration in serum ($\mu$g/mL), and the horizontal axis represents the number of days after administration of Tocilizumab.

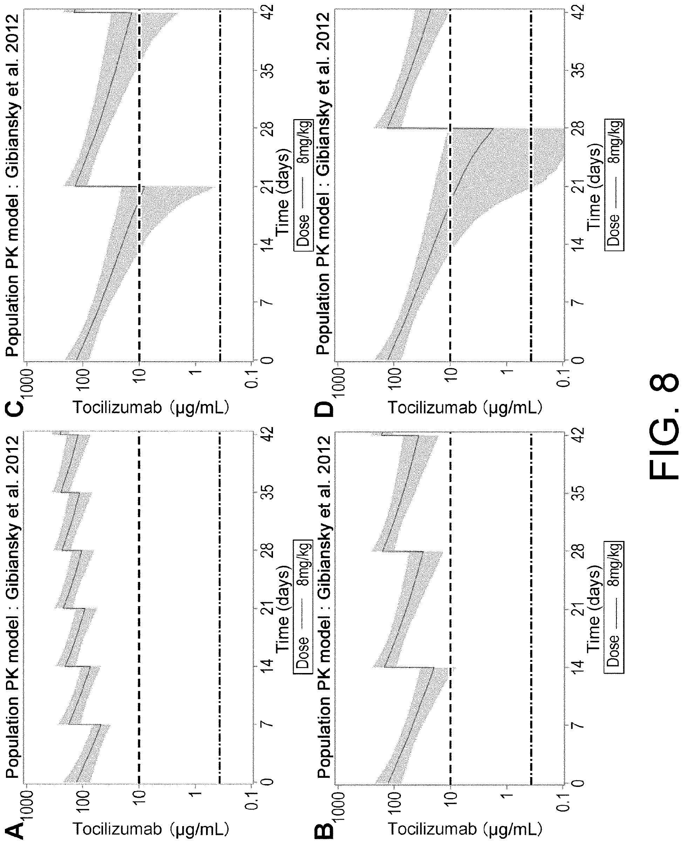

[Fig. 8] Fig. 8 is a simulation of changes in serum Tocilizumab concentration when Tocilizumab is administered (IV) at 8 mg/kg per dose from Day 0, at intervals of once every week (QW), once every 2 weeks (Q2W), once every 3 weeks (Q3W), or 4 times every 4 weeks (Q4W) based on the population pharmacokinetic model reported in the article by Gibiansky et al. Figs. 8A, 8B, 8C, and 8D show the simulation results of QW, Q2W, Q3W, and Q4W, respectively. The vertical axis represents the serum Tocilizumab concentration ($\mu$g/mL), and the horizontal axis represents the number of days after administration of Tocilizumab. The dashed line shows the target concentration of Tocilizumab (10 $\mu$g/mL), and the alternate long and short dash line depicts, as a reference, the Michaelis coefficient (Km value) (0.367 $\mu$g/mL) in the article by Gibiansky et al.

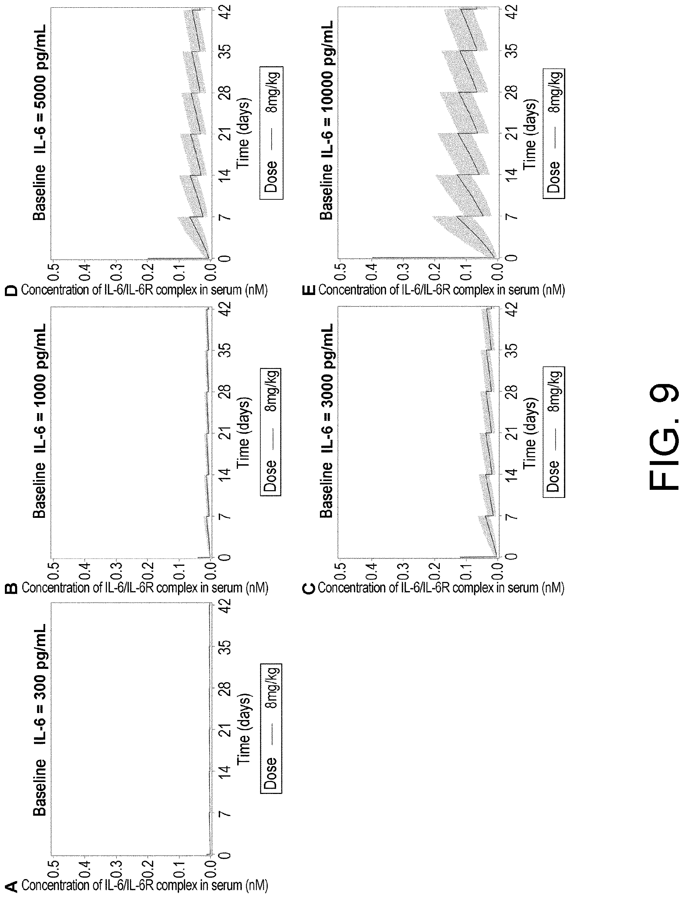

[Fig. 9] Fig. 9 is a simulation of changes in the concentration of the IL-6/IL-6R complex in serum when Tocilizumab is administered (IV) at 8 mg/kg per dose from Day 0, at intervals of once every week (QW), based on the population pharmacokinetic model reported in the article by Gibiansky et al.

Figs. 9A, 9B, 9C, 9D, and 9E show the simulation results when the serum IL-6 concentrations are 300 pg/mL, 1000 pg/mL, 3000 pg/mL, 5000 pg/mL, and 10000 pg/mL, respectively. The vertical axis represents the concentration of the IL-6/IL-6R complex (nM), and the horizontal axis represents the number of days after starting the administration of Tocilizumab.

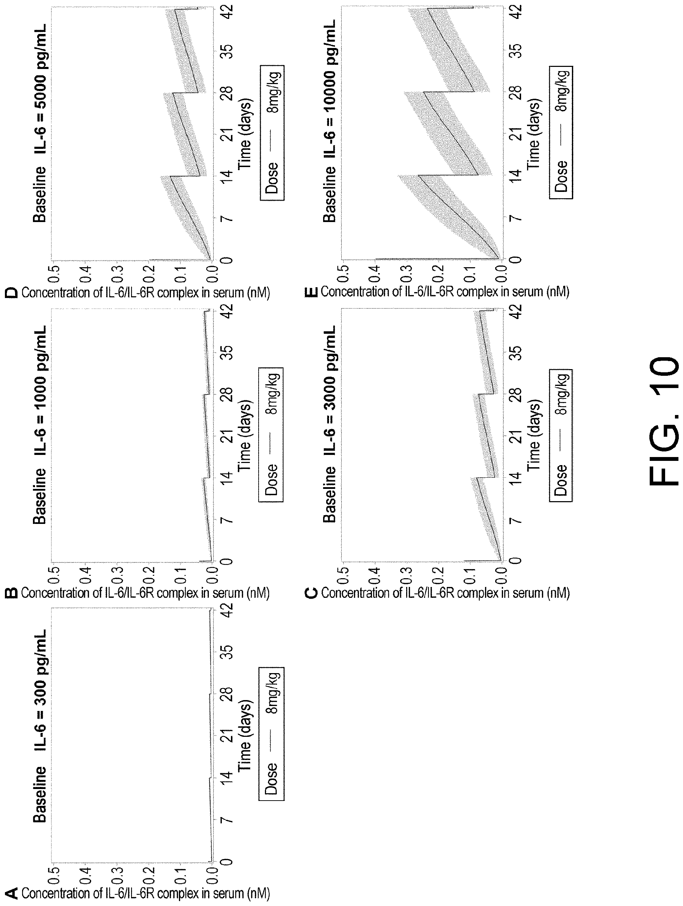

[Fig. 10] Fig. 10 is a simulation of changes in the concentration of the IL-6/IL-6R complex in serum when Tocilizumab is administered (IV) at 8 mg/kg per dose from Day 0, at intervals of once every 2 weeks (Q2W), based on the population pharmacokinetic model reported in the article by Gibiansky et al.

Figs. 10A, 10B, 10C, 10D, and 10E show the simulation results when the serum IL-6 concentrations are 300 pg/mL, 1000 pg/mL, 3000 pg/mL, 5000 pg/mL, and 10000 pg/mL, respectively.

The vertical axis represents the concentration of the IL-6/IL-6R complex (nM), and the horizontal axis represents the number of days after starting the administration of Tocilizumab.

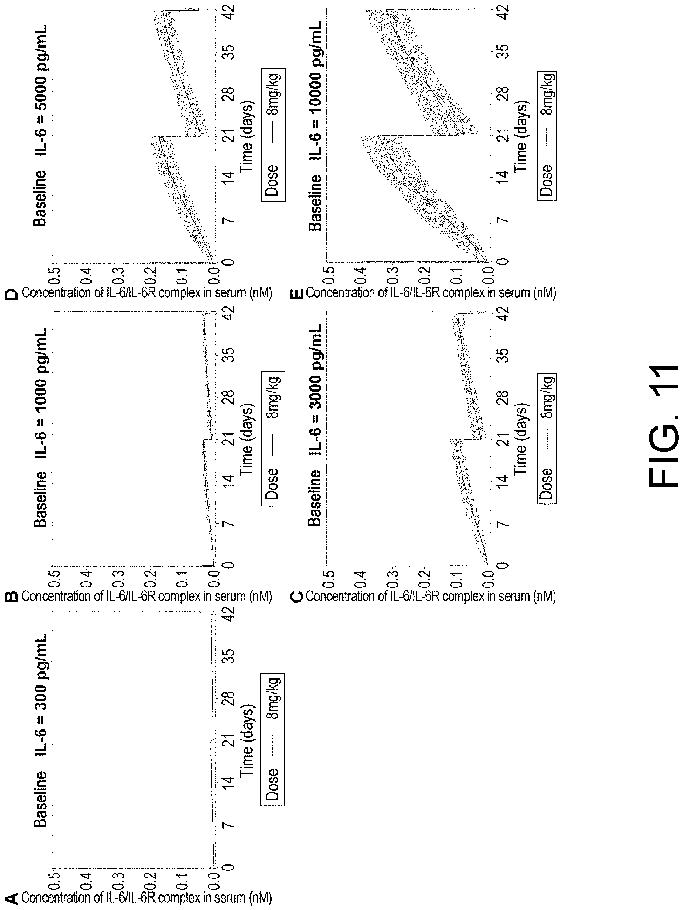

[Fig. 11] Fig. 11 is a simulation of changes in the concentration of the IL-6/IL-6R complex in serum when Tocilizumab is administered (IV) at 8 mg/kg per dose from Day 0, at intervals of once every 3 weeks (Q3W), based on the population pharmacokinetic model reported in the article by Gibiansky et al.

Figs. 11A, 11B, 11C, 11D, and 11E show the simulation results when the serum IL-6 concentrations are 300 pg/mL, 1000 pg/mL, 3000 pg/mL, 5000 pg/mL, and 10000 pg/mL, respectively.

The vertical axis represents the concentration of the IL-6/IL-6R complex (nM), and the horizontal axis represents the number of days after starting the administration of Tocilizumab.

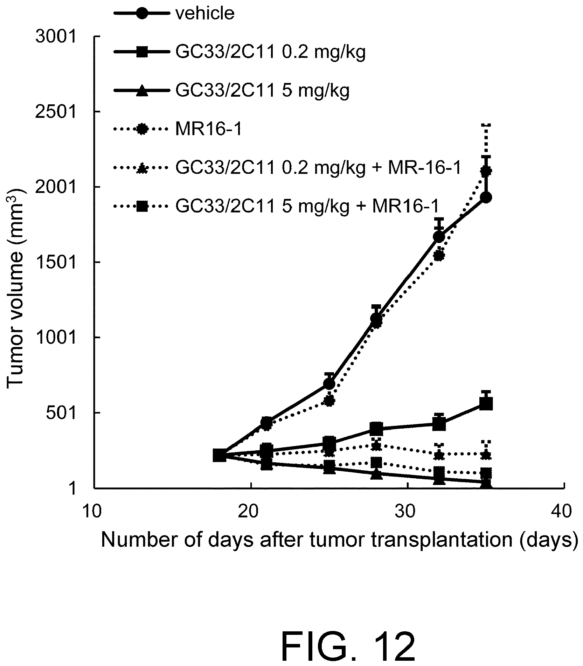

[Fig. 12] Fig. 12 shows the antitumor effects in a syngeneic mouse model (i) when the ERY974 surrogate antibody alone was administered, (ii) when the anti-mouse IL6 receptor antibody (MR-16-1) was administered, and (iii) when the ERY974 surrogate antibody was administered after pre-administration of the anti-mouse IL6 receptor antibody (MR-16-1).

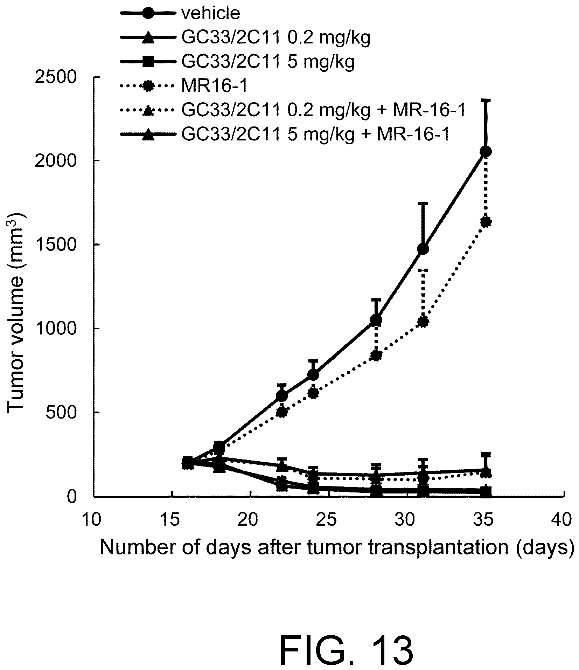

[Fig. 13] Fig. 13 shows the antitumor effects in a syngeneic mouse model (i) when the ERY974 surrogate antibody alone was administered, (ii) when the anti-mouse IL6 receptor antibody (MR-16-1) was administered, and (iii) when the anti-mouse IL6 receptor antibody (MR-16-1) was administered after administration of the ERY974 surrogate antibody.

[Description of Embodiments]

I. Definition

The definitions below are provided to help understanding of the present invention illustrated herein.

The term "specific" means that one of molecules involved in specific binding does not show any significant binding to molecules other than a single or a number of binding partner molecules. Furthermore, the term is also used when a domain containing an antibody variable region is specific to a particular epitope among multiple epitopes in an antigen. When an epitope bound by a domain containing an antibody variable region is included in a number of different antigens, antigen-binding molecules comprising the antibody variable region-containing domain can bind to various antigens that have the epitope.

The term "binding activity" refers to the strength of the sum total of noncovalent interactions between one or more binding sites of a molecule (e.g., an antibody) and its binding partner (e.g., an antigen). Herein, "binding activity" is not strictly limited to a 1:1 interaction between members of a binding pair (e.g., antibody and antigen). For example, when the members of a binding pair reflect a monovalent 1:1 interaction, the binding activity is particularly called the intrinsic binding affinity (affinity). When a member of a binding pair is capable of both monovalent binding and multivalent binding, the binding activity is the sum of each binding strength. The binding activity of a molecule X for its partner Y can generally be represented by the dissociation constant (KD) or "binding amount of analyte per unit amount of ligand" (hereinbelow, may be referred to as "binding amount"). Those skilled in the art would understand that, generally, lower value of dissociation constant (KD) means higher binding activity; and higher value of "binding amount of analyte per unit amount of ligand" or "binding amount" means higher binding activity. Binding activity can be measured by common methods known in the art, including those described herein. Specific illustrative and exemplary embodiments for measuring binding activity (including affinity) are described in the following. Specific illustrative and exemplary embodiments for measuring binding affinity are described in the following.

The terms "anti-'target antigen'-binding antigen-binding molecule" (herein 'target antigen' is any antigen protein that can be targeted, and examples include a T cell antigen, cancer antigen, cytokine, and cytokine receptor) and "an antigen-binding molecule that binds to a target antigen" refers to an antigen-binding molecule that is capable of binding a target antigen with sufficient affinity such that the antigen-binding molecule is useful as a diagnostic and/or therapeutic agent when the antigen-binding molecule targets the antigen. In one embodiment, the extent of binding of an antigen-binding molecule to an unrelated, non-target antigen protein is less than about 10% of the binding of the antigen-binding molecule to the target antigen as measured, e.g., by a radioimmunoassay (RIA). In certain embodiments, an antigen-binding molecule that binds to the target protein has a dissociation constant (Kd) of 1 micro M or less, 100 nM or less, 10 nM or less, 1 nM or less, 0.1 nM or less, 0.01 nM or less, or 0.001 nM or less (e.g. $10^{-8}$ M or less, e.g. from $10^{-8}$ M

to $10^{-13}$ M, e.g., from $10^{-9}$ M to $10^{-13}$ M). In certain embodiments, an antigen-binding molecule binds to an epitope of the target antigen that is conserved among the target antigen from different species.

An antigen-binding molecule "that binds to the same epitope" as a reference antigen-binding molecule refers to an antigen-binding molecule that blocks binding of the reference antigen-binding molecule to its antigen in a competition assay by 50% or more, and conversely, the reference antigen-binding molecule blocks binding of the antigen-binding molecule to its antigen in a competition assay by 50% or more. An exemplary competition assay is provided herein.

In an exemplary competition assay, an immobilized antigen (for example, GPC3, CD3, and/or IL-6 receptor) is incubated in a solution comprising a first labeled antigen-binding molecule that binds to the antigen and a second unlabeled antigen-binding molecule that is being tested for its ability to compete with the first antigen-binding molecule for binding to the antigen. The second antigen-binding molecule may be present in a hybridoma supernatant. As a control, the immobilized antigen is incubated in a solution comprising the first labeled antigen-binding molecule but not the second unlabeled antigen-binding molecule. After incubation under conditions permissive for binding of the first antigen-binding molecule to the antigen, excess unbound antigen-binding molecule is removed, and the amount of label associated with the immobilized antigen is measured. If the amount of label associated with the immobilized antigen is substantially reduced in the test sample relative to the control sample, then that indicates that the second antigen-binding molecule is competing with the first antigen-binding molecule for binding to the antigen. See Harlow and Lane (1988) Antibodies: A Laboratory Manual ch.14 (Cold Spring Harbor Laboratory, Cold Spring Harbor, NY).

The term "antigen-binding molecule" herein is used in the broadest sense and encompasses various antibody structures, including but not limited to monoclonal antibodies, polyclonal antibodies, multispecific antibodies (e.g., bispecific antibodies), antibody derivatives, and antibody fragments so long as they exhibit the desired antigen-binding activity.

In certain embodiments, an antibody provided herein may be further modified to contain additional nonproteinaceous moieties that are known in the art and readily available (this is called "antibody derivatives"). The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called alpha, delta, epsilon, gamma, and mu, respectively.

Constant regions of the isotypes IgG1, IgG2, IgG3, and IgG4 are called Cγ1, Cy2, Cy3, and Cy4, respectively. The amino acid sequences of Fc domain polypeptides forming human Cγ1, Cy2, Cy3, and Cy4 are exemplified in SEQ ID NO: 23, 24, 25, and 26, respectively. The relationship between amino acid residues forming each amino acid sequence and Kabat's EU numbering (herein also referred to as EU INDEX) are shown in Fig. 1.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The term "Fc receptor" or "FcR" refers to a receptor that binds to the Fc region of an antibody. In some embodiments, an FcR is a native human FcR. In some embodiments, an FcR is one which binds an IgG antibody (a gamma receptor) and includes receptors of the Fc gamma RI, Fc gamma RII, and Fc gamma RIII subclasses, including allelic variants and alternatively spliced forms of those receptors. Fc gamma RII receptors include Fc gamma RIIA (an "activating receptor") and Fc gamma RIIB (an "inhibiting receptor"), which have similar amino acid sequences that differ primarily in the cytoplasmic domains thereof. Activating receptor Fc gamma RIIA contains an immunoreceptor tyrosine-based activation motif (ITAM) in its cytoplasmic domain. Inhibiting receptor Fc gamma RIIB contains an immunoreceptor tyrosine-based inhibition motif (ITIM) in its cytoplasmic domain. (see, *e.g.,* Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, e.g., Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton et al.).

Binding to human FcRn *in vivo* and plasma half life of human FcRn high affinity binding polypeptides can be assayed, e.g., in transgenic mice or transfected human cell lines expressing human FcRn, or in primates to which the polypeptides with a variant Fc region are administered. WO 2000/42072 (Presta) describes antibody variants with increased or decreased binding to FcRs. See also, *e.g.,* Shields et al. J. Biol. Chem. 9(2):6591-6604 (2001).

The term "Fc region-comprising antibody" refers to an antibody that comprises an Fc region. The C-terminal lysine (residue 447 according to the EU numbering system) or C-terminal glycine-lysine (residues 446-447) of the Fc region may be removed, for example, during purification of the antibody or by recombinant engineering of the nucleic acid encoding the antibody. Accordingly, a composition comprising an antibody having an Fc region according to this invention can comprise an antibody with G446-K447, with G446 and without K447, with all G446-K447 removed, or a mixture of three types of antibodies described above.

Fc regions with reduced Fc receptor-binding activity include Fc regions with reduced binding activity to any of Fcγ receptors FcγI, FcγIIA, FcγIIB, FcγIIIA, and/or FcγIIIB. The reduced binding activity to any of the Fcγ receptors FcγI, FcγIIA, FcγIIB, FcγIIIA, and/or FcγIIIB can be assessed by using FACS and ELISA formats known to those skilled in the art as well as ALPHA screen (Amplified Luminescent Proximity Homogeneous Assay) and surface plasmon resonance (SPR)-based BIACORE method (Proc. Natl. Acad. Sci. USA (2006) 103(11), 4005-4010).

Antigen-binding molecules or antibodies comprising an Fc region with reduced Fc receptor-binding activity include antigen-binding molecules or antibodies with reduced effector function. Antigen-binding molecules or antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with increased or decreased binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either increased or decreased) C1q binding and/or Complement Dependent Cytotoxicity (CDC), e.g., as described in US Patent No. 6,194,551, WO 99/51642, and Idusogie et al. J. Immunol. 164: 4178-4184 (2000).

In some embodiments, Fc regions with reduced Fc receptor-binding activity include Fc mutants of the Fc regions illustrated herein.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region," "HVR," or "CDR" as used herein refers to each of the regions of an antibody variable domain which are hypervariable in sequence ("complementarity determining regions" or "complementarity determining regions") and/or form structurally defined loops ("hypervariable loops") and/or contain the antigen-contacting residues ("antigen contacts"). Generally, antibodies comprise six CDRs: three in the VH (H1, H2, H3), and three in the VL (L1, L2, L3). Exemplary CDRs herein include:

(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including amino acid residues 46-56 (L2), 47-56 (L2), 48-56 (L2), 49-56 (L2), 26-35 (H1), 26-35b (H1), 49-65 (H2), 93-102 (H3), and 94-102 (H3). Unless otherwise indicated, CDR residues and other residues in the variable domain (e.g., FR residues) are numbered herein according to Kabat et al., *supra.*

An "individual," "subject," or "patient" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" antigen-binding molecule is one which has been separated from a component of its natural environment. In some embodiments, an antigen-binding molecule is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an antigen-binding molecule" refers to one or more nucleic acid molecules encoding one or two or more polypeptide chains or its fragments of antigen-binding molecule (in case of antibody, antibody heavy and light chains or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies composing the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared.

The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

In certain embodiments, an antigen-binding molecule (or antibody) provided herein is a multispecific antibody, e.g. a bispecific antibody. Multispecific antibodies are monoclonal antibodies that have binding specificities for at least two different sites. Multispecific antibodies (e.g., bispecific antibodies) can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBOJ. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); crosslinking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see, e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antigen-binding molecule or fragment herein also includes a "Dual Acting Fab" or "DAF" comprising an antigen binding site that binds to a specific antigen as well as another, different antigen (see, US 2008/0069820, for example).

II. Anti-T cell antigen-binding molecules and pharmaceutical compositions

In one embodiment, "anti-T cell antigen-binding molecules" refer to antigen-binding molecules that bind to antigens on T cells, and include antigen-binding molecules that bind to T cell receptor complexes. In one embodiment, anti-T cell antigen-binding molecules are multispecific antigen-binding molecules. In one embodiment, anti-T cell antigen-binding molecules are bispecific antigen-binding molecules, preferably bispecific antibodies comprising "a domain comprising an antibody variable region having T cell receptor complex-binding activity" and "a domain comprising an antibody variable region having cancer antigen-binding activity". In one embodiment, the bispecific antibodies may have a structure of a single chain antibody, such as a structure in which antibody variable regions are linked by linkers. In one embodiment, anti-T cell antigen-binding molecules further comprise an Fc region with reduced Fcγ receptor-binding activity.

Furthermore, in one embodiment, a domain comprising an antibody variable region having T cell receptor complex-binding activity is preferably a domain comprising an antibody variable region having T cell receptor-binding activity, and more preferably a domain comprising an antibody variable region having CD3-binding activity. Furthermore, in one embodiment, the above "domain comprising an antibody variable region" is provided by one or more variable domains of an antibody, and preferably the domain comprising an antibody variable region comprises an antibody light chain variable region (VL) and an antibody heavy chain variable region (VH). Suitable examples of such domains comprising antibody variable regions include various antibody fragments such as "scFv (single chain Fv)", "single chain antibody", "Fv", "single chain Fv 2 (scFv2)", "Fab", and "F(ab')2".

Domain comprising an antibody variable region having cancer antigen-binding activity

Herein, the "domain comprising an antibody variable region having cancer antigen-binding activity" refers to a portion of an antibody comprising a region that specifically binds to and is complementary to all or a part of a cancer antigen.

Herein, a "cancer-specific antigen" refers to an antigen expressed by cancer cells, which enables one to distinguish between cancer cells and healthy cells; and for example, it includes antigens that are expressed as cells become malignant, or abnormal sugar chains that appear on protein molecules or cell surface when cells become cancerous. Specific examples include GPC3; ALK receptor (pleiotrophin receptor); pleiotrophin; KS 1/4 pancreas carcinoma antigen; ovarian carcinoma antigen (CA125); prostatic acid phosphate; prostate-specific antigen (PSA); melanoma-associated antigen p97; melanoma antigen gp75; high molecular weight melanoma antigen (HMW-MAA); prostate-specific membrane antigen; carcinoembryonic antigen (CEA); polymorphic epithelial mucin antigen; human milk fat globule antigen; colorectal tumor-associated antigens such as CEA, TAG-72, CO17-1A, GICA 19-9, CTA-1, and LEA; Burkitt's lymphoma antigen-38.13; CD19; human B-lymphoma antigen-CD20; CD33; melanoma-specific antigens such as ganglioside GD2, ganglioside GD3, ganglioside GM2, and ganglioside GM3; tumor-specific transplantation type cell-surface antigen (TSTA); virus-induced tumor antigens including T antigen and envelope antigens of DNA tumor viruses and RNA tumor viruses; CEA of colon; oncofetal antigens such as5T4 oncofetal trophoblast glycoprotein and bladder tumor oncofetal antigen; α-fetoprotein; differentiation antigens such as human lung carcinoma antigens L6 and L20; antigens of fibrosarcoma; human leukemia T cell antigen-Gp37; neoglycoprotein; sphingolipids; breast cancer antigens such as EGFR (epidermal growth factor receptor); NY-BR-16; NY-BR-16 and HER2 antigen (p185HER2); polymorphic epithelial mucin (PEM); malignant human lymphocyte antigen-APO-1; differentiation antigens such as I antigen found in fetal erythrocytes; primary endoderm I antigen found in adult erythrocytes; preimplantation embryos; I(Ma) found in gastric cancer; M18 and M39 found in mammary epithelium; SSEA-1, VEP8, VEP9, Myl, and VIM-D5 found in myeloid cells; D156-22 found in colorectal cancer; TRA-1-85 (blood group H); SCP-1 found in testis and ovarian cancer; C14 found in colon cancer; F3 found in lung cancer; AH6 found in gastric cancer; Y hapten; Ley found in embryonal carcinoma cells; TL5 (blood group A); EGF receptor found in A431 cells; E1 series (blood group B) found in pancreatic cancer; FC10.2 found in embryonal carcinoma cells; gastric cancer antigen; CO-514 (blood group Lea) found in adenocarcinomas; NS-10 found in adenocarcinomas; CO-43 (blood group Leb); G49 found in EGF receptor of A431 cells; MH2 (blood group ALeb/Ley) found in colon cancer; 19.9 found in colon cancer; gastric cancer mucins; T5A7 found in myeloid cells; R24 found in melanoma; 4.2, GD3, D1.1, OFA-1, GM2, OFA-2, GD2, and M1:22:25:8 found in embryonal carcinoma cells as well as SSEA-3 and SSEA-4 found in 4 to 8-cell stage embryos; subcutaneous T cell lymphoma antigen; MART-1 antigen; sialyl Tn (STn) antigen; colon cancer antigen NY-CO-45; lung cancer antigen NY-LU-12 variant A; adenocarcinoma antigen ART1; paraneoplastic associated brain-testis-cancer antigen (onconeuronal antigen MA2; paraneoplastic neuronal antigen); Neuro-oncological ventral antigen 2 (NOVA2); hemocyte carcinoma antigen gene 520; tumor-associated antigen CO-029; tumor-associated antigens MAGE-C1 (cancer/testis antigen CT7), MAGE-B1 (MAGE-XP antigen), MAGE-B2 (DAM6), MAGE-2, MAGE-4a, MAGE-4b and MAGE-X2; Cancer-Testis Antigen (NY-EOS-1); YKL-40, fragments of any of the aforementioned polypeptides, or structures produced by modification thereof (for example, the above-mentioned modified phosphate group or sugar chain); EpCAM; EREG; CA19-9; CA15-3; sialyl SSEA-1(SLX); HER2; PSMA; CEA; and CLEC12A. Cancer-specific antigens which become targets of the cancer-specific antigen-binding domains of the present invention are, in particular, preferably those expressed on cell surface, and examples of such cancer-specific antigens include CD19, CD20, EGFR, HER2, EpCAM, and EREG.

A domain comprising an antibody variable region having glypican 3 (GPC3)-binding activity

Herein, the phrase "a domain comprising an antibody variable region having glypican 3 (GPC3)-binding activity" refers to an antibody portion that comprises a region that specifically binds to the above-mentioned GPC3 protein, or to all or a portion of a partial peptide of the GPC3 protein, and is also complementary thereto.

Herein, glypican 3 (GPC3) is a protein that belongs to the glypican family, i.e., a group of heparan sulfate proteoglycans bound to cell surface *via* glycosylphosphatidylinositol (Filmus, J. Clin. Invest., 2001, 108, 497-501). Glypicans play an important role in cell proliferation, differentiation, and migration. GPC3 is expressed in 70% or more of hepatoma tissues obtained by surgical excision or biopsy, and is hardly or not at all expressed in neighboring nonneoplastic hepatic lesions and most adult tissues (Zhu-Zu-W, Gut, 2001, 48, 558-564 Yamauchi, Mod. Pathol., 2005, 18, 1591-1598).

A domain comprising an antibody variable region having T-cell receptor complex-binding activity

Herein, the phrase "a domain comprising an antibody variable region having T-cell receptor complex-binding activity" refers to a T-cell receptor complex-binding antibody portion that comprises a region that specifically binds to all or a portion of a T-cell receptor complex and is also complementary thereto. The T-cell receptor complex may be a T-cell receptor itself, or an adaptor molecule constituting a T-cell receptor complex along with a T-cell receptor. CD3 is suitable as an adaptor molecule.

A domain comprising an antibody variable region that has T-cell receptor-binding activity

Herein, the phrase "a domain comprising an antibody variable region having T-cell receptor-binding activity" refers to a T-cell receptor-binding antibody portion produced by including a region that specifically binds to all or a portion of a T-cell receptor and is also complementary thereto.

The portion of a T cell receptor to which the domain of the present invention binds may be a variable region or a constant region, but an epitope present in the constant region is preferred. Examples of the constant region sequence include the T cell receptor α chain of RefSeq Accession No. CAA26636.1 (SEQ ID NO: 9), the T cell receptor β chain of RefSeq Accession No. C25777 (SEQ ID NO: 10), the T cell receptor γ1 chain of RefSeq Accession No. A26659 (SEQ ID NO: 11), the T cell receptor γ2 chain of RefSeq Accession No. AAB63312.1 (SEQ ID NO: 12), and the T cell receptor δ chain of RefSeq Accession No. AAA61033.1 (SEQ ID NO: 13).

A domain comprising an antibody variable region that has CD3-binding activity

Herein, the phrase "a domain comprising an antibody variable region that has CD3-binding activity" refers to a CD3-binding antibody portion produced by including a region that specifically binds to all or a portion of CD3 and is also complementary thereto.

The domain comprising an antibody variable region that has CD3-binding activity of the present invention may be any epitope-binding domain as long as the epitope exists in the γ-chain, δ-chain, or ε-chain sequence that constitutes human CD3. In the present invention, preferably, a domain comprising an anti-CD3 antibody light-chain variable region (VL) and an anti-CD3 antibody heavy-chain variable region (VH) that bind to an epitope present in the extracellular region of the ε chain of the human CD3 complex is suitably used. Besides the anti-CD3 antibody light chain variable region (VL) and anti-CD3 antibody heavy chain variable region (VH) described in the Examples, various known CD3-binding domains containing a CD3-binding antibody light chain variable region (VL) and a CD3-binding antibody heavy chain variable region (VH), and those of the OKT3 antibody (Proc. Natl. Acad. Sci. USA (1980) 77, 4914-4917) are suitably used as such domains. One may appropriately use an antibody variable region-containing domain derived from the anti-CD3 antibody having desired properties, which is obtained by immunizing a desired animal by the above-mentioned method using the γ-chain, δ-chain, or ε-chain constituting the human CD3. Human antibodies and properly humanized antibodies as described above may be appropriately used as the anti-CD3 antibody to give rise to the domain containing the antibody variable region having CD3-binding activity. Regarding the structure of the γ-chain, δ-chain, or ε-chain constituting CD3, their polynucleotide sequences are shown in SEQ ID NOs: 9 (NM_000073.2), 10 (NM_000732.4), and 11 (NM_000733.3), and their polypeptide sequences are shown in SEQ ID NOs: 12 (NP_000064.1), 13 (NP_000723.1), and 14 (NP_000724.1) (the RefSeq accession number is shown in parentheses).

Antibody variable region-containing domains in antigen binding molecules of the present invention may bind to the same epitope. Herein, the same epitope may be present in a protein comprising the amino acid sequence of SEQ ID NO: 2 or 14. Alternatively, antibody variable region-containing domains in antigen binding molecules of the present invention may bind to different epitopes, respectively. Herein, the different epitopes may be present in a protein comprising the amino acid sequence of SEQ ID NO: 2 or 14.

Fc region with reduced Fcγ receptor-binding activity

In one embodiment, "Fcγ receptor-binding activity is reduced" means, for example, that the binding activity of a test antigen-binding molecule is 50% or less, preferably 45% or less, 40% or less, 35% or less, 30% or less, 20% or less, or 15% or less, and particularly preferably 10% or less, 9% or less, 8% or less, 7% or less, 6% or less, 5% or less, 4% or less, 3% or less, 2% or less, or 1% or less than the binding activity of a control antigen-binding molecule.

Antigen-binding molecules comprising the Fc domain of a monoclonal IgG1, IgG2, IgG3, or IgG4 antibody can be appropriately used as control antigen-binding molecules. Furthermore, when an antigen-binding molecule comprising an Fc domain mutant of an antibody of a particular isotype is used as a test substance, the effect of the mutation of the mutant on the Fcγ receptor-binding activity is assessed using as a control an antigen-binding molecule comprising an Fc domain of the same isotype. As described above, antigen-binding molecules comprising an Fc domain mutant whose Fcγ receptor-binding activity has been judged to be reduced are appropriately prepared.

Such known mutants include, for example, mutants having a deletion of amino acids 231A-238S (EU numbering) (WO 2009/011941), as well as mutants C226S, C229S, P238S, (C220S) (J. Rheumatol (2007) 34, 11); C226S and C229S (Hum. Antibod. Hybridomas (1990) 1(1), 47-54); C226S, C229S, E233P, L234V, and L235A (Blood (2007) 109, 1185-1192).

Specifically, the preferred antigen-binding molecules include those comprising an Fc domain with a substitution of the amino acid at position 220, 226, 229, 231, 232, 233, 234, 235, 236, 237, 238, 239, 240, 264, 265, 266, 267, 269, 270, 295, 296, 297, 298, 299, 300, 325, 327, 328, 329, 330, 331, or 332 (EU numbering) in the amino acids forming the Fc domain of an antibody of a particular isotype. The isotype of antibody from which the Fc domain originates is not particularly limited, and it is possible to use an appropriate Fc domain derived from a monoclonal IgG1, IgG2, IgG3, or IgG4 antibody. It is preferable to use Fc domains derived from IgG1 antibodies.

The preferred antigen-binding molecules include, for example, those comprising an Fc domain which has any one of the substitutions shown below, whose positions are specified according to EU numbering (each number represents the position of an amino acid residue in the EU numbering; and the one-letter amino acid symbol before the number represents the amino acid residue before substitution, while the one-letter amino acid symbol after the number represents the amino acid residue before the substitution) in the amino acids forming the Fc domain of IgG1 antibody:

(a) L234F, L235E, P331S;
(b) C226S, C229S, P238S;
(c) C226S, C229S;
(d) C226S, C229S, E233P, L234V, L235A;
(e) L234A, L235A or L235R, N297A;
(f) L235A or L235R, S239K, N297A

as well as those having an Fc domain which has a deletion of the amino acid sequence at positions 231 to 238.

Furthermore, the preferred antigen-binding molecules also include those comprising an Fc domain that has any one of the substitutions shown below, whose positions are specified according to EU numbering in the amino acids forming the Fc domain of an IgG2 antibody:

(g) H268Q, V309L, A330S, and P331S;
(h) V234A;
(i) G237A;
(j) V234A and G237A;
(k) A235E and G237A;
(l) V234A, A235E, and G237A. Each number represents the position of an amino acid residue in EU numbering; and the one-letter amino acid symbol before the number represents the amino acid residue before substitution, while the one-letter amino acid symbol after the number represents the amino acid residue before the substitution.

Furthermore, the preferred antigen-binding molecules also include those comprising an Fc domain that has any one of the substitutions shown below, whose positions are specified according to EU numbering in the amino acids forming the Fc domain of an IgG3 antibody:

(m) F241A;
(n) D265A;
(o) V264A. Each number represents the position of an amino acid residue in EU numbering; and the one-letter amino acid symbol before the number represents the amino acid residue before substitution, while the one-letter amino acid symbol after the number represents the amino acid residue before the substitution.

Furthermore, the preferred antigen-binding molecules also include those comprising an Fc domain that has any one of the substitutions shown below, whose positions are specified according to EU numbering in the amino acids forming the Fc domain of an IgG4 antibody:

(p) L235A, G237A, and E318A;
(q) L235E;
(r) F234A and L235A. Each number represents the position of an amino acid residue in EU numbering; and the one-letter amino acid symbol before the number represents the amino acid residue before substitution, while the one-letter amino acid symbol after the number represents the amino acid residue before the substitution.

The other preferred antigen-binding molecules include, for example, those comprising an Fc domain in which any amino acid at position 233, 234, 235, 236, 237, 327, 330, or 331 (EU numbering) in the amino acids forming the Fc domain of an IgG1 antibody is substituted with an amino acid of the corresponding position in EU numbering in the corresponding IgG2 or IgG4.

The preferred antigen-binding molecules also include, for example, those comprising an Fc domain in which any one or more of the amino acids at positions 234, 235, and 297 (EU numbering) in the amino acids forming the Fc domain of an IgG1 antibody is substituted with other amino acids. The type of amino acid after substitution is not particularly limited; however, the antigen-binding molecules comprising an Fc domain in which any one or more of the amino acids at positions 234, 235, and 297 are substituted with alanine are particularly preferred.

The preferred antigen-binding molecules also include, for example, those comprising an Fc domain in which an amino acid at position 265 (EU numbering) in the amino acids forming the Fc domain of an IgG1 antibody is substituted with another amino acid. The type of amino acid after substitution is not particularly limited; however, antigen-binding molecules comprising an Fc domain in which an amino acid at position 265 is substituted with alanine are particularly preferred.

Anti-glypican 3 (GPC3)/T-cell receptor complex bispecific antibody

In one embodiment, an anti-T cell antigen-binding molecule is a bispecific antibody comprising:

(1) a domain comprising an antibody variable region having glypican 3-binding activity;
(2) a domain comprising an antibody variable region having T-cell receptor complex-binding activity; and
(3) a domain comprising an Fc region with reduced Fcγ receptor-binding activity.

In one embodiment, for the antibody L-chain variable regions contained in the antibody variable region having glypican 3-binding activity and the antibody variable region having T-cell receptor complex-binding activity of the present invention, it is preferable to obtain a common L chain that may provide a binding ability to the H chain having glypican3-binding activity and to the H chain having T-cell receptor complex-binding activity, and to use this as the common L-chain variable region of the bispecific antigen-binding molecule.

Examples of a preferred antibody H-chain variable region of the present disclosure contained in the antibody variable region having glypican 3-binding activity comprises the antibody H-chain variable regions of Table 1, or antibody H-chain variable regions having CDR sequences whose CDR1, CDR2, and CDR3 amino acid sequences are the same as the CDR1, CDR2, and CDR3 amino acid sequences contained in the H-chain variable regions of Table 1, or antibody H-chain variable regions which are functionally equivalent to the above-mentioned variable regions.

[Table 1]

| Sequence Name | SEQ ID NO: |
|---|---|
| H0000 | 40 |
| GCH003 | 170 |
| GCH005 | 171 |
| GCH006 | 172 |
| GCH007 | 173 |
| GCH008 | 174 |
| GCH010 | 175 |
| GCH012 | 176 |
| GCH013 | 177 |
| GCH014 | 178 |
| GCH015 | 179 |
| GCH016 | 180 |
| GCH019 | 181 |
| GCH022 | 182 |
| GCH023 | 183 |
| GCH025 | 184 |
| GCH026 | 185 |
| GCH027 | 186 |
| GCH029 | 187 |
| GCH032 | 188 |
| GCH034 | 189 |
| GCH035 | 190 |
| GCH039 | 191 |
| GCH040 | 192 |
| GCH042 | 193 |

(continued)

| Sequence Name | SEQ ID NO: |
|---|---|
| GCH043 | 194 |
| GCH045 | 195 |
| GCH053 | 196 |
| GCH054 | 197 |
| GCH055 | 198 |
| GCH056 | 199 |
| GCH057 | 200 |
| GCH059 | 201 |
| GCH060 | 202 |
| GCH061 | 203 |
| GCH062 | 204 |
| GCH064 | 205 |
| GCH065 | 206 |
| GCH066 | 207 |
| GCH067 | 208 |
| GCH068 | 209 |
| GCH073 | 210 |
| GCH094 | 211 |
| GCH098 | 212 |
| GCH099 | 213 |
| GCH100 | 214 |
| H0610 | 215 |

Examples of a preferred antibody variable region having T-cell receptor complex-binding activity in the present disclosure include antibody variable regions having T-cell receptor-binding activity. Of the T-cell receptors, CD3 is preferred, and CD3ε is particularly preferred. Examples of an antibody H-chain variable region contained in such antibody variable regions include the antibody H-chain variable regions of Table 2, antibody H-chain variable regions having CDR sequences whose CDR1, CDR2, and CDR3 amino acid sequences are the same as the CDR1, CDR2, and CDR3 amino acid sequences contained in the antibody H-chain variable regions of Table 2, and antibody H-chain variable regions that are functionally equivalent to the above-mentioned variable regions.

[Table 2]

| Sequence Name | SEQ ID NO: |
|---|---|
| hCE115HA | 52 |
| CE115HA177 | 64 |
| CE115HA178 | 65 |
| CE115HA179 | 66 |
| CE115HA180 | 67 |
| hCE115HAa | 68 |
| TR01H006 | 69 |
| TR01H007 | 70 |

(continued)

| Sequence Name | SEQ ID NO: |
|---|---|
| TR01H008 | 71 |
| TR01H009 | 72 |
| TR01H010 | 73 |
| TR01H011 | 74 |
| TR01H012 | 75 |
| TR01H013 | 76 |
| TR01H014 | 77 |
| TR01H015 | 78 |
| TR01H016 | 79 |
| TR01H017 | 80 |
| TR01H018 | 81 |
| TR01H019 | 82 |
| TR01H020 | 83 |
| TR01H021 | 84 |
| TR01H022 | 85 |
| TR01H023 | 86 |
| TR01H024 | 87 |
| TR01H025 | 88 |
| TR01H026 | 89 |
| TR01H027 | 90 |
| TR01H028 | 91 |
| TR01H029 | 92 |
| TR01H030 | 93 |
| TR01H031 | 94 |
| TR01H032 | 95 |
| TR01H033 | 96 |
| TR01H034 | 97 |
| TR01H035 | 98 |
| TR01H036 | 99 |
| TR01H037 | 100 |
| TR01H038 | 101 |
| TR01H039 | 102 |
| TR01H040 | 103 |
| TR01H041 | 104 |
| TR01H042 | 105 |
| TR01H043 | 106 |
| TR01H044 | 107 |
| TR01H045 | 108 |

(continued)

| Sequence Name | SEQ ID NO: |
|---|---|
| TR01H046 | 109 |
| TR01H047 | 110 |
| TR01H048 | 111 |
| TR01H049 | 112 |
| TR01H050 | 113 |
| TR01H051 | 114 |
| TR01H052 | 115 |
| TR01H053 | 116 |
| TR01H054 | 117 |
| TR01H055 | 118 |
| TR01H056 | 119 |
| TR01H057 | 120 |
| TR01H058 | 121 |
| TR01H061 | 122 |
| TR01H062 | 123 |
| TR01H063 | 124 |
| TR01H064 | 125 |
| TR01H065 | 126 |
| TR01H066 | 127 |
| TR01H067 | 128 |
| TR01H068 | 129 |
| TR01H069 | 130 |
| TR01H070 | 131 |
| TR01H071 | 132 |
| TR01H072 | 133 |
| TR01H073 | 134 |
| TR01H074 | 135 |
| TR01H075 | 136 |
| TR01H076 | 137 |
| TR01H077 | 138 |
| TR01H079 | 139 |
| TR01H080 | 140 |
| TR01H081 | 141 |
| TR01H082 | 142 |
| TR01H083 | 143 |
| TR01H084 | 144 |
| TR01H090 | 145 |
| TR01H091 | 146 |

(continued)

| Sequence Name | SEQ ID NO: |
|---|---|
| TR01H092 | 147 |
| TR01H093 | 148 |
| TR01H094 | 149 |
| TR01H095 | 150 |
| TR01H096 | 151 |
| TR01H097 | 152 |
| TR01H098 | 153 |
| TR01H099 | 154 |
| TR01H100 | 155 |
| TR01H101 | 156 |
| TR01H102 | 157 |
| TR01H103 | 158 |
| TR01H104 | 159 |
| TR01H105 | 160 |
| TR01H106 | 161 |
| TR01H107 | 162 |
| TR01H108 | 163 |
| TR01H109 | 164 |
| TR01H110 | 165 |
| TR01H111 | 166 |
| TR01H112 | 167 |
| TR01H113 | 168 |
| TR01H114 | 169 |
| TR01H001 | 420 |
| TR01H002 | 421 |
| TR01H003 | 422 |
| TR01H004 | 423 |
| rCE115H | 424 |
| CE115HA121 | 425 |
| CE115HA122 | 426 |
| CE115HA124 | 427 |
| CE115HA192 | 428 |
| CE115HA236 | 429 |
| CE115HA251 | 430 |
| CE115HA252 | 431 |

The relationship between the CDR regions of the amino acid residues constituting the antibody H chain amino acid sequence and Kabat numbering is as shown in Fig. 2.

Examples of the common L-chain variable region to be used in the present invention include the L-chain variable regions of Table 3, antibody L-chain variable regions having CDR sequences whose CDR1, CDR2, and CDR3 amino acid sequences are the same as the CDR1, CDR2, and CDR3 amino acid sequences contained in the antibody L-chain variable regions of Table 3, and antibody L-chain variable regions that are functionally equivalent to the above-mentioned variable regions.

[Table 3]

| Sequence Name | SEQ ID NO: |
|---|---|
| L0000 | 53 |
| L0002 | 217 |
| L0003 | 218 |
| L0006 | 219 |
| L0007 | 220 |
| L0008 | 221 |
| L0009 | 222 |
| L0011 | 223 |
| L0012 | 224 |
| L0013 | 225 |
| L0014 | 226 |
| L0015 | 227 |
| L0016 | 228 |
| L0032 | 229 |
| L0038 | 230 |
| L0039 | 231 |
| L0041 | 232 |
| L0042 | 233 |
| L0043 | 234 |
| L0044 | 235 |
| L0045 | 236 |
| L0046 | 237 |
| L0047 | 238 |
| L0062 | 239 |
| L0063 | 240 |
| L0064 | 241 |
| L0065 | 242 |
| L0066 | 243 |
| L0069 | 244 |
| L0075 | 245 |
| L0079 | 246 |
| L0082 | 247 |
| L0085 | 248 |
| L0089 | 249 |
| L0090 | 250 |

(continued)

| Sequence Name | SEQ ID NO: |
|---|---|
| L0091 | 251 |
| L0093 | 252 |
| L0104 | 253 |
| L0106 | 254 |
| L0107 | 255 |
| L0109 | 256 |
| L0113 | 257 |
| L0115 | 258 |
| L0117 | 259 |
| L0120 | 260 |
| L0122 | 261 |
| L0123 | 262 |
| L0124 | 263 |
| L0125 | 264 |
| L0126 | 265 |
| L0127 | 266 |
| L0129 | 267 |
| L0132 | 268 |
| L0134 | 269 |
| L0136 | 270 |
| L0137 | 271 |
| L0138 | 272 |
| L0139 | 273 |
| L0140 | 274 |
| L0141 | 275 |
| L0143 | 276 |
| L0144 | 277 |
| L0145 | 278 |
| L0147 | 279 |
| L0148 | 280 |
| L0149 | 281 |
| L0151 | 282 |
| L0152 | 283 |
| L0154 | 284 |
| L0155 | 285 |
| L0157 | 286 |
| L0160 | 287 |
| L0161 | 288 |

(continued)

| Sequence Name | SEQ ID NO: |
|---------------|------------|
| L0163 | 289 |
| L0167 | 290 |
| L0168 | 291 |
| L0173 | 292 |
| L0175 | 293 |
| L0180 | 294 |
| L0181 | 295 |
| L0186 | 296 |
| L0187 | 297 |
| L0200 | 298 |
| L0201 | 299 |
| L0202 | 300 |
| L0203 | 301 |
| L0204 | 302 |
| L0205 | 303 |
| L0206 | 304 |
| L0207 | 305 |
| L0208 | 306 |
| L0209 | 307 |
| L0210 | 308 |
| L0211 | 309 |
| L0212 | 310 |
| L0213 | 311 |
| L0214 | 312 |
| L0215 | 313 |
| L0216 | 314 |
| L0217 | 315 |
| L0218 | 316 |
| L0219 | 317 |
| L0220 | 318 |
| L0222 | 319 |
| L0223 | 320 |
| L0224 | 321 |
| L0226 | 322 |
| L0227 | 323 |
| L0228 | 324 |
| L0229 | 325 |
| L0230 | 326 |

(continued)

| Sequence Name | SEQ ID NO: |
|---|---|
| L0231 | 327 |
| L0232 | 328 |
| L0233 | 329 |
| L0234 | 330 |
| L0235 | 331 |
| L0236 | 332 |
| L0237 | 333 |
| L0238 | 334 |
| L0239 | 335 |
| L0240 | 336 |
| L0241 | 337 |
| L0242 | 338 |
| L0243 | 339 |
| L0246 | 340 |
| L0247 | 341 |
| L0248 | 342 |
| L0249 | 343 |
| L0250 | 344 |
| L0258 | 345 |
| L0259 | 346 |
| L0260 | 347 |
| L0261 | 348 |
| L0262 | 349 |
| L0263 | 350 |
| L0264 | 351 |
| L0265 | 352 |
| L0266 | 353 |
| L0267 | 354 |
| L0268 | 355 |
| L0269 | 356 |
| L0270 | 357 |
| L0271 | 358 |
| L0272 | 359 |

The relationship between the CDR regions of the amino acid residues constituting the antibody L-chain amino acid sequence and Kabat numbering is as shown in Fig. 3.

In preferred embodiment, examples of a combination of the antibody variable region having glypican 3-binding activity and the antibody variable region having T-cell receptor complex binding activity, include the combinations of antibody H-chain variable regions shown in Table 4, combinations of antibody H-chain variable regions having CDR sequences whose CDR1, CDR2, and CDR3 amino acid sequences are the same as the CDR1, CDR2, and CDR3 amino acid sequences carried by the antibody H-chain variable regions of Table 4, and combinations of antibody H-chain variable regions functionally equivalent to these variable regions.

[Table 4]

| GPC3 side / T cell receptor complex side | SEQ ID NO: |
|---|---|
| H0000/hCE115HA | 40/52 |
| H0000/CE115HA251 | 40/430 |
| H0000/CE115HA236 | 40/429 |
| H0000/TR01H002 | 40/421 |
| H0000/CE115HA122 | 40/426 |
| H0610/rCE115H | 215/424 |
| H0610/TR01H040 | 215/103 |
| H0610/TR01H061 | 215/122 |
| H0610/TR01H068 | 215/129 |
| H0610/TR01H071 | 215/132 |
| GCH054/TR01 H067 | 197/128 |
| GCH094/TR01 H082 | 211/142 |
| GCH094/TR01 H084 | 211/144 |
| GCH065/TR01 H084 | 206/144 |
| GCH065/TR01 H082 | 206/142 |
| GCH094/TR01 H109 | 211/164 |
| GCH065/TR01H109 | 206/164 |
| GCH094/TR01 H113 | 211/168 |
| GCH065/TR01 H113 | 206/168 |

A preferred common L chain for the above combinations of an antibody variable region having glypican 3-binding activity and an antibody variable region having T-cell receptor complex binding activity includes, for example, L0000, L0011, L0201, L0203, L0204, L0206, L0208, L0209, L0211, L0212, L0222, and a common L chain having CDR sequences (CDR1, CDR2, and CDR3 amino acid sequences) identical to the CDR1, CDR2, and CDR3 amino acid sequences as in the above common L chain. Specific preferred combinations include, for example, the combinations of antibody H-chain variable regions and a common L chain shown in Table 5, combinations of antibody variable regions having CDR sequences (CDR1, CDR2, and CDR3 amino acid sequences) identical to the amino acid sequences of CDR1, CDR2, and CDR3 carried by the antibody variable regions and a common L chain of Table 5, and combinations of antibody H-chain variable regions and a common L chain functionally equivalent to these variable regions.

[Table 5]

| GPC3 side / T cell receptor complex side / common L chain | SEQ ID NO: |
|---|---|
| H0610/rCE115H/L0000 | 215/424/53 |
| H0610/TR01H040/L0000 | 215/103/53 |
| H0610/TR01H040/L0201 | 215/103/299 |
| H0610/TR01H040/L0203 | 215/103/301 |
| H0610/TR01H040/L0204 | 215/103/302 |
| H0610/TR01H040/L0206 | 215/103/304 |
| H0610/TR01H040/L0208 | 215/103/306 |

(continued)

| GPC3 side / T cell receptor complex side / common L chain | SEQ ID NO: |
|---|---|
| H0610/TR01H040/L0209 | 215/103/307 |
| H0610/TR01H040/L0211 | 215/103/309 |
| H0610/TR01H061/L0000 | 215/122/53 |
| H0610/TR01H068/L0000 | 215/129/53 |
| H0610/TR01H071/L0000 | 215/132/53 |
| GCH054/TR01 H067/L0201 | 197/128/299 |
| GCH054/TR01 H067/L0212 | 197/128/310 |
| GCH054/TR01 H067/L0222 | 197/128/319 |
| GCH054/TR01 H067/L0000 | 197/128/53 |
| GCH094/TR01 H082/L0201 | 211/142/299 |
| GCH094/TR01 H082/L0011 | 211/142/223 |
| GCH094/TR01 H084/L0011 | 211/144/223 |
| GCH065/TR01 H084/L0011 | 206/144/223 |
| GCH065/TR01 H082/L0011 | 206/142/223 |
| GCH094/TR01 H1 09/L0011 | 211/164/223 |
| GCH065/TR01H109/L0011 | 206/164/223 |
| GCH094/TR01 H113/L0011 | 211/168/223 |
| GCH065/TR01 H113/L0011 | 206/168/223 |

The Fc region comprised in the anti-T cell antigen-binding molecule (preferably the bispecific antibody) is not particularly limited as long as it is an Fc region having reduced Fcγ receptor-binding activity, but examples of a preferred Fc region include a combination of the Fc-region portion of E22Hh and the Fc-region portion of E22Hk, a combination of the Fc-region portion of E2702GsKsc and the Fc-region portion of E2704sEpsc, and a combination of the Fc-region portion of E2702sKsc and the Fc-region portion of E2704sEpsc.

In one embodiment, the anti-T cell antigen-binding molecule of the present disclosure is ERY974. ERY974 is a bispecific antibody comprising (1) a domain comprising an antibody variable region having glypican 3-binding activity, (2) a domain comprising an antibody variable region having T cell receptor complex-binding activity, and (3) a domain comprising an Fc region with reduced Fcγ receptor-binding activity. This bispecific antibody comprises TR01H113 (SEQ ID NO: 168) as the CD3-side heavy chain variable region, E2702sKsc (SEQ ID NO: 62) as the CD3-side heavy chain constant region, GCH065 (SEQ ID NO: 206) as the GPC3-side heavy chain variable region, E2704sEpsc (SEQ ID NO: 61) as the GPC3-side heavy chain constant region, L0011 (SEQ ID NO: 223) as the common light chain variable region, and k0 (SEQ ID NO: 63) as the common light chain constant region. In another embodiment, of ERY974, the CD3-side heavy chain comprises the amino acid sequence of SEQ ID NO: 402, the GPC3-side heavy chain comprises the amino acid sequence of SEQ ID NO: 385, and the common light chain comprises the amino acid sequence of SEQ ID NO: 410, respectively.

In one embodiment, amino acids contained in the amino acid sequences of the present disclosure may be subjected to post-translational modification. Modification well-known to those skilled in the art is, for example, conversion of an N-terminal glutamine residue (Q) to pyroglutamic acid (pGlu) by pyroglutamylation. Even when amino acids are post-translationally modified this way, they are, of course, included in the amino acid sequences described in the present disclosure.

Other preferred anti-T cell antigen-binding molecules in the present disclosure include bispecific antibodies having an antibody variable region having glypican 3-binding activity and an antibody variable region having CD3ε-binding activity. More preferably, the cytotoxic activity is equal to or higher than that of the bispecific antibody GPC3_ERY22_rCE115 (disclosed in Example 1 of WO2015156268). Examples of such bispecific antibodies include the bispecific antibody having an H chain and an L chain described in Example 3 (Table 13) of WO2015156268, or a bispecific antibody that binds to an epitope overlapping with an epitope to which the antibody binds and has an Fc region with reduced Fcγ receptor-binding activity.

In the present disclosure, the phrase "functionally equivalent" means that the binding affinities for an antigen are equivalent, or alternatively, it means that the cytotoxic activities against glypican 3-expressing cells or tissues containing these cells are equivalent when it is used as a bispecific antibody. The binding affinity and cytotoxic activity can be measured based on the description herein. The cells used for measurement of cytotoxic activity may be the desired GPC3-expressing cells or a desired tissue containing these cells, and for example, PC-10 or NCI-H446 which are GPC3-expressing human cancer cell lines can be used. Regarding the antibody constant regions, the phrase may mean that the decreases in Fcγ receptor-binding activity are equivalent.

For example, an antibody H-chain variable region functionally equivalent to the antibody H chain variable region described herein (i.e., the original H chain variable region) means that this region has the same binding affinity when it is combined with the antibody L-chain variable region described herein which forms a pair with the original H chain, or alternatively that the region has the same cytotoxic activity towards glypican 3-expressing cells or a tissue containing these cells when used for a bispecific antibody. Furthermore, an antibody L-chain variable region functionally equivalent to the antibody L-chain variable region described herein (i.e., the original L-chain variable region) means that this region has the same binding affinity when it is combined with the antibody H-chain variable region described herein which forms a pair with the original L chain, or alternatively that the region has the same cytotoxic activity towards glypican 3-expressing cells or a tissue containing these cells when used for a bispecific antibody.

The term "equivalent" does not necessarily have to mean the same degree of activity, and the activity may be enhanced. Specifically, for antigen-binding affinity, examples include the case where the value (KD value / parent KD value) obtained by comparison to the binding affinity of the antibody variable region serving as the control (parent KD value) is 1.5 or less. The value of KD value / parent KD value is preferably 1.3 or less, more preferably 1.2 or less, 1.1 or less, 1.0 or less, 0.9 or less, 0.8 or less, 0.7 or less, 0.6 or less, or 0.5 or less. While there is no lower limit, examples include $10^{-1}$, $10^{-2}$, $10^{-3}$, $10^{-4}$, $10^{-5}$, or $10^{-6}$. More specifically, in the present invention, the value of KD value / parent KD value is preferably $10^{-6}$ to $1.5 \times 10^{-0}$, more preferably $10^{-6}$ to $10^{-1}$, even more preferably $10^{-6}$ to $10^{-2}$, and yet even more preferably $10^{-6}$ to $10^{-3}$. For cytotoxic activity, examples include the case where the value (cell growth inhibition rate / parent cell growth inhibition rate) obtained by comparison to the cell growth inhibition rate of the bispecific antibody serving as the control (parent cell growth inhibition rate) is 0.7 or more. The concentration of the added multispecific antigen-binding molecule can be determined appropriately, but is preferably, for example, 0.01 nM, 0.05 nM, 0.1 nM, 0.5 nM, or 1 nM; and preferably, measurements are taken at 0.05 nM or 0.1 nM. The value for cell growth inhibition rate / parent cell growth inhibition rate is preferably 0.8 or higher, more preferably 0.9 or higher, 1.0 or higher, 1.2 or higher, 1.5 or higher, 2 or higher, 3 or higher, 5 or higher, 10 or higher, or 20 or higher. While there is no upper limit, the value may be 10, $10^2$, $10^3$, $10^4$, $10^5$, or $10^6$.

Furthermore, for cytotoxic activity, examples include the case where the value (concentration for 50% inhibition of cell growth / parent concentration for 50% inhibition of cell growth) obtained by comparison to the concentration of the original bispecific antibody for 50% inhibition of cell growth (parent concentration for 50% inhibition of cell growth) is 1.5 or less. Concentration for 50% growth inhibition refers to the concentration of the multispecific antigen-binding molecule necessary for reducing the cell proliferation rate to one half compared to when the multispecific antigen-binding molecule is not added. The value of "concentration for 50% inhibition of cell growth / parent concentration for 50% inhibition of cell growth" is preferably 1.3 or less, more preferably 1.2 or less, 1.1 or less, 1.0 or less, 0.9 or less, 0.8 or less, 0.7 or less, 0.6 or less, or 0.5 or less. While there is no lower limit, the value may be, for example, $10^{-1}$, $10^{-2}$, $10^{-3}$, $10^{-4}$, $10^{-5}$, or $10^{-6}$. Specifically, the value is preferably $10^{-6}$ to $1.5 \times 10^{-0}$, more preferably $10^{-6}$ to $10^{-1}$, even more preferably $10^{-6}$ to $10^{-2}$, and yet even more preferably $10^{-6}$ to $10^{-3}$.

Regarding the domain comprising an antibody variable region having GPC3-binding activity, the KD value towards GPC3 (for example, human GPC3) may be, for example, $5 \times 10^{-9}$ M or less, preferably $4 \times 10^{-9}$ M or less, such as $3 \times 10^{-9}$ M or less, $2 \times 10^{-9}$ M or less, $1 \times 10^{-9}$ M or less, $8 \times 10^{-10}$ M or less, $5 \times 10^{-10}$ M or less, $4 \times 10^{-10}$ M or less, $3 \times 10^{-10}$ M or less, $2 \times 10^{-10}$ M or less, $1 \times 10^{-10}$ M or less, $8 \times 10^{-11}$ M or less, $5 \times 10^{-11}$ M or less, $4 \times 10^{-11}$ M or less, $3 \times 10^{-11}$ M or less, $2 \times 10^{-11}$ M or less, $1 \times 10^{-11}$ M or less, $8 \times 10^{-12}$ M or less, $5 \times 10^{-12}$ M or less, $4 \times 10^{-12}$ M or less, $3 \times 10^{-12}$ M or less, $2 \times 10^{-12}$ M or less, $1 \times 10^{-12}$ M or less, $8 \times 10^{-13}$ M or less, $5 \times 10^{-13}$ M or less, $4 \times 10^{-13}$ M or less, $3 \times 10^{-13}$ M or less, $2 \times 10^{-13}$ M or less, or $1 \times 10^{-13}$ M or less.

Regarding the domain comprising an antibody variable region having T-cell receptor complex-binding activity, the KD value towards a human T-cell receptor complex such as a human T cell receptor, or more specifically for example human CD3ε may be, for example, $2 \times 10^{-7}$ M or less, preferably $1.5 \times 10^{-7}$ M or less, such as $1.4 \times 10^{-7}$ M or less, $1.3 \times 10^{-7}$ M or less, $1.2 \times 10^{-7}$ M or less, $1 \times 10^{-7}$ M or less, $3 \times 10^{-8}$ M or less, $2 \times 10^{-8}$ M or less, $1 \times 10^{-8}$ M or less, $8 \times 10^{-9}$ M or less, $5 \times 10^{-9}$ M or less, $4 \times 10^{-9}$ M or less, $3 \times 10^{-9}$ M or less, $2 \times 10^{-9}$ M or less, $1 \times 10^{-9}$ M or less, $8 \times 10^{-10}$ M or less, $5 \times 10^{-10}$ M or less, $4 \times 10^{-10}$ M or less, $3 \times 10^{-10}$ M or less, $2 \times 10^{-10}$ M or less, $1 \times 10^{-10}$ M or less, $8 \times 10^{-11}$ M or less, $5 \times 10^{-11}$ M or less, $4 \times 10^{-11}$ M or less, $3 \times 10^{-11}$ M or less, $2 \times 10^{-11}$ M or less, $1 \times 10^{-11}$ M or less, $8 \times 10^{-12}$ M or less, $5 \times 10^{-12}$ M or less, $4 \times 10^{-12}$ M or less, $3 \times 10^{-12}$ M or less, $2 \times 10^{-12}$ M or less, or $1 \times 10^{-12}$ M or less.

The bispecific antibodies of the present disclosure preferably have KD values toward human GPC3 and human T-cell receptor complex (for example, human CD3$\epsilon$ chain) that are 5 x 10$^{-9}$ M or less and 2 x 10$^{-7}$ M or less, respectively, and more preferably 1 x 10$^{-9}$ M or less and 5 x 10$^{-8}$ M or less, respectively.

In the present invention, antibody variable regions that are "functionally equivalent" are not particularly limited as long as they are antibody H-chain and/or antibody L-chain variable regions that satisfy the above-described conditions. Examples of such antibody variable regions include regions produced by introducing substitution, deletion, addition, and/or insertion of one or more amino acids (for example, 1, 2, 3, 4, 5, or 10 amino acids) into the amino acid sequences of the variable regions of Tables 1 to 3 mentioned above. A method well known to those skilled in the art for introducing one or more amino-acid substitutions, deletions, additions, and/or insertions into an amino acid sequence is a method of introducing mutations into proteins. For example, those skilled in the art can prepare variable regions that are functionally equivalent to the antibody variable regions having the above-mentioned functions by appropriately introducing mutations into amino acid sequences using methods such as site-directed mutagenesis (Hashimoto-Gotoh, T., Mizuno, T., Ogasahara, Y., and Nakagawa, M. (1995) An oligodeoxyribonucleotide-directed dual amber method for site-directed mutagenesis. Gene 152, 271-275; Zoller, M.J., and Smith, M. (1983) Oligonucleotide-directed mutagenesis of DNA fragments cloned into M13 vectors. Methods Enzymol. 100, 468-500; Kramer, W., Drutsa, V., Jansen, H.W., Kramer, B., Pflugfelder, M., and Fritz, H.J. (1984) The gapped duplex DNA approach to oligonucleotide-directed mutation construction. Nucleic Acids Res. 12, 9441-9456; Kramer, W., and Fritz, H.J. (1987) Oligonucleotide-directed construction of mutations via gapped duplex DNA Methods. Enzymol. 154, 350-367; and Kunkel, T.A. (1985) Rapid and efficient site-specific mutagenesis without phenotypic selection. Proc Natl Acad. Sci. U S A. 82, 488-492).

When an amino acid residue is altered, the amino acid is preferably mutated into a different amino acid(s) that conserves the properties of the amino acid side-chain. Examples of amino-acid side chain properties are: hydrophobic amino acids (A, I, L, M, F, P, W, Y, and V), hydrophilic amino acids (R, D, N, C, E, Q, G, H, K, S, and T), amino acids containing aliphatic side chains (G, A, V, L, I, and P), amino acids containing hydroxyl group-containing side chains (S, T, and Y), amino acids containing sulfur atom-containing side chains (C and M), amino acids containing carboxylic acid- and amide-containing side chains (D, N, E, and Q), amino acids containing basic side chains (R, K, and H), and amino acids containing aromatic side chains (H, F, Y, and W) (amino acids are represented by one-letter codes in parentheses). Amino acid substitutions within each of these groups are called conservative substitutions. It is already known that a polypeptide containing a modified amino acid sequence in which one or more amino acid residues in a given amino acid sequence are deleted, added, and/or substituted with other amino acids can retain the original biological activity (Mark, D. F. et al., Proc. Natl. Acad. Sci. USA; (1984) 81: 5662-6; Zoller, M. J. and Smith, M., Nucleic Acids Res. (1982) 10: 6487-500; Wang, A. et al., Science (1984) 224: 1431-3; Dalbadie-McFarland, G. et al., Proc. Natl. Acad. Sci. USA (1982) 79: 6409-13). Variable regions of the present invention containing such amino acid modifications have an amino acid sequence identity of at least 70%, more preferably at least 75%, even more preferably at least 80%, still more preferably at least 85%, yet more preferably at least 90%, and most preferably at least 95%, with the amino acid sequence of the CDR sequences, FR sequences, or whole variable regions of the variable region prior to modification. Herein, sequence identity is defined as the percentage of residues identical to those in the original amino acid sequence of the H-chain variable region or L-chain variable region determined after the sequences are aligned, and gaps are appropriately introduced to maximize the sequence identity as necessary. The identity of amino acid sequences can be determined by the method described below.

Furthermore, a "functionally equivalent antibody variable region" can be obtained, for example, from nucleic acids that hybridize under stringent conditions with nucleic acids comprising a nucleotide sequence encoding the amino acid sequence of a variable region in Tables 1 to 3 mentioned above. Stringent hybridization conditions for isolating a nucleic acid that hybridizes under stringent conditions with a nucleic acid comprising a nucleotide sequence encoding the amino acid sequence of a variable region include, for example, the conditions of 6 M urea, 0.4% SDS, 0.5x SSC, and 37°C, or hybridization conditions with a stringency equivalent thereto. Isolation of nucleic acids with a much higher homology can be expected with more stringent conditions, for example, the conditions of 6 M urea, 0.4% SDS, 0.1x SSC, and 42°C. The washing conditions following the hybridization are, for example, washing using 0.5x SSC (1x SSC is 0.15 M NaCl and 0.015 M sodium citrate at pH7.0) and 0.1% SDS at 60°C, more preferably washing using 0.2x SSC and 0.1% SDS at 60°C, even more preferably washing using 0.2x SSC and 0.1% SDS at 62°C, yet even more preferably washing using 0.2x SSC and 0.1% SDS at 65°C, and still more preferably washing using 0.1x SSC and 0.1% SDS at 65°C. The sequences of the isolated nucleic acids can be determined by the known methods described below. The overall nucleotide sequence homology of the isolated nucleic acid is at least 50% or higher, preferably 70% or higher, and more preferably 90% or higher (for example, 95%, 96%, 97%, 98%, 99%, or higher) sequence identity.

Nucleic acids that hybridize under stringent conditions to a nucleic acid comprising a nucleotide sequence encoding the amino acid sequence of a variable region can also be isolated by using, instead of the above-described methods using hybridization techniques, gene amplification methods such as polymerase chain reaction (PCR) that uses primers synthesized based on information of the nucleotide sequence encoding the variable-region amino acid sequence.

The identity of one nucleotide sequence or amino acid sequence to another can be determined using the algorithm BLAST, by Karlin and Altschul (Proc. Natl. Acad. Sci. USA (1993) 90: 5873-7). Programs called BLASTN and BLASTX were developed based on this algorithm (Altschul et al., J. Mol. Biol. (1990) 215: 403-10). To analyze nucleotide sequences according to BLASTN based on BLAST, the parameters are set, for example, as score = 100 and wordlength = 12. On the other hand, parameters used for the analysis of amino acid sequences by BLASTX based on BLAST include, for example, score = 50 and wordlength = 3. Default parameters for each program are used when using the BLAST and Gapped BLAST programs. Specific techniques for such analyses are known in the art (see the website of the National Center for Biotechnology Information (NCBI), Basic Local Alignment Search Tool (BLAST); http://www.ncbi.nlm.nih.gov).

Pharmaceutical compositions comprising an anti-T cell antigen-binding molecule

From another viewpoint, the present disclosure provides pharmaceutical compositions comprising as the active ingredient an anti-T cell antigen-binding molecule, preferably a bispecific antibody that comprises: (1) a domain comprising an antibody variable region having glypican 3-binding activity, (2) a domain comprising an antibody variable region having T-cell receptor complex-binding activity, and (3) a domain comprising an Fc region with reduced binding activity towards an Fcγ receptor. Furthermore, the present disclosure relates to pharmaceutical compositions that induce cell injury, which comprise the anti-T cell antigen-binding molecule as an active ingredient. Pharmaceutical compositions of the present disclosure which induce the described cell injury, particularly T-cell-dependent cellular cytotoxicity, are preferably administered to an individual suffering from a disease for which the activities are needed for prevention or treatment, or an individual in which the disease is possible to relapse.

Furthermore, in the present disclosure, cytotoxicity-inducing agents and cell growth-inhibiting agents comprising as the active ingredient a multispecific antigen-binding molecule that comprises:

(1) a domain comprising an antibody variable region having glypican 3-binding activity,
(2) a domain comprising an antibody variable region having T-cell receptor complex-binding activity, and
(3) a domain comprising an Fc region with reduced binding activity towards an Fcγ receptor can be presented as a method for inducing cell injury which comprises the step of administering the anti-T cell antigen-binding molecule to an individual, or it can be presented as use of the anti-T cell antigen-binding molecule in the manufacture of a cytotoxicity-inducing agent and a cell growth-inhibiting agent.

In the present disclosure "comprising as the active ingredient a multispecific antigen-binding molecule that comprises (1) a domain comprising an antibody variable region having glypican 3-binding activity, (2) a domain comprising an antibody variable region having T-cell receptor complex-binding activity, and (3) a domain comprising an Fc region with reduced binding activity towards an Fcγ receptor" means comprising the anti-T cell antigen-binding molecule as a major active component, without limitation to the content ratio of the anti-T cell antigen-binding molecule.

If necessary, anti-T cell antigen-binding molecules, preferably bispecific antibodies of the present invention may be encapsulated in microcapsules (e.g., those made of hydroxymethylcellulose, gelatin, and poly(methylmetacrylate)), or incorporated as components of a colloidal drug delivery system (e.g., liposomes, albumin microspheres, microemulsion, nanoparticles, and nanocapsules) (see, for example, "Remington's Pharmaceutical Science 16th edition", Oslo Ed. (1980)). Methods for preparing the pharmaceutical agents as controlled-release pharmaceutical agents are also well known, and such methods may be applied to the anti-T cell antigen-binding molecules of the present invention (J. Biomed. Mater. Res. (1981) 15: 267-277; Chemtech. (1982) 12: 98-105; U.S. Patent No. 3,773,719; European Patent Application Publication Nos. EP 58,481 and EP 133,988; Biopolymers (1983) 22: 547-556).

The pharmaceutical compositions comprising the anti-T cell antigen-binding molecule of the present disclosure may be administered to patients by oral or parenteral administration, and parenteral administration is preferred. Specific examples of the administration method include administration by injection, transnasal administration, transpulmonary administration, and transdermal administration. Examples of administration by injection include intravenous injection, intramuscular injection, intraperitoneal injection, and subcutaneous injection. A pharmaceutical composition of the present invention or a cytotoxicity-inducing agent and a cell growth-inhibiting agent can be administered systemically or locally, for example, through administration by injection. The method of administration can be selected appropriately according to the age and symptoms of the patient. The dose can be selected from the range of 0.0001 mg to 1000 mg per kilogram body weight for a single administration. Alternatively, for example, the dose may be selected from the range of 0.001 mg/body to 100000 mg/body per patient. However, the pharmaceutical compositions of the present invention or a cytotoxicity-inducing agent and cell growth-inhibiting agent are not limited to these doses.

The pharmaceutical compositions comprising the anti-T cell antigen-binding molecule of the present disclosure can be formulated according to conventional methods (for example, Remington's Pharmaceutical Science, latest edition, Mark Publishing Company, Easton, U.S.A), and may also contain pharmaceutically acceptable carriers and additives. Examples include, but are not limited to surfactants, excipients, coloring agents, perfumes, preservatives, stabilizers, buffers, suspending agents, isotonization agents, binders, disintegrants, lubricants, fluidity promoting agents, and flavoring agents; and other commonly used carriers can be suitably used. Specific examples of the carriers include light anhydrous silicic acid, lactose, crystalline cellulose, mannitol, starch, carmellose calcium, carmellose sodium, hydroxypropyl cellulose, hydroxypropyl methyl cellulose, polyvinylacetal diethylaminoacetate, polyvinylpyrrolidone, gelatin, medium chain fatty acid triglyceride, polyoxyethylene hardened castor oil 60, saccharose, carboxymethyl cellulose, corn starch, inorganic salt, and such.

In one embodiment, the present disclosure relates to pharmaceutical compositions comprising an anti-T cell antigen-binding molecule, such as a bispecific antibody, or preferably a bispecific antibody that binds to a cancer antigen and CD3, wherein the pharmaceutical composition is for use in its combination therapy with a cytokine inhibitor. In one embodiment, the cytokine inhibitor is an IL6 inhibitor, preferably an anti-human IL-6 receptor antibody, and most preferably Tocilizumab. Details of the combination therapy are disclosed in "V. Combination therapies" herein.

III. Cytokine inhibitors

In one embodiment, a "cytokine inhibitor" or "cytokine antagonist" refers to a substance capable of inhibiting or inactivating cytokines, or reducing the expression level or activity level of cytokines. A cytokine inhibitor is, for example, a compound that binds to cytokines and partially or completely blocks their activity, reduces, , prevents, delays activation of, inactivates, or desensitizes them, or down-regulates their activity or expression, such as an antagonist. In another example, a cytokine inhibitor is a compound that binds to cytokine receptors or inhibit the binding of cytokines to receptors to partially or completely block the activity of cytokines, reduce, prevent, delay the activation of, inactivate, or desensitize the cytokines, or down-regulate the activity. Cytokine inhibitors include polypeptide inhibitors such as antigen-binding molecules, antibodies, antibody derivatives, antibody fragments, and soluble receptors, and their derivatives; nucleic acid inhibitors such as siRNAs or antisense RNAs, and their derivatives; genetically modified forms of soluble factors such as forms having altered activity; as well as naturally occurring and synthetic soluble factor antagonists; and small molecule compounds, but are not limited thereto.

In one embodiment, the cytokine inhibitor is an inhibitor of one or more cytokines selected from IL-1β, IL-2, IL-4, IL-5, IL-6, IL-8, IL-10, IL-12, IL-13, IL-15, IL-17, IL-IRa, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE. In one embodiment, the cytokine inhibitor is an antibody, antibody derivative, or antibody fragment that binds to one or more cytokines selected from IL-1β, IL-2, IL-4, IL-5, IL-6, IL-8, IL-10, IL-12, IL-13, IL-15, IL-17, IL-IRa, IL-2R, IFN-α, IFN-γ, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE, or receptors thereof. In one embodiment, the cytokine inhibitor is an inhibitor of a cytokine whose plasma concentration increases when cytokine release syndrome (CRS) develops in an individual. Preferably, the cytokine inhibitor is an IL-6 inhibitor, and more preferably, it is an anti-IL-6 receptor antibody. Most preferably, the cytokine inhibitor is Tocilizumab.

IL-6 inhibitors

In one embodiment, a cytokine inhibitor is an inhibitor of IL-6 signaling, such as an inhibitor of IL-6 or IL-6 receptor. In one embodiment, an inhibitor may be an anti-IL-6 antigen-binding molecule, an anti-IL-6 antibody (including a chimeric, humanized, or human anti-IL-6 antibody), or an antigen-binding fragment thereof or an antibody derivative thereof. In another embodiment, an inhibitor may be an anti-IL-6 receptor (IL-6R) antigen-binding molecule, an anti-IL-6R antibody (including a chimeric, humanized, or human anti-IL-6R antibody), or an antigen-binding fragment thereof or an antibody derivative thereof. In one embodiment, these inhibitors may be a soluble gp130 (sgp130) or a fragment thereof that can block IL-6 signaling. In a further embodiment, sgp130 or a fragment thereof may be a fusion protein fused with a heterologous domain such as an Fc domain, for example, a gp130-Fc fusion protein such as FE301.

In one embodiment, an anti-IL-6 antibody includes, for example, Siltuximab, Olokizumab (CDP6038), Elsilimomab, Sirukumab (CNTO136), Clazakizumab (ALD518, BMS-945429), Gerilimzumab (ARGX 109), and FM101. In another embodiment, an anti-IL-6R antibody includes, for example, Tocilizumab, Satralizumab, and Sarilumab. In one embodiment, an inhibitor of IL-6 signaling includes small molecules such as CPSI-2364.

Anti-IL-6 receptor antibodies

In one embodiment, the anti-IL-6 receptor antibody is an antigen-binding molecule, an antibody, an antibody derivative, or an antibody fragment that can bind to human IL-6 receptors and block IL-6 signaling.

In a preferred embodiment, the anti-IL-6 receptor antibody is an antibody, an antibody fragment, or an antibody derivative that binds to human IL-6 receptors, and comprises the heavy chain CDR1 of SEQ ID NO: 433, the heavy chain CDR2 of SEQ ID NO: 434, and the heavy chain CDR3 of SEQ ID NO: 435, and the light chain CDR1 of SEQ ID NO: 436, the light chain CDR2 of SEQ ID NO: 437, and the light chain CDR3 of SEQ ID NO: 438.

In a preferred embodiment, the anti-IL-6 receptor antibody is an antibody, an antibody fragment, or an antibody derivative that binds to human IL-6 receptors, and comprises the heavy chain variable region of SEQ ID NO: 439 and the light chain variable region of SEQ ID NO: 440. In a different preferred embodiment, the anti-IL-6 receptor antibody is an antibody, an antibody fragment, or an antibody derivative that binds to human IL-6 receptors, and comprises the heavy chain variable region of SEQ ID NO: 441 and the light chain variable region of SEQ ID NO: 442. More preferably, the anti-IL-6 receptor antibody is an IgG antibody.

Tocilizumab

Tocilizumab (INN) is a humanized immunoglobulin G1 (IgG1) kappa anti-human IL-6R monoclonal antibody. Satralizumab is a humanized, immuno-immunoglobulin G2 (IgG2) kappa anti-human IL-6R monoclonal antibody. Tocilizumab and Satralizumab block the binding of IL-6 to soluble and membrane-bound IL-6 receptors (IL-6R); therefore, they inhibit classical and trans-IL-6 signaling.

The amino acid sequence of the heavy chain of Tocilizumab (SEQ ID NO: 441) is shown below. The part shown in bold is the heavy chain variable region of Tocilizumab (SEQ ID NO: 439), and the underlined parts are the heavy chain CDR1 (SEQ ID NO: 433), the heavy chain CDR2 (SEQ ID NO: 434), and the heavy chain CDR3 (SEQ ID NO: 435), in that order.

```
QVQLQESGPG  LVRPSQTLSL  TCTVSGYSIT  SDHAWSWVRQ  PPGRGLEWIG
YISYSGITTY  NPSLKSRVTM  LRDTSKNQFS  LRLSSVTAAD  TAVYYCARSL
ARTTAMDYWG  QGSLVTVSSA  STKGPSVFPL  APSSKSTSGG  TAALGCLVKD
YFPEPVTVSW  NSGALTSGVH  TFPAVLQSSG  LYSLSSVVTV  PSSSLGTQTY
ICNVNHKPSN  TKVDKKVEPK  SCDKTHTCPP  CPAPELLGGP  SVFLFPPKPK
DTLMISRTPE  VTCVVVDVSH  EDPEVKFNWY  VDGVEVHNAK  TKPREEQYNS
TYRVVSVLTV  LHQDWLNGKE  YKCKVSNKAL  PAPIEKTISK  AKGQPREPQV
YTLPPSRDEL  TKNQVSLTCL  VKGFYPSDIA  VEWESNGQPE  NNYKTTPPVL
DSDGSFFLYS  KLTVDKSRWQ  QGNVFSCSVM  HEALHNHYTQ  KSLSLSPG
```

The amino acid sequence of the light chain of Tocilizumab (SEQ ID NO: 442) is shown below. The part shown in bold is the light chain variable region of Tocilizumab (SEQ ID NO: 440), and the underlined parts are the light chain CDR1 (SEQ ID NO: 436), the light chain CDR2 (SEQ ID NO: 437), and the light chain CDR3 (SEQ ID NO: 438), in that order.

```
DIQMTQSPSS  LSASVGDRVT  ITCRASQDIS  SYLNWYQQKP  GKAPKLLIYY
TSRLHSGVPS  RFSGSGSGTD  FTFTISSLQP  EDIATYYCQQ  GNTLPYTFGQ
GTKVEIKRTV  AAPSVFIFPP  SDEQLKSGTA  SVVCLLNNFY  PREAKVQWKV
DNALQSGNSQ  ESVTEQDSKD  STYSLSSTLT  LSKADYEKHK  VYACEVTHQG
LSSPVTKSFN  RGEC
```

In one embodiment, the amino acids contained in the amino acid sequences of the present disclosure may be subjected to post-translational modification. For example, conversion of an N-terminal glutamine residue (Q) to pyroglutamic acid (pGlu) by pyroglutamylation is a modification well-known to those skilled in the art. Even when amino acids are post-translationally modified this way, they are, of course, included in the amino acid sequences described in the present disclosure.

Pharmaceutical compositions comprising a cytokine inhibitor

From another viewpoint, the present disclosure provides pharmaceutical compositions comprising as an active ingredient a cytokine inhibitor, preferably an anti-human IL-6 receptor antibody, and more preferably Tocilizumab. Furthermore, the present disclosure relates to pharmaceutical compositions comprising the cytokine inhibitor as the active ingredient, which prevent or treat cytokine release syndrome (CRS) associated with administration of an anti-T cell antigen-binding molecule. The pharmaceutical compositions of the present disclosure are preferably administered to an individual who may develop CRS associated with administration of an anti-T cell antigen-binding molecule, and/or an individual who has developed CRS or signs of CRS and needs treatment. More specifically, the present disclosure provides methods for treating cytokine release syndrome (CRS) associated with administration of an anti-T cell antigen-binding molecule, wherein the method comprises the steps of: administering an anti-T cell antigen-binding molecule to a subject; selecting a subject who has developed signs of CRS associated with the anti-T cell antigen-binding molecule and needs treatment; and administering a cytokine inhibitor to the selected subject.

Furthermore, in the present disclosure, a pharmaceutical composition comprising a cytokine inhibitor as an active ingredient can be expressed as a method for preventing or treating CRS associated with administration of an anti-T cell antigen-binding molecule, which comprises administering the cytokine inhibitor to an individual, or use of the cytokine inhibitor in producing an agent for CRS prevention or CRS treatment.

In the present disclosure, "comprising a cytokine inhibitor as an active ingredient" means that the cytokine inhibitor is comprised as a major active ingredient, and this does not limit the content percentage of the cytokine inhibitor.

If necessary, cytokine inhibitors of the present invention may be encapsulated in microcapsules (e.g., those made of hydroxymethylcellulose, gelatin, and poly(methylmetacrylate)), or incorporated as components of a colloidal drug delivery system (e.g., liposomes, albumin microspheres, microemulsion, nanoparticles, and nanocapsules) (see, for example, "Remington's Pharmaceutical Science 16th edition", Oslo Ed. (1980)). Methods for preparing the pharmaceutical agents as controlled-release pharmaceutical agents are also well known, and such methods may be applied to the cytokine inhibitors of the present invention (J. Biomed. Mater. Res. (1981) 15: 267-277; Chemtech. (1982) 12: 98-105; U.S. Patent No. 3,773,719; European Patent Application Publication Nos. EP 58,481 and EP 133,988; Biopolymers (1983) 22: 547-556).

The pharmaceutical compositions comprising the cytokine inhibitor of the present disclosure may be administered to patients by oral or parenteral administration, and parenteral administration is preferred. Specific examples of the administration method include administration by injection, transnasal administration, transpulmonary administration, and transdermal administration. Examples of administration by injection include intravenous injection, intramuscular injection, intraperitoneal injection, and subcutaneous injection. A pharmaceutical composition of the present invention or a cytotoxicity-inducing agent and a cell growth-inhibiting agent can be administered systemically or locally, for example, through administration by injection. The method of administration can be selected appropriately according to the age and symptoms of the patient. The dose can be selected from the range of 0.0001 mg to 1000 mg per kilogram body weight for a single administration. Alternatively, for example, the dose may be selected from the range of 0.001 mg/body to 100000 mg/body per patient. However, the pharmaceutical compositions of the present invention are not limited to these doses.

In one embodiment, when the cytokine inhibitor is Tocilizumab, Tocilizumab is administered at approximately 4 to 12 mg/kg per dose, such as 8 mg/kg or less per dose for a patient weighing 30 kg or more and at 12 mg/kg or less per dose for a patient weighing less than 30 kg, for example by intravenous injection over a course of 0.5 hours, 1 hour, 2 hours, or 3 hours. In a different embodiment, when Tocilizumab is administered for purposes of treating CRS, and the symptoms of CRS are not improved after the first administration of Tocilizumab to a patient, administrations of Tocilizumab can be added up to three times. In one example, when Tocilizumab is sequentially administered in this manner, the interval must be 8 hours or more from the previous administration.

The pharmaceutical compositions comprising the cytokine inhibitor of the present disclosure can be formulated according to conventional methods (for example, Remington's Pharmaceutical Science, latest edition, Mark Publishing Company, Easton, U.S.A), and may also contain pharmaceutically acceptable carriers and additives. Examples include, but are not limited to surfactants, excipients, coloring agents, perfumes, preservatives, stabilizers, buffers, suspending agents, isotonization agents, binders, disintegrants, lubricants, fluidity promoting agents, and flavoring agents; and other commonly used carriers can be suitably used. Specific examples of the carriers include light anhydrous silicic acid, lactose, crystalline cellulose, mannitol, starch, carmellose calcium, carmellose sodium, hydroxypropyl cellulose, hydroxypropyl methyl cellulose, polyvinylacetal diethylaminoacetate, polyvinylpyrrolidone, gelatin, medium chain fatty acid triglyceride, polyoxyethylene hardened castor oil 60, saccharose, carboxymethyl cellulose, corn starch, inorganic salt, and such.

In one embodiment, the present disclosure relates to pharmaceutical compositions comprising a cytokine inhibitor, preferably an IL-6 inhibitor, more preferably an IL-6 receptor antibody, and most preferably Tocilizumab, wherein the pharmaceutical composition is for use in its combination therapy with an anti-T cell antigen binding molecule. In one embodiment, the anti-T cell antigen-binding molecule is for example, a bispecific antibody, or preferably a bispecific antibody that binds to a cancer antigen and CD3. Details of the combination therapy are disclosed in "V. Combination therapies" herein.

IV. Cytokine Release Syndrome (CRS)

In one embodiment, cytokine release syndrome (CRS) is a serious and potentially life-threatening side effect that can occur when a pharmaceutical agent, for example, an antibody pharmaceutical (such as, an anti-T cell antibody) or a T cell pharmaceutical (such as, a chimeric antigen receptor (CAR) T cell (CAR-T cell)) is administered. Administration of antibody pharmaceuticals or T cell pharmaceuticals activates the immune response in the body more than necessary and releases inflammatory cytokines and such, which result in various symptoms such as chills, nausea, malaise, headache, fever, tachycardia, and blood pressure fluctuations. Severe cases, in particular, are sometimes referred to as cytokine storms. CRS results from high levels of immune activation when large numbers of lymphocytes and/or myeloid cells release inflammatory cytokines upon activation. The severity of CRS and the timing of onset of symptoms may vary depending on the magnitude of immune cell activation, the type of pharmaceutical agent administered, and/or the amount of systemic tumor tissue in the individual. In the case of T cell therapy for cancer, symptom onset is typically days to weeks after administration of the T cell therapy, for example, when there is a peak of T cell proliferation *in vivo*. See, for example, Lee et al. Blood. 124.2 (2014): 188-95.

In one embodiment, the symptoms of CRS may include neurologic toxicity, disseminated intravascular coagulation, cardiac dysfunction, adult respiratory distress syndrome, renal failure, and/or hepatic failure. For example, the symptoms of CRS may include fever/high fever with or without chills, fatigue, discomfort, muscle pain, vomiting, headache, nausea, loss of appetite, joint pain, diarrhea, rash, hypoxia, tachypnea, hypotension, widened pulse pressure, potentially diminished cardiac output (late), increased cardiac output (early), azotemia, hypofibrinogenemia with or without bleeding, elevated D-dimer, hyperbilirubinemia, transaminitis, confusion, delirium, changes in mental status, hallucinations, tremor (trembling), seizures, altered gait, word finding difficulty, obvious aphasia, or dementia.

In one embodiment, CRS is characterized by increased concentrations of several cytokines in an individual. In one embodiment, the cytokines include IL-1$\beta$, IL-2, IL-4, IL-5, IL-6, IL-8, IL-10, IL-12, IL-13, IL-15, IL-17, IL-1Ra, IL-2R, IFN-$\alpha$, IFN-$\gamma$, MIP-1$\alpha$, MIP-1$\beta$, MCP-1, TNF$\alpha$, GM-CSF, G-CSF, CXCL9, CXCL10, CXCR factor, VEGF, RANTES, eotaxin, EGF, HGF, FGF-$\beta$, CD30, CD30L, CD40, CD40L, ferritin, and RAGE, but are not limited thereto. Preferably, the cytokine comprises IL-6, IL-1 beta, or TNF-alpha, or any combination thereof. Most preferably, the cytokine is IL-6. In one embodiment, patients with large tumor volumes have a higher incidence and severity of cytokine syndrome.

In one embodiment, those skilled in the art will understand that the term "cytokine concentration" includes a measured concentration value, magnitude of fold change, magnitude of percent (%) change, or magnitude of rate change. In addition, methods for measuring cytokines in blood, saliva, serum, urine, plasma, and/or serum are well known in the art.

Severity (Grade) of CRS

In one embodiment, CRS can be classified by severity (Grade) from 1 to 5. In one embodiment, for Grade 1 CRS, only symptomatic treatment is needed (for example, nausea, fever, fatigue, muscle pain, malaise, and headache), and the symptoms are not life threatening. For Grade 2 CRS, the symptoms require moderate intervention and generally respond to moderate intervention. Individuals with Grade 2 CRS develop hypotension that is responsive to either fluids or one type of low-dose vasopressor; or they develop grade 2 organ toxicity or mild respiratory symptoms that are responsive to low flow oxygen (less than 40% oxygen). In individuals with Grade 3 CRS, hypotension generally cannot be reversed by fluid therapy or one type of low-dose vasopressor. These individuals generally require lower flow of oxygen and have grade 3 organ toxicity (for example, renal or cardiac dysfunction or blood coagulation disorder) and/or grade 4 transaminitis. Individuals with Grade 3 CRS require more aggressive intervention, such as oxygen of 40% or higher, a high-dose vasopressor, and/or multiple vasopressors. Individuals with Grade 4 CRS suffer from immediately life-threatening symptoms, including grade 4 organ toxicity or a need for mechanical artificial ventilation. Individuals with Grade 4 CRS generally do not have transaminitis. In individuals with Grade 5 CRS, the toxicity causes death.

In one embodiment, the CRS grading is based on the Common Terminology Criteria for Adverse Events (CTCAE) v4.03 shown in Table 6, the Common Terminology Criteria for Adverse Events (CTCAE) v5.0 shown in Table 7, Lee's criteria of 2014 (Lee DW, et al. Current concepts in the diagnosis and management of cytokine release syndrome. Blood, 124 (2014), pp. 188-195) shown in Table 8, the 2019 ASTCT CRS Consensus Grading (Lee DW, et al. ASTCT Consensus Grading for Cytokine Release Syndrome and Neurologic Toxicity Associated with Immune Effector Cells. Biol Blood Marrow Transplant. 2019 Apr;25(4):625-638) shown in Table 9, Penn's criteria, MSKCC's criteria, or CARTOX's criteria (Biol Blood Marrow Transplant. 2019 Apr;25(4):625-638 indicated above). Preferably, CRS grading is based on CTCAE v4.03, CTCAE v5.0, or Lee's criteria of 2014. Unless otherwise specified, CRS as used herein refers to CRS according to the criteria in Table 8 (Lee's criteria of 2014).

[Table 6]

| (CTCAE v 4.03) | |
|---|---|
| Grade I | Mild reaction; not requiring interruption of infusion; not requiring treatment |

(continued)

| (CTCAE v 4.03) | |
|---|---|
| Grade II | Requiring interruption of treatment or infusion. However, responding quickly to symptomatic treatment (for example: antihistamines, NSAIDs, narcotic pharmaceutical agents, and intravenous infusion); requiring prophylactic medications in 24 hours or less |
| Grade III | Prolonged (for example: not rapidly responsive to symptomatic treatment and/or brief interruption of infusion); recurrence after initial improvement; requiring hospitalization due to sequela(e) (for example: renal dysfunction, and pulmonary infiltration) |
| Grade IV | Life-threatening; requiring positive-pressure breathing or mechanical ventilation |
| Grade V | Death |

[Table 7]

| (CTCAE v 5.0) | |
|---|---|
| Grade I | Fever with or without constitutional symptoms |
| Grade II | Hypotension responding to infusion; hypoxia responding to <40% oxygen administration |
| Grade III | Hypotension manageable by a single vasopressor; hypoxia requiring ≥40% oxygen administration |
| Grade IV | Life-threatening; requiring emergency treatment |
| Grade V | Death |

[Table 8]

| (Lee's criteria) | |
|---|---|
| Grade I | Not life-threatening; requiring symptomatic treatments only (fever, nausea, fatigue, headache, muscle pain, discomfort) |
| Grade II | Requiring moderate intervention; hypoxia responding to >40% oxygen administration; hypotension responding to intravenous infusion or low-dose of a single vasopressor; grade 2 or higher organ toxicity |
| Grade III | Requiring aggressive intervention; hypoxia responding to ≤40% oxygen administration; hypotension responding to high-dose or multiple vasopressors; grade 3 or higher organ toxicity or grade 4 or higher transaminitis |
| Grade IV | Life-threatening; requiring mechanical artificial ventilation; grade 4 or higher organ toxicity (except transaminitis) |
| Grade V | Death |

[Table 9]

| (ASTCT CRS Consensus Grading) | | | |
|---|---|---|---|
| | Fever | | Hypotension and/or hypoxia |
| Grade I | 38°C or higher | and | Hypotension: none, hypoxia: none |
| Grade II | 38°C or higher | and | Hypotension not requiring a vasopressor and/or hypoxia requiring a low-flow nasal cannula or blow-by |
| Grade III | 38°Corhigher | and | Hypotension requiring a single vasopressor (regardless of combined use of vasopressin), and/or hypoxia requiring high-flow nasal cannula, face mask, nonrebreathing mask, or Venturi mask |

(continued)

| (ASTCT CRS Consensus Grading) | | | |
|---|---|---|---|
| Grade IV | 38°C or higher | and | Hypotension requiring multiple vasopressors (excluding vasopressin), and/or hypoxia requiring positive pressure (for example, CPAP, BiPAP, intubation, and mechanical artificial ventilation) |

In one embodiment, the symptoms of CRS develop within minutes, hours, or days after starting to administer the pharmaceutical agent, but some symptoms are delayed. Preferably, the symptoms of CRS develop within about 96 hours, about 72 hours, or about 48 hours after starting to administer the anti-T cell antigen-binding molecule, and more preferably, the symptoms develop from the day of administration of the anti-T cell antigen-binding molecule to the next day. In another embodiment, the severity of CRS symptoms correlates with peak cytokine concentrations.

Signs of CRS

In one embodiment, signs of CRS refer to CRS-like symptoms that are precursors to the above-mentioned CRS (regardless of Grade). Specific examples include, but are not limited to, fever or hypotension that initially develops after administration of the anti-T cell antigen binding molecule.

Treatment of CRS

In one embodiment, examples of treatment methods for CRS include inflammatory cytokine inhibitors, IL-6 inhibitors or IL-6 receptor (IL-6R) inhibitors (such as, Tocilizumab or Satralizumab), bazedoxifene, SGP130 blockers, vasoactive pharmaceutical agents, systemic adrenal cortex hormones (for example, corticosteroids), immunosuppressants, and mechanical artificial ventilation.

In one embodiment, the inflammatory cytokine inhibitor is an inhibitor or an antagonistic inhibitor of one or more cytokines selected from IL-1$\beta$, IL-2, IL-4, IL-5, IL-6, IL-8, IL-10, IL-12, IL-13, IL-15, IL-17, IL-IRa, IL-2R, IFN-$\alpha$, IFN-y, MIP-1$\alpha$, MIP-1$\beta$, MCP-1, TNF$\alpha$, GM-CSF, G-CSF, CXCL9, CXCL10, CXCR factor, VEGF, RANTES, eotaxin, EGF, HGF, FGF-$\beta$, CD30, CD30L, CD40, CD40L, ferritin, and RAGE. Preferred cytokine inhibitors are disclosed in "III. Cytokine Inhibitors" herein.

In one embodiment, when the cytokine inhibitor used for CRS treatment is Tocilizumab, Tocilizumab is administered by intravenous injection at approximately 4 to 12 mg/kg per dose, such as 8 mg/kg or less per dose for a patient weighing 30 kg or more and at 12 mg/kg or less per dose for a patient weighing less than 30 kg, for example over a course of 0.5 hours, 1 hour, 2 hours, or 3 hours. In a different embodiment, when Tocilizumab is administered for purposes of treating CRS, and the symptoms of CRS are not improved after the first administration of Tocilizumab to a patient, administration of Tocilizumab can be added up to three times. In one example, when Tocilizumab is sequentially administered in this manner, the interval must be 8 hours or more from the previous administration.

Exemplary vasoactive pharmaceutical agents include but are not limited to angiotensin-11, endothelin-1, alpha adrenergic agonists, rostanoids, phosphodiesterase inhibitors, endothelin antagonists, circulatory agonists (for example, adrenaline, dobutamine, isoprenaline, and ephedrine), vasopressors (for example, noradrenaline, vasopressin, metaraminol, vasopressin, and methylene blue), inodilators (for example, milrinone and levosimendan), and dopamine.

Exemplary vasopressors include but are not limited to norepinephrine, dopamine, phenylephrine, epinephrine, and vasopressin. In one embodiment, vasopressors include high-dose vasopressors and low-dose vasopressors. In one embodiment, a high-dose vasopressor includes one or more of the following: norepinephrine monotherapy at 20 $\mu$g/min or more, dopamine monotherapy at 10 ($\mu$g/kg/min or more, phenylephrine monotherapy at 200 $\mu$g/min or more, and/or epinephrine monotherapy at 10 $\mu$g/min or more. In some embodiments, if an individual is on vasopressin, a high-dose vasopressor includes vasopressin + norepinephrine equivalent at 10 $\mu$g/min or more (where the dose of norepinephrine equivalent = [norepinephrine ($\mu$g/min)] + [dopamine ($\mu$g/kg/min)/2] + [epinephrine ($\mu$g/min)] + [phenylephrine ($\mu$g/min)/10]). In some embodiments, if the individual is on combination vasopressors (not vasopressin), a high-dose vasopressor includes norepinephrine equivalent at 20 $\mu$g/min or more (where the dose of norepinephrine equivalent = [norepinephrine ($\mu$g/min)] + [dopamine ($\mu$g/kg/min)/2] + [epinephrine ($\mu$g/min)] + [phenylephrine ($\mu$g/min)/10]. See for example, *idem.*

In one embodiment, a low-dose vasopressor is a vasopressor administered at a dose less than one or more of the doses listed above for high-dose vasopressors.

Exemplary corticosteroids include but are not limited to dexamethasone, hydrocortisone, and methylprednisolone. In one embodiment, a dose of 0.5 mg/kg of dexamethasone is used by oral or intravenous administration. In one embodiment, 8 to 20 mg of dexamethasone per dose, a pharmaceutically acceptable salt thereof, or a derivative thereof is used by oral or intravenous administration. It will be understood by those skilled in the art that the dose of dexamethasone is not limited to the above and may be appropriately changed depending on the condition of the individual and the severity of CRS.

An exemplary immunosuppressant includes a TNF-alpha inhibitor or an IL-1 inhibitor. In one embodiment, TNF-alpha inhibitors include anti-TNF-alpha antibodies, for example, a monoclonal antibody, such as Infliximab. In one embodiment, TNF-alpha inhibitors include soluble TNF-alpha receptors (for example, Etanercept). In embodiments, IL-1 or IL-1R inhibitors include Anakinra.

V. Combination therapies

In one embodiment, the present disclosure relates to the combined use of an anti-T cell antigen-binding molecule and a cytokine inhibitor. In another embodiment, the present disclosure relates to a pharmaceutical composition comprising an anti-T cell antigen binding molecule, wherein the pharmaceutical composition is for use in its combination therapy with a cytokine inhibitor. In a further embodiment, the present disclosure relates to a pharmaceutical composition comprising a cytokine inhibitor, wherein the pharmaceutical composition is for use in its combination therapy with an anti-T cell antigen-binding molecule. The present disclosure relates to a method for treating cancer in an individual, comprising administering an anti-T cell antigen-binding molecule and a cytokine inhibitor.

In one embodiment, administration of a cytokine inhibitor prevents, alleviates, or treats cytokine release syndrome (CRS) associated with administration of an anti-T cell antigen-binding molecule in an individual. Therefore, the present disclosure may be rephrased as a method for preventing, alleviating, or treating development of CRS associated with administration of an anti-T cell antigen-binding molecule, which comprises administering a cytokine inhibitor. Alternatively, the present disclosure provides a method for any of the purposes selected from prevention, alleviation, and treatment of CRS associated with the administration of an anti-T cell antigen-binding molecule, or a combination thereof, wherein the method comprises administering a cytokine inhibitor.

In one embodiment, a cytokine inhibitor is administered subcutaneously or intravenously to an individual before, simultaneously with, or after administration of an anti-T cell antigen binding molecule. Preferably, a cytokine inhibitor is administered intravenously to an individual before, simultaneously with, or after administration of an anti-T cell antigen-binding molecule. Although not intended to be limiting, when a cytokine inhibitor is administered before or simultaneously with the administration of an anti-T cell antigen-binding molecule, it has the effect of preventing or alleviating development of CRS associated with administration of the anti-T cell antigen-binding molecule. In one embodiment, a cytokine inhibitor is administered 6 days, 5 days, 4 days, 3 days, 2 days, or 1 day before administration of the anti-T cell antigen-binding molecule, or on the same day as but before the administration. Alternatively, a cytokine inhibitor may be administered on the same day as and simultaneously with administration of the anti-T cell antigen-binding molecule (in the present disclosure, administration of a cytokine inhibitor before administration of the anti-T cell antigen-binding molecule, and administration of a cytokine inhibitor simultaneously with administration of the anti-T cell antigen-binding molecules are collectively referred to as "pre-administration of a cytokine inhibitor"). In one embodiment, pre-administration of a cytokine inhibitor prevents or alleviates development of CRS associated with administration of an anti-T cell antigen-binding molecule, without significantly impairing the efficacy of the anti-T cell antigen-binding molecule (for example, T cell-dependent cellular cytotoxicity (TDCC) and antitumor effect).

Furthermore, when a cytokine inhibitor is administered after administration of the anti-T cell antigen-binding molecule and before development of CRS, it has the effect of preventing or alleviating CRS that may develop thereafter. In addition, when a cytokine inhibitor is administered after administration of an anti-T cell antigen-binding molecule and after development of CRS or signs of CRS, it has the effect of treating CRS and alleviating their symptoms.

In one embodiment, the anti-T cell antigen-binding molecule used in the combination therapy is a bispecific antigen-binding molecule comprising "a domain comprising an antibody variable region having T cell receptor complex-binding activity" and "a domain comprising an antibody variable region having cancer antigen-binding activity", and is preferably a bispecific antibody. In one embodiment, the bispecific antibody may have the structure of a single chain antibody, for example, a structure in which antibody variable regions are linked by linkers. In one embodiment, the anti-T cell antigen-binding molecule further comprises an Fc region with reduced Fcγ receptor-binding activity. Preferably, the anti-T cell antigen-binding molecule is a bispecific antibody comprising (1) a domain comprising an antibody variable region having a glypican 3-binding activity, (2) a domain comprising an antibody variable region having T cell receptor complex-binding activity, and (3) a domain comprising an Fc region with reduced Fcγ receptor-binding activity.

In one embodiment, a cytokine inhibitor used in the combination therapy is an IL-6 inhibitor, preferably an anti-IL-6 receptor antibody, and most preferably Tocilizumab. In one embodiment, when Tocilizumab is administered before or simultaneously with administration of the anti-T cell antigen binding molecule, Tocilizumab is administered to human adults and children, for example, at a dose selected from 5 mg/kg to 100 mg/kg, for example, 5 mg/kg to 90 mg/kg, 5 mg/kg to 80 mg/kg, 5 mg/kg to 70 mg/kg, 5 mg/kg to 60 mg/kg, 5 mg/kg to 50 mg/kg, 5 mg/kg to 40 mg/kg, 5 mg/kg to 30 mg/kg, 5 mg/kg to 20 mg/kg, 5 mg/kg to 15 mg/kg, and for example, 10 mg/kg to 100 mg/kg, 20 mg/kg to 100 mg/kg, 30 mg/kg to 100 mg/kg, 40 mg/kg to 100 mg/kg, 50 mg/kg to 100 mg/kg, 60 mg/kg to 100 mg/kg, 70 mg/kg to 100 mg/kg, 80 mg/kg to 100 mg/kg, 90 mg/kg to 100 mg/kg, and the like, per dose. In addition, although it is specified to avoid doubt, for example, when described as 5 mg to 100 mg/kg, any dose included between 5 mg to 100 mg/kg varying by 0.1 mg/kg, such as 5.0 mg/kg, 5.1 mg/kg, 5.2 mg/kg, 5.3 mg/kg, 5.4 mg/kg, 49.9 mg, 50 mg/kg, 50.1 mg/kg, 50.2 mg/kg,... 99.8 mg/kg, 99.9 mg/kg, and 100 mg/kg, is intended to be individually and specifically described herein. In one embodiment, Tocilizumab is administered before or simultaneously with administration of the anti-T cell antigen-binding molecule, at 8 mg/kg or less, 0.5 to 8 mg/kg, 1 to 8 mg/kg, 2 to 8 mg/kg, 3 to 8 mg/kg, 4 to 8 mg/kg, or preferably 4 to 8 mg/kg per dose for a patient weighing 30 kg or more. In another embodiment, Tocilizumab is administered before or simultaneously with administration of the anti-T cell antigen-binding molecule, at 12 mg/kg or less, 0.5 to 12 mg/kg, 1 to 12 mg/kg, 3 to 12 mg/kg, 5 to 12 mg/kg, 6 to 12 mg/kg, or preferably 6 to 12 kg/kg per dose for a patient weighing less than 30 kg.

The combination therapies of the present disclosure may further include administration of corticosteroids. In one embodiment, for the purpose of preventing or alleviating CRS associated with administration of the anti-T cell antigen-binding molecule, corticosteroids are orally or intravenously administered to an individual before administration of the anti-T cell antigen-binding molecule, 2 days, 1 day, or 0 days before (on the same day as) the administration of the anti-T cell antigen-binding molecule. Alternatively, the corticosteroid is orally or intravenously administered to an individual simultaneously with the administration of the anti-T cell antigen-binding molecule (these may be referred to as "pre-administration of a corticosteroid" or "pre-administration of a steroid"). Preferably, pre-administration of a corticosteroid is performed in addition to pre-administration of a cytokine inhibitor (in combination with pre-administration of a cytokine inhibitor). Although not intended to be limiting, examples of preferred corticosteroids include dexamethasone, hydrocortisone, and methylprednisolone. Preferably, the corticosteroid is dexamethasone, a pharmaceutically acceptable salt thereof, or a derivative thereof, which is administered orally or intravenously. It will be understood by those skilled in the art that the dose of dexamethasone is not limited to the above and may be appropriately changed depending on the condition of the individual, the development of CRS, and the like.

In one embodiment, the combination therapy is carried out on the same day as administration of an anti-T cell antigen-binding molecule in the following order: (1) pre-administration of a corticosteroid, (2) pre-administration of a cytokine inhibitor, and (3) administration of an anti-T cell antigen-binding molecule. In this case, for example, administration of a corticosteroid to an individual is completed 1 hour or more, 2 hours or more, 3 hours or more, 4 hours or more, or 5 hours or more, or preferably 2 hours or more before starting the administration of a cytokine inhibitor; furthermore, administration of a cytokine inhibitor to the individual is completed 1 hour or more, 2 hours or more, 3 hours or more, 4 hours or more, or 5 hours or more, or preferably 2 hours or more before starting the administration of the anti-T cell antigen-binding molecule. In another embodiment, administration is performed on the same day as administration of an anti-T cell antigen-binding molecule in the following order: (1) pre-administration of a cytokine inhibitor, (2) pre-administration of a corticosteroid, and (3) administration of an anti-T cell antigen-binding molecule. In this case, for example, administration of a cytokine inhibitor to an individual is completed 1 hour or more, 2 hours or more, 3 hours or more, 4 hours or more, or 5 hours or more, or preferably 2 hours or more before starting the administration of a corticosteroid; furthermore, administration of the corticosteroid to the individual is completed 1 hour or more, 2 hours or more, 3 hours or more, 4 hours or more, or 5 hours or more, or preferably 2 hours or more before starting the administration of the anti-T cell antigen-binding molecule.

In a different embodiment, the combination therapy of the present disclosure does not include pre-administration of corticosteroids. Although not intended to be limiting, pre-administration of a cytokine inhibitor prevents or alleviates development of CRS associated with administration of the anti-T cell antigen-binding molecule, and administration of corticosteroids for purposes of preventing or alleviating CRS becomes unnecessary.

It is naturally understood by those skilled in the art that the anti-T cell antigen-binding molecules, cytokine inhibitors, and/or other pharmaceutical agents in the present disclosure may all have a given "permissible range" for the timing of administration, the dosing interval, and the dose, respectively, and those skilled in the art can appropriately determine the permissible range. For example, performing timely increase or decrease of the dosing interval or appropriate increase or decrease of the dose of anti-T cell antigen-binding molecules, cytokine inhibitors, and/or other pharmaceutical agents at the discretion of the doctor, based on symptoms and such of the individual, is within the above "permissible range".

Below, non-limiting specific examples of combination therapies comprising anti-T cell antigen-binding molecules and cytokine inhibitors are described.

Administration of an anti-T cell antigen-binding molecule, accompanied with pre-administration of a cytokine inhibitor

In one embodiment, the present disclosure provides a pharmaceutical composition comprising an anti-T cell antigen-binding molecule, which is for use in its combination therapy with a cytokine inhibitor. In a different embodiment, the present disclosure provides a pharmaceutical composition comprising a cytokine inhibitor, which is for use in its combination therapy with an anti-T cell antigen binding molecule. In a further embodiment, a cytokine inhibitor is administered to an individual before or simultaneously with administration of the anti-T cell antigen binding molecule. Although not intended to be limiting, when a cytokine inhibitor is administered before or simultaneously with administration of the anti-T cell antigen-binding molecule, it has the effect of preventing or alleviating development of CRS associated with administration of the anti-T cell antigen-binding molecule. In one embodiment, a cytokine inhibitor is administered 6 days, 5 days, 4 days, 3 days, 2 days, or 1 day before administration of the anti-T cell antigen-binding molecule, or on the same day as but before the administration of the bispecific antibody. Alternatively, a cytokine inhibitor is administered simultaneously with the anti-T cell antigen-binding molecule on the same day as administration of the anti-T cell antigen-binding molecule. In one embodiment, pre-administration of a cytokine inhibitor prevents or alleviates development of CRS associated with administration of an anti-T cell antigen-binding molecule, without significantly impairing the efficacy of the anti-T cell antigen-binding molecule (for example, T cell-dependent cellular cytotoxicity (TDCC) and antitumor effect).

In one embodiment, the combination therapy of the present disclosure comprises, in addition to pre-administration of a cytokine inhibitor, further administering the cytokine inhibitor to an individual 1 day, 2 days, 3 days, 4 days, or 5 days after administration of the anti-T cell antigen-binding molecule.

In one embodiment, the combination therapy of the present disclosure comprises, in addition to pre-administration of a cytokine inhibitor, further administering the cytokine inhibitor to an individual when CRS or signs of CRS develop after administration of the anti-T cell antigen-binding molecule. Here, the individual may be (i) one who has received a pre-administration of the cytokine inhibitor, or (ii) one who has received both a pre-administration of the cytokine inhibitor and an administration of the cytokine inhibitor after the administration of the anti-T cell antigen-binding molecule. In one embodiment, the CRS is CRS in Grade 2 or higher or Grade 3 or higher. Alternatively, the combination therapy of the present disclosure comprises the steps of administering an anti-T cell antigen-binding molecule to a subject, selecting a subject who has developed CRS or signs of CRS after administration of the anti-T cell antigen-binding molecule; and administering a cytokine inhibitor to the selected subject.

Administration of an anti-T cell antigen-binding molecule (repeated administration), accompanied with pre-administration of a cytokine inhibitor

In one embodiment, in the combination therapy of the present disclosure, (i) an anti-T cell antigen-binding molecule is administered repeatedly, and (ii) a cytokine inhibitor is administered before or simultaneously with each administration of the repeated administrations. Preferably, the anti-T cell antigen-binding molecule is administered repeatedly at the same dose.

When pre-administration of a cytokine inhibitor is performed for each (every) anti-T cell antigen-binding molecule administration

(Repeated administration of the anti-T cell antigen-binding molecule at intervals of once every week)

In one embodiment, in the combination therapy of the present disclosure, (i) an anti-T cell antigen-binding molecule is administered repeatedly at intervals of once every week, and (ii) a cytokine inhibitor is administered before administration of the anti-T cell antigen-binding molecule, which is 6 days, 5 days, 4 days, 3 days, 2 days, or 1 day before, or on the same day as each administration of the repeated administrations. Alternatively, (i) an anti-T cell antigen-binding molecule is administered repeatedly at intervals of once every week, and (ii) a cytokine inhibitor is administered simultaneously with administration of the anti-T cell antigen-binding molecule on the same day as each administration of the repeated administrations.

In a further embodiment, in the combination therapy of the present disclosure, in addition to pre-administration of a cytokine inhibitor, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- on the same day as administration of the anti-T cell antigen-binding molecule, when pre-administration of the cytokine inhibitor is performed 6 days before the administration of the anti-T cell antigen-binding molecule;
- on the same day as or 1 day after administration of the anti-T cell antigen-binding molecule, when pre-administration of the cytokine inhibitor is performed 5 days before the administration of the anti-T cell antigen-binding molecule;
- on the same day as, or 1 day or 2 days after administration of the anti-T cell antigen-binding molecule, when pre-administration of the cytokine inhibitor is performed 4 days before the administration of the anti-T cell antigen-binding molecule;
- on the same day as, or 1 day, 2 days, or 3 days after administration of the anti-T cell antigen-binding molecule,

when pre-administration of the cytokine inhibitor is performed 3 days before the administration of the anti-T cell antigen-binding molecule;

- on the same day as, or 1 day, 2 days, 3 days, or 4 days after administration of the anti-T cell antigen-binding molecule, when pre-administration of the cytokine inhibitor is performed 2 days before the administration of the anti-T cell antigen-binding molecule;
- on the same day as, or 1 day, 2 days, 3 days, 4 days, or 5 days after administration of the T cell antigen-binding molecule, when pre-administration of the cytokine inhibitor is performed 1 day before the administration of the anti-T cell antigen-binding molecule; or
- 1 day, 2 days, 3 days, 4 days, 5 days, or 6 days after administration of the T cell antigen-binding molecule, when pre-administration of the cytokine inhibitor is performed on the same day as the administration of the anti-T cell antigen-binding molecule.

(Repeated administration of the anti-T cell antigen-binding molecule at intervals of once every 2 weeks)

In one embodiment, in the combination therapy of the present disclosure, (i) an anti-T cell antigen-binding molecule is administered repeatedly at intervals of once every 2 weeks, and (ii) a cytokine inhibitor is administered before administration of the anti-T cell antigen-binding molecule, which is 6 days, 5 days, 4 days, 3 days, 2 days, or 1 day before, or on the same day as each administration of the repeated administrations. Alternatively, (i) an anti-T cell antigen-binding molecule is administered repeatedly at intervals of once every 2 weeks, and (ii) a cytokine inhibitor is administered simultaneously with administration of the anti-T cell antigen-binding molecule on the same day as each administration of the repeated administrations.
In a further embodiment, in the combination therapy of the present disclosure, in addition to pre-administration of a cytokine inhibitor, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- on the same day as, or 1 day, 2 days, 3 days, 4 days, 5 days, or 6 days after administration of the anti-T cell antigen-binding molecule (when pre-administration of the cytokine inhibitor is performed 6 days, 5 days, 4 days, 3 days, 2 days, or 1 day before the administration of the anti-T cell antigen-binding molecule); or
- 1 day, 2 days, 3 days, 4 days, 5 days, or 6 days after administration of the anti-T cell antigen-binding molecule (when pre-administration of the cytokine inhibitor is performed on the same day as the administration of the anti-T cell antigen-binding molecule).

(Repeated administration of the anti-T cell antigen-binding molecule at intervals of once every 3 weeks)

In one embodiment, in the combination therapy of the present disclosure, (i) an anti-T cell antigen-binding molecule is administered repeatedly at intervals of once every 3 weeks, and (ii) a cytokine inhibitor is administered before administration of the anti-T cell antigen-binding molecule, which is 6 days, 5 days, 4 days, 3 days, 2 days, or 1 day before, or on the same day as each administration of the repeated administrations. Alternatively, (i) an anti-T cell antigen-binding molecule is administered repeatedly at intervals of once every 3 weeks, and (ii) a cytokine inhibitor is administered simultaneously with administration of the anti-T cell antigen-binding molecule on the same day as each administration of the repeated administrations.
In a further embodiment, in the combination therapy of the present disclosure, in addition to pre-administration of a cytokine inhibitor, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- on the same day as, or 1 day, 2 days, 3 days, 4 days, 5 days, or 6 days after administration of the anti-T cell antigen-binding molecule (when pre-administration of the cytokine inhibitor is performed 6 days, 5 days, 4 days, 3 days, 2 days, or 1 day before the administration of the anti-T cell antigen-binding molecule); or
- 1 day, 2 days, 3 days, 4 days, 5 days, or 6 days after administration of the anti-T cell antigen-binding molecule (when pre-administration of the cytokine inhibitor is performed on the same day as the administration of the anti-T cell antigen-binding molecule).

(Repeated administration of the anti-T cell antigen-binding molecule at intervals of once every 4 weeks)

In one embodiment, in the combination therapy of the present disclosure, (i) an anti-T cell antigen-binding molecule is administered repeatedly at intervals of once every 4 weeks, and (ii) a cytokine inhibitor is administered before administration of the anti-T cell antigen-binding molecule, which is 6 days, 5 days, 4 days, 3 days, 2 days, or 1 day before, or on the same day as each administration of the repeated administrations. Alternatively, (i) an anti-T cell antigen-binding molecule is administered repeatedly at intervals of once every 4 weeks, and (ii) a cytokine inhibitor is administered simultaneously with administration of the anti-T cell antigen-binding molecule on the same day as each administration of the repeated administrations.

In a further embodiment, in the combination therapy of the present disclosure, in addition to pre-administration of a cytokine inhibitor, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- on the same day as, or 1 day, 2 days, 3 days, 4 days, 5 days, or 6 days after administration of the anti-T cell antigen-binding molecule (when pre-administration of the cytokine inhibitor is performed 6 days, 5 days, 4 days, 3 days, 2 days, or 1 day before the administration of the anti-T cell antigen-binding molecule); or
- 1 day, 2 days, 3 days, 4 days, 5 days, or 6 days after administration of the anti-T cell antigen-binding molecule (when pre-administration of the cytokine inhibitor is performed on the same day as the administration of the anti-T cell antigen-binding molecule).

(Repeated administration of the anti-T cell antigen-binding molecule at intervals of once every 5 weeks)

In one embodiment, in the combination therapy of the present disclosure, (i) an anti-T cell antigen-binding molecule is administered repeatedly at intervals of once every 5 weeks, and (ii) a cytokine inhibitor is administered before administration of the anti-T cell antigen-binding molecule, which is 6 days, 5 days, 4 days, 3 days, 2 days, or 1 day before, or on the same day as each administration of the repeated administrations. Alternatively, (i) an anti-T cell antigen-binding molecule is administered repeatedly at intervals of once every 5 weeks, and (ii) a cytokine inhibitor is administered simultaneously with administration of the anti-T cell antigen-binding molecule on the same day as each administration of the repeated administrations.

In a further embodiment, in the combination therapy of the present disclosure, in addition to pre-administration of a cytokine inhibitor, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- on the same day as, or 1 day, 2 days, 3 days, 4 days, 5 days, or 6 days after administration of the anti-T cell antigen-binding molecule (when pre-administration of the cytokine inhibitor is performed 6 days, 5 days, 4 days, 3 days, 2 days, or 1 day before the administration of the anti-T cell antigen-binding molecule); or
- 1 day, 2 days, 3 days, 4 days, 5 days, or 6 days after administration of the anti-T cell antigen-binding molecule (when pre-administration of the cytokine inhibitor is performed on the same day as the administration of the anti-T cell antigen-binding molecule).

When pre-administration of a cytokine inhibitor is performed for the first or multiple administrations of an anti-T cell antigen-binding molecule

(Repeated administration of the anti-T cell antigen-binding molecule at intervals of once every week)

In one embodiment, in the combination therapy of the present disclosure, an anti-T cell antigen-binding molecule is administered repeatedly at intervals of once every week, and pre-administration of a cytokine inhibitor is performed for the first or multiple administrations of the repeated administrations. Specifically, for example, an anti-T cell antigen-binding molecule is administered repeatedly at intervals of once every week, and pre-administration of a cytokine inhibitor is performed according to the following embodiment:

- the cytokine inhibitor is administered before administration of the anti-T cell antigen-binding molecule, which is 6 days before the first administration or each administration of the first to second, the first to third, the first to fourth, or the first to fifth administrations of the repeated administrations;
- the cytokine inhibitor is administered before administration of the anti-T cell antigen-binding molecule, which is 5 days before the first administration or each administration of the first to second, the first to third, the first to fourth, or the first to fifth administrations of the repeated administrations;
- the cytokine inhibitor is administered before administration of the anti-T cell antigen-binding molecule, which is 4 days before the first administration or each administration of the first to second, the first to third, the first to fourth, or the first to fifth administrations of the repeated administrations;

- the cytokine inhibitor is administered before administration of the anti-T cell antigen-binding molecule, which is 3 days before the first administration or each administration of the first to second, the first to third, the first to fourth, or the first to fifth administrations of the repeated administrations;
- the cytokine inhibitor is administered before administration of the anti-T cell antigen-binding molecule, which is 2 days before the first administration or each administration of the first to second, the first to third, the first to fourth, or the first to fifth administrations of the repeated administrations;
- the cytokine inhibitor is administered before administration of the anti-T cell antigen-binding molecule, which is 1 day before the first administration or each administration of the first to second, the first to third, the first to fourth, or the first to fifth administrations of the repeated administrations;
- the cytokine inhibitor is administered before administration of the anti-T cell antigen-binding molecule, on the same day as the first administration or each administration of the first to second, the first to third, the first to fourth, or the first to fifth administrations of the repeated administrations; or
- the cytokine inhibitor is administered simultaneously with administration of the anti-T cell antigen-binding molecule, on the same day as the first administration or each administration of the first to second, the first to third, the first to fourth, or the first to fifth administrations of the repeated administrations.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. Specifically, the cytokine inhibitor may be further administered as follows:

- on the same day as the first administration or each administration of the first to second, the first to third, the first to fourth, or the first to fifth administrations of the anti-T cell antigen-binding molecule (when pre-administration of the cytokine inhibitor is performed 6 days before the said first administration or each administration of the first to second, the first to third, the first to fourth, or the first to fifth administrations of the anti-T cell antigen-binding molecule, respectively);
- on the same day as or 1 day after the first administration or each administration of the first to second, the first to third, the first to fourth, or the first to fifth administrations of the anti-T cell antigen-binding molecule (when pre-administration of the cytokine inhibitor is performed 5 days before the said first administration or each administration of the first to second, the first to third, the first to fourth, or the first to fifth administrations of the anti-T cell antigen-binding molecule, respectively);
- on the same day as, or 1 day or 2 days after the first administration or each administration of the first to second, the first to third, the first to fourth, or the first to fifth administrations of the anti-T cell antigen-binding molecule (when pre-administration of the cytokine inhibitor is performed 4 days before the said first administration or each administration of the first to second, the first to third, the first to fourth, or the first to fifth administrations of the anti-T cell antigen-binding molecule, respectively);
- on the same day as, or 1 day, 2 days, or 3 days after the first administration or each administration of the first to second, the first to third, the first to fourth, or the first to fifth administrations of the anti-T cell antigen-binding molecule (when pre-administration of the cytokine inhibitor is performed 3 days before the said first administration or each administration of the first to second, the first to third, the first to fourth, or the first to fifth administrations of the anti-T cell antigen-binding molecule, respectively);
- on the same day as, or 1 day, 2 days, 3 days, or 4 days after the first administration or each administration of the first to second, the first to third, the first to fourth, or the first to fifth administrations of the anti-T cell antigen-binding molecule (when pre-administration of the cytokine inhibitor is performed 2 days before the said first administration or each administration of the first to second, the first to third, the first to fourth, or the first to fifth administrations of the anti-T cell antigen-binding molecule, respectively);
- on the same day as, or 1 day, 2 days, 3 days, 4 days, or 5 days after the first administration or each administration of the first to second, the first to third, the first to fourth, or the first to fifth administrations of the anti-T cell antigen-binding molecule (when pre-administration of the cytokine inhibitor is performed 1 day before the said first administration or each administration of the first to second, the first to third, the first to fourth, or the first to fifth administrations of the anti-T cell antigen-binding molecule, respectively); or
- 1 day, 2 days, 3 days, 4 days, 5 days, or 6 days after the first administration or each administration of the first to second, the first to third, the first to fourth, or the first to fifth administrations of the anti-T cell antigen-binding molecule (when pre-administration of the cytokine inhibitor is performed on the same day as the said first administration or each administration of the first to second, the first to third, the first to fourth, or the first to fifth administrations of the anti-T cell antigen-binding molecule, respectively).

(Repeated administration of the anti-T cell antigen-binding molecule at intervals of once every 2 weeks)

In one embodiment, in the combination therapy of the present disclosure, an anti-T cell antigen-binding molecule is administered repeatedly at intervals of once every 2 weeks, and pre-administration of a cytokine inhibitor is performed for the first or multiple administrations of the repeated administrations. Specifically, an anti-T cell antigen-binding molecule is administered repeatedly at intervals of once every 2 weeks, and pre-administration of a cytokine inhibitor is performed according to the following embodiment:

- the cytokine inhibitor is administered before administration of the anti-T cell antigen-binding molecule, which is 6 days before the first administration or each administration of the first to second, the first to third, the first to fourth, or the first to fifth administrations of the repeated administrations;
- the cytokine inhibitor is administered before administration of the anti-T cell antigen-binding molecule, which is 5 days before the first administration or each administration of the first to second, the first to third, the first to fourth, or the first to fifth administrations of the repeated administrations;
- the cytokine inhibitor is administered before administration of the anti-T cell antigen-binding molecule, which is 4 days before the first administration or each administration of the first to second, the first to third, the first to fourth, or the first to fifth administrations of the repeated administrations;
- the cytokine inhibitor is administered before administration of the anti-T cell antigen-binding molecule, which is 3 days before the first administration or each administration of the first to second, the first to third, the first to fourth, or the first to fifth administrations of the repeated administrations;
- the cytokine inhibitor is administered before administration of the anti-T cell antigen-binding molecule, which is 2 days before the first administration or each administration of the first to second, the first to third, the first to fourth, or the first to fifth administrations of the repeated administrations;
- the cytokine inhibitor is administered before administration of the anti-T cell antigen-binding molecule, which is 1 day before the first administration or each administration of the first to second, the first to third, the first to fourth, or the first to fifth administrations of the repeated administrations;
- the cytokine inhibitor is administered before administration of the anti-T cell antigen-binding molecule, on the same day as the first administration or each administration of the first to second, the first to third, the first to fourth, or the first to fifth administrations of the repeated administrations; or
- the cytokine inhibitor is administered simultaneously with administration of the anti-T cell antigen-binding molecule, on the same day as the first administration or each of the first to second, the first to third, the first to fourth, or the first to fifth administrations of the repeated administrations.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. Specifically, the cytokine inhibitor may be further administered as follows:

- on the same day as, or 1 day, 2 days, 3 days, 4 days, 5 days, or 6 days after the first administration or each administration of the first to second, the first to third, the first to fourth, or the first to fifth administrations of the anti-T cell antigen-binding molecule (when pre-administration of the cytokine inhibitor is performed 6 days, 5 days, 4 days, 3 days, 2 days, or 1 day before the said first administration or each of the first to second, the first to third, the first to fourth, or the first to fifth administrations of the anti-T cell antigen-binding molecule); or
- 1 day, 2 days, 3 days, 4 days, 5 days, or 6 days after the first administration or each administration of the first to second, the first to third, the first to fourth, or the first to fifth administrations of the anti-T cell antigen-binding molecule (when pre-administration of the cytokine inhibitor is performed on the same day as the said first administration or each administration of the first to second, the first to third, the first to fourth, or the first to fifth administrations of the anti-T cell antigen-binding molecule).

(Repeated administration of the anti-T cell antigen-binding molecule at intervals of once every 3 weeks)

In one embodiment, in the combination therapy of the present disclosure, an anti-T cell antigen-binding molecule is administered repeatedly at intervals of once every 3 weeks, and pre-administration of a cytokine inhibitor is performed for the first or multiple administrations of the repeated administrations. Specifically, an anti-T cell antigen-binding molecule is administered repeatedly at intervals of once every 3 weeks, and pre-administration of a cytokine inhibitor is performed according to the following embodiment:

- the cytokine inhibitor is administered before administration of the anti-T cell antigen-binding molecule, which is 6 days before the first administration- or each administration of the first to second, the first to third, the first to fourth, or the first to fifth administrations of the repeated administrations;

- the cytokine inhibitor is administered before administration of the anti-T cell antigen-binding molecule, which is 5 days before the first administration or each administration of the first to second, the first to third, the first to fourth, or the first to fifth administrations of the repeated administrations;

- the cytokine inhibitor is administered before administration of the anti-T cell antigen-binding molecule, which is 4 days before the first administration or each administration of the first to second, the first to third, the first to fourth, or the first to fifth administrations of the repeated administrations;
- the cytokine inhibitor is administered before administration of the anti-T cell antigen-binding molecule, which is 3 days before the first administration or each administration of the first to second, the first to third, the first to fourth, or the first to fifth administrations of the repeated administrations;
- the cytokine inhibitor is administered before administration of the anti-T cell antigen-binding molecule, which is 2 days before the first administration or each administration of the first to second, the first to third, the first to fourth, or the first to fifth administrations of the repeated administrations;
- the cytokine inhibitor is administered before administration of the anti-T cell antigen-binding molecule, which is 1 day before the first administration or each administration of the first to second, the first to third, the first to fourth, or the first to fifth administrations of the repeated administrations;
- the cytokine inhibitor is administered before administration of the anti-T cell antigen-binding molecule, on the same day as the first administration or each administration of the first to second, the first to third, the first to fourth, or the first to fifth administrations of the repeated administrations; or
- the cytokine inhibitor is administered simultaneously with administration of the anti-T cell antigen-binding molecule, on the same day as the first administration or each administration of the first to second, the first to third, the first to fourth, or the first to fifth administrations of the repeated administrations.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. Specifically, the cytokine inhibitor may be further administered as follows:

- on the same day as, or 1 day, 2 days, 3 days, 4 days, 5 days, or 6 days after the first administration or each administration of the first to second, the first to third, the first to fourth, or the first to fifth administrations of the anti-T cell antigen-binding molecule (when pre-administration of the cytokine inhibitor is performed 6 days, 5 days, 4 days, 3 days, 2 days, or 1 day before the said first administration or each administration of the first to second, the first to third, the first to fourth, or the first to fifth administrations of the anti-T cell antigen-binding molecule); or
- 1 day, 2 days, 3 days, 4 days, 5 days, or 6 days after the first administration or each administration of the first to second, the first to third, the first to fourth, or the first to fifth administrations of the anti-T cell antigen-binding molecule (when pre-administration of the cytokine inhibitor is performed on the same day as the said first administration or each administration of the first to second, the first to third, the first to fourth, or the first to fifth administrations of the anti-T cell antigen-binding molecule).

(Repeated administration of the anti-T cell antigen-binding molecule at intervals of once every 4 weeks)

In one embodiment, in the combination therapy of the present disclosure, an anti-T cell antigen-binding molecule is administered repeatedly at intervals of once every 4 weeks, and pre-administration of a cytokine inhibitor is performed for the first or multiple administrations of the repeated administrations. Specifically, an anti-T cell antigen-binding molecule is administered repeatedly at intervals of once every 4 weeks, and pre-administration of a cytokine inhibitor is performed according to the following embodiment:

- the cytokine inhibitor is administered before administration of the anti-T cell antigen-binding molecule, which is 6 days before the first administration or each administration of the first to second, the first to third, the first to fourth, or the first to fifth administrations of the repeated administrations;
- the cytokine inhibitor is administered before administration of the anti-T cell antigen-binding molecule, which is 5 days before the first administration or each administration of the first to second, the first to third, the first to fourth, or the first to fifth administrations of the repeated administrations;
- the cytokine inhibitor is administered before administration of the anti-T cell antigen-binding molecule, which is 4 days before the first administration or each administration of the first to second, the first to third, the first to fourth, or the first to fifth administrations of the repeated administrations;

- the cytokine inhibitor is administered before administration of the anti-T cell antigen-binding molecule, which is 3 days before the first administration or each administration of the first to second, the first to third, the first to fourth, or the first to fifth administrations of the repeated administrations;
- the cytokine inhibitor is administered before administration of the anti-T cell antigen-binding molecule, which is 2 days before the first administration or each administration of the first to second, the first to third, the first to fourth, or the first to fifth administrations of the repeated administrations;
- the cytokine inhibitor is administered before administration of the anti-T cell antigen-binding molecule, which is 1 day before the first administration or each administration of the first to second, the first to third, the first to fourth, or the first to fifth administrations of the repeated administrations;
- the cytokine inhibitor is administered before administration of the anti-T cell antigen-binding molecule, on the same day as the first administration or each administration of the first to second, the first to third, the first to fourth, or the first to fifth administrations of the repeated administrations; or
- the cytokine inhibitor is administered simultaneously with administration of the anti-T cell antigen-binding molecule, on the same day as the first administration or each administration of the first to second, the first to third, the first to fourth, or the first to fifth administrations of the repeated administrations.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. Specifically, the cytokine inhibitor may be further administered as follows:

- on the same day as, or 1 day, 2 days, 3 days, 4 days, 5 days, or 6 days after the first administration or each administration of the first to second, the first to third, the first to fourth, or the first to fifth administrations of the anti-T cell antigen-binding molecule (when pre-administration of the cytokine inhibitor is performed 6 days, 5 days, 4 days, 3 days, 2 days, or 1 day before the said first administration or each administration of the first to second, the first to third, the first to fourth, or the first to fifth administrations of the anti-T cell antigen-binding molecule); or
- 1 day, 2 days, 3 days, 4 days, 5 days, or 6 days after the first administration or each administration of the first to second, the first to third, the first to fourth, or the first to fifth administrations of the anti-T cell antigen-binding molecule (when pre-administration of the cytokine inhibitor is performed on the same day as the said first administration or each of the first to second, the first to third, the first to fourth, or the first to fifth administrations of the anti-T cell antigen-binding molecule).

(Repeated administration of the anti-T cell antigen-binding molecule at intervals of once every 5 weeks)

In one embodiment, in the combination therapy of the present disclosure, an anti-T cell antigen-binding molecule is administered repeatedly at intervals of once every 5 weeks, and pre-administration of a cytokine inhibitor is performed for the first or multiple administrations of the repeated administrations. Specifically, an anti-T cell antigen-binding molecule is administered repeatedly at intervals of once every 5 weeks, and pre-administration of a cytokine inhibitor is performed according to the following embodiment:

- the cytokine inhibitor is administered before administration of the anti-T cell antigen-binding molecule, which is 6 days before the first administration or each administration of the first to second, the first to third, the first to fourth, or the first to fifth administrations of the repeated administrations;
- the cytokine inhibitor is administered before administration of the anti-T cell antigen-binding molecule, which is 5 days before the first administration or each administration of the first to second, the first to third, the first to fourth, or the first to fifth administrations of the repeated administrations;
- the cytokine inhibitor is administered before administration of the anti-T cell antigen-binding molecule, which is 4 days before the first administration or each administration of the first to second, the first to third, the first to fourth, or the first to fifth administrations of the repeated administrations;
- the cytokine inhibitor is administered before administration of the anti-T cell antigen-binding molecule, which is 3 days before the first administration or each administration of the first to second, the first to third, the first to fourth, or the first to fifth administrations of the repeated administrations;
- the cytokine inhibitor is administered before administration of the anti-T cell antigen-binding molecule, which is 2 days before the first administration or each administration of the first to second, the first to third, the first to fourth, or the first to fifth administrations of the repeated administrations;
- the cytokine inhibitor is administered before administration of the anti-T cell antigen-binding molecule, which is 1 day before the first administration or each administration of the first to second, the first to third, the first to fourth, or the first to fifth administrations of the repeated administrations;

- the cytokine inhibitor is administered before administration of the anti-T cell antigen-binding molecule, on the same day as the first administration or each administration of the first to second, the first to third, the first to fourth, or the first to fifth administrations of the repeated administrations; or
- the cytokine inhibitor is administered simultaneously with administration of the anti-T cell antigen-binding molecule, on the same day as the first administration or each administration of the first to second, the first to third, the first to fourth, or the first to fifth administrations of the repeated administrations.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. Specifically, the cytokine inhibitor may be further administered as follows:

- on the same day as, or 1 day, 2 days, 3 days, 4 days, 5 days, or 6 days after the first administration or each administration of the first to second, the first to third, the first to fourth, or the first to fifth administrations of the anti-T cell antigen-binding molecule (when pre-administration of the cytokine inhibitor is performed 6 days, 5 days, 4 days, 3 days, 2 days, or 1 day before the said first administration or each administration of the first to second, the first to third, the first to fourth, or the first to fifth administrations of the anti-T cell antigen-binding molecule); or
- 1 day, 2 days, 3 days, 4 days, 5 days, or 6 days after the first administration or each administration of the first to second, the first to third, the first to fourth, or the first to fifth administrations of the anti-T cell antigen-binding molecule (when pre-administration of the cytokine inhibitor is performed on the same day as the said first administration or each administration of the first to second, the first to third, the first to fourth, or the first to fifth administrations of the anti-T cell antigen-binding molecule).

When pre-administration of a cytokine inhibitor is performed in every dosing cycle of an anti-T cell antigen-binding molecule

(Repeated administration of the anti-T cell antigen-binding molecule at intervals of once every week)

In one embodiment, in the combination therapy of the present disclosure, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every week, and (iii) pre-administration of a cytokine inhibitor is performed for the first administration of the anti-T cell antigen-binding molecule in each of the dosing cycles. As specific examples, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every week, and (iii) pre-administration of a cytokine inhibitor is performed according to the following embodiment:

- the cytokine inhibitor is administered before administration of the anti-T cell antigen-binding molecule, which is 6 days before the first administration in each of the dosing cycles;
- the cytokine inhibitor is administered before administration of the anti-T cell antigen-binding molecule, which is 5 days before the first administration in each of the dosing cycles;
- the cytokine inhibitor is administered before administration of the anti-T cell antigen-binding molecule, which is 4 days before the first administration in each of the dosing cycles;
- the cytokine inhibitor is administered before administration of the anti-T cell antigen-binding molecule, which is 3 days before the first administration in each of the dosing cycles;
- the cytokine inhibitor is administered before administration of the anti-T cell antigen-binding molecule, which is 2 days before the first administration in each of the dosing cycles;
- the cytokine inhibitor is administered before administration of the anti-T cell antigen-binding molecule, which is 1 day before the first administration in each of the dosing cycles;
- the cytokine inhibitor is administered before administration of the anti-T cell antigen-binding molecule, on the same day as the first administration in each of the dosing cycles; or
- the cytokine inhibitor is administered simultaneously with administration of the anti-T cell antigen-binding molecule, on the same day as the first administration in each of the dosing cycles.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- on the same day as, or 1 day, 2 days, 3 days, 4 days, 5 days, or 6 days after the first administration of the anti-T cell antigen-binding molecule in each of the dosing cycles (when the anti-T cell antigen-binding molecule is administered repeatedly in dosing cycles where 2, 3, 4, or 5 administrations constitute one cycle, and pre-administration of the cytokine inhibitor is performed 6 days, 5 days, 4 days, 3 days, 2 days, or 1 day before the said first administration of the anti-T cell antigen-binding molecule in each of the dosing cycles); or
- 1 day, 2 days, 3 days, 4 days, 5 days, or 6 days after the first administration of the anti-T cell antigen-binding molecule in each of the dosing cycles (when the anti-T cell antigen-binding molecule is administered repeatedly in dosing cycles where 2, 3, 4, or 5 administrations constitute one cycle, and pre-administration of the cytokine inhibitor is performed on the same day as the said first administration of the anti-T cell antigen-binding molecule in each of the dosing cycles).

In another embodiment, in the combination therapy of the present disclosure, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every week, and (iii) pre-administration of a cytokine inhibitor is performed (a) for each (every) administration of the anti-T cell antigen-binding molecule in the first dosing cycle, and (b) for the first administration in each of the second and subsequent dosing cycles. As specific examples, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every week, and (iii) pre-administration of a cytokine inhibitor is performed according to the following embodiment:

- the cytokine inhibitor is administered before administration of the anti-T cell antigen-binding molecule, which is (a) 6 days before each (every) administration of the anti-T cell antigen-binding molecule in the first dosing cycle, and (b) 6 days before the first administration in each of the second and subsequent dosing cycles;
- the cytokine inhibitor is administered before administration of the anti-T cell antigen-binding molecule, which is (a) 5 days before each (every) administration of the anti-T cell antigen-binding molecule in the first dosing cycle, and (b) 5 days before the first administration in each of the second and subsequent dosing cycles;
- the cytokine inhibitor is administered before administration of the anti-T cell antigen-binding molecule, which is (a) 4 days before each (every) administration of the anti-T cell antigen-binding molecule in the first dosing cycle, and (b) 4 days before the first administration in each of the second and subsequent dosing cycles;
- the cytokine inhibitor is administered before administration of the anti-T cell antigen-binding molecule, which is (a) 3 days before each (every) administration of the anti-T cell antigen-binding molecule in the first dosing cycle, and (b) 3 days before the first administration in each of the second and subsequent dosing cycles;
- the cytokine inhibitor is administered before administration of the anti-T cell antigen-binding molecule, which is (a) 2 days before each (every) administration of the anti-T cell antigen-binding molecule in the first dosing cycle, and (b) 2 days before the first administration in each of the second and subsequent dosing cycles;
- the cytokine inhibitor is administered before administration of the anti-T cell antigen-binding molecule, which is (a) 1 day before each (every) administration of the anti-T cell antigen-binding molecule in the first dosing cycle, and (b) 1 day before the first administration in each of the second and subsequent dosing cycles;
- the cytokine inhibitor is administered before administration of the anti-T cell antigen-binding molecule (a) on the same day as each (every) administration of the anti-T cell antigen-binding molecule in the first dosing cycle, and (b) on the same day as the first administration in each of the second and subsequent dosing cycles; or - the cytokine inhibitor is administered simultaneously with administration of the anti-T cell antigen-binding molecule (a) on the same day as each (every) administration of the anti-T cell antigen-binding molecule in the first dosing cycle, and (b) on the same day as the first administration in each of the second and subsequent dosing cycles.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- (a) on the same day as each (every) administration of the anti-T cell antigen-binding molecule in the first dosing cycle, and (b) on the same day as the first administration in each of the second and subsequent dosing cycles (when the anti-T cell antigen-binding molecule is administered repeatedly in dosing cycles where 2, 3, 4, or 5 administrations constitute one cycle, and pre-administration of the cytokine inhibitor is performed (a) 6 days before said each (every) administration of the anti-T cell antigen-binding molecule in the first dosing cycle, and (b) 6 days before the said first administration in each of the second and subsequent dosing cycles);
- (a) on the same day as or 1 day after each (every) administration of the anti-T cell antigen-binding molecule in the first dosing cycle, and (b) on the same day as or 1 day after the first administration in each of the second and subsequent dosing cycles (when the anti-T cell antigen-binding molecule is administered repeatedly in dosing cycles where 2, 3, 4, or 5 administrations constitute one cycle, and pre-administration of the cytokine inhibitor is performed (a) 5 days before said each (every) administration of the anti-T cell antigen-binding molecule in the first dosing cycle, and (b) 5 days before the said first administration in each of the second and subsequent dosing cycles);

- (a) on the same day as, or 1 day or 2 days after each (every) administration of the anti-T cell antigen-binding molecule in the first dosing cycle, and (b) on the same day as, or 1 day or 2 days after the first administration in each of the second and subsequent dosing cycles (when the anti-T cell antigen-binding molecule is administered repeatedly in dosing cycles where 2, 3, 4, or 5 administrations constitute one cycle, and pre-administration of the cytokine inhibitor is performed (a) 4 days before said each (every) administration of the anti-T cell antigen-binding molecule in the first dosing cycle, and (b) 4 days before the said first administration in each of the second and subsequent dosing cycles);

- (a) on the same day as, or 1 day, 2 days, or 3 days after each (every) administration of the anti-T cell antigen-binding molecule in the first dosing cycle, and (b) on the same day as, or 1 day, 2 days, or 3 days after the first administration in each of the second and subsequent dosing cycles (when the anti-T cell antigen-binding molecule is administered repeatedly in dosing cycles where 2, 3, 4, or 5 administrations constitute one cycle, and pre-administration of the cytokine inhibitor is performed (a) 3 days before said each (every) administration of the anti-T cell antigen-binding molecule in the first dosing cycle, and (b) 3 days before the said first administration in each of the second and subsequent dosing cycles);

- (a) on the same day as, or 1 day, 2 days, 3 days, or 4 days after each (every) administration of the anti-T cell antigen-binding molecule in the first dosing cycle, and (b) on the same day as, or 1 day, 2 days, 3 days, or 4 days after the first administration in each of the second and subsequent dosing cycles (when the anti-T cell antigen-binding molecule is administered repeatedly in dosing cycles where 2, 3, 4, or 5 administrations constitute one cycle, and pre-administration of the cytokine inhibitor is performed (a) 2 days before said each (every) administration of the anti-T cell antigen-binding molecule in the first dosing cycle, and (b) 2 days before the said first administration in each of the second and subsequent dosing cycles);

- (a) on the same day as, or 1 day, 2 days, 3 days, 4 days, or 5 days after each (every) administration of the anti-T cell antigen-binding molecule in the first dosing cycle, and (b) on the same day as, or 1 day, 2 days, 3 days, 4 days, or 5 days after the first administration in each of the second and subsequent dosing cycles (when the anti-T cell antigen-binding molecule is administered repeatedly in dosing cycles where 2, 3, 4, or 5 administrations constitute one cycle, and pre-administration of the cytokine inhibitor is performed (a) 1 day before said each (every) administration of the anti-T cell antigen-binding molecule in the first dosing cycle, and (b) 1 day before the said first administration in each of the second and subsequent dosing cycles); or

- (a) 1 day, 2 days, 3 days, 4 days, 5 days, or 6 days after each (every) administration of the anti-T cell antigen-binding molecule in the first dosing cycle, and (b) 1 day, 2 days, 3 days, 4 days, 5 days, or 6 days after the first administration in each of the second and subsequent dosing cycles (when the anti-T cell antigen-binding molecule is administered repeatedly in dosing cycles where 2, 3, 4, or 5 administrations constitute one cycle, and pre-administration of the cytokine inhibitor is performed (a) on the same day as said each (every) administration of the anti-T cell antigen-binding molecule in the first dosing cycle, and (b) on the same day as the said first administration in each of the second and subsequent dosing cycles).

(Repeated administration of the anti-T cell antigen-binding molecule at intervals of once every 2 weeks)

In one embodiment, in the combination therapy of the present disclosure, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 2 weeks, and (iii) pre-administration of a cytokine inhibitor is performed for the first administration of the anti-T cell antigen-binding molecule in each of the dosing cycles. As specific examples, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 2 weeks, and (iii) pre-administration of a cytokine inhibitor is performed according to the following embodiment:

- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 6 days before the first administration in each of the dosing cycles;

- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 5 days before the first administration in each of the dosing cycles;

- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 4 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 3 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 2 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 1 day before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, on the same day as the first administration in each of the dosing cycles; or
- the anti-T cell antigen-binding molecule is administered repeatedly in dosing cycles where 2, 3, 4, or 5 administrations constitute one cycle, and the cytokine inhibitor is administered simultaneously with administration of the anti-T cell antigen-binding molecule, on the same day as the first administration in each of the dosing cycles.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- on the same day as, or 1 day, 2 days, 3 days, 4 days, 5 days, or 6 days after the first administration of the anti-T cell antigen-binding molecule in each of the dosing cycles (when the anti-T cell antigen-binding molecule is administered repeatedly at intervals of once every 2 weeks, in dosing cycles where 2, 3, 4, or 5 administrations constitute one cycle, and pre-administration of the cytokine inhibitor is performed 6 days, 5 days, 4 days, 3 days, 2 days, or 1 day before the said first administration of the anti-T cell antigen-binding molecule in each of the dosing cycles); or
- 1 day, 2 days, 3 days, 4 days, 5 days, or 6 days after the first administration of the anti-T cell antigen-binding molecule in each of the dosing cycles (when the anti-T cell antigen-binding molecule is administered repeatedly at intervals of once every 2 weeks, in dosing cycles where 2, 3, 4, or 5 administrations constitute one cycle, and pre-administration of the cytokine inhibitor is performed on the same day as the said first administration of the anti-T cell antigen-binding molecule in each of the dosing cycles).

In another embodiment, in the combination therapy of the present disclosure, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 2 weeks, and (iii) pre-administration of a cytokine inhibitor is performed (a) for each (every) administration of the anti-T cell antigen-binding molecule in the first dosing cycle, and (b) for the first administration in each of the second and subsequent dosing cycles. As specific examples, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 2 weeks, and (iii) pre-administration of a cytokine inhibitor is performed according to the following embodiment:

- the cytokine inhibitor is administered before administration of the anti-T cell antigen-binding molecule, which is (a) 6 days before each (every) administration of the anti-T cell antigen-binding molecule in the first dosing cycle, and (b) 6 days before the first administration in each of the second and subsequent dosing cycles;
- the cytokine inhibitor is administered before administration of the anti-T cell antigen-binding molecule, which is (a) 5 days before each (every) administration of the anti-T cell antigen-binding molecule in the first dosing cycle, and (b) 5 days before the first administration in each of the second and subsequent dosing cycles;
- the cytokine inhibitor is administered before administration of the anti-T cell antigen-binding molecule, which is (a) 4 days before each (every) administration of the anti-T cell antigen-binding molecule in the first dosing cycle, and (b) 4 days before the first administration in each of the second and subsequent dosing cycles;
- the cytokine inhibitor is administered before administration of the anti-T cell antigen-binding molecule, which is (a) 3 days before each (every) administration of the anti-T cell antigen-binding molecule in the first dosing cycle, and (b) 3 days before the first administration in each of the second and subsequent dosing cycles;
- the cytokine inhibitor is administered before administration of the anti-T cell antigen-binding molecule, which is (a) 2 days before each (every) administration of the anti-T cell antigen-binding molecule in the first dosing cycle, and (b) 2 days before the first administration in each of the second and subsequent dosing cycles;

- the cytokine inhibitor is administered before administration of the anti-T cell antigen-binding molecule, which is (a) 1 day before each (every) administration of the anti-T cell antigen-binding molecule in the first dosing cycle, and (b) 1 day before the first administration in each of the second and subsequent dosing cycles;
- the cytokine inhibitor is administered before administration of the anti-T cell antigen-binding molecule (a) on the same day as each (every) administration of the anti-T cell antigen-binding molecule in the first dosing cycle, and (b) on the same day as the first administration in each of the second and subsequent dosing cycles; or
- the cytokine inhibitor is administered simultaneously with administration of the anti-T cell antigen-binding molecule (a) on the same day as each (every) administration of the anti-T cell antigen-binding molecule in the first dosing cycle, and (b) on the same day as the first administration in each of the second and subsequent dosing cycles.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- (a) on the same day as, or 1 day, 2 days, 3 days, 4 days, 5 days, or 6 days after each (every) administration of the anti-T cell antigen-binding molecule in the first dosing cycle, and (b) on the same day as, or 1 day, 2 days, 3 days, 4 days, 5 days, or 6 days after the first administration in each of the second and subsequent dosing cycles (when the anti-T cell antigen-binding molecule is administered repeatedly at intervals of once every 2 weeks, in dosing cycles where 2, 3, 4, or 5 administrations constitute one cycle, and pre-administration of the cytokine inhibitor is performed (a) 6 days, 5 days, 4 days, 3 days, 2 days, or 1 day before said each (every) administration of the anti-T cell antigen-binding molecule in the first dosing cycle, and (b) 6 days, 5 days, 4 days, 3 days, 2 days, or 1 day before the said first administration in each of the second and subsequent dosing cycles); or
- (a) 1 day, 2 days, 3 days, 4 days, 5 days, or 6 days after each (every) administration of the anti-T cell antigen-binding molecule in the first dosing cycle, and (b) 1 day, 2 days, 3 days, 4 days, 5 days, or 6 days after the first administration in each of the second and subsequent dosing cycles (when the anti-T cell antigen-binding molecule is administered repeatedly at intervals of once every 2 weeks, in dosing cycles where 2, 3, 4, or 5 administrations constitute one cycle, and pre-administration of the cytokine inhibitor is performed (a) on the same day as said each (every) administration of the anti-T cell antigen-binding molecule in the first dosing cycle, and (b) on the same day as the said first administration in each of the second and subsequent dosing cycles).

(Repeated administration of the anti-T cell antigen-binding molecule at intervals of once every 3 weeks)

In one embodiment, in the combination therapy of the present disclosure, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 3 weeks, and (iii) pre-administration of a cytokine inhibitor is performed for the first administration of the anti-T cell antigen-binding molecule in each of the dosing cycles. As specific examples, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 3 weeks, and (iii) pre-administration of a cytokine inhibitor is performed according to the following embodiment:

- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 6 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 5 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 4 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 3 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 2 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 1 day before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, on the same day as the first administration in each of the dosing cycles; or
- the anti-T cell antigen-binding molecule is administered repeatedly in dosing cycles where 2, 3, 4, or 5 administrations constitute one cycle, and the cytokine inhibitor is administered simultaneously with administration of the anti-T cell antigen-binding molecule, on the same day as the first administration in each of the dosing cycles.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- on the same day as, or 1 day, 2 days, 3 days, 4 days, 5 days, or 6 days after the first administration of the anti-T cell antigen-binding molecule in each of the dosing cycles (when the anti-T cell antigen-binding molecule is administered repeatedly at intervals of once every 3 weeks, in dosing cycles where 2, 3, 4, or 5 administrations constitute one cycle, and pre-administration of the cytokine inhibitor is performed 6 days, 5 days, 4 days, 3 days, 2 days, or 1 day before the said first administration of the anti-T cell antigen-binding molecule in each of the dosing cycles); or
- 1 day, 2 days, 3 days, 4 days, 5 days, or 6 days after the first administration of the anti-T cell antigen-binding molecule in each of the dosing cycles (when the anti-T cell antigen-binding molecule is administered repeatedly at intervals of once every 3 weeks, in dosing cycles where 2, 3, 4, or 5 administrations constitute one cycle, and pre-administration of the cytokine inhibitor is performed on the same day as the said first administration of the anti-T cell antigen-binding molecule in each of the dosing cycles).

In another embodiment, in the combination therapy of the present disclosure, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 3 weeks, and (iii) pre-administration of a cytokine inhibitor is performed (a) for each (every) administration of the anti-T cell antigen-binding molecule in the first dosing cycle, and (b) for the first administration in each of the second and subsequent dosing cycles. As specific examples, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 3 weeks, and (iii) pre-administration of a cytokine inhibitor is performed according to the following embodiment:

- the cytokine inhibitor is administered before administration of the anti-T cell antigen-binding molecule, which is (a) 6 days before each (every) administration of the anti-T cell antigen-binding molecule in the first dosing cycle, and (b) 6 days before the first administration in each of the second and subsequent dosing cycles;
- the cytokine inhibitor is administered before administration of the anti-T cell antigen-binding molecule, which is (a) 5 days before each (every) administration of the anti-T cell antigen-binding molecule in the first dosing cycle, and (b) 5 days before the first administration in each of the second and subsequent dosing cycles;
- the cytokine inhibitor is administered before administration of the anti-T cell antigen-binding molecule, which is (a) 4 days before each (every) administration of the anti-T cell antigen-binding molecule in the first dosing cycle, and (b) 4 days before the first administration in each of the second and subsequent dosing cycles;
- the cytokine inhibitor is administered before administration of the anti-T cell antigen-binding molecule, which is (a) 3 days before each (every) administration of the anti-T cell antigen-binding molecule in the first dosing cycle, and (b) 3 days before the first administration in each of the second and subsequent dosing cycles;
- the cytokine inhibitor is administered before administration of the anti-T cell antigen-binding molecule, which is (a) 2 days before each (every) administration of the anti-T cell antigen-binding molecule in the first dosing cycle, and (b) 2 days before the first administration in each of the second and subsequent dosing cycles;
- the cytokine inhibitor is administered before administration of the anti-T cell antigen-binding molecule, which is (a) 1 day before each (every) administration of the anti-T cell antigen-binding molecule in the first dosing cycle, and (b) 1 day before the first administration in each of the second and subsequent dosing cycles;
- the cytokine inhibitor is administered before administration of the anti-T cell antigen-binding molecule (a) on the same day as each (every) administration of the anti-T cell antigen-binding molecule in the first dosing cycle, and (b) on the same day as the first administration in each of the second and subsequent dosing cycles; or

- the cytokine inhibitor is administered simultaneously with administration of the anti-T cell antigen-binding molecule (a) on the same day as each (every) administration of the anti-T cell antigen-binding molecule in the first dosing cycle, and (b) on the same day as the first administration in each of the second and subsequent dosing cycles.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- (a) on the same day as, or 1 day, 2 days, 3 days, 4 days, 5 days, or 6 days after each (every) administration of the anti-T cell antigen-binding molecule in the first dosing cycle, and (b) on the same day as, or 1 day, 2 days, 3 days, 4 days, 5 days, or 6 days after the first administration in each of the second and subsequent dosing cycles (when the anti-T cell antigen-binding molecule is administered repeatedly at intervals of once every 3 weeks, in dosing cycles where 2, 3, 4, or 5 administrations constitute one cycle, and pre-administration of the cytokine inhibitor is performed (a) 6 days, 5 days, 4 days, 3 days, 2 days, or 1 day before said each (every) administration of the anti-T cell antigen-binding molecule in the first dosing cycle, and (b) 6 days, 5 days, 4 days, 3 days, 2 days, or 1 day before the said first administration in each of the second and subsequent dosing cycles); or
- (a) 1 day, 2 days, 3 days, 4 days, 5 days, or 6 days after each (every) administration of the anti-T cell antigen-binding molecule in the first dosing cycle, and (b) 1 day, 2 days, 3 days, 4 days, 5 days, or 6 days after the first administration in each of the second and subsequent dosing cycles (when the anti-T cell antigen-binding molecule is administered repeatedly at intervals of once every 3 weeks, in dosing cycles where 2, 3, 4, or 5 administrations constitute one cycle, and pre-administration of the cytokine inhibitor is performed (a) on the same day as said each (every) administration of the anti-T cell antigen-binding molecule in the first dosing cycle, and (b) on the same day as the said first administration in each of the second and subsequent dosing cycles).

(Repeated administration of the anti-T cell antigen-binding molecule at intervals of once every 4 weeks)

In one embodiment, in the combination therapy of the present disclosure, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 4 weeks, and (iii) pre-administration of a cytokine inhibitor is performed for the first administration of the anti-T cell antigen-binding molecule in each of the dosing cycles. As specific examples, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 4 weeks, and (iii) pre-administration of a cytokine inhibitor is performed according to the following embodiment:

- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 6 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 5 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 4 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 3 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 2 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 1 day before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, on the same day as the first administration in each of the dosing cycles; or
- the anti-T cell antigen-binding molecule is administered repeatedly in dosing cycles where 2, 3, 4, or 5 administrations constitute one cycle, and the cytokine inhibitor is administered simultaneously with administration of the anti-T cell antigen-binding molecule, on the same day as the first administration in each of the dosing cycles.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- on the same day as, or 1 day, 2 days, 3 days, 4 days, 5 days, or 6 days after the first administration of the anti-T cell antigen-binding molecule in each of the dosing cycles (when the anti-T cell antigen-binding molecule is administered repeatedly at intervals of once every 4 weeks, in dosing cycles where 2, 3, 4, or 5 administrations constitute one cycle, and pre-administration of the cytokine inhibitor is performed 6 days, 5 days, 4 days, 3 days, 2 days, or 1 day before the said first administration of the anti-T cell antigen-binding molecule in each of the dosing cycles); or
- 1 day, 2 days, 3 days, 4 days, 5 days, or 6 days after the first administration of the anti-T cell antigen-binding molecule in each of the dosing cycles (when the anti-T cell antigen-binding molecule is administered repeatedly at intervals of once every 4 weeks, in dosing cycles where 2, 3, 4, or 5 administrations constitute one cycle, and pre-administration of the cytokine inhibitor is performed on the same day as the said first administration of the anti-T cell antigen-binding molecule in each of the dosing cycles).

In another embodiment, in the combination therapy of the present disclosure, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 4 weeks, and (iii) pre-administration of a cytokine inhibitor is performed (a) for each (every) administration of the anti-T cell antigen-binding molecule in the first dosing cycle, and (b) for the first administration in each of the second and subsequent dosing cycles. As specific examples, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 4 weeks, and (iii) pre-administration of a cytokine inhibitor is performed according to the following embodiment:

- the cytokine inhibitor is administered before administration of the anti-T cell antigen-binding molecule, which is (a) 6 days before each (every) administration of the anti-T cell antigen-binding molecule in the first dosing cycle, and (b) 6 days before the first administration in each of the second and subsequent dosing cycles;
- the cytokine inhibitor is administered before administration of the anti-T cell antigen-binding molecule, which is (a) 5 days before each (every) administration of the anti-T cell antigen-binding molecule in the first dosing cycle, and (b) 5 days before the first administration in each of the second and subsequent dosing cycles;
- the cytokine inhibitor is administered before administration of the anti-T cell antigen-binding molecule, which is (a) 4 days before each (every) administration of the anti-T cell antigen-binding molecule in the first dosing cycle, and (b) 4 days before the first administration in each of the second and subsequent dosing cycles;
- the cytokine inhibitor is administered before administration of the anti-T cell antigen-binding molecule, which is (a) 3 days before each (every) administration of the anti-T cell antigen-binding molecule in the first dosing cycle, and (b) 3 days before the first administration in each of the second and subsequent dosing cycles;
- the cytokine inhibitor is administered before administration of the anti-T cell antigen-binding molecule, which is (a) 2 days before each (every) administration of the anti-T cell antigen-binding molecule in the first dosing cycle, and (b) 2 days before the first administration in each of the second and subsequent dosing cycles;
- the cytokine inhibitor is administered before administration of the anti-T cell antigen-binding molecule, which is (a) 1 day before each (every) administration of the anti-T cell antigen-binding molecule in the first dosing cycle, and (b) 1 day before the first administration in each of the second and subsequent dosing cycles;
- the cytokine inhibitor is administered before administration of the anti-T cell antigen-binding molecule (a) on the same day as each (every) administration of the anti-T cell antigen-binding molecule in the first dosing cycle, and (b) on the same day as the first administration in each of the second and subsequent dosing cycles; or
- the cytokine inhibitor is administered simultaneously with administration of the anti-T cell antigen-binding molecule (a) on the same day as each (every) administration of the anti-T cell antigen-binding molecule in the first dosing cycle, and (b) on the same day as the first administration in each of the second and subsequent dosing cycles.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- (a) on the same day as, or 1 day, 2 days, 3 days, 4 days, 5 days, or 6 days after each (every) administration of the anti-T cell antigen-binding molecule in the first dosing cycle, and (b) on the same day as, or 1 day, 2 days, 3 days, 4 days, 5 days, or 6 days after the first administration in each of the second and subsequent dosing cycles (when the anti-T cell antigen-binding molecule is administered repeatedly at intervals of once every 4 weeks, in dosing cycles where 2, 3, 4, or 5 administrations constitute one cycle, and pre-administration of the cytokine inhibitor is performed (a) 6 days, 5 days, 4 days, 3 days, 2 days, or 1 day before said each (every) administration of the anti-T cell antigen-binding molecule in the first dosing cycle, and (b) 6 days, 5 days, 4 days, 3 days, 2 days, or 1 day before the said first administration in each of the second and subsequent dosing cycles); or
- (a) 1 day, 2 days, 3 days, 4 days, 5 days, or 6 days after each (every) administration of the anti-T cell antigen-binding molecule in the first dosing cycle, and (b) 1 day, 2 days, 3 days, 4 days, 5 days, or 6 days after the first administration in each of the second and subsequent dosing cycles (when the anti-T cell antigen-binding molecule is administered repeatedly at intervals of once every 4 weeks, in dosing cycles where 2, 3, 4, or 5 administrations constitute one cycle, and pre-administration of the cytokine inhibitor is performed (a) on the same day as said each (every) administration of the anti-T cell antigen-binding molecule in the first dosing cycle, and (b) on the same day as the said first administration in each of the second and subsequent dosing cycles).

(Repeated administration of the anti-T cell antigen-binding molecule at intervals of once every 5 weeks)

In one embodiment, in the combination therapy of the present disclosure, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 5 weeks, and (iii) pre-administration of a cytokine inhibitor is performed for the first administration of the anti-T cell antigen-binding molecule in each of the dosing cycles. As specific examples, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 5 weeks, and (iii) pre-administration of a cytokine inhibitor is performed according to the following embodiment:

- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 6 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 5 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 4 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 3 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 2 days before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, which is 1 day before the first administration in each of the dosing cycles;
- the anti-T cell antigen-binding molecule is administered repeatedly in dosing cycles where 2, 3, 4, or 5 administrations constitute one cycle, and the cytokine inhibitor is administered before administration of the anti-T cell antigen-binding molecule, on the same day as the first administration in each of the dosing cycles; or
- the anti-T cell antigen-binding molecule is administered repeatedly in dosing cycles where 2, 3, 4, or 5 administrations constitute one cycle, and the cytokine inhibitor is administered simultaneously with administration of the anti-T cell antigen-binding molecule, on the same day as the first administration in each of the dosing cycles.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- on the same day as, or 1 day, 2 days, 3 days, 4 days, 5 days, or 6 days after the first administration of the anti-T cell antigen-binding molecule in each of the dosing cycles (when the anti-T cell antigen-binding molecule is administered repeatedly at intervals of once every 5 weeks, in dosing cycles where 2, 3, 4, or 5 administrations constitute one cycle, and pre-administration of the cytokine inhibitor is performed 6 days, 5 days, 4 days, 3 days, 2 days, or 1 day before the said first administration of the anti-T cell antigen-binding molecule in each of the dosing cycles); or

- 1 day, 2 days, 3 days, 4 days, 5 days, or 6 days after the first administration of the anti-T cell antigen-binding molecule in each of the dosing cycles (when the anti-T cell antigen-binding molecule is administered repeatedly at intervals of once every 5 weeks, in dosing cycles where 2, 3, 4, or 5 administrations constitute one cycle, and pre-administration of the cytokine inhibitor is performed on the same day as the said first administration of the anti-T cell antigen-binding molecule in each of the dosing cycles).

In another embodiment, in the combination therapy of the present disclosure, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 5 weeks, and (iii) pre-administration of a cytokine inhibitor is performed (a) for each (every) administration of the anti-T cell antigen-binding molecule in the first dosing cycle, and (b) for the first administration in each of the second and subsequent dosing cycles. As specific examples, (i) an anti-T cell antigen-binding molecule is administered repeatedly in dosing cycles where 2, 3, 4, or 5 administrations constitute one cycle, (ii) intervals of repeated administrations of the anti-T cell antigen-binding molecule are once every 5 weeks, and (iii) pre-administration of a cytokine inhibitor is performed according to the following embodiment:

- the cytokine inhibitor is administered before administration of the anti-T cell antigen-binding molecule, which is (a) 6 days before each (every) administration of the anti-T cell antigen-binding molecule in the first dosing cycle, and (b) 6 days before the first administration in each of the second and subsequent dosing cycles;
- the cytokine inhibitor is administered before administration of the anti-T cell antigen-binding molecule, which is (a) 5 days before each (every) administration of the anti-T cell antigen-binding molecule in the first dosing cycle, and (b) 5 days before the first administration in each of the second and subsequent dosing cycles;
- the cytokine inhibitor is administered before administration of the anti-T cell antigen-binding molecule, which is (a) 4 days before each (every) administration of the anti-T cell antigen-binding molecule in the first dosing cycle, and (b) 4 days before the first administration in each of the second and subsequent dosing cycles;
- the cytokine inhibitor is administered before administration of the anti-T cell antigen-binding molecule, which is (a) 3 days before each (every) administration of the anti-T cell antigen-binding molecule in the first dosing cycle, and (b) 3 days before the first administration in each of the second and subsequent dosing cycles;
- the cytokine inhibitor is administered before administration of the anti-T cell antigen-binding molecule, which is (a) 2 days before each (every) administration of the anti-T cell antigen-binding molecule in the first dosing cycle, and (b) 2 days before the first administration in each of the second and subsequent dosing cycles;
- the cytokine inhibitor is administered before administration of the anti-T cell antigen-binding molecule, which is (a) 1 day before each (every) administration of the anti-T cell antigen-binding molecule in the first dosing cycle, and (b) 1 day before the first administration in each of the second and subsequent dosing cycles;
- the cytokine inhibitor is administered before administration of the anti-T cell antigen-binding molecule (a) on the same day as each (every) administration of the anti-T cell antigen-binding molecule in the first dosing cycle, and (b) on the same day as the first administration in each of the second and subsequent dosing cycles; or
- the cytokine inhibitor is administered simultaneously with administration of the anti-T cell antigen-binding molecule (a) on the same day as each (every) administration of the anti-T cell antigen-binding molecule in the first dosing cycle, and (b) on the same day as the first administration in each of the second and subsequent dosing cycles.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after administration of the anti-T cell antigen-binding molecule. As specific examples, the cytokine inhibitor may be further administered as follows:

- (a) on the same day as, or 1 day, 2 days, 3 days, 4 days, 5 days, or 6 days after each (every) administration of the anti-T cell antigen-binding molecule in the first dosing cycle, and (b) on the same day as, or 1 day, 2 days, 3 days, 4 days, 5 days, or 6 days after the first administration in each of the second and subsequent dosing cycles (when the anti-T cell antigen-binding molecule is administered repeatedly at intervals of once every 5 weeks, in dosing cycles where 2, 3, 4, or 5 administrations constitute one cycle, and pre-administration of the cytokine inhibitor is performed (a) 6 days, 5 days, 4 days, 3 days, 2 days, or 1 day before said each (every) administration of the anti-T cell antigen-binding molecule in the first dosing cycle, and (b) 6 days, 5 days, 4 days, 3 days, 2 days, or 1 day before the said first administration in each of the second and subsequent dosing cycles); or

- (a) 1 day, 2 days, 3 days, 4 days, 5 days, or 6 days after each (every) administration of the anti-T cell antigen-binding molecule in the first dosing cycle, and (b) 1 day, 2 days, 3 days, 4 days, 5 days, or 6 days after the first administration in each of the second and subsequent dosing cycles (when the anti-T cell antigen-binding molecule is administered repeatedly at intervals of once every 5 weeks, in dosing cycles where 2, 3, 4, or 5 administrations constitute one cycle, and pre-administration of the cytokine inhibitor is performed (a) on the same day as said each (every) administration of the anti-T cell antigen-binding molecule in the first dosing cycle, and (b) on the same day as the said first administration in each of the second and subsequent dosing cycles).

Administration of an anti-T cell antigen-binding molecule (comprising a step-up dosing period and a subsequent maintenance dosing period), accompanied with pre-administration of a cytokine inhibitor

In one embodiment, in the combination therapy of the present disclosure, (i) a step-up dosing period and a subsequent maintenance dosing period are comprised for an anti-T cell antigen-binding molecule, and (ii) a cytokine inhibitor is administered before or simultaneously with each administration during the step-up dosing period and the maintenance dosing period.

In one embodiment, the dose of an anti-T cell antigen-binding molecule is increased stepwise during the step-up dosing period. Here, when the dose is "increased stepwise", it means that the (n+1)th dose is larger than the nth dose. For example, when the anti-T cell antigen-binding molecule is administered twice during the step-up dosing period, the second dose is larger than the first dose. When the anti-T cell antigen-binding molecule is administered three times during the step-up dosing period, the second dose is larger than the first dose and the third dose is larger than the second dose. When the anti-T cell antigen-binding molecule is administered four times during the step-up dosing period, the second dose is larger than the first dose, the third dose is larger than the second dose, and the fourth dose is larger than the third dose. When the anti-T cell antigen-binding molecule is administered 5 times during the step-up dosing period, the second dose is larger than the first dose, the third dose is larger than the second dose, and the fourth dose is larger than the third dose, and the fifth dose is larger than the fourth dose. In one embodiment, the anti-T cell antigen-binding molecule is administered 2, 3, 4, 5, 6, 7, 8, 9, or 10 times during the step-up dosing period. In one embodiment, during the step-up dosing period, the anti-T cell antigen-binding molecule is administered at intervals of once every week, once every 2 weeks, once every 3 weeks, once every 4 weeks, or once every 5 weeks.

In one embodiment, the anti-T cell antigen-binding molecule is repeatedly administered at the same dose during the maintenance dosing period. In one embodiment, during the maintenance dosing period, the anti-T cell antigen-binding molecule is administered at intervals of once every week, once every 2 weeks, once every 3 weeks, once every 4 weeks, or once every 5 weeks. In a further embodiment, the dosing interval of the anti-T cell antigen-binding molecule during the maintenance dosing period is the same as the dosing interval of the anti-T cell antigen binding molecule during the step-up dosing period.

When pre-administration of a cytokine inhibitor is performed for each (every) anti-T cell antigen-binding molecule administration

(Repeated administration of the anti-T cell antigen-binding molecule at intervals of once every week)

In one embodiment, in the combination therapy of the present disclosure (i) a step-up dosing period and a subsequent maintenance dosing period are comprised for an anti-T cell antigen-binding molecule, (ii) the anti-T cell antigen-binding molecule is administered repeatedly at intervals of once every week during the step-up dosing period and the maintenance dosing period, and (iii) pre-administration of a cytokine inhibitor is performed for each administration during the step-up dosing period and the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every week during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is 6 days before each administration during the step-up dosing period and the maintenance dosing period;
- the cytokine inhibitor is administered before administration of the anti-T cell antigen-binding molecule, which is 5 days before each administration during the step-up dosing period and the maintenance dosing period;
- the cytokine inhibitor is administered before administration of the anti-T cell antigen-binding molecule, which is 4 days before each administration during the step-up dosing period and the maintenance dosing period;
- the cytokine inhibitor is administered before administration of the anti-T cell antigen-binding molecule, which is 3 days before each administration during the step-up dosing period and the maintenance dosing period;

- the cytokine inhibitor is administered before administration of the anti-T cell antigen-binding molecule, which is 2 days before each administration during the step-up dosing period and the maintenance dosing period;
- the cytokine inhibitor is administered before administration of the anti-T cell antigen-binding molecule, which is 1 day before each administration during the step-up dosing period and the maintenance dosing period;
- the cytokine inhibitor is administered before administration of the anti-T cell antigen-binding molecule, on the same day as each administration during the step-up dosing period and the maintenance dosing period; or
- the cytokine inhibitor is administered simultaneously with administration of the anti-T cell antigen-binding molecule, on the same day as each administration during the step-up dosing period and the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- on the same day as the administration of the anti-T cell antigen-binding molecule (when the anti-T cell antigen-binding molecule is administered repeatedly at intervals of once every week, and pre-administration of the cytokine inhibitor is performed 6 days before each administration during the step-up dosing period and the maintenance dosing period);
- on the same day as or 1 day after the administration of the anti-T cell antigen-binding molecule (when the anti-T cell antigen-binding molecule is administered repeatedly at intervals of once every week, and pre-administration of the cytokine inhibitor is performed 5 days before each administration during the step-up dosing period and the maintenance dosing period);
- on the same day as, or 1 day or 2 days after the administration of the anti-T cell antigen-binding molecule (when the anti-T cell antigen-binding molecule is administered repeatedly at intervals of once every week, and pre-administration of the cytokine inhibitor is performed 4 days before each administration during the step-up dosing period and the maintenance dosing period);
- on the same day as, or 1 day, 2 days, or 3 days after the administration of the anti-T cell antigen-binding molecule (when the anti-T cell antigen-binding molecule is administered repeatedly at intervals of once every week, and pre-administration of the cytokine inhibitor is performed 3 days before each administration during the step-up dosing period and the maintenance dosing period);
- on the same day as, or 1 day, 2 days, 3 days, or 4 days after the administration of the anti-T cell antigen-binding molecule (when the anti-T cell antigen-binding molecule is administered repeatedly at intervals of once every week, and pre-administration of the cytokine inhibitor is performed 2 days before each administration during the step-up dosing period and the maintenance dosing period);
- on the same day as, or 1 day, 2 days, 3 days, 4 days, or 5 days after the administration of the anti-T cell antigen-binding molecule (when the anti-T cell antigen-binding molecule is administered repeatedly at intervals of once every week, and pre-administration of the cytokine inhibitor is performed 1 day before each administration during the step-up dosing period and the maintenance dosing period); or
- 1 day, 2 days, 3 days, 4 days, 5 days, or 6 days after the administration of the anti-T cell antigen-binding molecule (when the anti-T cell antigen-binding molecule is administered repeatedly at intervals of once every week, and pre-administration of the cytokine inhibitor is performed on the same day as each administration during the step-up dosing period and the maintenance dosing period).

(Repeated administration of the anti-T cell antigen-binding molecule at intervals of once every 2 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 2 weeks during the step-up dosing period and the maintenance dosing period, and (iii) pre-administration of a cytokine inhibitor is performed for each administration during the step-up dosing period and the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 2 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is 6 days before each administration during the step-up dosing period and the maintenance dosing period;
- the cytokine inhibitor is administered before administration of the anti-T cell antigen-binding molecule, which is 5 days before each administration during the step-up dosing period and the maintenance dosing period;

- the cytokine inhibitor is administered before administration of the anti-T cell antigen-binding molecule, which is 4 days before each administration during the step-up dosing period and the maintenance dosing period;
- the cytokine inhibitor is administered before administration of the anti-T cell antigen-binding molecule, which is 3 days before each administration during the step-up dosing period and the maintenance dosing period;
- the cytokine inhibitor is administered before administration of the anti-T cell antigen-binding molecule, which is 2 days before each administration during the step-up dosing period and the maintenance dosing period;
- the cytokine inhibitor is administered before administration of the anti-T cell antigen-binding molecule, which is 1 day before each administration during the step-up dosing period and the maintenance dosing period;
- the cytokine inhibitor is administered before administration of the anti-T cell antigen-binding molecule, on the same day as each administration during the step-up dosing period and the maintenance dosing period; or
- the cytokine inhibitor is administered simultaneously with administration of the anti-T cell antigen-binding molecule, on the same day as each administration during the step-up dosing period and the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- on the same day as, or 1 day, 2 days, 3 days, 4 days, 5 days, or 6 days after the administration of the anti-T cell antigen-binding molecule (when the anti-T cell antigen-binding molecule is administered repeatedly at intervals of once every 2 weeks, and pre-administration of the cytokine inhibitor is performed 6 days, 5 days, 4 days, 3 days, 2 days, or 1 day before each administration during the step-up dosing period and the maintenance dosing period); or
- 1 day, 2 days, 3 days, 4 days, 5 days, or 6 days after the administration of the anti-T cell antigen-binding molecule (when the anti-T cell antigen-binding molecule is administered repeatedly at intervals of once every 2 weeks, and pre-administration of the cytokine inhibitor is performed on the same day as each administration during the step-up dosing period and the maintenance dosing period).

(Repeated administration of the anti-T cell antigen-binding molecule at intervals of once every 3 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 3 weeks during the step-up dosing period and the maintenance dosing period, and (iii) pre-administration of a cytokine inhibitor is performed for each administration during the step-up dosing period and the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 3 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is 6 days before each administration during the step-up dosing period and the maintenance dosing period;
- the cytokine inhibitor is administered before administration of the anti-T cell antigen-binding molecule, which is 5 days before each administration during the step-up dosing period and the maintenance dosing period;
- the cytokine inhibitor is administered before administration of the anti-T cell antigen-binding molecule, which is 4 days before each administration during the step-up dosing period and the maintenance dosing period;
- the cytokine inhibitor is administered before administration of the anti-T cell antigen-binding molecule, which is 3 days before each administration during the step-up dosing period and the maintenance dosing period;
- the cytokine inhibitor is administered before administration of the anti-T cell antigen-binding molecule, which is 2 days before each administration during the step-up dosing period and the maintenance dosing period;
- the cytokine inhibitor is administered before administration of the anti-T cell antigen-binding molecule, which is 1 day before each administration during the step-up dosing period and the maintenance dosing period;
- the cytokine inhibitor is administered before administration of the anti-T cell antigen-binding molecule, on the same day as each administration during the step-up dosing period and the maintenance dosing period; or
- the cytokine inhibitor is administered simultaneously with administration of the anti-T cell antigen-binding molecule, on the same day as each administration during the step-up dosing period and the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- on the same day as, or 1 day, 2 days, 3 days, 4 days, 5 days, or 6 days after the administration of the anti-T cell antigen-binding molecule (when the anti-T cell antigen-binding molecule is administered repeatedly at intervals of once every 3 weeks, and pre-administration of the cytokine inhibitor is performed 6 days, 5 days, 4 days, 3 days, 2 days, or 1 day before each administration during the step-up dosing period and the maintenance dosing period); or
- 1 day, 2 days, 3 days, 4 days, 5 days, or 6 days after the administration of the anti-T cell antigen-binding molecule (when the anti-T cell antigen-binding molecule is administered repeatedly at intervals of once every 3 weeks, and pre-administration of the cytokine inhibitor is performed on the same day as each administration during the step-up dosing period and the maintenance dosing period).

(Repeated administration of the anti-T cell antigen-binding molecule at intervals of once every 4 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 4 weeks during the step-up dosing period and the maintenance dosing period, and (iii) pre-administration of a cytokine inhibitor is performed for each administration during the step-up dosing period and the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 4 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is 6 days before each administration during the step-up dosing period and the maintenance dosing period;
- the cytokine inhibitor is administered before administration of the anti-T cell antigen-binding molecule, which is 5 days before each administration during the step-up dosing period and the maintenance dosing period;
- the cytokine inhibitor is administered before administration of the anti-T cell antigen-binding molecule, which is 4 days before each administration during the step-up dosing period and the maintenance dosing period;
- the cytokine inhibitor is administered before administration of the anti-T cell antigen-binding molecule, which is 3 days before each administration during the step-up dosing period and the maintenance dosing period;
- the cytokine inhibitor is administered before administration of the anti-T cell antigen-binding molecule, which is 2 days before each administration during the step-up dosing period and the maintenance dosing period;
- the cytokine inhibitor is administered before administration of the anti-T cell antigen-binding molecule, which is 1 day before each administration during the step-up dosing period and the maintenance dosing period;
- the cytokine inhibitor is administered before administration of the anti-T cell antigen-binding molecule, on the same day as each administration during the step-up dosing period and the maintenance dosing period; or
- the cytokine inhibitor is administered simultaneously with administration of the anti-T cell antigen-binding molecule, on the same day as each administration during the step-up dosing period and the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- on the same day as, or 1 day, 2 days, 3 days, 4 days, 5 days, or 6 days after the administration of the anti-T cell antigen-binding molecule (when the anti-T cell antigen-binding molecule is administered repeatedly at intervals of once every 4 weeks, and pre-administration of the cytokine inhibitor is performed 6 days, 5 days, 4 days, 3 days, 2 days, or 1 day before each administration during the step-up dosing period and the maintenance dosing period); or
- 1 day, 2 days, 3 days, 4 days, 5 days, or 6 days after the administration of the anti-T cell antigen-binding molecule (when the anti-T cell antigen-binding molecule is administered repeatedly at intervals of once every 4 weeks, and pre-administration of the cytokine inhibitor is performed on the same day as each administration during the step-up dosing period and the maintenance dosing period).

(Repeated administration of the anti-T cell antigen-binding molecule at intervals of once every 5 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 5 weeks during the step-up dosing period and the maintenance dosing period, and (iii) pre-administration of a cytokine inhibitor is performed for each administration during the step-up dosing period and the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 5 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is 6 days before each administration during the step-up dosing period and the maintenance dosing period;
- the cytokine inhibitor is administered before administration of the anti-T cell antigen-binding molecule, which is 5 days before each administration during the step-up dosing period and the maintenance dosing period;
- the cytokine inhibitor is administered before administration of the anti-T cell antigen-binding molecule, which is 4 days before each administration during the step-up dosing period and the maintenance dosing period;
- the cytokine inhibitor is administered before administration of the anti-T cell antigen-binding molecule, which is 3 days before each administration during the step-up dosing period and the maintenance dosing period;
- the cytokine inhibitor is administered before administration of the anti-T cell antigen-binding molecule, which is 2 days before each administration during the step-up dosing period and the maintenance dosing period;
- the cytokine inhibitor is administered before administration of the anti-T cell antigen-binding molecule, which is 1 day before each administration during the step-up dosing period and the maintenance dosing period;
- the cytokine inhibitor is administered before administration of the anti-T cell antigen-binding molecule, on the same day as each administration during the step-up dosing period and the maintenance dosing period; or
- the cytokine inhibitor is administered simultaneously with administration of the anti-T cell antigen-binding molecule, on the same day as each administration during the step-up dosing period and the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- on the same day as, or 1 day, 2 days, 3 days, 4 days, 5 days, or 6 days after the administration of the anti-T cell antigen-binding molecule (when the anti-T cell antigen-binding molecule is administered repeatedly at intervals of once every 5 weeks, and pre-administration of the cytokine inhibitor is performed 6 days, 5 days, 4 days, 3 days, 2 days, or 1 day before each administration during the step-up dosing period and the maintenance dosing period); or
- 1 day, 2 days, 3 days, 4 days, 5 days, or 6 days after the administration of the anti-T cell antigen-binding molecule (when the anti-T cell antigen-binding molecule is administered repeatedly at intervals of once every 5 weeks, and pre-administration of the cytokine inhibitor is performed on the same day as each administration during the step-up dosing period and the maintenance dosing period).

When pre-administration of a cytokine inhibitor is performed for each (every) anti-T cell antigen-binding molecule administration during the step-up dosing period

(Repeated administration of the anti-T cell antigen-binding molecule at intervals of once every week)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every week during the step-up dosing period and the maintenance dosing period, and (iii) (a) pre-administration of a cytokine inhibitor is performed for each administration during the step-up dosing period, and (b) pre-administration of the cytokine inhibitor is not performed during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every week during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is 6 days before each administration during the step-up dosing period;
- the cytokine inhibitor is administered before administration of the anti-T cell antigen-binding molecule, which is 5 days before each administration during the step-up dosing period;
- the cytokine inhibitor is administered before administration of the anti-T cell antigen-binding molecule, which is 4 days before each administration during the step-up dosing period;
- the cytokine inhibitor is administered before administration of the anti-T cell antigen-binding molecule, which is 3 days before each administration during the step-up dosing period;
- the cytokine inhibitor is administered before administration of the anti-T cell antigen-binding molecule, which is 2 days before each administration during the step-up dosing period;
- the cytokine inhibitor is administered before administration of the anti-T cell antigen-binding molecule, which is 1 day before each administration during the step-up dosing period;
- the cytokine inhibitor is administered before administration of the anti-T cell antigen-binding molecule, on the same day as each administration during the step-up dosing period; or
- the cytokine inhibitor is administered simultaneously with administration of the anti-T cell antigen-binding molecule, on the same day as each administration during the step-up dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- on the same day as each administration during the step-up dosing period (when the anti-T cell antigen-binding molecule is administered repeatedly at intervals of once every week, and the cytokine inhibitor is pre-administered 6 days before said each administration during the step-up dosing period);
- on the same day as or 1 day after each administration during the step-up dosing period (when the anti-T cell antigen-binding molecule is administered repeatedly at intervals of once every week, and pre-administration of the cytokine inhibitor is performed 5 days before said each administration during the step-up dosing period);
- on the same day as, or 1 day or 2 days after each administration during the step-up dosing period (when the anti-T cell antigen-binding molecule is administered repeatedly at intervals of once every week, and pre-administration of the cytokine inhibitor is performed 4 days before said each administration during the step-up dosing period);
- on the same day as, or 1 day, 2 days, or 3 days after each administration during the step-up dosing period (when the anti-T cell antigen-binding molecule is administered repeatedly at intervals of once every week, and pre-administration of the cytokine inhibitor is performed 3 days before said each administration during the step-up dosing period);
- on the same day as, or 1 day, 2 days, 3 days, or 4 days after each administration during the step-up dosing period (when the anti-T cell antigen-binding molecule is administered repeatedly at intervals of once every week, and pre-administration of the cytokine inhibitor is performed 2 days before said each administration during the step-up dosing period);
- on the same day as, or 1 day, 2 days, 3 days, 4 days, or 5 days after each administration during the step-up dosing period (when the anti-T cell antigen-binding molecule is administered repeatedly at intervals of once every week, and pre-administration of the cytokine inhibitor is performed 1 day before said each administration during the step-up dosing period); or
- 1 day, 2 days, 3 days, 4 days, 5 days, or 6 days after each administration during the step-up dosing period (when the anti-T cell antigen-binding molecule is administered repeatedly at intervals of once every week, and pre-administration of the cytokine inhibitor is performed on the same day as said each administration during the step-up dosing period).

(Repeated administration of the anti-T cell antigen-binding molecule at intervals of once every 2 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 2 weeks during the step-up dosing period and the maintenance dosing period, and (iii) (a) pre-administration of a cytokine inhibitor is performed for each administration during the step-up dosing period, and (b) pre-administration of the cytokine inhibitor is not performed during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 2 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is 6 days before each administration during the step-up dosing period;
- the cytokine inhibitor is administered before administration of the anti-T cell antigen-binding molecule, which is 5 days before each administration during the step-up dosing period;
- the cytokine inhibitor is administered before administration of the anti-T cell antigen-binding molecule, which is 4 days before each administration during the step-up dosing period;
- the cytokine inhibitor is administered before administration of the anti-T cell antigen-binding molecule, which is 3 days before each administration during the step-up dosing period;
- the cytokine inhibitor is administered before administration of the anti-T cell antigen-binding molecule, which is 2 days before each administration during the step-up dosing period;
- the cytokine inhibitor is administered before administration of the anti-T cell antigen-binding molecule, which is 1 day before each administration during the step-up dosing period;
- the cytokine inhibitor is administered before administration of the anti-T cell antigen-binding molecule, on the same day as each administration during the step-up dosing period; or
- the cytokine inhibitor is administered simultaneously with administration of the anti-T cell antigen-binding molecule, on the same day as each administration during the step-up dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- on the same day as, or 1 day, 2 days, 3 days, 4 days, 5 days, or 6 days after each administration during the step-up dosing period (when the anti-T cell antigen-binding molecule is administered repeatedly at intervals of once every 2 weeks, and pre-administration of the cytokine inhibitor is performed 6 days, 5 days, 4 days, 3 days, 2 days, or 1 day before said each administration during the step-up dosing period); or
- 1 day, 2 days, 3 days, 4 days, 5 days, or 6 days after each administration during the step-up dosing period (when the anti-T cell antigen-binding molecule is administered repeatedly at intervals of once every 2 weeks, and pre-administration of the cytokine inhibitor is performed on the same day as said each administration during the step-up dosing period).

(Repeated administration of the anti-T cell antigen-binding molecule at intervals of once every 3 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 3 weeks during the step-up dosing period and the maintenance dosing period, and (iii) (a) pre-administration of a cytokine inhibitor is performed for each administration during the step-up dosing period, and (b) pre-administration of the cytokine inhibitor is not performed during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 3 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is 6 days before each administration during the step-up dosing period;
- the cytokine inhibitor is administered before administration of the anti-T cell antigen-binding molecule, which is 5 days before each administration during the step-up dosing period;
- the cytokine inhibitor is administered before administration of the anti-T cell antigen-binding molecule, which is 4 days before each administration during the step-up dosing period;
- the cytokine inhibitor is administered before administration of the anti-T cell antigen-binding molecule, which is 3 days before each administration during the step-up dosing period;
- the cytokine inhibitor is administered before administration of the anti-T cell antigen-binding molecule, which is 2 days before each administration during the step-up dosing period;
- the cytokine inhibitor is administered before administration of the anti-T cell antigen-binding molecule, which is 1 day before each administration during the step-up dosing period;

- the cytokine inhibitor is administered before administration of the anti-T cell antigen-binding molecule, on the same day as each administration during the step-up dosing period; or
- the cytokine inhibitor is administered simultaneously with administration of the anti-T cell antigen-binding molecule, on the same day as each administration during the step-up dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- on the same day as, or 1 day, 2 days, 3 days, 4 days, 5 days, or 6 days after each administration during the step-up dosing period (when the anti-T cell antigen-binding molecule is administered repeatedly at intervals of once every 3 weeks, and pre-administration of the cytokine inhibitor is performed 6 days, 5 days, 4 days, 3 days, 2 days, or 1 day before said each administration during the step-up dosing period); or
- 1 day, 2 days, 3 days, 4 days, 5 days, or 6 days after each administration during the step-up dosing period (when the anti-T cell antigen-binding molecule is administered repeatedly at intervals of once every 3 weeks, and pre-administration of the cytokine inhibitor is performed on the same day as said each administration during the step-up dosing period).

(Repeated administration of the anti-T cell antigen-binding molecule at intervals of once every 4 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 4 weeks during the step-up dosing period and the maintenance dosing period, and (iii) (a) pre-administration of a cytokine inhibitor is performed for each administration during the step-up dosing period, and (b) pre-administration of the cytokine inhibitor is not performed during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 4 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is 6 days before each administration during the step-up dosing period;
- the cytokine inhibitor is administered before administration of the anti-T cell antigen-binding molecule, which is 5 days before each administration during the step-up dosing period;
- the cytokine inhibitor is administered before administration of the anti-T cell antigen-binding molecule, which is 4 days before each administration during the step-up dosing period;
- the cytokine inhibitor is administered before administration of the anti-T cell antigen-binding molecule, which is 3 days before each administration during the step-up dosing period;
- the cytokine inhibitor is administered before administration of the anti-T cell antigen-binding molecule, which is 2 days before each administration during the step-up dosing period;
- the cytokine inhibitor is administered before administration of the anti-T cell antigen-binding molecule, which is 1 day before each administration during the step-up dosing period;
- the cytokine inhibitor is administered before administration of the anti-T cell antigen-binding molecule, on the same day as each administration during the step-up dosing period; or
- the cytokine inhibitor is administered simultaneously with administration of the anti-T cell antigen-binding molecule, on the same day as each administration during the step-up dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- on the same day as, or 1 day, 2 days, 3 days, 4 days, 5 days, or 6 days after each administration during the step-up dosing period (when the anti-T cell antigen-binding molecule is administered repeatedly at intervals of once every 4 weeks, and pre-administration of the cytokine inhibitor is performed 6 days, 5 days, 4 days, 3 days, 2 days, or 1 day before said each administration during the step-up dosing period); or

- 1 day, 2 days, 3 days, 4 days, 5 days, or 6 days after each administration during the step-up dosing period (when the anti-T cell antigen-binding molecule is administered repeatedly at intervals of once every 4 weeks, and pre-administration of the cytokine inhibitor is performed on the same day as said each administration during the step-up dosing period).

(Repeated administration of the anti-T cell antigen-binding molecule at intervals of once every 5 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 5 weeks during the step-up dosing period and the maintenance dosing period, and (iii) (a) pre-administration of a cytokine inhibitor is performed for each administration during the step-up dosing period, and (b) pre-administration of the cytokine inhibitor is not performed during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 5 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is 6 days before each administration during the step-up dosing period;
- the cytokine inhibitor is administered before administration of the anti-T cell antigen-binding molecule, which is 5 days before each administration during the step-up dosing period;
- the cytokine inhibitor is administered before administration of the anti-T cell antigen-binding molecule, which is 4 days before each administration during the step-up dosing period;
- the cytokine inhibitor is administered before administration of the anti-T cell antigen-binding molecule, which is 3 days before each administration during the step-up dosing period;
- the cytokine inhibitor is administered before administration of the anti-T cell antigen-binding molecule, which is 2 days before each administration during the step-up dosing period;
- the cytokine inhibitor is administered before administration of the anti-T cell antigen-binding molecule, which is 1 day before each administration during the step-up dosing period;
- the cytokine inhibitor is administered before administration of the anti-T cell antigen-binding molecule, on the same day as each administration during the step-up dosing period; or
- the cytokine inhibitor is administered simultaneously with administration of the anti-T cell antigen-binding molecule, on the same day as each administration during the step-up dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- on the same day as, or 1 day, 2 days, 3 days, 4 days, 5 days, or 6 days after each administration during the step-up dosing period (when the anti-T cell antigen-binding molecule is administered repeatedly at intervals of once every 5 weeks, and pre-administration of the cytokine inhibitor is performed 6 days, 5 days, 4 days, 3 days, 2 days, or 1 day before said each administration during the step-up dosing period); or
- 1 day, 2 days, 3 days, 4 days, 5 days, or 6 days after each administration during the step-up dosing period (when the anti-T cell antigen-binding molecule is administered repeatedly at intervals of once every 5 weeks, and pre-administration of the cytokine inhibitor is performed on the same day as said each administration during the step-up dosing period).

When pre-administration of a cytokine inhibitor is performed for administration of an anti-T cell antigen-binding molecule for each (every) administration during the step-up dosing period, and for the first or multiple administrations during the maintenance dosing period

(Repeated administration of the anti-T cell antigen-binding molecule at intervals of once every week)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every week during the step-up dosing period and the maintenance dosing period, and (iii) pre-administration of a cytokine inhibitor is performed (a) for each administration during the step-up dosing period, and (b) for the first administration or the first to second, the first to third, the first to fourth, or the first to fifth administrations during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every week during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is (a) 6 days before each administration during the step-up dosing period, and (b) 6 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 5 days before each administration during the step-up dosing period, and (b) 5 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 4 days before each administration during the step-up dosing period, and (b) 4 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 3 days before each administration during the step-up dosing period, and (b) 3 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 2 days before each administration during the step-up dosing period, and (b) 2 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 1 day before each administration during the step-up dosing period, and (b) 1 day before the first administration or each of the first to second, the first to third, the first to fourth, or the first to fifth administrations during the maintenance dosing period;
- the cytokine inhibitor is administered before administration of the anti-T cell antigen-binding molecule (a) on the same day as each administration during the step-up dosing period, and (b) on the same day as the first administration or each of the first to second, the first to third, the first to fourth, or the first to fifth administrations during the maintenance dosing period; or
- the cytokine inhibitor is administered simultaneously with administration of the anti-T cell antigen-binding molecule (a) on the same day as each administration during the step-up dosing period, and (b) on the same day as the first administration or each of the first to second, the first to third, the first to fourth, or the first to fifth administrations during the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- (a) on the same day as each administration during the step-up dosing period, and (b) on the same day as the first administration or each of the first to second, the first to third, the first to fourth, or the first to fifth administrations during the maintenance dosing period (when the anti-T cell antigen-binding molecule is administered repeatedly at intervals of once every week, and pre-administration of the cytokine inhibitor is performed (a) 6 days before said each administration during the step-up dosing period and (b) 6 days before the said first administration or each of the first to second, the first to third, the first to fourth, or the first to fifth administrations during the maintenance dosing period);
- (a) on the same day as or 1 day after each administration during the step-up dosing period, and (b) on the same day as or 1 day after the first administration or each of the first to second, the first to third, the first to fourth, or the first to fifth administrations during the maintenance dosing period (when the anti-T cell antigen-binding molecule is administered repeatedly at intervals of once every week, and pre-administration of the cytokine inhibitor is performed (a) 5 days before said each administration during the step-up dosing period and (b) 5 days before the said first administration or each of the first to second, the first to third, the first to fourth, or the first to fifth administrations during the maintenance dosing period);

- (a) on the same day as, or 1 day or 2 days after each administration during the step-up dosing period, and (b) on the same day as, or 1 day or 2 days after the first administration or each of the first to second, the first to third, the first to fourth, or the first to fifth administrations during the maintenance dosing period (when the anti-T cell antigen-binding molecule is administered repeatedly at intervals of once every week, and pre-administration of the cytokine inhibitor is performed (a) 4 days before said each administration during the step-up dosing period and (b) 4 days before the said first administration or each of the first to second, the first to third, the first to fourth, or the first to fifth administrations during the maintenance dosing period);

- (a) on the same day as, or 1 day, 2 days, or 3 days after each administration during the step-up dosing period, and (b) on the same day as, or 1 day, 2 days, or 3 days after the first administration or each of the first to second, the first to third, the first to fourth, or the first to fifth administrations during the maintenance dosing period (when the anti-T cell antigen-binding molecule is administered repeatedly at intervals of once every week, and pre-administration of the cytokine inhibitor is performed (a) 3 days before said each administration during the step-up dosing period and (b) 3 days before the said first administration or each of the first to second, the first to third, the first to fourth, or the first to fifth administrations during the maintenance dosing period);

- (a) on the same day as, or 1 day, 2 days, 3 days, or 4 days after each administration during the step-up dosing period, and (b) on the same day as, or 1 day, 2 days, 3 days, or 4 days after the first administration or each of the first to second, the first to third, the first to fourth, or the first to fifth administrations during the maintenance dosing period (when the anti-T cell antigen-binding molecule is administered repeatedly at intervals of once every week, and pre-administration of the cytokine inhibitor is performed (a) 2 days before said each administration during the step-up dosing period and (b) 2 days before the said first administration or each of the first to second, the first to third, the first to fourth, or the first to fifth administrations during the maintenance dosing period);

- (a) on the same day as, or 1 day, 2 days, 3 days, 4 days, or 5 days after each administration during the step-up dosing period, and (b) on the same day as, or 1 day, 2 days, 3 days, 4 days, or 5 days after the first administration or each of the first to second, the first to third, the first to fourth, or the first to fifth administrations during the maintenance dosing period (when the anti-T cell antigen-binding molecule is administered repeatedly at intervals of once every week, and pre-administration of the cytokine inhibitor is performed (a) 1 day before said each administration during the step-up dosing period and (b) 1 day before the said first administration or each of the first to second, the first to third, the first to fourth, or the first to fifth administrations during the maintenance dosing period); or

- (a) 1 day, 2 days, 3 days, 4 days, 5 days, or 6 days after each administration during the step-up dosing period, and (b) 1 day, 2 days, 3 days, 4 days, 5 days, or 6 days after the first administration or each administration of the first to second, the first to third, the first to fourth, or the first to fifth administrations during the maintenance dosing period (when the anti-T cell antigen-binding molecule is administered repeatedly at intervals of once every week, and pre-administration of the cytokine inhibitor is performed (a) on the same day as said each administration during the step-up dosing period and (b) on the same day as the said first administration or each of the first to second, the first to third, the first to fourth, or the first to fifth administrations during the maintenance dosing period).

(Repeated administration of the anti-T cell antigen-binding molecule at intervals of once every 2 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 2 weeks during the step-up dosing period and the maintenance dosing period, and (iii) pre-administration of a cytokine inhibitor is performed (a) for each administration during the step-up dosing period, and (b) for the first administration or the first to second, the first to third, the first to fourth, or the first to fifth administrations during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 2 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is (a) 6 days before each administration during the step-up dosing period, and (b) 6 days before the first administration or each of the first to second, the first to third, the first to fourth, or the first to fifth administrations during the maintenance dosing period;

- the cytokine inhibitor is administered before administration of the anti-T cell antigen-binding molecule, which is (a) 5 days before each administration during the step-up dosing period, and (b) 5 days before the first administration or each of the first to second, the first to third, the first to fourth, or the first to fifth administrations during the maintenance dosing period;

- the cytokine inhibitor is administered before administration of the anti-T cell antigen-binding molecule, which is (a) 4 days before each administration during the step-up dosing period, and (b) 4 days before the first administration or each of the first to second, the first to third, the first to fourth, or the first to fifth administrations during the maintenance dosing period;

- the cytokine inhibitor is administered before administration of the anti-T cell antigen-binding molecule, which is (a) 3 days before each administration during the step-up dosing period, and (b) 3 days before the first administration or each of the first to second, the first to third, the first to fourth, or the first to fifth administrations during the maintenance dosing period;

- the cytokine inhibitor is administered before administration of the anti-T cell antigen-binding molecule, which is (a) 2 days before each administration during the step-up dosing period, and (b) 2 days before the first administration or each of the first to second, the first to third, the first to fourth, or the first to fifth administrations during the maintenance dosing period;

- the cytokine inhibitor is administered before administration of the anti-T cell antigen-binding molecule, which is (a) 1 day before each administration during the step-up dosing period, and (b) 1 day before the first administration or each of the first to second, the first to third, the first to fourth, or the first to fifth administrations during the maintenance dosing period;

- the cytokine inhibitor is administered before administration of the anti-T cell antigen-binding molecule (a) on the same day as each administration during the step-up dosing period, and (b) on the same day as the first administration or each of the first to second, the first to third, the first to fourth, or the first to fifth administrations during the maintenance dosing period; or

- the cytokine inhibitor is administered simultaneously with administration of the anti-T cell antigen-binding molecule (a) on the same day as each administration during the step-up dosing period, and (b) on the same day as the first administration or each of the first to second, the first to third, the first to fourth, or the first to fifth administrations during the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- (a) on the same day as, or 1 day, 2 days, 3 days, 4 days, 5 days, or 6 days after each administration during the step-up dosing period, and (b) on the same day as, or 1 day, 2 days, 3 days, 4 days, 5 days, or 6 days after the first administration or each of the first to second, the first to third, the first to fourth, or the first to fifth administrations during the maintenance dosing period (when the anti-T cell antigen-binding molecule is administered repeatedly at intervals of once every 2 weeks, and pre-administration of the cytokine inhibitor is performed (a) 6 days, 5 days, 4 days, 3 days, 2 days, or 1 day before said each administration during the step-up dosing period and (b) 6 days, 5 days, 4 days, 3 days, 2 days, or 1 day before the said first administration or each of the first to second, the first to third, the first to fourth, or the first to fifth administrations during the maintenance dosing period); or

- (a) 1 day, 2 days, 3 days, 4 days, 5 days, or 6 days after each administration during the step-up dosing period, and (b) 1 day, 2 days, 3 days, 4 days, 5 days, or 6 days after the first administration or each of the first to second, the first to third, the first to fourth, or the first to fifth administrations during the maintenance dosing period (when the anti-T cell antigen-binding molecule is administered repeatedly at intervals of once every 2 weeks, and pre-administration of the cytokine inhibitor is performed (a) on the same day as said each administration during the step-up dosing period and (b) on the same day as the said first administration or each of the first to second, the first to third, the first to fourth, or the first to fifth administrations during the maintenance dosing period).

(Repeated administration of the anti-T cell antigen-binding molecule at intervals of once every 3 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 3 weeks during the step-up dosing period and the maintenance dosing period, and (iii) pre-administration of a cytokine inhibitor is performed (a) for each administration during the step-up dosing period, and (b) for the first administration or the first to second, the first to third, the first to fourth, or the first to fifth administrations during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 3 weeks during the step-up dosing period and the maintenance dosing period, and

77

(iii) pre-administration of a cytokine inhibitor is performed according to the following embodiment:

- the cytokine inhibitor is administered before administration of the anti-T cell antigen-binding molecule, which is (a) 6 days before each administration during the step-up dosing period, and (b) 6 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 5 days before each administration during the step-up dosing period, and (b) 5 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 4 days before each administration during the step-up dosing period, and (b) 4 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 3 days before each administration during the step-up dosing period, and (b) 3 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 2 days before each administration during the step-up dosing period, and (b) 2 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 1 day before each administration during the step-up dosing period, and (b) 1 day before the first administration or each of the first to second, the first to third, the first to fourth, or the first to fifth administrations during the maintenance dosing period;
- the cytokine inhibitor is administered before administration of the anti-T cell antigen-binding molecule (a) on the same day as each administration during the step-up dosing period, and (b) on the same day as the first administration or each of the first to second, the first to third, the first to fourth, or the first to fifth administrations during the maintenance dosing period; or
- the cytokine inhibitor is administered simultaneously with administration of the anti-T cell antigen-binding molecule (a) on the same day as each administration during the step-up dosing period, and (b) on the same day as the first administration or each of the first to second, the first to third, the first to fourth, or the first to fifth administrations during the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- (a) on the same day as, or 1 day, 2 days, 3 days, 4 days, 5 days, or 6 days after each administration during the step-up dosing period, and (b) on the same day as, or 1 day, 2 days, 3 days, 4 days, 5 days, or 6 days after the first administration or each of the first to second, the first to third, the first to fourth, or the first to fifth administrations during the maintenance dosing period (when the anti-T cell antigen-binding molecule is administered repeatedly at intervals of once every 3 weeks, and pre-administration of the cytokine inhibitor is performed (a) 6 days, 5 days, 4 days, 3 days, 2 days, or 1 day before said each administration during the step-up dosing period and (b) 6 days, 5 days, 4 days, 3 days, 2 days, or 1 day before the said first administration or each of the first to second, the first to third, the first to fourth, or the first to fifth administrations during the maintenance dosing period); or
- (a) 1 day, 2 days, 3 days, 4 days, 5 days, or 6 days after each administration during the step-up dosing period, and (b) 1 day, 2 days, 3 days, 4 days, 5 days, or 6 days after the first administration or each of the first to second, the first to third, the first to fourth, or the first to fifth administrations during the maintenance dosing period (when the anti-T cell antigen-binding molecule is administered repeatedly at intervals of once every 3 weeks, and pre-administration of the cytokine inhibitor is performed (a) on the same day as said each administration during the step-up dosing period and (b) on the same day as the said first administration or each of the first to second, the first to third, the first to fourth, or the first to fifth administrations during the maintenance dosing period).

(Repeated administration of the anti-T cell antigen-binding molecule at intervals of once every 4 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 4 weeks during the step-up dosing period and the maintenance dosing period, and (iii) pre-administration of a cytokine inhibitor is performed (a) for each administration during the step-up dosing period, and (b) for the first administration or the first to second, the first to third, the first to fourth, or the first to fifth administrations during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 4 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is (a) 6 days before each administration during the step-up dosing period, and (b) 6 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 5 days before each administration during the step-up dosing period, and (b) 5 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 4 days before each administration during the step-up dosing period, and (b) 4 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 3 days before each administration during the step-up dosing period, and (b) 3 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 2 days before each administration during the step-up dosing period, and (b) 2 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 1 day before each administration during the step-up dosing period, and (b) 1 day before the first administration or each of the first to second, the first to third, the first to fourth, or the first to fifth administrations during the maintenance dosing period;
- the cytokine inhibitor is administered before administration of the anti-T cell antigen-binding molecule (a) on the same day as each administration during the step-up dosing period, and (b) on the same day as the first administration or each of the first to second, the first to third, the first to fourth, or the first to fifth administrations during the maintenance dosing period; or
- the cytokine inhibitor is administered simultaneously with administration of the anti-T cell antigen-binding molecule (a) on the same day as each administration during the step-up dosing period, and (b) on the same day as the first administration or each of the first to second, the first to third, the first to fourth, or the first to fifth administrations during the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- (a) on the same day as, or 1 day, 2 days, 3 days, 4 days, 5 days, or 6 days after each administration during the step-up dosing period, and (b) on the same day as, or 1 day, 2 days, 3 days, 4 days, 5 days, or 6 days after the first administration or each of the first to second, the first to third, the first to fourth, or the first to fifth administrations during the maintenance dosing period (when the anti-T cell antigen-binding molecule is administered repeatedly at intervals of once every 4 weeks, and pre-administration of the cytokine inhibitor is performed (a) 6 days, 5 days, 4 days, 3 days, 2 days, or 1 day before said each administration during the step-up dosing period and (b) 6 days, 5 days, 4 days, 3 days, 2 days, or 1 day before the said first administration or each of the first to second, the first to third, the first to fourth, or the first to fifth administrations during the maintenance dosing period); or

- (a) 1 day, 2 days, 3 days, 4 days, 5 days, or 6 days after each administration during the step-up dosing period, and (b) 1 day, 2 days, 3 days, 4 days, 5 days, or 6 days after the first administration or each of the first to second, the first to third, the first to fourth, or the first to fifth administrations during the maintenance dosing period (when the anti-T cell antigen-binding molecule is administered repeatedly at intervals of once every 4 weeks, and pre-administration of the cytokine inhibitor is performed (a) on the same day as said each administration during the step-up dosing period and (b) on the same day as the said first administration or each of the first to second, the first to third, the first to fourth, or the first to fifth administrations during the maintenance dosing period).

(Repeated administration of the anti-T cell antigen-binding molecule at intervals of once every 5 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 5 weeks during the step-up dosing period and the maintenance dosing period, and (iii) pre-administration of a cytokine inhibitor is performed (a) for each administration during the step-up dosing period, and (b) for the first administration or the first to second, the first to third, the first to fourth, or the first to fifth administrations during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 5 weeks during the step-up dosing period and the maintenance dosing period, and (iii) pre-administration of a cytokine inhibitor is performed according to the following embodiment:

- the cytokine inhibitor is administered before administration of the anti-T cell antigen-binding molecule, which is (a) 6 days before each administration during the step-up dosing period, and (b) 6 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 5 days before each administration during the step-up dosing period, and (b) 5 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 4 days before each administration during the step-up dosing period, and (b) 4 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 3 days before each administration during the step-up dosing period, and (b) 3 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 2 days before each administration during the step-up dosing period, and (b) 2 days before the first administration or each of the first to second, the first to third, the first to fourth, or the first to fifth administrations during the maintenance dosing period;
- the cytokine inhibitor is administered before administration of the anti-T cell antigen-binding molecule, which is (a) 1 day before each administration during the step-up dosing period, and (b) 1 day before the first administration or each of the first to second, the first to third, the first to fourth, or the first to fifth administrations during the maintenance dosing period;
- the cytokine inhibitor is administered before administration of the anti-T cell antigen-binding molecule (a) on the same day as each administration during the step-up dosing period, and (b) on the same day as the first administration or each of the first to second, the first to third, the first to fourth, or the first to fifth administrations during the maintenance dosing period; or
- the cytokine inhibitor is administered simultaneously with administration of the anti-T cell antigen-binding molecule (a) on the same day as each administration during the step-up dosing period, and (b) on the same day as the first administration or each of the first to second, the first to third, the first to fourth, or the first to fifth administrations during the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- (a) on the same day as, or 1 day, 2 days, 3 days, 4 days, 5 days, or 6 days after each administration during the step-up dosing period, and (b) on the same day as, or 1 day, 2 days, 3 days, 4 days, 5 days, or 6 days after the first administration or each of the first to second, the first to third, the first to fourth, or the first to fifth administrations during the maintenance dosing period (when the anti-T cell antigen-binding molecule is administered repeatedly at intervals of once every 5 weeks, and pre-administration of the cytokine inhibitor is performed (a) 6 days, 5 days, 4 days, 3 days, 2 days, or 1 day before said each administration during the step-up dosing period and (b) 6 days, 5 days, 4 days, 3 days, 2 days, or 1 day before the said first administration or each of the first to second, the first to third, the first to fourth, or the first to fifth administrations during the maintenance dosing period); or
- (a) 1 day, 2 days, 3 days, 4 days, 5 days, or 6 days after each administration during the step-up dosing period, and (b) 1 day, 2 days, 3 days, 4 days, 5 days, or 6 days after the first administration or each of the first to second, the first to third, the first to fourth, or the first to fifth administrations during the maintenance dosing period (when the anti-T cell antigen-binding molecule is administered repeatedly at intervals of once every 5 weeks, and pre-administration of the cytokine inhibitor is performed (a) on the same day as said each administration during the step-up dosing period and (b) on the same day as the said first administration or each of the first to second, the first to third, the first to fourth, or the first to fifth administrations during the maintenance dosing period).

When pre-administration of a cytokine inhibitor is performed for administration of an anti-T cell antigen-binding molecule for each (every) administration during the step-up dosing period, and for every dosing cycle during the maintenance dosing period

(Repeated administration of the anti-T cell antigen-binding molecule at intervals of once every week)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every week during the step-up dosing period, (iii) the anti-T cell antigen-binding molecule is administered repeatedly at intervals of once every week, in dosing cycles where 2, 3, 4, or 5 administrations constitute one cycle during the maintenance dosing period, and (iv) pre-administration of a cytokine inhibitor is performed (a) for each administration during the step-up dosing period, and (b) for the first administration in each of the dosing cycles during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every week during the step-up dosing period, (iii) the anti-T cell antigen-binding molecule is administered repeatedly at intervals of once every week, in dosing cycles where 2, 3, 4, or 5 administrations constitute one cycle during the maintenance dosing period, and (iv) pre-administration of a cytokine inhibitor is performed according to the following embodiment:

- the cytokine inhibitor is administered before administration of the anti-T cell antigen-binding molecule, which is (a) 6 days before each administration during the step-up dosing period, and (b) 6 days before the first administration in each of the dosing cycles during the maintenance dosing period;
- the cytokine inhibitor is administered before administration of the anti-T cell antigen-binding molecule, which is (a) 5 days before each administration during the step-up dosing period, and (b) 5 days before the first administration in each of the dosing cycles during the maintenance dosing period;
- the cytokine inhibitor is administered before administration of the anti-T cell antigen-binding molecule, which is (a) 4 days before each administration during the step-up dosing period, and (b) 4 days before the first administration in each of the dosing cycles during the maintenance dosing period;
- the cytokine inhibitor is administered before administration of the anti-T cell antigen-binding molecule, which is (a) 3 days before each administration during the step-up dosing period, and (b) 3 days before the first administration in each of the dosing cycles during the maintenance dosing period;
- the cytokine inhibitor is administered before administration of the anti-T cell antigen-binding molecule, which is (a) 2 days before each administration during the step-up dosing period, and (b) 2 days before the first administration in each of the dosing cycles during the maintenance dosing period;
- the cytokine inhibitor is administered before administration of the anti-T cell antigen-binding molecule, which is (a) 1 day before each administration during the step-up dosing period, and (b) 1 day before the first administration in each of the dosing cycles during the maintenance dosing period;
- the cytokine inhibitor is administered before administration of the anti-T cell antigen-binding molecule (a) on the same day as each administration during the step-up dosing period, and (b) on the same day as the first administration in each of the dosing cycles during the maintenance dosing period; or

- the cytokine inhibitor is administered simultaneously with administration of the anti-T cell antigen-binding molecule (a) on the same day as each administration during the step-up dosing period, and (b) on the same day as the first administration in each of the dosing cycles during the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- (a) on the same day as administration during the step-up dosing period, and (b) on the same day as the first administration in each of the dosing cycles during the maintenance dosing period (when the anti-T cell antigen-binding molecule is administered repeatedly at intervals of once every week, and pre-administration of the cytokine inhibitor is performed (a) 6 days before each administration during the step-up dosing period and (b) 6 days before the said first administration in each of the dosing cycles during the maintenance dosing period);
- (a) on the same day as or 1 day after administration during the step-up dosing period, and (b) on the same day as or 1 day after the first administration in each of the dosing cycles during the maintenance dosing period (when the anti-T cell antigen-binding molecule is administered repeatedly at intervals of once every week, and pre-administration of the cytokine inhibitor is performed (a) 5 days before each administration during the step-up dosing period and (b) 5 days before the said first administration in each of the dosing cycles during the maintenance dosing period);
- (a) on the same day as, or 1 day or 2 days after administration during the step-up dosing period, and (b) on the same day as, or 1 day or 2 days after the first administration in each of the dosing cycles during the maintenance dosing period (when the anti-T cell antigen-binding molecule is administered repeatedly at intervals of once every week, and pre-administration of the cytokine inhibitor is performed (a) 4 days before each administration during the step-up dosing period and (b) 4 days before the said first administration in each of the dosing cycles during the maintenance dosing period);
- (a) on the same day as, or 1 day, 2 days, or 3 days after administration during the step-up dosing period, and (b) on the same day as, or 1 day, 2 days, or 3 days after the first administration in each of the dosing cycles during the maintenance dosing period (when the anti-T cell antigen-binding molecule is administered repeatedly at intervals of once every week, and pre-administration of the cytokine inhibitor is performed (a) 3 days before each administration during the step-up dosing period and (b) 3 days before the said first administration in each of the dosing cycles during the maintenance dosing period);
- (a) on the same day as, or 1 day, 2 days, 3 days, or 4 days after administration during the step-up dosing period, and (b) on the same day as, or 1 day, 2 days, 3 days, or 4 days after the first administration in each of the dosing cycles during the maintenance dosing period (when the anti-T cell antigen-binding molecule is administered repeatedly at intervals of once every week, and pre-administration of the cytokine inhibitor is performed (a) 2 days before each administration during the step-up dosing period and (b) 2 days before the said first administration in each of the dosing cycles during the maintenance dosing period);
- (a) on the same day as, or 1 day, 2 days, 3 days, 4 days, or 5 days after administration during the step-up dosing period, and (b) on the same day as, or 1 day, 2 days, 3 days, 4 days, or 5 days after the first administration in each of the dosing cycles during the maintenance dosing period (when the anti-T cell antigen-binding molecule is administered repeatedly at intervals of once every week, and pre-administration of the cytokine inhibitor is performed (a) 1 day before each administration during the step-up dosing period and (b) 1 day before the said first administration in each of the dosing cycles during the maintenance dosing period); or
- (a) 1 day, 2 days, 3 days, 4 days, 5 days, or 6 days after administration during the step-up dosing period, and (b) 1 day, 2 days, 3 days, 4 days, 5 days, or 6 days after the said first administration in each of the dosing cycles during the maintenance dosing period (when the anti-T cell antigen-binding molecule is administered repeatedly at intervals of once every week, and pre-administration of the cytokine inhibitor is performed (a) on the same day as each administration during the step-up dosing period and (b) on the same day as the said first administration in each of the dosing cycles during the maintenance dosing period).

(Repeated administration of the anti-T cell antigen-binding molecule at intervals of once every 2 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 2 weeks during the step-up dosing period, (iii) the anti-T cell antigen-binding molecule is administered repeatedly at intervals of once every 2 weeks, in dosing cycles where 2, 3, 4, or 5 administrations constitute one cycle during the maintenance dosing period, and (iv) pre-administration of a cytokine inhibitor is performed (a) for each administration during the step-up dosing period, and (b) for the first administration in each of the dosing cycles during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 2 weeks during the step-up dosing period, (iii) the anti-T cell antigen-binding molecule is administered repeatedly at intervals of once every 2 weeks, in dosing cycles where 2, 3, 4, or 5 administrations constitute one cycle during the maintenance dosing period, and (iv) pre-administration of a cytokine inhibitor is performed according to the following embodiment:

- the cytokine inhibitor is administered before administration of the anti-T cell antigen-binding molecule, which is (a) 6 days before each administration during the step-up dosing period, and (b) 6 days before the first administration in each of the dosing cycles during the maintenance dosing period;
- the cytokine inhibitor is administered before administration of the anti-T cell antigen-binding molecule, which is (a) 5 days before each administration during the step-up dosing period, and (b) 5 days before the first administration in each of the dosing cycles during the maintenance dosing period;
- the cytokine inhibitor is administered before administration of the anti-T cell antigen-binding molecule, which is (a) 4 days before each administration during the step-up dosing period, and (b) 4 days before the first administration in each of the dosing cycles during the maintenance dosing period;
- the cytokine inhibitor is administered before administration of the anti-T cell antigen-binding molecule, which is (a) 3 days before each administration during the step-up dosing period, and (b) 3 days before the first administration in each of the dosing cycles during the maintenance dosing period;
- the cytokine inhibitor is administered before administration of the anti-T cell antigen-binding molecule, which is (a) 2 days before each administration during the step-up dosing period, and (b) 2 days before the first administration in each of the dosing cycles during the maintenance dosing period;
- the cytokine inhibitor is administered before administration of the anti-T cell antigen-binding molecule, which is (a) 1 day before each administration during the step-up dosing period, and (b) 1 day before the first administration in each of the dosing cycles during the maintenance dosing period;
- the cytokine inhibitor is administered before administration of the anti-T cell antigen-binding molecule (a) on the same day as each administration during the step-up dosing period, and (b) on the same day as the first administration in each of the dosing cycles during the maintenance dosing period; or
- the cytokine inhibitor is administered simultaneously with administration of the anti-T cell antigen-binding molecule (a) on the same day as each administration during the step-up dosing period, and (b) on the same day as the first administration in each of the dosing cycles during the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- (a) on the same day as, or 1 day, 2 days, 3 days, 4 days, 5 days, or 6 days after administration during the step-up dosing period, and (b) on the same day as, or 1 day, 2 days, 3 days, 4 days, 5 days, or 6 days after the first administration in each of the dosing cycles during the maintenance dosing period (when the anti-T cell antigen-binding molecule is administered repeatedly at intervals of once every 2 weeks, and pre-administration of the cytokine inhibitor is performed (a) 6 days, 5 days, 4 days, 3 days, 2 days, or 1 day before each administration during the step-up dosing period and (b) 6 days, 5 days, 4 days, 3 days, 2 days, or 1 day before the said first administration in each of the dosing cycles during the maintenance dosing period); or
- (a) 1 day, 2 days, 3 days, 4 days, 5 days, or 6 days after administration during the step-up dosing period, and (b) 1 day, 2 days, 3 days, 4 days, 5 days, or 6 days after the first administration in each of the dosing cycles during the maintenance dosing period (when the anti-T cell antigen-binding molecule is administered repeatedly at intervals of once every 2 weeks, and pre-administration of the cytokine inhibitor is performed (a) on the same day as each administration during the step-up dosing period and (b) on the same day as the said first administration in each of the dosing cycles during the maintenance dosing period).

(Repeated administration of the anti-T cell antigen-binding molecule at intervals of once every 3 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 3 weeks during the step-up dosing period, (iii) the anti-T cell antigen-binding molecule is administered repeatedly at intervals of once every 3 weeks, in dosing cycles where 2, 3, 4, or 5 administrations constitute one cycle during the maintenance dosing period, and (iv) pre-administration of a cytokine inhibitor is performed (a) for each administration during the step-up dosing period, and (b) for the first administration in each of the dosing cycles during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 3 weeks during the step-up dosing period, (iii) the anti-T cell antigen-binding molecule is administered repeatedly at intervals of once every 3 weeks, in dosing cycles where 2, 3, 4, or 5 administrations constitute one cycle during the maintenance dosing period, and (iv) pre-administration of a cytokine inhibitor is performed according to the following embodiment:

- the cytokine inhibitor is administered before administration of the anti-T cell antigen-binding molecule, which is (a) 6 days before each administration during the step-up dosing period, and (b) 6 days before the first administration in each of the dosing cycles during the maintenance dosing period;
- the cytokine inhibitor is administered before administration of the anti-T cell antigen-binding molecule, which is (a) 5 days before each administration during the step-up dosing period, and (b) 5 days before the first administration in each of the dosing cycles during the maintenance dosing period;
- the cytokine inhibitor is administered before administration of the anti-T cell antigen-binding molecule, which is (a) 4 days before each administration during the step-up dosing period, and (b) 4 days before the first administration in each of the dosing cycles during the maintenance dosing period;
- the cytokine inhibitor is administered before administration of the anti-T cell antigen-binding molecule, which is (a) 3 days before each administration during the step-up dosing period, and (b) 3 days before the first administration in each of the dosing cycles during the maintenance dosing period;
- the cytokine inhibitor is administered before administration of the anti-T cell antigen-binding molecule, which is (a) 2 days before each administration during the step-up dosing period, and (b) 2 days before the first administration in each of the dosing cycles during the maintenance dosing period;
- the cytokine inhibitor is administered before administration of the anti-T cell antigen-binding molecule, which is (a) 1 day before each administration during the step-up dosing period, and (b) 1 day before the first administration in each of the dosing cycles during the maintenance dosing period;
- the cytokine inhibitor is administered before administration of the anti-T cell antigen-binding molecule (a) on the same day as each administration during the step-up dosing period, and (b) on the same day as the first administration in each of the dosing cycles during the maintenance dosing period; or
- the cytokine inhibitor is administered simultaneously with administration of the anti-T cell antigen-binding molecule (a) on the same day as each administration during the step-up dosing period, and (b) on the same day as the first administration in each of the dosing cycles during the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- (a) on the same day as, or 1 day, 2 days, 3 days, 4 days, 5 days, or 6 days after administration during the step-up dosing period, and (b) on the same day as, or 1 day, 2 days, 3 days, 4 days, 5 days, or 6 days after the first administration in each of the dosing cycles during the maintenance dosing period (when the anti-T cell antigen-binding molecule is administered repeatedly at intervals of once every 3 weeks, and pre-administration of the cytokine inhibitor is performed (a) 6 days, 5 days, 4 days, 3 days, 2 days, or 1 day before each administration during the step-up dosing period and (b) 6 days, 5 days, 4 days, 3 days, 2 days, or 1 day before the said first administration in each of the dosing cycles during the maintenance dosing period); or
- (a) 1 day, 2 days, 3 days, 4 days, 5 days, or 6 days after administration during the step-up dosing period, and (b) 1 day, 2 days, 3 days, 4 days, 5 days, or 6 days after the first administration in each of the dosing cycles during the maintenance dosing period (when the anti-T cell antigen-binding molecule is administered repeatedly at intervals of once every 3 weeks, and pre-administration of the cytokine inhibitor is performed (a) on the same day as each administration during the step-up dosing period and (b) on the same day as the said first administration in each of the dosing cycles during the maintenance dosing period).

(Repeated administration of the anti-T cell antigen-binding molecule at intervals of once every 4 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 4 weeks during the step-up dosing period, (iii) the anti-T cell antigen-binding molecule is administered repeatedly at intervals of once every 4 weeks, in dosing cycles where 2, 3, 4, or 5 administrations constitute one cycle during the maintenance dosing period, and (iv) pre-administration of a cytokine inhibitor is performed (a) for each administration during the step-up dosing period, and (b) for the first administration in each of the dosing cycles during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 4 weeks during the step-up dosing period, (iii) the anti-T cell antigen-binding molecule is administered repeatedly at intervals of once every 4 weeks, in dosing cycles where 2, 3, 4, or 5 administrations constitute one cycle during the maintenance dosing period, and (iv) pre-administration of a cytokine inhibitor is performed according to the following embodiment:

- the cytokine inhibitor is administered before administration of the anti-T cell antigen-binding molecule, which is (a) 6 days before each administration during the step-up dosing period, and (b) 6 days before the first administration in each of the dosing cycles during the maintenance dosing period;
- the cytokine inhibitor is administered before administration of the anti-T cell antigen-binding molecule, which is (a) 5 days before each administration during the step-up dosing period, and (b) 5 days before the first administration in each of the dosing cycles during the maintenance dosing period;
- the cytokine inhibitor is administered before administration of the anti-T cell antigen-binding molecule, which is (a) 4 days before each administration during the step-up dosing period, and (b) 4 days before the first administration in each of the dosing cycles during the maintenance dosing period;
- the cytokine inhibitor is administered before administration of the anti-T cell antigen-binding molecule, which is (a) 3 days before each administration during the step-up dosing period, and (b) 3 days before the first administration in each of the dosing cycles during the maintenance dosing period;
- the cytokine inhibitor is administered before administration of the anti-T cell antigen-binding molecule, which is (a) 2 days before each administration during the step-up dosing period, and (b) 2 days before the first administration in each of the dosing cycles during the maintenance dosing period;
- the cytokine inhibitor is administered before administration of the anti-T cell antigen-binding molecule, which is (a) 1 day before each administration during the step-up dosing period, and (b) 1 day before the first administration in each of the dosing cycles during the maintenance dosing period;
- the cytokine inhibitor is administered before administration of the anti-T cell antigen-binding molecule (a) on the same day as each administration during the step-up dosing period, and (b) on the same day as the first administration in each of the dosing cycles during the maintenance dosing period; or
- the cytokine inhibitor is administered simultaneously with administration of the anti-T cell antigen-binding molecule (a) on the same day as each administration during the step-up dosing period, and (b) on the same day as the first administration in each of the dosing cycles during the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- (a) on the same day as, or 1 day, 2 days, 3 days, 4 days, 5 days, or 6 days after administration during the step-up dosing period, and (b) on the same day as, or 1 day, 2 days, 3 days, 4 days, 5 days, or 6 days after the first administration in each of the dosing cycles during the maintenance dosing period (when the anti-T cell antigen-binding molecule is administered repeatedly at intervals of once every 4 weeks, and pre-administration of the cytokine inhibitor is performed (a) 6 days, 5 days, 4 days, 3 days, 2 days, or 1 day before each administration during the step-up dosing period and (b) 6 days, 5 days, 4 days, 3 days, 2 days, or 1 day before the said first administration in each of the dosing cycles during the maintenance dosing period); or
- (a) 1 day, 2 days, 3 days, 4 days, 5 days, or 6 days after administration during the step-up dosing period, and (b) 1 day, 2 days, 3 days, 4 days, 5 days, or 6 days after the first administration in each of the dosing cycles during the maintenance dosing period (when the anti-T cell antigen-binding molecule is administered repeatedly at intervals of once every 4 weeks, and pre-administration of the cytokine inhibitor is performed (a) on the same day as each administration during the step-up dosing period and (b) on the same day as the said first administration in each of the dosing cycles during the maintenance dosing period).

(Repeated administration of the anti-T cell antigen-binding molecule at intervals of once every 5 weeks)

In one embodiment, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 5 weeks during the step-up dosing period, (iii) the anti-T cell antigen-binding molecule is administered repeatedly at intervals of once every 5 weeks, in dosing cycles where 2, 3, 4, or 5 administrations constitute one cycle during the maintenance dosing period, and (iv) pre-administration of a cytokine inhibitor is performed (a) for each administration during the step-up dosing period, and (b) for the first administration in each of the dosing cycles during the maintenance dosing period. As specific examples, in the combination therapy of the present disclosure, (i) a step-up dosing period and a subsequent maintenance dosing period are comprised for an anti-T cell antigen-binding molecule, (ii) the anti-T cell antigen-binding molecule is administered repeatedly at intervals of once every 5 weeks during the step-up dosing period, (iii) the anti-T cell antigen-binding molecule is administered repeatedly at intervals of once every 5 weeks, in dosing cycles where 2, 3, 4, or 5 administrations constitute one cycle during the maintenance dosing period, and (iv) pre-administration of a cytokine inhibitor is performed according to the following embodiment:

- the cytokine inhibitor is administered before administration of the anti-T cell antigen-binding molecule, which is (a) 6 days before each administration during the step-up dosing period, and (b) 6 days before the first administration in each of the dosing cycles during the maintenance dosing period;
- the cytokine inhibitor is administered before administration of the anti-T cell antigen-binding molecule, which is (a) 5 days before each administration during the step-up dosing period, and (b) 5 days before the first administration in each of the dosing cycles during the maintenance dosing period;
- the cytokine inhibitor is administered before administration of the anti-T cell antigen-binding molecule, which is (a) 4 days before each administration during the step-up dosing period, and (b) 4 days before the first administration in each of the dosing cycles during the maintenance dosing period;
- the cytokine inhibitor is administered before administration of the anti-T cell antigen-binding molecule, which is (a) 3 days before each administration during the step-up dosing period, and (b) 3 days before the first administration in each of the dosing cycles during the maintenance dosing period;
- the cytokine inhibitor is administered before administration of the anti-T cell antigen-binding molecule, which is (a) 2 days before each administration during the step-up dosing period, and (b) 2 days before the first administration in each of the dosing cycles during the maintenance dosing period;
- the cytokine inhibitor is administered before administration of the anti-T cell antigen-binding molecule, which is (a) 1 day before each administration during the step-up dosing period, and (b) 1 day before the first administration in each of the dosing cycles during the maintenance dosing period;
- the cytokine inhibitor is administered before administration of the anti-T cell antigen-binding molecule (a) on the same day as each administration during the step-up dosing period, and (b) on the same day as the first administration in each of the dosing cycles during the maintenance dosing period; or
- the cytokine inhibitor is administered simultaneously with administration of the anti-T cell antigen-binding molecule (a) on the same day as each administration during the step-up dosing period, and (b) on the same day as the first administration in each of the dosing cycles during the maintenance dosing period.

In a further embodiment, in the combination therapy of the present disclosure, in addition to pre-administration of a cytokine inhibitor according to the above embodiment, the cytokine inhibitor may be further administered to an individual on the same day as, or 1 day, 2 days, 3 days, 4 days, 5 days, or 6 days after the administration of the anti-T cell antigen-binding molecule. As specific examples, the cytokine inhibitor may be further administered as follows:

- (a) on the same day as, or 1 day, 2 days, 3 days, 4 days, 5 days, or 6 days after administration during the step-up dosing period, and (b) on the same day as, or 1 day, 2 days, 3 days, 4 days, 5 days, or 6 days after the first administration in each of the dosing cycles during the maintenance dosing period (when the anti-T cell antigen-binding molecule is administered repeatedly at intervals of once every 5 weeks, and pre-administration of the cytokine inhibitor is performed (a) 6 days, 5 days, 4 days, 3 days, 2 days, or 1 day before each administration during the step-up dosing period and (b) 6 days, 5 days, 4 days, 3 days, 2 days, or 1 day before the said first administration in each of the dosing cycles during the maintenance dosing period); or
- (a) 1 day, 2 days, 3 days, 4 days, 5 days, or 6 days after administration during the step-up dosing period, and (b) 1 day, 2 days, 3 days, 4 days, 5 days, or 6 days after the first administration in each of the dosing cycles during the maintenance dosing period (when the anti-T cell antigen-binding molecule is administered repeatedly at intervals of once every 5 weeks, and pre-administration of the cytokine inhibitor is performed (a) on the same day as each administration during the step-up dosing period and (b) on the same day as the said first administration in each of the dosing cycles during the maintenance dosing period).

VI. Combination therapies with an anti-glypican 3 (GPC3)/ T cell receptor complex bispecific antibody and a cytokine inhibitor

In one embodiment, the present disclosure provides a pharmaceutical composition comprising an anti-GPC3/ T cell receptor complex bispecific antibody, wherein the pharmaceutical composition is for use in its combination therapy with a cytokine inhibitor. In a different embodiment, the present disclosure provides a pharmaceutical composition comprising a cytokine inhibitor, wherein the pharmaceutical composition is for use in its combination therapy with an anti-glypican 3 (GPC3)/ T cell receptor complex bispecific antibody. In one embodiment, the cytokine inhibitor is administered to an individual before, simultaneously with, or after administration of the anti-GPC3/ T cell receptor complex bispecific antibody. In another embodiment, if CRS or signs of CRS develop in the individual after administration of the anti-GPC3/ T cell receptor complex bispecific antibody, the cytokine inhibitor is administered to the individual with an aim to treat the CRS.

Although not intended to be limiting, when a cytokine inhibitor is administered before or simultaneously with administration of an anti-GPC3/ T cell receptor complex bispecific antibody, the inhibitor has effects of preventing or alleviating the development of CRS associated with administration of the anti-GPC3/ T cell receptor complex bispecific antibody. In one embodiment, the cytokine inhibitor is administered 6 days, 5 days, 4 days, 3 days, 2 days, or 1 day before the administration of the anti-GPC3/ T cell receptor complex bispecific antibody, or on the same day as but before the administration of the bispecific antibody. Alternatively, the cytokine inhibitor is administered on the same day as administration of the anti-GPC3/T cell receptor complex bispecific antibody, and simultaneously with the bispecific antibody (pre-administration of the cytokine inhibitor). For specific examples of the combination therapy comprising pre-administration of the cytokine inhibitor, see the various embodiments described above. Furthermore, when the cytokine inhibitor is administered after administration of the anti-GPC3/ T cell receptor complex bispecific antibody and before development of CRS, it has effects of preventing or alleviating the CRS which may develop afterward. Furthermore, when the cytokine inhibitor is administered after administration of the anti-GPC3/ T cell receptor complex bispecific antibody and after development of CRS or signs of CRS, the inhibitor has effects of treating CRS and alleviating the symptoms of CRS. In one embodiment, pre-administration of the cytokine inhibitor prevents or alleviates development of CRS associated with administration of the anti-GPC3/ T cell receptor complex bispecific antibody without significantly compromising the drug efficacy of the anti-GPC3/ T cell receptor complex bispecific antibody (for example, T cell-dependent cellular cytotoxicity (TDCC) and antitumor effects).

VII. Production Methods and Kits

The present invention also provides a kit for use in the method of the present invention, which comprises the multispecific antigen-binding molecule of the present invention or the multispecific antigen-binding molecule produced by the production method of the present invention. In addition, the kit may include in its package a pharmaceutically acceptable carrier, medium, and instructions describing the method of use, and such.

The present invention also relates to a multispecific antigen-binding molecule of the present invention or a multispecific antigen-binding molecule produced by the production method of the present invention for use in the method of the present invention.

The present invention also relates to nucleic acids encoding these molecules, vectors into which the nucleic acids have been introduced, cells containing the nucleic acids or the vectors, methods for producing the molecules by culturing the cells, and molecules produced by the methods.

For example, if the T-cell antigen-binding molecule is a multispecific antigen-binding molecule, the multispecific antigen-binding molecule can be produced by introducing into an appropriate host cell, a nucleic acid encoding the multispecific antigen-binding molecule or a vector into which the nucleic acid has been introduced and t culturing the host cell. When the multispecific antigen-binding molecule is secreted, the multispecific antigen-binding molecule of interest can be recovered from the culture supernatant. A method for purifying the multispecific antigen-binding molecule of interest from the culture is also known. On the other hand, if the cytokine inhibitor is a protein component such as an antibody, it can be produced in the same manner as the multispecific antigen-binding molecule by introducing into an appropriate host cell, the nucleic acid encoding the cytokine inhibitor molecule or the vector into which the nucleic acid is introduced, and culturing the host cell.

In the present disclosure, the T cell antigen-binding molecule and the cytokine inhibitor can be independently packaged with a pharmaceutically acceptable carrier, medium, instructions describing the method of use, and such. Alternatively, both can be formulated and then combined to form a kit. Specifically, the kit can include, for example, a package containing a formulated T cell antigen-binding molecule and a package containing a formulated cytokine inhibitor. When each of the formulations constituting the kit is a lyophilized one or a concentrated liquid formulation, a liquid medium for dissolving or diluting the formulation can be combined with the kit.

It should be noted that all prior art documents cited herein are incorporated herein by reference.

[Example]

[Example 1]

ERY974 single-drug dose-escalation study in patients with Glypican-3 (GPC3)-positive advanced cancer

The ERY101EG study is a phase I clinical trial targeting patients with Glypican-3-positive advanced solid tumors, and the dose was escalated from the initial dose of 0.0031 μg/kg as determined by the Minimum Anticipated Biological Effect Level (MABEL) and NOAEL. In the present study, the tolerability of ERY974 was evaluated in one to three subjects per cohort, and the dose (or target dose) was gradually increased when proceeding from one cohort to the next.

In the clinical trial, ERY974 was administered intravenously once every week. The intravenous administration of ERY974 was performed for three hours, and then, flush with physiological saline solution was performed for one hour. When the clinical trial investigator determined that there was no problem with the safety of the subjects, the administration duration of ERY974 was allowed to be shortened to a minimum of two hours.

In the present study, pre-administration of a steroid was performed in all cases to handle the cytokine release syndrome. Specifically, the pre-administration was defined such that at the first and second administrations of ERY974, dexamethasone is orally administered in the afternoon of the day before the ERY974 administrations; one hour before the start of the ERY974 administrations, dexamethasone is further administered intravenously, and antihistamine and paracetamol are pre-administered.

According to the rules of the protocol, up to the first case of cohort 5, for the first and second administrations of ERY974, the dose of dexamethasone pre-administered in the afternoon of the day before the ERY974 administrations was 8 mg by oral administration, and at the first administration of ERY974, the dose of dexamethasone pre-administered one hour before the start of ERY974 administration was 8 mg by intravenous administration. For subjects from the second case in cohort 5 to cohort 8A, the dose of dexamethasone pre-administered one hour before the start of ERY974 administration was increased to 20 mg. The dose and dosing schedule of dexamethasone in each cohort were those recommended by the Safety Monitoring Committee (SMC) and decided by the sponsor for use during dose escalation.

(1-1) Fixed dose regimen

For cohorts 1-5, the dose of ERY974 decided for each cohort was repeatedly administered by intravenous drip infusion at intervals of once every week (QW) until the discontinuation criteria were met. Then, the dose limiting toxicity (DLT) was evaluated using the period from the first administration to the seventh day after the third administration (including the administration day) as the DLT evaluation period. This is called a fixed dose regimen. The administered dose for each cohort in cohorts 1-5 is shown in Table 10.

[Table 10]

| | |
|---|---|
| Cohort 1 | 0.0031 μg/kg |
| Cohort 2 | 0. 009 μg/kg |
| Cohort 3 | 0. 027 μg/kg |
| Cohort 4 | 0.081 μg/kg |
| Cohort 5 | 0. 24 μg/kg |

(1-2) Step-up dosing regimen

From cohort 6 and beyond, ERY974 was repeatedly administered by intravenous drip infusion at QW in the same manner as in cohorts 1 to 5, but the dose was increased sequentially for the first, second, third, and/or fourth administrations. That is, first, a low dose of ERY974 was administered to the subjects for the first (Day 1) administration (0.081 μg/kg in cohort 6 and 0.12 μg/kg in cohort 7 and beyond). Then, in the second and subsequent administrations, the dose to the subject was sequentially increased until the dose reached the target dose designated for each cohort.

Specifically, in cohort 6, 0.081 μg/kg was administered on Day 1 and 0.24 μg/kg was administered from Day 8 and beyond, and in cohort 7, 0.12 μg/kg was administered on Day 1 and 0.24 μg/kg was administered on Day 8 and beyond. In cohorts 8A, 9A, and 10A, 0.12 μg/kg was administered in the first (Day 1) administration and the target dose designated for each cohort was administered in the second (Day 8) administration (this is called the Fixed Day 1 regimen). Next, in cohorts 8B and 9B, 0.12 $\mu$g/kg was administered in the first (Day 1) administration and second (Day 8) administration, 0.24 $\mu$g/kg was administered in the third (Day 15) administration, and the target dose designated for each cohort was administered in the fourth (Day 22) administration (this is called the Fixed Day 15 regimen). DLT was evaluated for the Fixed Day1 regimen, by setting the DLT evaluation period from the first administration to the seventh day after the third administration (including the administration day), and for the Fixed Day15 regimen, by setting the DLT evaluation period from the first administration to the seventh day after the fourth administration (including the administration day).

In the present study, the target doses were 0.24 $\mu$g/kg for cohorts 6 and 7, 0.36 $\mu$g/kg for cohorts 8A and 8B, 0.54 $\mu$g/kg for cohorts 9A and 9B, and 0.81 $\mu$g/kg for cohort 10A. From cohort 1 to cohort 10A, a total of 29 subjects were administered ERY974.

[Example 2]

Evaluation items and evaluation results in the ERY101EG study

The ERY101EG mainly evaluated the occurrence of adverse events (AE) and the development of dose limiting toxicity (DLT) in the dosage and administration for each cohort.

(2-1) Adverse event (AE)

The main AEs associated with ERY974 administration (manifested in four or more out of 29 patients) were cytokine release syndrome (CRS, 66%), fever (24%), nausea (17%), and fatigue (14%). The severity of the CRS was mostly Grade 1/2, while Grade 3 events were also seen in four patients. Most of the CRS manifested within two days after ERY974 administration. Accordingly, major AE associated with ERY974 was evaluated to be CRS. The severity of CRS was evaluated based on the National Cancer Institute Common Terminology Criteria for Adverse Events (CTCAE) v4.03 (the above Table 6), in a similar manner to other adverse events at the start of the study, and for cohort 8B and beyond, it was evaluated based on Lee's criteria in 2014 (the above Table 8).

(2-2) Dose limiting toxicity (DLT)

The severity of AE was evaluated based on CTCAE v4.03. Among the AEs manifested during the DLT evaluation period, those predetermined to be events for which a causal relationship with ERY974 cannot be ruled out and that interfere with dose increase and/or continued administration of ERY974 were defined as DLT, and manifestation of DLT in each cohort was evaluated.

DLT was not observed in cohorts 1 to 9. DLT was reported in two out of three enrolled in cohort 10A. In both of the two patients, due to cytokine release syndrome (one case of Grade 2 and one case of Grade 3) that manifested after administration of 0.81 $\mu$g/kg of ERY974 on the second (Day 8) administration, the third (Day 15) administration of ERY974 had to be postponed. Therefore, this corresponded to the above-mentioned predetermined DLT standard, and it was judged to be DLT. In cohort 10A, one subject who did not show DLT also manifested cytokine release syndrome (Grade 2) after administration of ERY974 at 0.81 $\mu$g/kg in the second (Day 8) administration, and the dose of ERY974 was reduced in the third (Day 15) administration. Therefore, the regimen/dose of cohort 10A was concluded to be dosage and administration intolerable under steroid pre-administration conditions.

(2-3) Comparison of Fixed Day 1 regimen with Fixed Day 15 regimen

Furthermore, the Fixed Day 1 regimen was compared with the Fixed Day 15 regimen when the target doses were the same. Specifically, in cohorts 8A and 9A (Fixed Day 1 regimen) and cohorts 8B and 9B (Fixed Day 15 regimen), the manifestation frequency and severity of CRS, the main AE of ERY974, were compared after the first two ERY974 administrations at the target dose (ERY974 administrations on the second (Day 8) and third (Day 15) administrations in the Fixed Day 1 regimen, or on the fourth (Day 22) and fifth (Day 29) administrations in the Fixed Day 15 regimen). As a result, the number of cases manifesting CRS was 1, 0, 3, and 3 case(s), which are Grade 1 or 2, in cohorts 8A, 8B, 9A, and 9B (three patients each), respectively, and no clear difference was observed between cohorts 8A and 8B and between cohorts 9A and 9B. Since cohort 10B (Fixed Day 15 regimen, target dose of 0.81 $\mu$g/kg) was not tested in the ERY101EG study, DLT detection was not performed in the Fixed Day 15 regimen examination, and therefore one cannot conclude that cohort 10B had intolerable dosage and administration. However, there were no indications from the results up to cohort 9B suggesting that the Fixed Day 15 regimen was superior to the Fixed Day 1 regimen in terms of CRS suppression.

(2-4) Antitumor effect

Of the 29 patients enrolled in this study, partial response (PR) was observed in one patient enrolled in cohort 9B (0.54 mg/kg). Other patients had stable disease (SD) or progressive disease (PD). When the blood concentration of ERY974 in the subject who showed a partial response was checked, it corresponded to the EC10-EC30 concentration calculated from the cell growth inhibitory activity *in vitro.* On the other hand, to reach the dose to attain the target blood concentration in 70% of the subjects, achieving a higher dose seemed necessary.

(2-5) Discussion

Given the course of the above study, observation results that DLT manifested in cohort 10A, and the dose at which drug efficacy is estimated, adopting a method for managing CRS manifestation separate from a different Fixed Day regimen examination seemed necessary.

[Example 3]

Analysis of cytokine release syndrome (CRS) developed in the ERY101EG study, and increase of IL-6 and use of Tocilizumab in the ERY101EG study

(3-1) Cytokine level after ERY974 administration

In the ERY101EG study, an increase in IL-6 associated with ERY974 administration was confirmed in some subjects who manifested cytokine release syndrome (CRS). Three subjects enrolled in cohort 10A manifested CRS requiring interruption or dose reduction of ERY974 after the second (Day 8) administration of ERY974 (after administration of the first target dose of 0.81 $\mu$g/kg) (2 cases of Grade 2 and 1 case of Grade 1), and in these 3 patients, increase in IL-6, IL-8 and IL-10 was observed within 24 hours after the end of the second (Day 8) study drug administration (Figs. 4A, 4B and 4C) (the lower limit of quantification was plotted if the increase was at or below the lower limit of quantification). CRS was observed in two of the three patients (subject IDs 840010006 and 840010007) after the second (Day 8) study drug administration, and Tocilizumab was used as described below. One of them (subject ID 840010007) showed another increase in cytokine levels including IL-6 after the third (Day 15) administration, but did not manifest CRS symptoms. In the other subject, CRS (Grade 3) manifested again after re-administration.

(3-2) Use of Tocilizumab for the treatment of CRS

In the ERY101EG study, Tocilizumab was used alone or in combination with a steroid and such for the treatment of CRS in some patients at the discretion of the clinical trial investigator (Table 11). The severity of CRS ranged from Grade 1 to Grade 3. Tocilizumab was administered on the same day as the manifestation of CRS, with the exception of two patients, two cases (cohort 10A, 840010006, 840010007). In all of the Tocilizumab-administered cases, CRS recovered four days after the day of Tocilizumab administration.

[Table 11]

## List of patients for which Tocilizumab was used in the 101EG study

| Patient ID | Cohort | Number of the administrations of this agent immediately before Tocilizumab administration (Dose) | Grade of CRS | Timing of Tocilizumab administration | Dose of Tocilizumab | Duration from Tocilizumab administration to recovery from CRS | Number of the administrations of this agent after Tocilizumab administration (Dose) | Manifestation of CRS after Tocilizumab administration and until the next administration of this agent | Presence or absence of CRS recurrence |
|---|---|---|---|---|---|---|---|---|---|
| 840020017 | 8A | 1st administration (0. 12 $\mu$ g/kg) | 2 (Non-critical) | Same day as CRS manifestation | Not known | 0 days | 3 times (0. 36 $\mu$ g/kg) | None | Absent |
| 840060002 | 8B | 3rd administration (0. 24 $\mu$ g/kg) | 2 | Same day as CRS manifestation | 8mg/kg | 2 days | 9 times (0. 36 $\mu$ g/kg) | None | Absent |
| 840020021 | 9A | 3rd administration (0. 54 $\mu$ g/kg) | 1 | Same day as CRS manifestation | 4mg/kg | 0 days | 9 times (0. 54 $\mu$ g/kg) | None | Absent |
| 840020022 | 9A | 3rd administration (0. 54 $\mu$ g/kg) | 2 | Within 2 hours from CRS manifestation | 4mg/kg | 0 days | 3 times (0. 54 $\mu$ g/kg) | None | Absent |
| 840020023 | 9B | 4th administration (0. 54 $\mu$ g/kg) | 2 | Same day as CRS manifestation | 4mg/kg | 0 days | 19 times (0. 54 $\mu$ g/kg) | None | Absent |
| 840010006 | 10A | 2nd administration (0. 81 $\mu$ g/kg) | 3 | 3 days after CRS manifestation | 8mg/kg | 4 days | 1 time (0. 54 $\mu$ g/kg) | None | Present |
| | | 3rd administration (0. 54 $\mu$ g/kg) | 3 | Same day as CRS manifestation | 8mg/kg | 3 days | 0 times | Not applicable | Not applicable |
| 840010007 | 10A | 2nd administration (0. 81 $\mu$ g/kg) | 2 | 6 days after CRS manifestation | 8mg/kg | 1 day | 2 times (0. 54, 0. 81 $\mu$ g/kg) | None | Absent |

[Example 4]

Simulation of pre-administration of Tocilizumab in ERY974 monotherapy

(4-1) Setting estimated effective concentration of Tocilizumab

It is known that the elimination of Tocilizumab involves not only the elimination pathway of non-specific antibodies but also the elimination mediated by binding with the target IL-6R. The elimination mediated by the binding with IL-6R was shown saturated at high concentration of Tocilizumab, and the saturation of this elimination is considered to be the saturation of the binding between Tocilizumab and IL-6R (IL-6R). Therefore, it is presumed that saturation of the binding with IL-6R is a sufficient condition for IL-6-mediated signal inhibition, and it was considered important for the serum concentration of Tocilizumab to maintain the concentration at which the binding between Tocilizumab and IL-6R is considered to be always saturated, that is, the concentration at which elimination of Tocilizumab mediated by binding with IL-6R is saturated.

In a report by a population pharmacokinetic model incorporating the Michaelis-Menten equation for the elimination of Tocilizumab from serum, the results of analysis by Frey et al. (Frey, N., Grange, S. and Woodworth, T. (2010), Population Pharmacokinetic Analysis of Tocilizumab in Patients with Rheumatoid Arthritis. The Journal of Clinical Pharmacology, 50: 754-766) (hereinafter referred to as "the article by Frey et al.") report 2.7 $\mu$g/mL as a Michaelis coefficient (Km) for target-mediated elimination. From the $K_m$ value, the serum concentration of Tocilizumab required for binding to 80% or more of IL-6R is theoretically 10.8 $\mu$g/mL or more, and the simulation results showed non-linear elimination below 10 $\mu$g/mL (Fig. 5A).

Furthermore, Gibiansky et al. have reported a population pharmacokinetic model that expresses the process of formation and elimination of IL-6R in serum (IL-6R has membranebound IL-6R and soluble IL-6R, and IL-6R in serum is the latter) as indirect response model and incorporates binding to Tocilizumab for the elimination of the unbound IL-6R (referring to IL-6R that does not form a complex with IL-6) (Gibiansky, L. and Frey, N., Linking interleukin-6 receptor blockade with tocilizumab and its hematological effects using a modeling approach. J Pharmacokinet Pharmacodyn. 2012 Feb; 39 (1): 5-16) (hereafter referred to as "the article by Gibiansky et al."). In the report by Gibiansky et al., non-linear elimination is clearly visible when the median serum concentration of Tocilizumab is 10 $\mu$g/mL or less (Fig. 5B). From the above, the estimated effective serum concentration (target concentration) of Tocilizumab was set to 10 $\mu$g/mL.

(4-2) Route of Tocilizumab administration

In the ERY101EG study, most of CRS following ERY974 administration was reported from the day of the administration to the following day; therefore, the route selected for administration of Tocilizumab was intravenous administration that allows the serum concentration of Tocilizumab to be maintained at a high concentration by 48 hours after ERY974 administration (Figs. 6A and 6B).

(4-3) Setting the dose and dosing frequency of Tocilizumab that allow maintenance of the target concentration

Tocilizumab has been approved by the US Food and Drug Administration (FDA) for use in the treatment of severe or life-threatening CRS associated with CAR-T cell therapy, and administration of 8 mg/kg/8h to patients weighing 30 kg or more has been accepted. Therefore, 8 mg/kg was selected as a dose of Tocilizumab per administration. According to the simulation results (Figs. 5A and 5B), exposure exceeding the target concentration could be obtained in 95% of the patients between 7 and 14 days after administration, and in 50% of the patients between 14 and 21 days after administration. Based on the above, when 8 mg/kg was selected as a dose of Tocilizumab per administration, patients who can maintain a trough concentration (referring to the lowest blood drug concentration under steady state when the drug is administered repeatedly) exceeding the target of 10 $\mu$g/mL at 21 days or more after administration, were less than half of the patients in any model; therefore, the recommended dosing interval of Tocilizumab was three weeks or less.

In addition, the model reported in the above article by Gibiansky et al. allows simulation of unbound IL-6R, and change in the unbound IL-6R over time when Tocilizumab is administered at a dose of 8 mg/kg per administration is shown in Fig. 7. According to the simulation results, the unbound IL-6R was 1.0 to 2.3 ng/mL in 95% of the patients up to 7 days after the administration, whereas it was increased to 3.9 to 8.3 ng/mL at 14 days after the administration, and to 4.5 to 54 ng/mL at 21 days after the administration. At 28 days after the administration, patients having nearly the same amount of unbound IL-6R as before the administration were estimated to be about 30%. The above suggested that, when 8 mg/kg is selected as a dose of Tocilizumab per administration, the number of patients in which the inhibitory effect of sIL-6R cannot be expected increases beyond three weeks after administration.

On the other hand, long-term high exposure to Tocilizumab is not recommended due to concerns of side effects such as infection, and the highest number of reported CRS manifestation in the ERY101EG study was during the first to third administrations (for example, see Example 3-2). Therefore, it was decided to intravenously administer Tocilizumab at 8 mg/kg before the first administration of ERY974 at the start of the ERY102JP study (details are shown in Example 5), and to examine the inhibitory effect the Tocilizumab administration on CRS until the third administration of ERY974.

(4-4) Estimated value of IL-6 concentration that increases after ERY974 administration and examination of dosing frequency of Tocilizumab

In the ERY101EG study, the maximum serum IL-6 concentration was about 10 ng/mL (approximately 0.4 nM) (see Example 3), which is higher than the concentration reported for rheumatic patients (0.7-981 pg/mL) (see the above article by Frey et al.). This suggested that it may not be possible to sufficiently inhibit the IL-6 signal increased by CRS even at concentrations where efficacy of Tocilizumab can be demonstrated in rheumatic patients. Therefore, assuming that IL-6 increases after ERY974 administration, the IL-6/IL-6R complex concentrations were calculated assuming situations where IL-6 concentrations are 300, 1000, 3000, 5000, and 10000 pg/mL in the presence of unbound sIL-6R calculated from the Gibiansky model (it is understood that the higher the IL-6/IL-6R complex concentrations are, the more IL-6 signaling occurs). The calculation was performed based on the following formula, presuming that the reaction between IL-6 and IL-6R is under equilibrium. The Kd value used is the value reported by Baran et al. (Baran P, Hansen S, Waetzig GH, et al. The balance of interleukin (IL)-6, IL-6 soluble IL-6 receptor (sIL-6R), and IL-6 sIL- 6R sgp130 complexes allows simultaneous classic and trans-signaling. J Biol Chem. 2018; 293 (18): 6762-6775.) (hereinafter referred to as "the article by Baren et al."), which is 22 nM.

$$Kd = \frac{complex(IL-6_{bound}, sIL-6R_{bound})}{IL-6_{unbound} \cdot sIL-6R_{unbound}}$$

Assuming that CRS will continue to occur during QW administration of ERY974, the dosing interval of Tocilizumab (8 mg/kg per dose) was examined for administration once every week (QW), once every 2 weeks (Q2W), once every 3 weeks (Q3W), and once every 4 weeks (Q4W), and the results are shown in Figures 8A to 8D. Here, it is presumed that a certain concentration of the IL-6/IL-6R complex is necessary for the development of CRS; however, the concentration that causes CRS has not been specified by the findings so far. However, at the same serum Tocilizumab concentration, the speculation is that the higher the IL-6 concentration is, the higher the IL-6/IL-6R complex concentration is, and the more easily CRS is initiated (Fig. 11). This suggests that the target concentration estimated from rheumatic patients may not be sufficient for suppressing CRS after ERY974 administration. Thus, if the target concentration of Tocilizumab estimated from rheumatoid patients is not sufficient for suppressing CRS after ERY974 administration, administering Tocilizumab multiple times to keep the IL-6/IL-6R complex concentration low, is expected to yield effects of suppressing IL-6-mediated signals (Figs. 9 and 10).

As described above, at the start of the ERY102JP study (its details are shown in Example 5), 8 mg/kg of Tocilizumab will be intravenously administered before the first administration of ERY974, and the inhibitory effect of the Tocilizumab administration on CRS until the third administration of ERY974 will be examined. However, if an increase in IL-6 caused at the time of the second or subsequent administration of ERY974 is observed and CRS is reported in this study, administering Tocilizumab multiple times is expected to suppress IL-6-mediated signals even if the serum IL-6 concentration is high, as described above (Figs. 9 to 11). Therefore, in such a case, it is assumed that the number of administrations of Tocilizumab and/or the dosing interval are changed.

(4-5) Examination of validity and dose change for pre-administration of tocilizumab

The direct action of ERY974 is activation of CD3+T cells, but increase in IL-6, thought to be causing CRS, has been reported to be contributed by T cell activation and also by secondary activation of immune cells (see, for example, Giavridis, Theodoros et al., CAR T cellinduced cytokine release syndrome is mediated by macrophages and abated by IL-1 blockade. Nature medicine vol. 24,6 (2018): 731-738.; Barrett, David M et al., Toxicity management for patients receiving novel T-cell engaging therapies. Current opinion in pediatrics vol. 26,1 (2014): 43-9.; and Yoshika Iwata, et al., Different players generate positive responses in two in vitro cytokine assay formats with aqueous and immobilized TGN1412 analog. Biochemical and Biophysical Research Communications, vol 502, 1 (2018): 91-7). Considered in this way, it is inferred that pre-administration of Tocilizumab can suppress the increase in serum IL-6 associated with ERY974 administration by suppressing the secondary activation of immune cells in advance. If pre-administration of Tocilizumab can suppress the increase in serum IL-6 associated with ERY974 administration, it will be theoretically possible to reduce the concentration of Tocilizumab required to suppress CRS. Therefore, even at concentrations lower than the dose of Tocilizumab per administration in applications for treating CRS (8 mg/kg for patients weighing 30 kg or more, see Example 4-3), CRS suppression effect can be expected from pre-administration of Tocilizumab. Based on the above hypothesis, in the ERY102JP study, the optimal dose of Tocilizumab per administration (for example, a dose per administration is set to be lower than 8 mg/kg) is assumed to be examined by taking into consideration the serum concentration of Tocilizumab, IL-6 concentration, and AE report that are obtained after the start of the study.

[Example 5]

Pre-administration of Tocilizumab in the clinical trials of ERY974

(5-1) Outline and purpose of the ERY102JP study

The ERY102JP study is a phase I clinical trial targeting patients with Glypican-3 (GPC3)-positive advanced solid tumors, comprising single-agent administration of ERY974. This study is a single-center, open-label, dose-escalation study that has been designed for evaluating the safety, tolerability, pharmacokinetics, and antitumor effects when patients with GPC3-positive advanced/recurrent solid tumors are subjected to single-agent administration of ERY974 after pre-administration of Tocilizumab. Furthermore, this enables evaluation of the safety, efficacy, and pharmacokinetics of Tocilizumab as a preventive measure against CRS associated with ERY974 administration.

The number of subjects scheduled to participate in this clinical trial is 15 to 30 (3 to 6 in each cohort, 5 cohorts in total) at the initial stage of preparing the clinical trial protocol, but the results of examining this clinical trial and results of overseas clinical trials and such may lead to change in the method of administration of ERY974 and/or Tocilizumab, and change in the planned number of subjects, including those examined for specific carcinomas.

The primary objective of this study is to evaluate the safety and tolerability and confirm the pharmacokinetics, as well as to evaluate the dose limiting toxicity (DLT) and determine the maximum tolerated dose (MTD) by subjecting patients with GPC3-positive advanced/recurrent solid tumors to single-agent administration of ERY974 at doses (or administration methods) that differ for each cohort after pre-administration of Tocilizumab. In addition, as a secondary objective, the antitumor effect of single-agent administration of ERY974 after pre-administration of Tocilizumab in patients with GPC3-positive advanced/recurrent solid tumors, is evaluated. Furthermore, for exploratory purposes, the evaluation of cytokines and pharmacodynamic markers, and association with disease status, safety, and antitumor effect resulting from single-agent administration of ERY974 after pre-administration of Tocilizumab in patients with GPC3-positive advanced/recurrent solid tumors are examined, and the safety, efficacy and pharmacodynamics of Tocilizumab as an agent to be pre-administered relative to ERY974 are confirmed.

(5-2) Dose of ERY974

In this study, ERY974 is administered intravenously at a dose per administration indicated in Table 12 shown below at intervals of once every week (QW) until the administration is judged to be ineffective or an unacceptable adverse event manifests. In this study, in principle, the dose of ERY974 per administration shall not be changed, but if clinically necessary due to development of adverse events and the like, the dose may be reduced at the discretion of the clinical trial investigator.

[Table 12]

| Doses of ERY974 | |
| --- | --- |
| Cohort | Dose of ERY974 |
| Cohort 1 | 0.12 $\mu$g/kg |
| Cohort 2 | 0.24 $\mu$g/kg |
| Cohort 3 | 0.50 $\mu$g/kg |
| Cohort 4 | 0.80 $\mu$g/kg |
| Cohort 5 | 1.20 $\mu$g/kg |

The intravenous administration of ERY974 is performed in three hours, and then flush with physiological saline solution is performed in one hour. If the clinical trial investigator judges that there is no problem regarding the safety of the subject, the administration duration of ERY974 can be shortened to a minimum of one hour.

(5-3) Pre-administration of Tocilizumab

All subjects receive a dose of Tocilizumab per administration, as shown in Table 13 below, by intravenous administration over a course of one hour, before the first administration of ERY974 (Cycle 1 Day 1). Administration of Tocilizumab is to be terminated no later than two hours before the start of ERY974 administration.

[Table 13]

| Doses of Tocilizumab | |
|---|---|
| When the body weight is 30 kg or more | Tocilizumab at 8 mg/kg |
| When the body weight is less than 30 kg | Tocilizumab at 12 mg/kg |

In subjects who manifest cytokine release syndrome (CRS) or an event judged to suggest CRS associated with ERY974 administration in the first (cycle 1) administration of ERY974, administration of Tocilizumab before ERY974 at the doses shown in Table 13 above is tolerated even in the second (cycle 2) or subsequent ERY974 administrations.

If, with advancement of the study, the trial sponsor decides that pre-administration of Tocilizumab is required even at the second (cycle 2) or subsequent administrations of ERY974 when proceeding to the next cohort (that is, when increasing the dose of ERY974), the study is designed to be such that for the cohort and beyond, , pre-administration of Tocilizumab can be made required for the second and subsequent designated ERY974 administrations in addition to the first ERY974 administration.

(5-4) Use of Tocilizumab and/or steroids for the treatment of CRS

In this study, if CRS or an event judged to suggest CRS is observed after ERY974 administration, therapeutic administration of Tocilizumab and/or systemic corticosteroids is allowed. The dose in this case should be in accordance with Table 13 shown above. If multiple doses of Tocilizumab are required for CRS treatment purposes, the intervals after the previous Tocilizumab administration have to be eight hours or more, and in principle, the number of administrations of Tocilizumab with respect to one ERY974 administration is up to a maximum of four administrations including the pre-administration, while five or more administrations are allowed in limited cases where the clinical trial investigator decides that it is necessary upon sufficiently examining the medical needs and risks.

(5-5) DLT evaluation

In the ERY102JP study, one cycle is 7 days, and 28 days spanning from the first (cycle 1 Day 1) administration of ERY974 to the day before the fifth (cycle 5 Day 1) administration of ERY974 (+3 days if the administration is postponed within the permissible range) is regarded as the DLT evaluation period. If the administration of ERY974 is postponed, the DLT evaluation period is extended, but the maximum DLT evaluation period shall not exceed 42 days (+3 days if the administration is postponed within the permissible range). Among the adverse events manifested during the DLT evaluation period, the events for which a predetermined causal relationship with the ERY974 cannot be ruled out are defined as DLT. Regarding nonhematologic toxicity other than hepatotoxicity as the main DLT event, the toxicity rated as Grade 3 or higher as specified in CTCAE ver.5.0 (Table 7), for which a causal relationship with ERY974 administration cannot be ruled out, is defined as DLT; however, regarding CRS, even if it is Grade 3 or higher, it is not defined as DLT if it improves or recovers to Grade 1 or lower within three days by appropriate treatment.

To confirm the tolerability of ERY974, three to six subjects are enrolled in each cohort to perform a DLT evaluation, and based on the DLT manifestation status observed during the DLT evaluation period, the clinical trial investigator and sponsor decide, upon discussion, whether or not to proceed to the next ERY974 dose-stage cohort. The maximum dose at which the rate of DLT manifestation becomes less than 33% is defined as MTD.

[Example 6]

Evaluation items and evaluation results in the ERY102JP study

(6-1) Main analysis

The main analysis of this clinical trial are carried out by the following procedure.

- Safety

Development of adverse event (AE), and development of dose-limiting toxicity (DLT) are assessed for each cohort, and the clinical test values and vital signs of the subjects are observed. For AE, particularly regarding CRS, the development frequency and severity (Grade) of CRS in each cohort are evaluated.

- Pharmacokinetics

Plasma ERY974 concentration and serum Tocilizumab concentration of each of the subjects are observed over time, and evaluation and comparison are carried out for each cohort. In addition, the pharmacokinetic parameters of ERY974 and Tocilizumab (AUC, Cmax, Tmax, etc.) are calculated, and the effects of anti-ERY974 antibody and anti-Tocilizumab antibody on their respective pharmacokinetics are evaluated.

- Efficacy (anti-tumor effect)

In determination of the antitumor effect of ERY974, that is, the tumor response, based on RECIST v1.1, and the overall response rate (ORR), disease control rate (DCR), progressionfree survival (PFS), and the duration of response (DoR) are determined.

(6-2) Expected results and beneficial effects

Pre-administration of Tocilizumab prevents or alleviates the development of CRS associated with ERY974 administration. In the ERY101EG study (without pre-administration of Tocilizumab), all three subjects enrolled in cohort 10A evaluated on the Fixed Day 1 regimen developed CRS (Grade 2 or 3) after administration of ERY974 at the target dose of 0.81 $\mu$g/kg, and required postponement or dose reduction of ERY974 administration (see Example 2-2). However, in the ERY102JP study in which Tocilizumab is pre-administered, it can be confirmed that even if ERY974 is administered at a dose of 0.80 $\mu$g/kg (cohort 4) or higher, CRS which is Grade 3 or higher and requires discontinuation of ERY974 administration does not develop. This can confirm that the pre-administration of Tocilizumab is effective in preventing or alleviating CRS associated with ERY974 administration. In addition, the effectiveness of pre-administration of Tocilizumab in preventing or alleviating CRS associated with ERY974 administration allows a dose of ERY974 per administration to be increased, and allows continuous administration at this dose until the administration is judged to be ineffective, or until an unacceptable adverse event other than CRS manifests. This makes it possible to administer a dose of ERY974 capable of exerting sufficient antitumor effects to a patient without causing serious CRS. In another aspect, when a pharmaceutical agent predicted to develop CRS is administered to a patient, the patient must usually be hospitalized and managed after the pharmaceutical agent is administered, but if Tocilizumab is pre-administered, hospitalization for management of the patient after ERY974 administration will become unnecessary, and this may contribute to improving the QOL of patients.

(6-3) One embodiment of ERY974 and Tocilizumab after approval

Although the embodiment is not limited, ERY974 is used in combination with Tocilizumab, and in the combination therapy, Tocilizumab is administered to an individual before or simultaneously with ERY974 administration. In one embodiment, Tocilizumab is administered 6 days, 5 days, 4 days, 3 days, 2 days, or 1 day before, or on the same day as but before administration of ERY974. Alternatively, Tocilizumab may be administered simultaneously with ERY974 on the day of ERY974 administration. At this time, a pharmaceutical composition comprising ERY974 may further contain a document instructing to use a cytokine inhibitor in combination. In addition, a pharmaceutical composition comprising Tocilizumab may further comprise a document instructing to use an anti-T cell antigen binding molecule in combination.

[Example 7]

Effect of pre- and post-administration of anti-mouse IL6 receptor antibody (MR-16-1) on the drug efficacy of ERY974 surrogate antibody (GC33/2C11)

(7-1) Pre-administration of anti-mouse IL6 receptor antibody (MR-16-1)

In a test using a syngeneic mouse model, a study for evaluating the effect of pre-administration of an anti-mouse IL6 receptor antibody, MR-16-1 (see Okazaki M, et al., Characterization of anti-mouse interleukin-6 receptor antibody, Immunol Lett. 2002 Dec 3;84(3):231-40; and Saito T, et al., Preparation of soluble murine IL-6 receptor and anti-murine IL-6 receptor antibodies, J. Immunol., 47 (1991), pp. 168-173), on the drug efficacy of ERY974 surrogate antibodies was carried out as follows. Hepa1-6/hGPC3 cancer cells, which are mouse cancer cell line Hepa1-6 made to highly express human GPC3, were transplanted subcutaneously in C57BL/6 mice. The day of transplantation was set to day 0. On day 18, grouping was performed according to tumor size and body weight. The group settings are as shown in Table 14. MR-16-1 was administered into the tail vein at 30 mg/kg on day 18. On day 19, the day after MR-16-1 administration, GC33/2C11 was administered into the tail vein at 0.2 and 5 mg/kg. MR-16-1 was administered six times in total (on days 18, 21, 25, 28, 32 and day 35). GC33/2C11 was administered once, only on day 19. GC33/2C11 was an antibody comprising the amino acid sequence of SEQ ID NO: 443 as the GPC3-side heavy chain, the amino acid sequence of SEQ ID NO: 444 as the GPC3-side light chain, the amino acid sequence of SEQ ID NO: 445 as the CD3-side heavy chain, and the amino acid sequence of SEQ ID NO: 446 as the CD3-side light chain.

As a result, pre-administration of MR16-1 did not inhibit the drug efficacy of GC33/2C11. (Table 14) (Fig. 12)

[Table 14]

| Group | Dose of the administered pharmaceutical agent (mg/kg) | | Day of administration of the pharmaceutical agent | Antitumor activity (%) |
|---|---|---|---|---|
| 1 | solvent | not applicable | day 19, 21, 25, 28, 32, 35 | not applicable |
| 2 | GC33/2C11 | 0.2 | day 19 | 80 |
| 3 | GC33/2C11 | 5 | day 19 | 110 |
| 4 | MR-16-1 | 30 (first administration), 15 (second and subsequent administrations) | day 18, 21, 25, 28, 32, 35 | -10 |
| 5 | GC33/2C11 | 0.2 | day 19 | 99 |
| | MR-16-1 | 30 (first administration), 15 (second and subsequent administrations) | day 18, 21, 25, 28, 32, 35 | |
| 6 | GC33/2C12 | 5 | day 19 | 107 |
| | MR-16-1 | 30 (first administration), 15 (second and subsequent administrations) | day 18, 21, 25, 28, 32, 35 | |

(7-2) Post-administration of anti-mouse IL6 receptor antibody (MR-16-1)

In a study using a syngeneic mouse model, a study for evaluating the effect of post-administration of an anti-mouse IL6 receptor antibody, MR-16-1, on the drug efficacy of ERY974 surrogate antibodies was carried out as follows. Hepa1-6/hGPC3 cancer cells, which are mouse cancer cell line Hepa1-6 made to highly express human GPC3, were transplanted subcutaneously in C57BL/6 mice. The day of transplantation was set to day 0. On day 16, grouping was performed according to tumor size and body weight. The group settings are as shown in Table XX. On day 17, GC33/2C11 was first administered into the tail vein at 0.2 or 5 mg/kg, and two hours later, MR-16-1 was administered into the tail vein at 30 mg/kg. MR-16-1 was administered six times in total (on days 17, 21, 24, 28, 31, and day 35). GC33/2C11 was administered once, only on day 17.

As a result, post-administration of MR16-1 did not inhibit the drug efficacy of GC33/2C11. (Table 15) (Fig. 13)

[Table 15]

| Group | Dose of the administered pharmaceutical agent (mg/kg) | | Day of administration of the pharmaceutical agent | Antitumor activity (%) |
|---|---|---|---|---|
| 1 | solvent | not applicable | day 17, 21, 24, 28, 31, 35 | not applicable |
| 2 | GC33/2C11 | 0.2 | day 17 | 102 |
| 3 | GC33/2C11 | 5 | day 17 | 109 |
| 4 | MR-16-1 | 30 (first administration), 15 (second and subsequent administrations) | day 17, 21, 24, 28, 31, 35 | 23 |
| 5 | GC33/2C11 | 0.2 | day 17 (2 hours before MR-16-1 administration) | 103 |
| | MR-16-1 | 30 (first administration), 15 (second and subsequent administrations) | day 17, 21, 24, 28, 31, 35 | |

(continued)

| Group | Dose of the administered pharmaceutical agent (mg/kg) | | Day of administration of the pharmaceutical agent | Antitumor activity (%) |
|---|---|---|---|---|
| 6 | GC33/2C12 | 5 | day 17 (2 hours before MR-16-1 administration) | 109 |
| | MR-16-1 | 30 (first administration), 15 (second and subsequent administrations) | day 17, 21, 24, 28, 31, 35 | |

[Industrial Applicability]

The present disclosure is useful for preventing, alleviating, or treating adverse reactions caused by cytokine production associated with administration of anti-T cell antigen-binding molecules. Anti-T cell antigen-binding molecules are drawing attention as means for treating cancer. Therefore, in some embodiments, the present disclosure is useful in treatment of cancer with anti-T cell antigen-binding molecules.

**Claims**

1. A pharmaceutical composition comprising an anti-T cell antigen-binding molecule, wherein the pharmaceutical composition is for use in its combination therapy with a cytokine inhibitor.

2. The pharmaceutical composition of claim 1, wherein the cytokine inhibitor is administered before, simultaneously with, or after administration of the pharmaceutical composition.

3. The pharmaceutical composition of claim 1 or 2, wherein the cytokine inhibitor is administered before or simultaneously with the administration of the pharmaceutical composition.

4. The pharmaceutical composition of any one of claims 1 to 3, wherein the cytokine inhibitor is administered 6 days, 5 days, 4 days, 3 days, 2 days, or 1 day before the administration of the pharmaceutical composition, or on the same day as but before said administration.

5. The pharmaceutical composition of any one of claims 1 to 4, wherein the cytokine inhibitor is further administered on the same day as, or 1 day, 2 days, 3 days, 4 days, 5 days, or 6 days after the administration of the pharmaceutical composition.

6. The pharmaceutical composition of any one of claims 1 to 5, wherein the cytokine inhibitor is further administered when CRS or signs of CRS develop after the administration of the pharmaceutical composition.

7. The pharmaceutical composition of claim 6, wherein the CRS is CRS in Grade 2 or higher or Grade 3 or higher.

8. The pharmaceutical composition of any one of claims 1 to 7, wherein the cytokine inhibitor is administered after the administration of the pharmaceutical composition.

9. The pharmaceutical composition of claim 1 or 8, wherein the cytokine inhibitor is administered when CRS or signs of CRS develop after the administration of the pharmaceutical composition.

10. The pharmaceutical composition of claim 8 or 9, wherein the CRS is CRS in Grade 2 or higher or Grade 3 or higher.

11. The pharmaceutical composition of any one of claims 1 to 10, wherein the cytokine inhibitor is an inhibitor of one or more cytokines selected from IL-1$\beta$, IL-2, IL-4, IL-5, IL-6, IL-8, IL-10, IL-12, IL-13, IL-15, IL-17, IL-IRa, IL-2R, IFN-$\alpha$, IFN-$\gamma$, MIP-1$\alpha$, MIP-1$\beta$, MCP-1, TNF$\alpha$, GM-CSF, G-CSF, CXCL9, CXCL10, CXCR factor, VEGF, RANTES, eotaxin, EGF, HGF, FGF-$\beta$, CD30, CD30L, CD40, CD40L, ferritin, and RAGE.

12. The pharmaceutical composition of any one of claims 1 to 11, wherein the cytokine inhibitor is an anti-IL6R antibody.

13. The pharmaceutical composition of any one of claims 1 to 12, wherein the cytokine inhibitor is Tocilizumab, and wherein Tocilizumab is administered at 8 mg/kg or less per dose for a patient weighing 30 kg or more and at 12 mg/kg or less per dose for a patient weighing less than 30 kg.

14. The pharmaceutical composition of any one of claims 1 to 13, wherein a corticosteroid is not administered before or simultaneously with the administration of the pharmaceutical composition.

15. The pharmaceutical composition of any one of claims 1 to 14, wherein a corticosteroid is further administered before, simultaneously with, or after the administration of the pharmaceutical composition.

```
Kabat      1                                                                                      2
EU index   1-2---------3----------4----------5----------6----------7----------8----------9----------0----------1----------2--
           8-0---------0----------0----------0----------0----------0----------0----------0----------0----------0----------0--
IgG1       ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCD-
IgG2       ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERK--CC
IgG3       ASTKGPSVFPLAPCSRSTSGGTAALGCLVKDYFPEPVTVSWNSGALTSGVHTFPAVLQSSGLYSLSSVVTVPSSSLGTQTYTCNVNHKPSNTKVDKRVELKTPLG
IgG4       ASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYG--

Kabat        2
EU index     2-----------------------------------------------------------------3----------4----------5----------6----------7----------8
             2----8---------------------------------------------------------------0----------0----------0----------0----------0----------0
IgG1       -KTHTCPP-------------------------------------------------CPAPELLGGPSVFLFPPKPKDTLMISRTPEVTCVVVDVSHEDPEVKFNWYVD
IgG2       -V-E-CPP-------------------------------------------------CPAPPVA-GPSVFLFPPKPKDTLMISRTPEVTCVVVDVSHEDPEVQFNWYVD
IgG3       DTTHTCPRCPEPKSCDTPPPCPRCPEPKSCDTPPPCPRCPEPKSCDTPPPCPRCPAPELLGGPSVFLFPPKPKDTLMISRTPEVTCVVVDVSHEDPEVQFKWYVD
IgG4       ---PPCPS-------------------------------------------------CPAPEFLGGPSVFLFPPKPKDTLMISRTPEVTCVVVDVSQEDPEVQFNWYVD

Kabat      2                     3
EU index   8---------9----------0----------1----------2----------3----------4----------5----------6----------7----------8-----
           1---------0----------0----------0----------0----------0----------0----------0----------0----------0----------0-----
IgG1       GVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNG
IgG2       GVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNG
IgG3       GVEVHNAKTKPREEQYNSTFRVVSVLTVLHQDWLNGKEYKCKVSNKALPAPIEKTISKTKGQPREPQVYTLPPSREEMTKNQVSLTCLVKGFYPSDIAVEWESSG
IgG4       GVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNG

Kabat      3                     4
EU index   8---9----------0----------1----------2----------3----------4
           6---0----------0----------0----------0----------0----------0------7
IgG1       QPENNYKTTPPVLDSDGSFFLYSKLTVDKSRWQQGNVFSCSVMHEALHNHYTQKSLSLSPGK  (SEQ ID NO: 23)
IgG2       QPENNYKTTPPMLDSDGSFFLYSKLTVDKSRWQQGNVFSCSVMHEALHNHYTQKSLSLSPGK  (SEQ ID NO: 24)
IgG3       QPENNYNTTPPMLDSDGSFFLYSKLTVDKSRWQQGNIFSCSVMHEALHNRFTQKSLSLSPGK  (SEQ ID NO: 25)
IgG4       QPENNYKTTPPVLDSDGSFFLYSRLTVDKSRWQEGNVFSCSVMHEALHNHYTQKSLSLSLGK  (SEQ ID NO: 26)
```

FIG. 1

EP 3 981 429 A1

EP 3 981 429 A1

| Antibody name | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 35a | 35b | 36 | 37 | 38 | 39 | 40 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|
| CE115HA000 | Q | V | Q | L | V | E | S | G | G | G | V | V | Q | P | G | R | S | L | R | L | S | C | A | A | S | G | F | T | F | S | N | A | W | M | H | - | - | W | V | R | Q | A |
| TR01H040 | Q | V | Q | L | V | E | S | G | G | G | L | V | Q | P | G | R | S | L | R | L | S | C | A | A | S | G | F | T | F | S | N | A | W | M | H | - | - | W | V | R | Q | A |
| TR01H071 | Q | V | Q | L | V | E | S | G | G | G | L | V | Q | P | G | R | S | L | R | L | S | C | A | A | S | G | F | T | F | S | N | A | W | M | H | - | - | W | V | R | Q | A |
| TR01H082 | Q | V | Q | L | V | E | S | G | G | G | L | V | Q | P | G | R | S | L | R | L | S | C | A | A | S | G | F | T | F | S | N | A | W | M | H | - | - | W | V | R | Q | A |
| TR01H084 | Q | V | Q | L | V | E | S | G | G | G | V | V | Q | P | G | G | S | L | R | L | S | C | A | A | S | G | F | T | F | S | N | A | W | M | H | - | - | W | V | R | Q | A |
| TR01H109 | Q | V | Q | L | V | E | S | G | G | G | L | V | Q | P | G | R | S | L | R | L | S | C | A | A | S | G | F | T | F | S | N | A | W | M | H | - | - | W | V | R | Q | A |
| TR01H113 | Q | V | Q | L | V | E | S | G | G | G | V | V | Q | P | G | G | S | L | R | L | S | C | A | A | S | G | F | T | F | S | N | A | W | M | H | - | - | W | V | R | Q | A |
| H0000 | Q | V | Q | L | V | Q | S | G | A | E | V | K | K | P | G | A | S | V | K | V | S | C | K | A | S | G | Y | T | F | T | D | Y | E | M | H | - | - | W | I | R | Q | P |
| H0610 | Q | V | Q | L | V | Q | S | G | A | E | V | K | K | P | G | A | S | V | K | V | S | C | K | A | S | G | Y | T | F | T | D | Y | E | M | H | - | - | W | I | R | Q | P |
| GCH054 | Q | V | Q | L | V | Q | S | G | A | E | V | K | K | P | G | A | S | V | T | V | S | C | K | A | S | G | Y | T | F | T | D | Y | E | M | H | - | - | W | I | R | Q | P |
| GCH065 | Q | V | Q | L | V | Q | S | G | A | E | V | K | K | P | G | A | S | V | T | V | S | C | K | A | S | G | Y | T | F | T | D | Y | E | M | H | - | - | W | I | R | Q | P |
| GCH094 | Q | V | Q | L | V | Q | S | G | A | E | V | K | K | P | G | A | S | V | T | V | S | C | K | A | S | G | Y | T | F | T | D | Y | E | M | H | - | - | W | V | R | Q | P |

Kabat CDR H1: positions 31–35b · Chothia CDR H1: positions 26–32 · Contact CDR H1: positions 30–35b

| Antibody name | 41 | 42 | 43 | 44 | 45 | 46 | 47 | 48 | 49 | 50 | 51 | 52 | 52a | 52b | 52c | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|
| CE115HA000 | P | G | K | G | L | E | W | V | A | Q | I | K | A | K | S | N | N | Y | A | T | Y | Y | A | E | S | V | K | G | R | F | T | I | S | R | D | D | S | K | N | S | L | Y |
| TR01H040 | P | G | K | G | L | E | W | V | A | Q | I | K | D | K | S | Q | N | Y | A | T | Y | Y | A | E | S | V | K | G | R | F | T | I | S | R | A | D | S | K | N | S | I | Y |
| TR01H071 | P | G | K | G | L | E | W | V | A | Q | I | K | D | K | S | N | N | Y | A | T | Y | Y | A | E | S | V | K | G | R | F | T | I | S | R | A | D | S | K | N | S | I | Y |
| TR01H082 | P | G | K | G | L | E | W | V | A | Q | I | K | D | K | S | Q | N | Y | A | T | Y | Y | A | E | S | V | K | G | R | F | T | I | S | R | D | D | S | K | N | S | L | Y |
| TR01H084 | P | G | K | G | L | E | W | V | A | Q | I | K | D | K | S | Q | N | Y | A | T | Y | Y | A | E | S | V | K | G | R | F | T | I | S | R | A | D | S | K | N | S | I | Y |
| TR01H109 | P | G | K | G | L | E | W | V | A | Q | I | K | D | K | S | Q | N | Y | A | T | Y | Y | A | E | S | V | K | G | R | F | T | I | S | R | D | D | S | K | N | S | L | Y |
| TR01H113 | P | G | K | G | L | E | W | V | A | Q | I | K | D | K | S | Q | N | Y | A | T | Y | V | A | E | S | V | K | G | R | F | T | I | S | R | A | D | S | K | N | S | I | Y |
| H0000 | P | G | Q | G | L | E | W | I | G | A | I | D | P | - | - | K | T | G | D | T | A | Y | S | Q | K | F | K | G | R | V | T | L | T | A | D | K | S | T | S | T | A | Y |
| H0610 | P | G | Q | G | L | E | W | I | G | A | I | D | G | - | - | K | T | P | D | T | A | Y | S | Q | K | F | K | G | R | V | T | L | T | A | D | K | S | T | S | T | A | Y |
| GCH054 | P | G | E | G | L | E | W | I | G | A | I | D | G | - | - | E | T | P | D | T | A | Y | S | E | K | F | K | G | R | V | T | L | T | A | D | K | S | T | S | T | A | Y |
| GCH065 | P | G | E | G | L | E | W | I | G | A | I | D | G | - | - | P | T | P | D | T | A | Y | S | E | K | F | K | G | R | V | T | L | T | A | D | K | S | T | S | T | A | Y |
| GCH094 | P | G | E | G | L | E | W | M | G | A | I | D | G | - | - | E | T | P | D | T | A | Y | S | E | K | F | K | G | R | V | T | L | T | A | D | K | S | T | S | T | A | Y |

Kabat CDR H2: positions 50–65 · Chothia CDR H2: positions 52–56 · Contact CDR H2: positions 47–58

FIG. 2-1

| | | | | | | | | | | | | | | | | | | | | | | | Kabat CDR H3 | | | | | | | | | | | | | | | | | |
| | | | | | | | | | | | | | | | | | | | | | | | Chothia CDR H3 | | | | | | | | | | | | | | | | | |
| | | | | | | | | | | | | | | | | | | | | | | | Contact CDR H3 | | | | | | | | | | | | | | | | | |
| Antibody name | 80 | 81 | 82 | 82a | 82b | 82c | 83 | 84 | 85 | 86 | 87 | 88 | 89 | 90 | 91 | 92 | 93 | 94 | 95 | 96 | 97 | 98 | 99 | 100 | 100a | 100b | 100c | 100d | 100e | 100f | 100g | 100h | 100i | 100j | 100k | 101 | 102 | 103 | 104 | 105 | 106 | 107 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|
| CE115HA000 | L | Q | M | N | S | L | K | T | E | D | T | A | V | Y | Y | Y | C | R | Y | V | H | Y | G | A | Y | Y | G | V | - | - | - | - | - | - | - | D | A | W | G | Q | G | T |
| TR01H040 | L | Q | M | N | S | L | K | T | E | D | T | A | V | Y | Y | Y | C | R | Y | V | H | Y | S | A | S | Y | G | V | - | - | - | - | - | - | - | D | A | W | G | Q | G | T |
| TR01H071 | L | Q | M | N | S | L | K | T | E | D | T | A | V | Y | Y | Y | C | R | Y | V | H | Y | A | A | S | Y | G | V | - | - | - | - | - | - | - | D | I | W | G | Q | G | T |
| TR01H082 | L | Q | M | N | S | L | K | T | E | D | T | A | V | Y | Y | Y | C | R | Y | V | H | Y | A | A | G | Y | G | V | - | - | - | - | - | - | - | D | A | W | G | Q | G | T |
| TR01H084 | L | Q | M | N | S | L | K | T | E | D | T | A | V | Y | Y | Y | C | R | Y | V | H | Y | A | A | G | Y | G | V | - | - | - | - | - | - | - | D | I | W | G | Q | G | T |
| TR01H109 | L | Q | M | N | S | L | K | T | E | D | T | A | V | Y | Y | Y | C | R | Y | V | H | Y | A | A | G | Y | G | V | - | - | - | - | - | - | - | D | A | W | G | Q | G | T |
| TR01H113 | L | Q | M | N | S | L | K | T | E | D | T | A | V | Y | Y | Y | C | R | Y | V | H | Y | A | A | G | Y | G | V | - | - | - | - | - | - | - | D | I | W | G | Q | G | T |
| H0000 | M | E | L | S | S | L | T | S | E | D | T | A | V | Y | Y | Y | C | T | R | F | Y | S | Y | - | - | - | - | - | - | - | - | - | - | - | - | T | Y | W | G | Q | G | T |
| H0610 | M | E | L | S | S | L | T | S | E | D | T | A | V | Y | Y | Y | C | T | R | F | Y | S | Y | - | - | - | - | - | - | - | - | - | - | - | - | T | Y | W | G | Q | G | T |
| GCH054 | M | E | L | S | S | L | T | S | E | D | T | A | V | Y | Y | Y | C | T | R | F | Y | S | Y | - | - | - | - | - | - | - | - | - | - | - | - | T | Y | W | G | Q | G | T |
| GCH065 | M | E | L | S | S | L | T | S | E | D | T | A | V | Y | Y | Y | C | T | R | F | Y | S | Y | - | - | - | - | - | - | - | - | - | - | - | - | T | Y | W | G | Q | G | T |
| GCH094 | M | E | L | S | S | L | T | S | E | D | T | A | V | Y | Y | Y | C | T | R | F | Y | S | Y | - | - | - | - | - | - | - | - | - | - | - | - | T | Y | W | G | Q | G | T |

| Antibody name | 108 | 109 | 110 | 111 | 112 | 113 |
|---|---|---|---|---|---|---|
| CE115HA000 | T | V | T | V | S | S |
| TR01H040 | T | V | T | V | S | S |
| TR01H071 | T | V | T | V | S | S |
| TR01H082 | T | V | T | V | S | S |
| TR01H084 | T | V | T | V | S | S |
| TR01H109 | T | V | T | V | S | S |
| TR01H113 | T | V | T | V | S | S |
| H0000 | L | V | T | V | S | S |
| H0610 | L | V | T | V | S | S |
| GCH054 | L | V | T | V | S | S |
| GCH065 | L | V | T | V | S | S |
| GCH094 | L | V | T | V | S | S |

FIG. 2-2

**FIG. 3**

Table 1 — Positions 1–36 (Kabat CDR L1 / Chothia CDR L1 ≈ 24–34; Contact CDR L1 ≈ 30–36)

| Antibody name | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 27a | 27b | 27c | 27d | 27e | 27f | 28 | 29 | 30 | 31 | 32 | 33 | 34 | 35 | 36 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|
| L0000 | D | - | V | M | T | Q | S | P | L | S | L | P | V | T | P | G | E | P | A | S | - | S | C | R | S | S | Q | S | L | V | H | S | - | N | R | N | T | Y | - | L | W | Y |
| L0011 | D | - | V | M | T | Q | S | P | L | S | L | P | V | T | P | G | E | P | A | S | - | S | C | R | S | S | Q | P | L | V | H | S | - | N | R | N | T | Y | - | L | W | Y |
| L0201 | D | - | V | M | T | Q | S | P | L | S | L | P | V | T | P | G | E | P | A | S | - | S | C | R | S | S | Q | P | P | V | H | S | - | N | R | N | T | Y | - | L | W | Y |
| L0203 | D | - | V | M | T | Q | S | P | L | S | L | P | V | T | P | G | E | P | A | S | - | S | C | R | S | S | Q | S | P | V | H | S | - | N | R | N | T | Y | - | L | W | Y |
| L0204 | D | - | V | M | T | Q | S | P | L | S | L | P | V | T | P | G | E | P | A | S | - | S | C | R | S | S | Q | S | P | V | H | S | - | N | R | N | T | Y | - | L | W | Y |
| L0206 | D | - | V | M | T | Q | S | P | L | S | L | P | V | T | P | G | E | P | A | S | - | S | C | R | S | S | Q | S | P | V | H | S | - | N | R | N | T | Y | - | L | W | Y |
| L0208 | D | - | V | M | T | Q | S | P | L | S | L | P | V | T | P | G | E | P | A | S | - | S | C | R | S | S | Q | S | P | V | H | S | - | N | R | N | T | Y | - | L | W | Y |
| L0209 | D | - | V | M | T | Q | S | P | L | S | L | P | V | T | P | G | E | P | A | S | - | S | C | R | S | S | Q | S | P | V | H | S | - | N | R | N | T | Y | - | L | W | Y |
| L0211 | D | - | V | M | T | Q | S | P | L | S | L | P | V | T | P | G | E | P | A | S | - | S | C | R | S | S | Q | S | P | V | H | S | - | N | R | N | T | Y | - | L | W | Y |
| L0212 | D | - | V | M | T | Q | S | P | L | S | L | P | V | T | P | G | E | P | A | S | - | S | C | R | P | S | Q | S | P | V | H | S | - | N | R | N | T | Y | - | L | W | Y |
| L0222 | D | - | V | M | T | Q | S | P | L | S | L | P | V | T | P | G | E | P | A | S | - | S | C | R | S | S | Q | S | P | V | H | S | - | N | R | N | T | Y | - | Q | W | Y |
| L0272 | D | - | V | M | T | Q | S | P | L | S | L | P | V | T | P | G | E | P | A | S | - | S | C | R | S | S | Q | S | L | V | H | S | - | N | R | Q | T | Y | - | H | W | Y |

Table 2 — Positions 37–78 (Kabat CDR L2 / Chothia CDR L2; Contact CDR L2 ≈ 46–52)

| Antibody name | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|
| L0000 | Q | Q | K | P | G | Q | A | P | R | L | L | I | Y | K | V | S | N | R | F | S | G | V | P | D | R | F | S | G | S | G | S | G | T | D | F | T | L | K | I | S | R | V |
| L0011 | Q | Q | K | P | G | Q | A | P | R | L | L | I | Y | K | V | S | N | R | F | S | G | V | P | D | R | F | S | G | S | G | S | G | T | D | F | T | L | K | I | S | R | V |
| L0201 | Q | Q | K | P | G | Q | A | P | R | L | L | I | Y | K | V | S | N | R | F | S | G | V | P | D | R | F | S | G | S | G | S | G | T | D | F | T | L | K | I | S | R | V |
| L0203 | Q | Q | K | P | G | Q | A | P | R | L | L | I | Y | K | V | S | N | R | F | S | G | V | P | D | R | F | S | G | S | G | S | G | T | D | F | T | L | K | I | S | R | V |
| L0204 | Q | Q | K | P | G | Q | A | P | R | L | L | I | Y | K | V | S | N | R | F | S | G | V | P | D | R | F | S | G | S | G | S | G | T | D | F | T | L | K | I | S | R | V |
| L0206 | Q | Q | K | P | G | Q | A | P | R | L | L | I | Y | K | V | S | N | R | F | R | G | V | P | D | R | F | S | G | S | G | S | G | T | D | F | T | L | K | I | S | R | V |
| L0208 | Q | Q | K | P | G | Q | A | P | R | L | L | I | Y | K | V | S | N | R | F | W | G | V | P | D | R | F | S | G | S | G | S | G | T | D | F | T | L | K | I | S | R | V |
| L0209 | Q | Q | K | P | G | Q | A | P | R | L | L | I | Y | K | V | S | N | R | F | S | G | V | P | D | R | F | S | G | S | G | S | G | T | D | F | T | L | K | I | S | R | V |
| L0211 | Q | Q | K | P | G | Q | A | P | R | L | L | I | Y | K | V | S | N | R | F | S | G | V | P | D | R | F | S | G | S | G | S | G | T | D | F | T | L | K | I | S | R | V |
| L0212 | Q | Q | K | P | G | Q | A | P | R | L | L | I | Y | K | V | S | N | R | F | S | G | V | P | D | R | F | S | G | S | G | S | G | T | D | F | T | L | K | I | S | R | V |
| L0222 | Q | Q | K | P | G | Q | A | P | R | L | L | I | Y | K | V | S | N | R | F | S | G | V | P | D | R | F | S | G | S | G | S | G | T | D | F | T | L | K | I | S | R | V |
| L0272 | Q | Q | K | P | G | Q | A | P | R | L | L | I | Y | K | V | S | N | R | F | S | G | V | P | D | R | F | S | G | S | G | S | G | T | D | F | T | L | K | I | S | R | V |

Table 3 — Positions 79–107 (Kabat CDR L3 / Chothia CDR L3 / Contact CDR L3)

| Antibody name | 79 | 80 | 81 | 82 | 83 | 84 | 85 | 86 | 87 | 88 | 89 | 90 | 91 | 92 | 93 | 94 | 95 | 95a | 95b | 95c | 95d | 95e | 95f | 96 | 97 | 98 | 99 | 100 | 101 | 102 | 103 | 104 | 105 | 106 | 107 |
|---|---|---|---|---|---|---|---|---|---|---|---|---|---|---|---|---|---|---|---|---|---|---|---|---|---|---|---|---|---|---|---|---|---|---|---|
| L0000 | E | A | E | D | V | G | V | Y | Y | C | G | Q | G | T | Q | V | P | P | - | - | - | - | - | Y | T | F | G | Q | G | T | K | L | E | - | K |
| L0011 | E | A | E | D | V | G | V | Y | Y | C | G | Q | G | T | Q | V | P | P | - | - | - | - | - | Y | T | F | G | Q | G | T | K | L | E | - | K |
| L0201 | E | A | E | D | V | G | V | Y | Y | C | G | Q | G | T | Q | V | P | P | - | - | - | - | - | Y | T | F | G | Q | G | T | K | L | E | - | K |
| L0203 | E | A | E | D | V | G | V | Y | Y | C | Y | Q | G | T | Q | V | P | P | - | - | - | - | - | Y | T | F | G | Q | G | T | K | L | E | - | K |
| L0204 | E | A | E | D | V | G | V | Y | Y | C | Y | Q | G | T | Q | V | P | P | - | - | - | - | - | Y | T | F | G | Q | G | T | K | L | E | - | K |
| L0206 | E | A | E | D | V | G | V | Y | Y | C | Y | Q | G | T | Q | V | P | P | - | - | - | - | - | Y | T | F | G | Q | G | T | K | L | E | - | K |
| L0208 | E | A | E | D | V | G | V | Y | Y | C | Y | Q | G | T | Q | V | P | P | - | - | - | - | - | Y | T | F | G | Q | G | T | K | L | E | - | K |
| L0209 | E | A | E | D | V | G | V | Y | Y | C | Y | Q | G | T | Q | V | P | P | - | - | - | - | - | Y | T | F | G | Q | G | T | K | L | E | - | K |
| L0211 | E | A | E | D | V | G | V | Y | Y | C | Y | Q | G | T | Q | V | P | P | - | - | - | - | - | Y | T | F | G | Q | G | T | K | L | E | - | K |
| L0212 | E | A | E | D | V | G | V | Y | Y | C | Y | Q | G | T | Q | V | P | P | - | - | - | - | - | Y | T | F | G | Q | G | T | K | L | E | - | K |
| L0222 | E | A | E | D | V | G | V | Y | Y | C | Y | Q | G | T | Q | V | P | P | - | - | - | - | - | Y | T | F | G | Q | G | T | K | L | E | - | K |
| L0272 | E | A | E | D | V | G | V | Y | Y | C | Y | Q | G | T | Q | V | P | P | - | - | - | - | - | Y | T | F | G | Q | G | T | K | L | E | - | K |

**840010006 Cohort 10A**

FIG. 4A

840010007 Cohort 10A

FIG. 4B

FIG. 4C

FIG. 5A

Population PK model : Gibiansky et al. 2012

FIG. 5B

FIG. 6A

## Population PK model : Frey et al. 2010

FIG. 6B

Population PK model : Gibiansuky et al. 2012

FIG. 7

EP 3 981 429 A1

FIG. 8

FIG. 9

FIG. 10

FIG. 11

FIG. 12

FIG. 13

## INTERNATIONAL SEARCH REPORT

International application No.

PCT/JP2020/022771

### A. CLASSIFICATION OF SUBJECT MATTER

A61K 39/395(2006.01)i; A61K 45/00(2006.01)i; A61P 35/00(2006.01)i; A61P 43/00(2006.01)i; C07K 16/28(2006.01)i; C07K 16/46(2006.01)i; C12N 15/13(2006.01)i; A61K 31/573(2006.01)i
FI:     A61K39/395 N ZNA; A61K39/395 D; A61K45/00; A61P35/00; A61P43/00
        111; A61P43/00 121; A61K31/573; C12N15/13; C07K16/28; C07K16/46

According to International Patent Classification (IPC) or to both national classification and IPC

### B. FIELDS SEARCHED

Minimum documentation searched (classification system followed by classification symbols)
A61K39/395; A61K45/00; A61P35/00; A61P43/00; C07K16/28; C07K16/46;
C12N15/13; A61K31/573

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/MEDLINE/EMBASE/BIOSIS (STN);
PubMed

### C. DOCUMENTS CONSIDERED TO BE RELEVANT

| Category* | Citation of document, with indication, where appropriate, of the relevant passages | Relevant to claim No. |
|---|---|---|
| X | TEACHEY, D. T. et al., "Cytokine release syndrome after blinatumomab treatment related to abnormal macrophage activation and ameliorated with cytokine-directed therapy", Blood, 2013, vol. 121, no. 26, pp. 5154–5157, DOI 10.1182/blood-2013-02-485623 abstract, fig. 1, 2, page 5155, left column, paragraph [0002], right column, paragraph [0001], page 5156, left column | 1–2, 5–15 |

☒ Further documents are listed in the continuation of Box C.     ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| | |
|---|---|
| Date of the actual completion of the international search | Date of mailing of the international search report |
| 20 August 2020 (20.08.2020) | 01 September 2020 (01.09.2020) |
| Name and mailing address of the ISA/ | Authorized officer |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
|---|
| PCT/JP2020/022771 |

C (Continuation). DOCUMENTS CONSIDERED TO BE RELEVANT

| Category* | Citation of document, with indication, where appropriate, of the relevant passages | Relevant to claim No. |
|---|---|---|
| X | ASSI, R. et al., "Safety and Efficacy of Blinatumomab in Combination With a Tyrosine Kinase Inhibitor for the Treatment of Relapsed Philadelphia Chromosome-positive Leukemia", Clinical lymphoma, myeloma & leukemia, 2017, vol. 17, no. 12, pp. 897-901, DOI http://dx.doi.org/10.1016/j.clml.2017.08.101 abstract, page 900, left column, section "Safety" | 1-2, 5-12, 14-15 |
| X | CHOUDHRY, J. et al., "A Retrospective Review of Tocilizumab for the Management of Blinatumomab (a Bispecific T Cell Engager)-Induced Cytokine Release Syndrome (CRS)", Blood, 2018, vol. 132, Supply no. 1, pp. 5211, DOI 10.1182/blood-2018-99-117353 results | 1-2, 5-12, 14-15 |
| X | BANNERJI, R. et al., "Emerging Clinical Activity of REGN1979, an Anti-CD20 x Anti-CD3 Bispecific Antibody, in Patients with Relapsed/Refractory Follicular Lymphoma (FL), Diffuse Large B-Cell Lymphoma (DLBCL), and Other B-Cell Non-Hodgkin Lymphoma (B-NHL) Subtypes", Blood, 2018, vol. 132, supply no. 1, p. 1690, DOI 10.1182/blood-2018-99-113328 Methods, Results | 1-2, 5-12, 14-15 |
| X | JACOBS, K. et al., "Management of Cytokine Release Syndrome in AML Patients Treated with Flotetuzumab, a CD123 x CD3 Bispecific Dart (R) Molecule for T-Cell Redirected Therapy", Blood, 2018, vol. 132, supply no. 1, p. 2738, DOI 10.1182/blood-2018-99-112615 Methods, Results, Conclusion | 1-2, 5-12, 14 |
| X | WESTERVELT, P. et al., "Phase 1 First-in-Human Trial of AMV564, a Bivalent Bispecific (2x2) CD33/CD3 T-Cell Engager, in Patients with Relapsed/ Refractory Acute Myeloid Leukemia (ANIL)", Blood, 2018, vol. 132, supply no. 1, p. 1455, DOI 10.1182/blood-2018-99-111529 Results | 1-2, 5-12, 14 |
| X  Y | WO 2018/093821 A1 (GENENTECH, INC.) 24.05.2018 (2018-05-24) claims (in particular, claims 1, 43, 51, 53, 59-69), page 24, paragraph [0004], page 27, paragraph [0001], page 31, paragraph [0001], example 2, page 34, paragraph [0003] | 1-15  3-4 |
| X  Y | SHINIABUKURO-V., A. et al., "Cytokine release syndrome", Journal for ImmunoTherapy of Cancer, 2018, vol. 6, no. 1, article no. 56, DOI 10.1186/s40425-018-0343-9 abstract, fig. 2, page 10, right column, paragraph [0002]-[0003], page 10, right column, paragraph [0003], page 10, right column, the bottom paragraph, page 11, left column, paragraph [0002] | 1-2, 5-15  3-4 |

Form PCT/ISA/210 (continuation of second sheet) (January 2015)

**INTERNATIONAL SEARCH REPORT**

| International application No. |
|---|
| PCT/JP2020/022771 |

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | GARCIA R., A. et al., "Cytokine release syndrome. Revlewrng a new entity in the intensive care unit", medicina intensiva, March 2019, vol. 43, no. 8, pp. 480-488 abstract, page 481, left column, paragraph [0002], fig. 3 | 1-2, 5-15<br>3-4 |
| X<br><br><br>Y | SHRESHTHA, S. et al., "Cytokine release syndrome: an overview on its features and management", Journal of Pure and Applied Microbiology, March 2019, vol. 13, no. 1, pp. 133-140, DOI 10.22207/jpam.13.1.14 abstract, page 134, right column, the bottom paragraph to page 135, left column, paragraph [0001], page 135, right column, paragraph [0003], page 136, right column, paragraph [0003] to page 137, left column, paragraph [0001], pp. 137-138, section "Targeting cytokines", page 318, tables, page 137, left column, paragraph [0003] | 1-2, 5-12, 14-15<br><br>3-4 |
| X<br><br><br>Y | KHADKA, R. H. et al., "Management of cytokine release syndrome: An update on emerging antigen-specific T cell engaging immunotherapies", Immunotherapy, 05 June 2019, vol. 11, no. 10, pp. 851-857, DOI 10.2217/imt-2019-0074 abstract, page 851, section "Emerging T cell-engaging immunotherapies associated with CRS", page 853, section "Bi-specific T cell engagers", pp. 854-856, section "Management of CRS: current perspective" | 1-2, 5-12, 14-15<br><br>3-4 |
| Y | ISHIGURO, T. et al., "An anti-glypican 3/CD3 bispecific T cell-redirecting antibody for treatment of solid tumors", Science Translational Medicine, 2017, vol. 9, no. 410, article number eaal4291, DOI 10.1126/scitranslmed.aal4291 abstract, fig. 6, 7 | 3-4 |
| Y | KADAUKE, S. et al., "Early Administration of Tocilizumab (Toci) for the Prevention of Grade 4 Cytokine Release Syndrome (CRS) after CD19-directed CAR T-cell Therapy (CTL019)", Cytotherapy, May 2019, vol. 21, no. 5 S, pp. e2-e3, DOI 10.1016/j.jcyt.2019.04.009 entire text | 3-4 |
| P, X | KAUER, J. et al., "Tocilizumab, but not dexamethasone, prevents CRS without affecting antitumor activity of bispecific antibodies", Journal for immunotherapy of cancer, May 2020, vol. 8, no. 1, article number e000621, DOI http://dx.doi.org/10.1136/jitc-2020-000621 abstract, page 2, section "Animal model", fig. 1, 2 | 1-15 |

Form PCT/ISA/210 (continuation of second sheet) (January 2015)

**INTERNATIONAL SEARCH REPORT**
Information on patent family members

| International application No. |
|---|
| PCT/JP2020/022771 |

| Patent Documents referred in the Report | Publication Date | Patent Family | Publication Date |
|---|---|---|---|
| WO 2018/093821 A1 | 24 May 2018 | JP 2020-503260 A<br>US 2018/0134798 A1<br>EP 3541843 A1<br>CN 109923128 A<br>KR 10-2019-0074300 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012073985 A
- WO 2016047722 A
- WO 200492219 A, Hinton
- WO 200042072 A, Presta
- US 6737056 B
- US 7332581 B
- WO 2004056312 A
- US 6194551 B
- WO 9951642 A
- WO 9308829 A
- US 5731168 A
- WO 2009089004 A1
- US 4676980 A
- US 20060025576 A1
- US 20080069820 A
- WO 2009011941 A
- WO 2015156268 A
- US 3773719 A
- EP 58481 A
- EP 133988 A

### Non-patent literature cited in the description

- *Nat. Biotechnol.,* 2005, vol. 23, 1073-1078
- *Eur J Pharm Biopharm,* 2005, vol. 59 (3), 389-396
- *Drug Des Devel Ther,* 2009, vol. 3, 7-16
- *Cancer Res,* 2010, vol. 16 (1), 11-20
- *Nature,* 1985, vol. 314 (6012), 628-31
- *Int J Cancer,* 1988, vol. 41 (4), 609-15
- *Proc Natl Acad Sci USA,* 1986, vol. 83 (5), 1453-7
- *Cancer J,* March 2014, vol. 20 (2), 119-122
- **ABDALLAH, HISHAM et al.** Pharmacokinetic and Pharmacodynamic Analysis of Subcutaneous Tocilizumab in Patients with Rheumatoid Arthritis From 2 Randomized, Controlled Trials: SUMMACTA and BREVACTA. *Journal of clinical pharmacology,* 2017, vol. 57 (4), 459-468
- **HARLOW ; LANE.** Antibodies: A Laboratory Manual. Cold Spring Harbor Laboratory, 1988
- **KABAT et al.** Sequences of Proteins of Immunological Interest. Public Health Service, National Institutes of Health, 1991
- **DAËRON.** *Annu. Rev. Immunol.,* 1997, vol. 15, 203-234
- **RAVETCH ; KINET.** *Annu. Rev. Immunol,* 1991, vol. 9, 457-92
- **CAPEL et al.** *Immunomethods,* 1994, vol. 4, 25-34
- **HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GHETIE ; WARD.** *Immunol. Today,* 1997, vol. 18 (12), 592-598
- **GHETIE et al.** *Nature Biotechnology,* 1997, vol. 15 (7), 637-640
- **HINTON et al.** *J. Biol. Chem.,* 2004, vol. 279 (8), 6213-6216
- **SHIELDS et al.** *J. Biol. Chem.,* 2001, vol. 9 (2), 6591-6604
- *Proc. Natl. Acad. Sci. USA,* 2006, vol. 103 (11), 4005-4010
- **IDUSOGIE et al.** *J. Immunol.,* 2000, vol. 164, 4178-4184
- **KABAT et al.** Sequences of Proteins of Immunological Interest. 1991, vol. 1-3
- **CHOTHIA ; LESK.** *J. Mol. Biol.,* 1987, vol. 196, 901-917
- **MACCALLUM et al.** *J. Mol. Biol.,* 1996, vol. 262, 732-745
- **FLATMAN et al.** *J. Chromatogr. B,* 2007, vol. 848, 79-87
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **MILSTEIN ; CUELLO.** *Nature,* 1983, vol. 305, 537
- **TRAUNECKER et al.** *EMBOJ,* 1991, vol. 10, 3655
- **BRENNAN et al.** *Science,* 1985, vol. 229, 81
- **KOSTELNY et al.** *J. Immunol.,* 1992, vol. 148 (5), 1547-1553
- **HOLLINGER et al.** *Proc. Natl. Acad. Sci. USA,* 1993, vol. 90, 6444-6448
- **GRUBER et al.** *J. Immunol.,* 1994, vol. 152, 5368
- **TUTT et al.** *J. Immunol.,* 1991, vol. 147, 60

- **FILMUS.** *J. Clin. Invest.,* 2001, vol. 108, 497-501
- **ZHU-ZU-W.** *Gut,* 2001, vol. 48, 558-564
- **YAMAUCHI.** *Mod. Pathol.,* 2005, vol. 18, 1591-1598
- *Proc. Natl. Acad. Sci. USA,* 1980, vol. 77, 4914-4917
- *J. Rheumatol,* 2007, vol. 34, 11
- *Hum. Antibod. Hybridomas,* 1990, vol. 1 (1), 47-54
- *Blood,* 2007, vol. 109, 1185-1192
- **HASHIMOTO-GOTOH, T. ; MIZUNO, T. ; OGASA-HARA, Y. ; NAKAGAWA, M.** An oligodeoxyribonucleotide-directed dual amber method for site-directed mutagenesi. *Gene,* 1995, vol. 152, 271-275
- **ZOLLER, M.J. ; SMITH, M.** Oligonucleotide-directed mutagenesis of DNA fragments cloned into M13 vectors. *Methods Enzymol,* 1983, vol. 100, 468-500
- **KRAMER, W. ; DRUTSA, V. ; JANSEN, H.W. ; KRAMER, B. ; PFLUGFELDER, M. ; FRITZ, H.J.** The gapped duplex DNA approach to oligonucleotide-directed mutation construction. *Nucleic Acids Res,* 1984, vol. 12, 9441-9456
- **KRAMER, W. ; FRITZ, H.J.** Oligonucleotide-directed construction of mutations via gapped duplex DNA Methods. *Enzymol,* 1987, vol. 154, 350-367
- **KUNKEL, T.A.** Rapid and efficient site-specific mutagenesis without phenotypic selection. *Proc Natl Acad. Sci. U S A.,* 1985, vol. 82, 488-492
- **MARK, D. F. et al.** *Proc. Natl. Acad. Sci. USA,* 1984, vol. 81, 5662-6
- **ZOLLER, M. J. ; SMITH, M.** *Nucleic Acids Res.,* 1982, vol. 10, 6487-500
- **WANG, A et al.** *Science,* 1984, vol. 224, 1431-3
- **DALBADIE-MCFARLAND, G et al.** *Proc. Natl. Acad. Sci. USA,* 1982, vol. 79, 6409-13
- **KARLIN ; ALTSCHUL.** *Proc. Natl. Acad. Sci. USA,* 1993, vol. 90, 5873-7
- **ALTSCHUL et al.** *J. Mol. Biol.,* 1990, vol. 215, 403-10
- Remington's Pharmaceutical Science. 1980
- *J. Biomed. Mater. Res.,* 1981, vol. 15, 267-277
- *Chemtech,* 1982, vol. 12, 98-105
- *Biopolymers,* 1983, vol. 22, 547-556
- Remington's Pharmaceutical Science. Mark Publishing Company
- **LEE et al.** *Blood,* 2014, vol. 124 (2), 188-95
- **LEE DW et al.** Current concepts in the diagnosis and management of cytokine release syndrome. *Blood,* 2014, vol. 124, 188-195
- **LEE DW et al.** ASTCT Consensus Grading for Cytokine Release Syndrome and Neurologic Toxicity Associated with Immune Effector Cells. *Biol Blood Marrow Transplant,* April 2019, vol. 25 (4), 625-638
- *Biol Blood Marrow Transplant,* April 2019, vol. 25 (4), 625-638
- **FREY, N. ; GRANGE, S. ; WOODWORTH, T.** Population Pharmacokinetic Analysis of Tocilizumab in Patients with Rheumatoid Arthritis. *The Journal of Clinical Pharmacology,* 2010, vol. 50, 754-766
- **GIBIANSKY, L. ; FREY, N.** Linking interleukin-6 receptor blockade with tocilizumab and its hematological effects using a modeling approach. *J Pharmacokinet Pharmacodyn,* February 2012, vol. 39 (1), 5-16
- **BARAN P ; HANSEN S ; WAETZIG GH et al.** The balance of interleukin (IL)-6, IL-6 soluble IL-6 receptor (sIL-6R), and IL-6 sIL- 6R sgp130 complexes allows simultaneous classic and trans-signaling. *J Biol Chem,* 2018, vol. 293 (18), 6762-6775
- **GIAVRIDIS, THEODOROS et al.** CAR T cellinduced cytokine release syndrome is mediated by macrophages and abated by IL-1 blockade. *Nature medicine,* 2018, vol. 24 (6), 731-738
- **BARRETT, DAVID M et al.** Toxicity management for patients receiving novel T-cell engaging therapies. *Current opinion in pediatrics,* 2014, vol. 26 (1), 43-9
- **YOSHIKA IWATA et al.** Different players generate positive responses in two in vitro cytokine assay formats with aqueous and immobilized TGN1412 analog. *Biochemical and Biophysical Research Communications,* 2018, vol. 502 (1), 91-7
- **OKAZAKI M et al.** Characterization of anti-mouse interleukin-6 receptor antibody. *Immunol Lett,* 03 December 2002, vol. 84 (3), 231-40
- **SAITO T et al.** Preparation of soluble murine IL-6 receptor and anti-murine IL-6 receptor antibodies. *J. Immunol.,* 1991, vol. 47, 168-173